# EUROPEAN PATENT SPECIFICATION

(11) **EP 2 827 890 B1**
(45) Date of publication and mention of the grant of the patent: **28.08.2019**
(21) Application number: 13764426.6
(22) Date of filing: 25.03.2013
(51) Int. Cl.: A61K 39/00, A61K 38/39, A61P 19/02, A61K 31/09

(54) **IMMUNOMODULATORY AGENT AND USES THEREFOR**
IMMUNOMODULATORISCHER WIRKSTOFF UND VERWENDUNGEN DAFÜR
AGENT IMMUNOMODULATEUR ET SES UTILISATIONS

(30) Priority: 23.03.2012 AU 2012901189
(43) Date of publication of application: 28.01.2015
(73) Proprietor: The University Of Queensland, St. Lucia, QLD 4072 (AU)
(72) Inventor: THOMAS, Ranjeny, Hawthorne Queensland 4171 (AU)
(74) Representative: Maschio, Antonio
(86) International application number: PCT/AU2013/000303
(87) International publication number: WO 2013/138871

(56) References cited:
- WO-A1-01/70253
- WO-A1-2014/141244
- WO-A1-2014/176604
- WO-A2-2014/160465
- CA-A1- 2 421 321
- SOI CHENG LAW ET AL: "T-cell autoreactivity to citrullinated autoantigenic peptides in rheumatoid arthritis patients carrying HLA-DRB1 shared epitope alleles", ARTHRITIS RESEARCH AND THERAPY, BIOMED CENTRAL, LONDON, GB, vol. 14, no. 3, 17 May 2012 (2012-05-17), page R118, XP021096214, ISSN: 1478-6354, DOI: 10.1186/AR3848
- M. CIECHOMSKA ET AL: "Antigen-specific B cells can acquire and present the rheumatoid arthritis candidate autoantigen aggrecan from a non-internalisable surface", ANNALS OF THE RHEUMATIC DISEASES, vol. 70, no. Suppl 2, 22 February 2011 (2011-02-22), pages A62-A62, XP055216996, ISSN: 0003-4967, DOI: 10.1136/ard.2010.149005.12
- STREET S ET AL: "PROINFLAMMATORY AND REGULATORY T CELL RESPONSES TO CITRULLINATED AUTOANTIGENIC PEPTIDES IN PERIPHERAL BLOOD OF SHARED EPITOPE plus RHEUMATOID ARTHRITIS PATIENTS", INTERNAL MEDICINE JOURNAL, BLACKWELL SCIENCE, AU, 1 January 2011 (2011-01-01), XP009186321, ISSN: 1444-0903
- BARDOS T. ET AL.: 'Continuous nasal administration of antigen is critical to maintain tolerance in adoptively transferred autoimmune arthritis in SCID mice' CLINICAL AND EXPERIMENTAL IMMUNOLOGY vol. 129, 2002, pages 224 - 231, XP055168605

## Description

### FIELD OF THE INVENTION

This invention relates generally to immunomodulatory agents that are useful for treating or preventing joint damage. More particularly, the present invention relates to the use of immune modulators that elicit an antigen-specific tolerogenic response to an aggrecan polypeptide including citrullinated forms thereof to treat or prevent joint damage, including joint damage in subjects with early RA or incipient RA.

### BACKGROUND OF THE INVENTION

Inflammatory arthritis is a prominent clinical manifestation in diverse autoimmune disorders including rheumatoid arthritis (RA), psoriatic arthritis (PsA), systemic lupus erythematosus (SLE), Sjogren's syndrome, and polymyositis. Most of these patients develop joint deformities on physical examination but typically only RA and PsA patients manifest bone erosions on imaging studies.

The pathogenesis of chronic inflammatory bone diseases, such as RA, is not fully elucidated. Such diseases are accompanied by bone loss around affected joints due to increased osteoclastic resorption, which is mediated largely by increased local production of pro-inflammatory cytokines (Teitelbaum, 2000. Science 289:1504-1508; Goldring and Gravallese, 2000. Arthritis Res. 2(1):33-37). These cytokines can act directly on cells in the osteoclast lineage or indirectly by affecting the production of the essential osteoclast differentiation factor, receptor activator of NF-κB ligand (RANKL), and/or its soluble decoy receptor, osteoprotegrin (OPG), by osteoblast/stromal cells (Hossbauer et al., 2000. J. Bone Min. Res. 15(1):2-12).

RA is a systemic inflammatory disease that affects approximately 0.5 to 1% of the adult population in northern Europe and North America, and a slightly lower proportion in other parts of the world (Alamanos and Drosos, 2005. Autoimmun. Rev. 4: 130-136). It is characterized by chronic inflammation of joint synovial tissue, which ultimately leads to loss of daily function due to chronic pain and fatigue. It is s a chronic inflammatory disease. The majority of patients also experience progressive deterioration of cartilage and bone in the affected joints, which may eventually lead to permanent disability. The long-term prognosis of RA is poor, with approximately 50% of patients experiencing significant functional disability within 10 years from the time of diagnosis.

Several autoantigens are described in RA, including a variety of proteins that become citrullinated in diseased joints. Citrullination is a physiological process of arginine deimination that occurs during apoptosis and inflammation. This process results in modification of arginine-containing proteins, which can give rise to sets of neo-self antigens in individuals bearing at-risk HLA alleles (Vossenaar ER, et al., 2004. Arthritis Res. Ther. 6:107-11). Specific HLA-DR gene variants mapping to amino acids 70-74 of the third hypervariable region of DRβ-chains, are highly associated with RA (Gregersen PK, et al., 1987. Arthritis Rheum. 30:1205-1213). This region encodes a conserved amino acid sequence that forms the fourth anchoring pocket (P4) in the HLA-DR antigen-binding groove. This "shared susceptibility epitope" (SE) is found in multiple RA-associated DR alleles, including DRB1^{∗}0401, DRB1^{∗}0404 and DRB^{∗}0101 in Caucasians (Gregersen PK, *et al*., 1987, *supra).* The SE-encoding HLA alleles are particularly associated with ACPA-positive RA (Klareskog L, et al., 2006. Arthritis Rheum. 54:38-46; van Gaalen FA, et al., 2004. Arthritis Rheum. 50:2113-21; Hida S, et al., 2004. J. Autoimmun. 23:141-50; Hill JA, et al., 2003. J. Immunol. 171:538-41).

The SE is highly positively charged, is situated in a region of the DRβ-chain that influences the specificity of the P4 amino acid of the bound ligand and would therefore preferentially bind peptides containing a negatively charged or non-polar amino acid at this position. Citrullination replaces charged arginine amino side-chain groups with an uncharged carbonyl group, and increases the binding affinity of a human vimentin peptide epitope to SE⁺ HLA DR molecules (Hill JA, *et al*., 2003, *supra;* Snir O, et al., 2011. Arthritis Rheum. n/a-n/a).

Approximately 70% of RA patient sera contain anti-citrullinated protein autoantibodies (ACPA) (Meyer O, et al., 2006. Arthritis Res. Ther. 8:R40). This reactivity reflects autoantibody production towards a group of citrullinated autoantigens modified post-translationally, including fibrinogen, vimentin, collagen type II and enolase (Wegner N, et al., 2010. Immunol. Rev. 233:34-54). ACPA develop up to 15 years prior to the onset of RA, with increasing titers and peptide reactivities as disease onset becomes imminent (van de Stadt LA, et al., 2011. Arthritis Rheum. n/a-n/a). Citrullinated proteins have been demonstrated in inflamed tissues in RA, and ACPA are induced in a number of mouse models of inflammatory arthritis (Masson-Bessiere C, et al., 2001. J. Immunol. 166:4177-84; Vossenaar ER, et al., 2003. Arthritis Rheum. 48:2489-500; Kuhn KA, et al., 2006. J. Clin. Invest. 116:961-73; Giant TT, et al., 2011. Arthritis Rheum. 63:1312-1321.). Although citrullination is ubiquitous in response to stress and inflammation, ACPA are highly specific for RA and are associated with more severe joint damage and radiographic outcome (Klareskog L, *et al*., 2006, *supra;;* van Gaalen FA, *et al*., 2004, *supra;* Meyer O, *et al*., 2006, *supra).*

Immunization of HLA-DRB1^{∗}0401 transgenic mice with citrullinated fibrinogen, but not native fibrinogen, induced inflammatory arthritis characterized by simultaneous B cell and T cell autoreactivity to citrullinated and native HLA-DR-restricted fibrinogen epitopes, which was not present in naïve HLA-DRB1^{∗}0401 transgenic mice. The capacity of citrullinated rather than native fibrinogen to induce arthritis suggested that one or more citrullinated neo-self epitopes broke T cell tolerance to corresponding native epitopes, either during priming or resulting from epitope spreading (Hill JA, et al., 2008. J. Exp. Med. 205:967-979). Furthermore, recent studies suggest that delivery of abatacept may restore tolerance towards citrullinated antigens (Yue D, et al., 2010. Arthritis Rheum. 62:2941-2952). Notwithstanding these studies in transgenic mice, citrulline-specific autoreactive T cells have been difficult to demonstrate in RA patients due to the weak proliferative responses made by autoreactive effector memory T cells *in vitro.* However, several recent papers have shown convincing cytokine responses made by RA patient T cells in response to citrullinated vimentin and aggrecan epitopes (Snir O, *et al*., 2011, *supra;* von Delwig A, et al., 2010. Arthritis Rheum. 62:143-149). In the case of vimentin, the immunogenicity of the epitope was dependent on the location of the citrulline modification within the peptide sequence (Snir O, *et al*., 2011, *supra).* CA2421321 describes a variety of citrullinated peptides and epitopes that bind to a shared susceptibility epitope (SE) in various MHC alleles associated with RA, wherein said binding may evoke a T cell response in the blood of a patient with or at risk of developing RA. Street S, *et al.* reports that cytokines secreted by citrullinated peptide-specific auto-reactive T cells results in suppressed T cell proliferation (Street S, et al., 2011. Internal Medicine Journal, AU). Bardos T, *et al.* describes a study using an aggrecan-induced arthritis mouse model (Bardos T, et al. 2002, Clin. Exp. Immunol. 129:224-231).

The present inventors have now profiled the responses of SE⁺ healthy controls and RA patients towards a set of citrullinated and un-modified (native) self-antigens, and characterized the responding T cells. Their aim was: (1) to identify citrullinated epitopes that may be of particular relevance in ACPA⁺ RA; (2) to determine the extent of individual variability among citrullinated autoantigenic T cell responses; and (3) to identify T cells that may contribute to the development of ACPA⁺ RA.

### SUMMARY OF THE INVENTION

The present invention is defined in independent claim 1 and certain optional features thereof are defined in the dependent claims. In so far as the terms "invention", "example" and "embodiment" are used herein, this will be interpreted in such a way that the only protection sought is for the invention as claimed.

The present invention stems in part from the unexpected discovery that RA patients with long-standing disease are more likely to make a T lymphocyte response to multiple citrullinated autoantigens, whereas patients with recent-onset RA (including those previously untreated) are more likely to respond either to no antigen or only to citrullinated aggrecan. Based on these observations, it is proposed that antigens corresponding in whole, or in part, to aggrecan, including citrullinated aggrecan, will be useful in immunotherapeutic strategies for treating or preventing joint damage, including in subjects with early RA (*e.g.*, when clinical symptoms such as swelling of the joints or pain are not yet present), or in subjects at risk of developing RA (*e.g*., incipient RA).

For illustration only, the present invention provides methods for treating or preventing joint damage in a subject. These methods generally comprise, consist or consist essentially of eliciting an antigen-specific tolerogenic response to an aggrecan polypeptide (*e.g.*, a citrullinated aggrecan polypeptide, which is also referred to interchangeably herein as "*cit*-aggrecan" or *"cit-agg"* polypeptide) in the subject, thereby treating or preventing the joint damage. In some embodiments, the subject has early RA or incipient RA and in illustrative examples of this type, the methods further comprise identifying that the subject has early RA or incipient RA, suitably prior to eliciting the antigen-specific tolerogenic response. In some embodiments, the subject is positive for the shared epitope (SE) and in illustrative examples of this type, the methods further comprise identifying that the subject is positive for SE prior to eliciting the antigen-specific tolerogenic response. Suitably, the subject has or is at risk of developing an immune response including an effector immune response (*e.g.*, an effector T lymphocyte response such as but not limited to a CD4⁺ effector T lymphocyte) to the aggrecan polypeptide (*e.g.*, a *cit*-agg polypeptide) or fragments thereof. In non-limiting examples the immune response comprises production (*e.g*., secretion) of at least one cytokine selected from the group consisting of interleukin-6 (IL-6), interferon-γ (IFN-γ), tumor necrosis factor (TNF), and interleukin-10 (IL-10). In some embodiments, the immune response includes a pro-inflammatory T lymphocyte response, which comprises, consists or consists essentially of production (*e.g*., secretion) of at least one cytokine selected from the group consisting of IL-6, IFN-γ and TNF. In illustrative examples of this type, the pro-inflammatory T lymphocyte response comprises, consists or consists essentially of production (*e.g*., secretion) of IL-6. Suitably, the immune response is produced at least in part by CD4⁺ CD28⁻ T lymphocytes. In some embodiments, the immune response is produced at least in part by CD4⁺ CD28⁺ T lymphocytes. In specific embodiments, the immune response is produced at least in part by CD4⁺ CD28⁻ T lymphocytes and CD4⁺ CD28⁺ T lymphocytes.

The antigen-specific tolerogenic response may be achieved using any suitable strategy, illustrative examples of which include:
(1) increasing the number of tolerogenic antigen-presenting cells in the subject, which present a peptide (*e.g*., an autoantigen) corresponding to a portion of the aggrecan polypeptide (also referred to herein as "aggrecan-specific tolerogenic antigen-presenting cells" or "agg-tolAPC"), wherein the portion is associated with a pro-inflammatory or autoreactive T lymphocyte response to the aggrecan polypeptide (*e.g*., a *cit*-agg polypeptide);
(2) inducing anergy or apoptosis in pro-inflammatory or autoreactive T lymphocytes (*e.g*., effector T lymphocytes) in the subject, which are reactive against the aggrecan polypeptide (*e.g*., a *cit*-agg polypeptide) or portion thereof; and
(3) increasing the number of regulatory or suppressor T lymphocytes in the subject, which suppress or otherwise reduce a pro-inflammatory or autoreactive T lymphocyte response to the aggrecan polypeptide.

Thus, in some embodiments, the methods of the present invention comprise, consist or consist essentially of increasing the number of agg-tolAPC in the subject to thereby treat or prevent the joint damage. In some embodiments, the agg-tolAPC stimulate the production of regulatory or suppressor T lymphocytes that suppress or otherwise reduce the pro-inflammatory or autoreactive T lymphocyte response to the aggrecan polypeptide (*e.g*., a cit-aggrecan polypeptide). Suitably, the regulatory or suppressor T lymphocyte expresses at least one marker (*e.g*., 1, 2, 3, 4, 5 etc.) of a constitutive regulatory or suppressor T lymphocyte (*e.g*., at least one marker selected from CD4, CD25, CD62L, GITR, CTLA4) and the transcription factor Forkhead box P3 (FoxP3). Illustrative regulatory or suppressor T lymphocytes include, but are not limited to, CD4⁺CD25⁺regulatory T lymphocytes, Tr1 lymphocytes, T*ₕ*3 lymphocytes, and CD8⁺ regulatory T lymphocytes. In specific embodiments, the regulatory T lymphocyte is CD4⁺CD25⁺. Non-limiting antigen-presenting cells include dendritic cells, macrophages, Langerhans cells, B lymphocytes and artificial antigen-presenting cells.

The aggrecan-specific tolerogenic antigen-presenting cells may be produced using any suitable strategy. In some embodiments, they are produced by contacting antigen-presenting cells (*e.g.*, dendritic cells, macrophages, Langerhans cells, B cells *etc.*) with: (1) at least one NF-κB inhibitor in an amount sufficient to inhibit the NF-κB pathway of the antigen-presenting cells; and/or (2) at least one mTOR inhibitor in an amount sufficient to inhibit mTOR of the antigen-presenting cells; and/or (3) at least one Syk inhibitor in an amount sufficient to inhibit the Syk pathway of the antigen-presenting cells, and with an antigenic molecule selected from an antigen that corresponds in whole, or in part, to an aggrecan polypeptide (*e.g*., a cit-aggrecan polypeptide) or a nucleic acid molecule from which the antigen is expressible, in an amount sufficient for the antigen-presenting cells to present the antigen or a processed form thereof on their surface. In illustrative examples of this type, the methods comprise, consist or consist essentially of co-administering to the subject (*e.g*., a subject having or at risk of developing joint damage such as a subject with early RA or incipient RA) an NF-κB inhibitor and an antigenic molecule selected from an antigen that corresponds in whole, or in part, to an aggrecan polypeptide (*e.g*., a cit-aggrecan polypeptide) or a nucleic acid molecule from which the antigen is expressible, wherein the inhibitor is administered in an amount sufficient to inhibit NF-κB activity in an antigen-presenting cell of the subject and wherein the antigenic molecule is administered in an amount sufficient for the NF-κB activity-inhibited antigen-presenting cell to present the antigen or a processed form thereof to the immune system of the subject. In other illustrative examples, the methods comprise, consist or consist essentially of co-administering to the subject (*e.g*., a subject having or at risk of developing joint damage such as a subject with early RA or incipient RA) an mTOR inhibitor and an antigenic molecule selected from an antigen that corresponds in whole, or in part, to an aggrecan polypeptide (*e.g*., a cit-aggrecan polypeptide) or a nucleic acid molecule from which the antigen is expressible, wherein the inhibitor is administered in an amount sufficient to inhibit mTOR activity in an antigen-presenting cell of the subject and wherein the antigenic molecule is administered in an amount sufficient for the mTOR activity-inhibited antigen-presenting cell to present the antigen or a processed form thereof to the immune system of the subject. In still other illustrative examples, the methods comprise, consist or consist essentially of co-administering to the subject (*e.g*., a subject having or at risk of developing joint damage such as a subject with early RA or incipient RA) a Syk inhibitor and an antigenic molecule selected from an antigen that corresponds in whole, or in part, to an aggrecan polypeptide (*e.g*., a *cit*-aggrecan polypeptide) or a nucleic acid molecule from which the antigen is expressible, wherein the inhibitor is administered in an amount sufficient to inhibit Syk activity in an antigen-presenting cell of the subject and wherein the antigenic molecule is administered in an amount sufficient for the NF-κB activity-inhibited antigen-presenting cell to present the antigen or a processed form thereof to the immune system of the subject. Suitably, when the antigenic molecule is a nucleic acid molecule from which the antigen is expressible, the nucleic acid molecule is generally in the form of a nucleic acid construct comprising a nucleotide sequence that encodes the antigen and that is operably connected to a regulatory element that is operable in the antigen-presenting cell. In these embodiments, one or more inhibitors (*e.g*., at least one NF-κB inhibitor and/or at least one mTOR inhibitor and/or at least one Syk inhibitor) and/or the antigenic molecule are generally in a form that is suitable for introduction (*e.g*., by transformation, internalization, endocytosis or phagocytosis) into the antigen-presenting cells or their precursors, which includes soluble and particulate forms of the inhibitor and/or antigenic molecule.

In some embodiments, the antigen-presenting cells are contacted with at least one inhibitor of the NF-κB pathway, illustrative examples of which are listed in Tables 2, 3A, 3B or 4 *infra.* In specific embodiments, the NF-κB inhibitor is curcumin or a curcumin derivative.

The aggrecan antigen may comprise, consist or consist essentially of an amino acid sequence corresponding to a putatively full-length citrullinated aggrecan polypeptide including unprocessed, partially processed and mature forms thereof. Alternatively, the aggrecan antigen may comprise, consist or consist essentially of a domain of a citrullinated aggrecan polypeptide such as but not limited to the G1, G2 and G3 domains. In some embodiments, the antigen comprises, consists or consists essentially of an amino acid sequence corresponding to a T cell epitope. In other embodiments, the antigen comprises, consists or consists essentially of a plurality of peptides wherein individual peptides comprise different portions of an amino acid sequence corresponding to an aggrecan polypeptide (*e.g*., a *cit*-aggrecan polypeptide). In illustrative examples of this type, the peptides are overlapping peptides.

In related embodiments, the inhibitor (*e.g*., NF-κB inhibitor, mTOR inhibitor and/or Syk inhibitor) and the antigenic molecule are co-administered in soluble or in particulate form (*e.g.*, the antigenic molecule and the inhibitor are both in soluble form, or one of the antigenic molecule or inhibitor is in soluble form and the other is in particulate form, or the antigenic molecule and the inhibitor are both in particulate form). In specific embodiments, both the inhibitor and the antigenic molecule are co-administered in particulate form. For example, the inhibitor and the antigenic molecule may be contained in one or more particles (*e.g*., nanoparticles or microparticles such as liposomes and polymeric particles), which are suitably capable of being taken up by an antigen-presenting cell. In specific embodiments, the inhibitor and the antigenic molecule are contained in the same particle. The inhibitor and the antigenic molecule may be administered by injection, by topical application or by the nasal or oral route including sustained-release modes of administration, over a period of time and in amounts which are suitably effective to suppress or otherwise reduce a T lymphocyte response to the aggrecan polypeptide or to ameliorate the symptoms of RA. In specific embodiments, the inhibitor and the antigenic molecule are co-administered subcutaneously.

In other embodiments, the agg-tolAPC are produced by expressing in B lymphocytes a nucleic acid molecule that encodes an antigen that corresponds in whole, or in part, to an aggrecan polypeptide (*e.g*., a *cit*-aggrecan polypeptide), wherein the expression of the nucleic acid molecule leads to presentation of the antigen or processed form thereof on the surface of the B lymphocytes. Desirably, in these examples, the nucleic acid molecule further encodes an immunoglobulin (*e.g.*, IgG) or a fragment (*e.g.*, Fv, Fab, Fab' and F(ab')₂ immunoglobulin fragments, immunoglobulin heavy chain *etc.*) of an immunoglobulin fused directly or indirectly to the antigen (*e.g*., adjacent to the C-terminus of the antigen).

In some embodiments, the methods comprise, consist or consist essentially of administering agg-tolAPC or precursors thereof to the subject in an amount effective to suppress or otherwise reduce the pro-inflammatory or autoreactive T lymphocyte response to the aggrecan polypeptide (*e.g*., a cit-aggrecan polypeptide) or to inhibit the development of that response. In illustrative examples of this type, the agg-tolAPC or their precursors are produced by harvesting antigen-presenting cells or antigen-presenting cell precursors from the subject or from a histocompatible donor and exposing them *ex vivo* to: (A1) at least one NF-κB inhibitor in an amount sufficient to inhibit the NF-κB pathway of the antigen-presenting cells; and/or (A2) at least one mTOR inhibitor in amounts sufficient to inhibit mTOR of the antigen-presenting cells; and/or (A3) at least one Syk inhibitor in an amount sufficient to inhibit the Syk pathway of the antigen-presenting cells, and to (B) an antigenic molecule selected from an antigen that corresponds in whole, or in part, to an aggrecan polypeptide (*e.g*., a *cit*-aggrecan polypeptide) or a nucleic acid molecule from which the antigen is expressible, in an amount sufficient for the antigen-presenting cells or their precursors to present the antigen or a processed form thereof to the immune system of the subject.

In other illustrative examples, the agg-tolAPC or their precursors are produced by harvesting B lymphocytes or B lymphocyte precursors from the subject or from a histocompatible donor and introducing into them *ex vivo* a nucleic acid molecule which is operably connected to a regulatory element that is operable in the B lymphocytes and which encodes an antigen that corresponds in whole, or in part, to an aggrecan polypeptide (*e.g*., a *cit*-aggrecan polypeptide), wherein expression of the nucleic acid molecule leads to presentation of the antigen or processed form thereof on the surface of the B lymphocytes. In specific embodiments, the nucleic acid molecule further encodes an immunoglobulin or an immunoglobulin fragment fused directly or indirectly to the antigen. Suitably, when precursors are used, the precursors are cultured for a time and under conditions sufficient to differentiate antigen-presenting cells from the precursors.

In other embodiments, the methods comprise, consist or consist essentially of administering to the subject a MHC-peptide complex consisting essentially of an antigen that corresponds in whole, or in part, to an aggrecan polypeptide (*e.g*., a *cit*-aggrecan polypeptide) and an isolated (*e.g*., soluble) MHC component having an antigen-binding site, wherein the antigen is associated with the antigen-binding site.

In still other embodiments, the methods comprise, consist or consist essentially of administering to the subject a chimeric construct comprising an immunomodulatory peptide and an immune- or T cell-binding ligand (I/TCBL), wherein the immunomodulatory peptide comprises, consists or consists essentially of an amino acid sequence corresponding to a portion (*e.g*., an autoantigen) of an aggrecan polypeptide (*e.g*., a cit-aggrecan polypeptide), which is suitably associated with rheumatoid arthritis (RA), and which binds to an antigen receptor (*e.g*., T cell receptor) on pro-inflammatory or autoreactive T lymphocytes, and wherein the I/TCBL binds to a class or subclass of T cell selected from the group consisting of helper T cells, suppressor T cells and cytotoxic T cells and modulates T cell activity. Suitably, the I/TCBL comprises at least a portion of a molecule selected from a MHC class I molecule, a MHC class II molecule, an accessory molecule such as β2-microglobulin, lymphocyte function associated molecule-3 (LFA-3), the Fc region of the heavy chain of an immunoglobulin molecule, Ia⁺ molecules, an antibody to CD2, an antibody to CD3, an antibody to CD4, an antibody to CD8, an antibody to lectin, a lymphokine.

In other embodiments, the methods comprise, consist or consist essentially of administering to the subject an altered peptide ligand (APL) that comprises, consists or consists essentially of an amino acid sequence corresponding to a portion (*e.g*., an autoantigen) of an aggrecan polypeptide (*e.g*., a *cit*-aggrecan polypeptide), which is suitably associated with rheumatoid arthritis (RA), and which binds to an antigen receptor (*e.g*., T cell receptor) on pro-inflammatory or autoreactive T lymphocytes, wherein the APL amino acid sequence is distinguished from the amino acid sequence of the portion by at least one amino acid substitution, deletion or addition, interferes with normal signaling through the antigen receptor and has at least one activity selected from: (i) antagonizing the response of the T lymphocytes to the aggrecan polypeptide (*e.g*., a *cit*-aggrecan polypeptide), (ii) inducing anergy in aggrecan- or citrullinated aggrecan-specific T lymphocytes, (iii) inducing apoptosis in aggrecan- or citrullinated aggrecan-specific T lymphocytes, (iv) stimulating or inducing a aggrecan- or citrullinated aggrecan-specific T*ₕ*2 immune response, (v) suppressing development of a aggrecan- or citrullinated aggrecan-specific T*ₕ*1 immune response including suppressing the production of pro-inflammatory cytokines, (vi) stimulating activation of aggrecan- or citrullinated aggrecan-specific regulatory lymphocytes (*e.g*., T regulatory lymphocytes (Treg)), or (vii) preventing or inhibiting the activation of aggrecan- or citrullinated aggrecan-specific antigen-presenting cells by an inflammatory stimulus.

In related aspects, the invention extends to the use of an agent for treating or preventing joint disease (*e.g*., early RA or incipient RA) or in the manufacture of a medicament for treating or preventing joint disease (*e.g*., early RA or incipient RA), wherein the agent is selected from: (1) an inhibitor of the NF-κB pathway and an antigenic molecule selected from an antigen that corresponds in whole, or in part, to an aggrecan polypeptide (*e.g*., a *cit*-aggrecan polypeptide) or a nucleic acid molecule from which the antigen is expressible, as broadly described above; (2) an mTOR inhibitor and an antigenic molecule selected from an antigen that corresponds in whole, or in part, to an aggrecan polypeptide (*e.g*., a *cit*-aggrecan polypeptide) or a nucleic acid molecule from which the antigen is expressible, as broadly described above; (3) an inhibitor of the Syk pathway and an antigenic molecule selected from an antigen that corresponds in whole, or in part, to an aggrecan polypeptide (*e.g*., a *cit*-aggrecan polypeptide) or a nucleic acid molecule from which the antigen is expressible, as broadly described above; (4) a nucleic acid molecule that encodes an antigen that corresponds in whole, or in part, to an aggrecan polypeptide (*e.g*., a *cit*-aggrecan polypeptide) for introduction into B lymphocytes, as broadly described above; (5) a MHC-peptide complex consisting essentially of an antigen that corresponds in whole, or in part, to an aggrecan polypeptide (*e.g*., a *cit-*aggrecan polypeptide) and an isolated (*e.g*., soluble) MHC component having an antigen-binding site, wherein the antigen is associated with the antigen-binding site, as broadly described above; (6) a chimeric construct as broadly described above; (7) aggrecan-specific tolerogenic antigen-presenting cells as broadly described above; or (8) an APL that comprises, consists or consists essentially of an amino acid sequence corresponding to a portion of an aggrecan polypeptide (*e.g*., a *cit*-aggrecan polypeptide), as broadly described above.

Still another aspect of the present invention relates to compositions, which are suitably useful for treating or preventing joint damage in a subject. These compositions generally comprise, consist or consist essentially of an agent selected from: (1) an inhibitor of the NF-κB pathway and an antigenic molecule selected from an antigen that corresponds in whole, or in part, to an aggrecan polypeptide (*e.g*., a *cit*-aggrecan polypeptide) or a nucleic acid molecule from which the antigen is expressible, as broadly described above; (2) an mTOR inhibitor and an antigenic molecule selected from an antigen that corresponds in whole, or in part, to an aggrecan polypeptide (*e.g.*, a *cit-*aggrecan polypeptide) or a nucleic acid molecule from which the antigen is expressible, as broadly described above; (3) an inhibitor of the Syk pathway and an antigenic molecule selected from an antigen that corresponds in whole, or in part, to an aggrecan polypeptide (*e.g*., a *cit*-aggrecan polypeptide) or a nucleic acid molecule from which the antigen is expressible, as broadly described above; (4) a nucleic acid molecule that encodes an antigen that corresponds in whole, or in part, to an aggrecan polypeptide (*e.g*., a *cit*-aggrecan polypeptide) for introduction into B lymphocytes, as broadly described above; (5) a MHC-peptide complex consisting essentially of an antigen that corresponds in whole, or in part, to an aggrecan polypeptide (*e.g*., a *cit*-aggrecan polypeptide) and an isolated (*e.g*., soluble) MHC component having an antigen-binding site, wherein the antigen is associated with the antigen-binding site, as broadly described above; (6) a chimeric construct as broadly described above; (7) aggrecan-specific tolerogenic antigen-presenting cells as broadly described above; or (8) an APL that comprises, consists or consists essentially of an amino acid sequence corresponding to a portion of an aggrecan polypeptide (*e.g*., a *cit*-aggrecan polypeptide), as broadly described above.

### BRIEF DESCRIPTION OF THE DRAWINGS

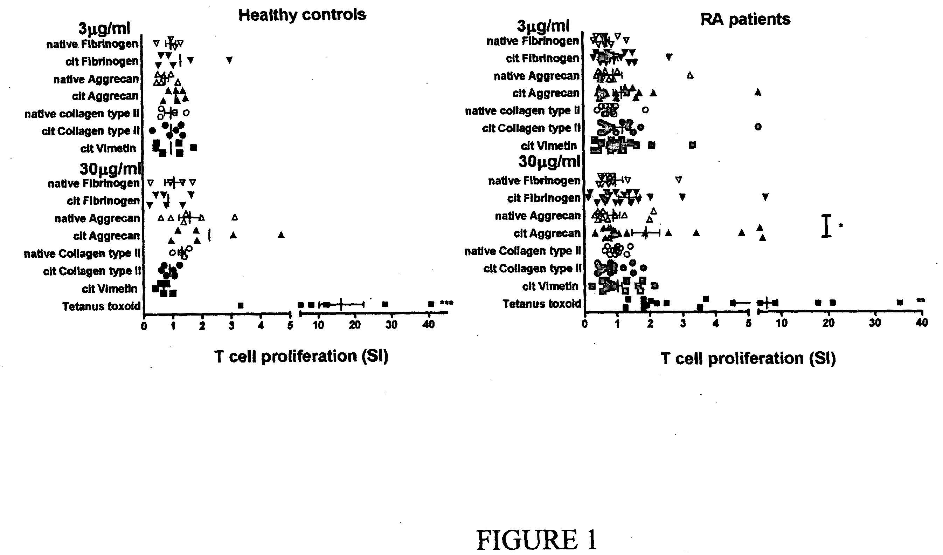

Figure 1 is a graphical representation showing the T cell proliferative response of healthy controls and RA patients stimulated with citrullinated and native peptides. PBMC and SFMC of 20 RA patients and 6 healthy controls were incubated with 0, 3 or 30 µg/mL peptides or 4 Lfi/mL tetanus toxoid, as shown, for 5 days. T cell proliferation was assessed by uptake of [³H] thymidine. Each dot represents one individual. ***p<0.001 for RA patients and **p<0.01 for healthy controls comparing multiple means (Kruskal-Wallis test), **p<0.01 comparing healthy controls and RA patient PBMC for the response to tetanus toxoid by (Mann-Whitney test). *p<0.05 comparing RA patients' response to citrullinated and native aggrecan peptides (Mann-Whitney test).

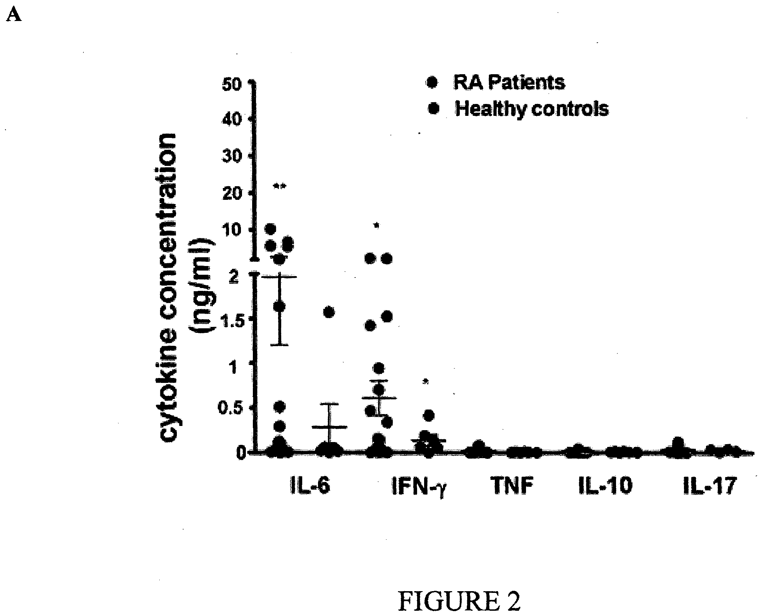

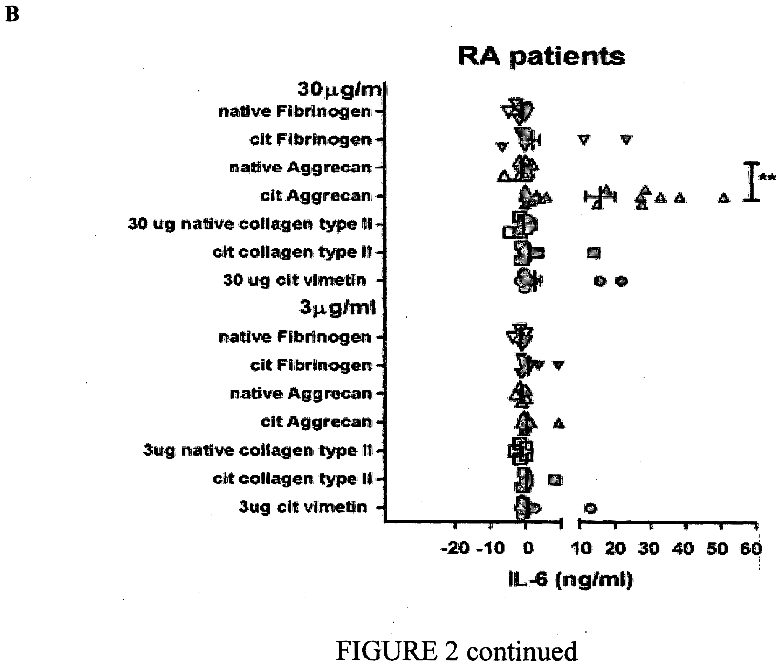

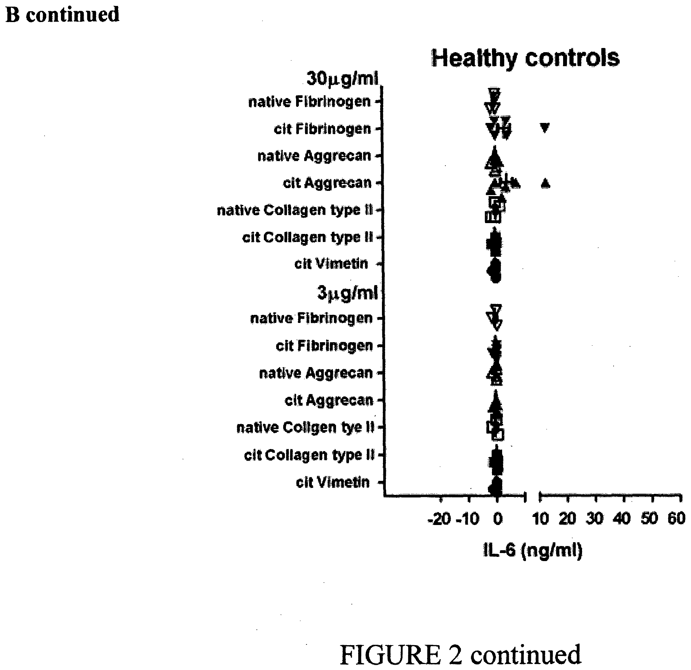

Figure 2 is a graphical representation showing a comparison of unstimulated cytokine secretion and net IL-6 secretion by RA and healthy control mononuclear cells stimulated with citrullinated and native peptides. A: PBMC from 17 RA patients and 6 healthy controls were incubated in medium for 5 days and cytokines were assessed in supernatants by CBA. **p<0.01 comparing production of IFN-γ to that of other cytokines (Kruskal-Wallis test with Dunns post-hoc correction) B: PBMC of 17 RA patients and 6 healthy controls were incubated with 0, 3 or 30 µg/mL peptides as shown, and IL-6 was assessed in supernatants by CBA. Net cytokine secretion was calculated as [IL-6 concentration with peptide stimulation] minus [IL-6 concentration without peptide stimulation]. Each dot represents one individual. Each dot represents one individual. *p<0.05, **p<0.01 comparing IL-6 response to citrullinated and native peptides (Wilcoxon signed rank test).

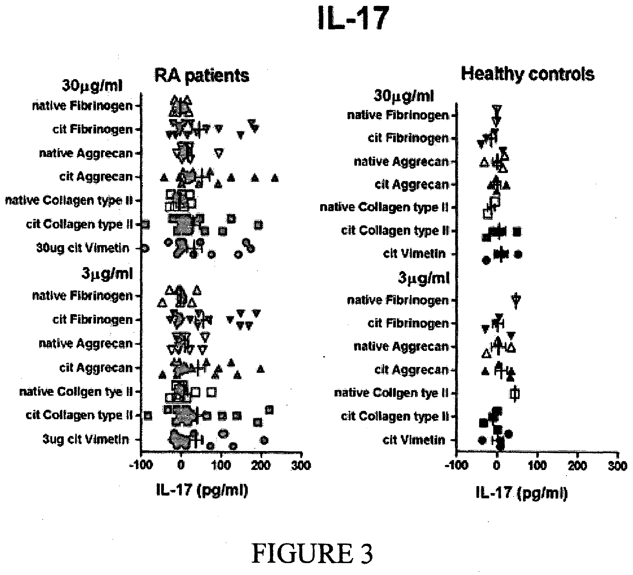

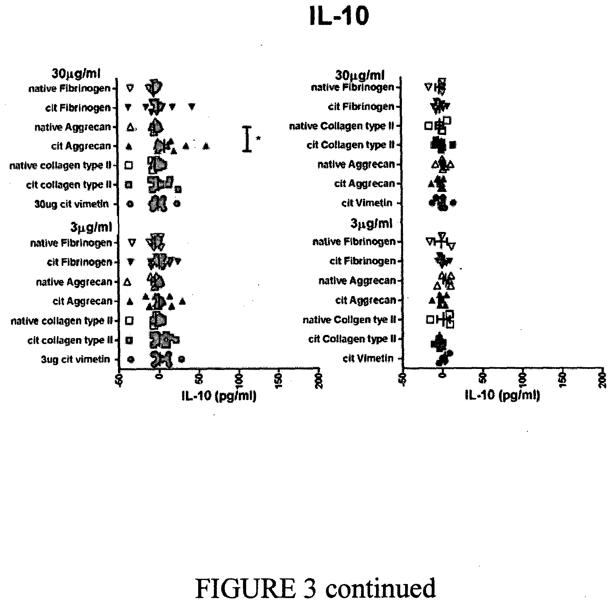

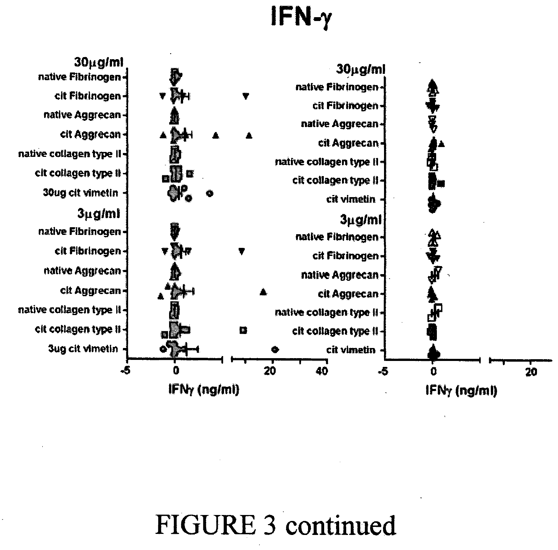

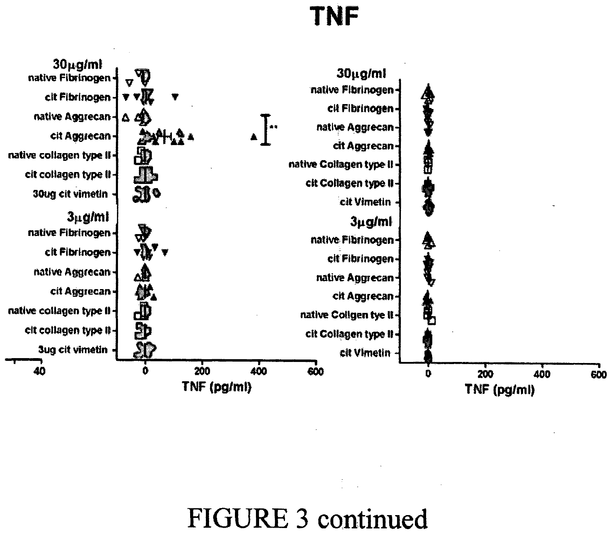

Figure 3 is a graphical representation showing net TNF, IFN-γ, IL-10 and IL-17 secretion by RA and healthy control PBMC stimulated with citrullinated and native peptides. PBMC of 17 RA patients and 6 healthy controls were incubated with 0, 3 or 30 µg/mL peptides as shown, and cytokines were assessed in supernatants by CBA. Net cytokine secretion was calculated as in Figure 2B. Each dot represents one individual. *p<0.05, comparing IL-10 response to citrullinated and native aggrecan (Wilcoxon signed rank test).

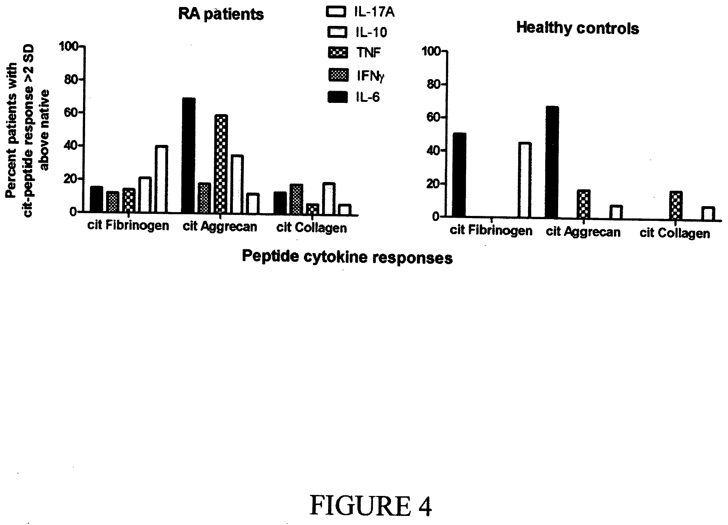

Figure 4 is a graphical representation showing the representation of cytokines produced by RA patients and healthy controls. The percentage of RA patients or of healthy controls with positive responses (>2 SD above the mean response towards the corresponding native peptide), was calculated for each cytokine, and is plotted for responses to citrullinated fibrinogen, aggrecan and collagen type II.

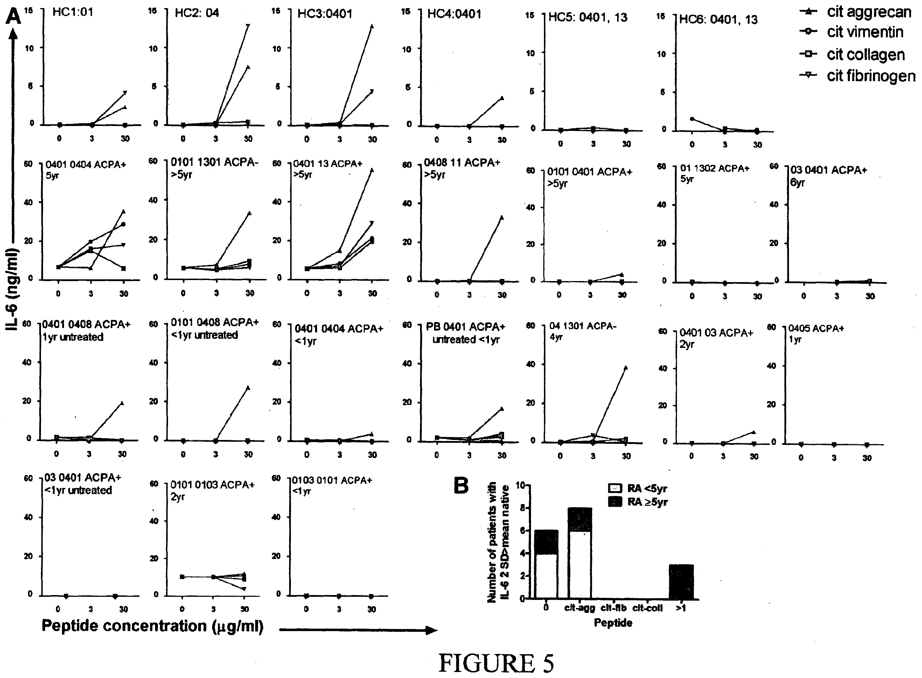

Figure 5 is a graphical representation showing the diversity of citrullinated peptide-reactive IL-6 response among RA patients and healthy controls. A: PBMC and SFMC of 17 RA patients and 6 healthy controls were incubated with 0, 3 or 30 µg/mL peptides as shown, and each individual's IL-6 response in supernatant was plotted. Disease duration and HLA type are indicated. B: The frequency of RA patients with either recent-onset or longstanding disease with positive responses (>2 SD above the mean response towards the corresponding native peptide) for each peptide is shown.

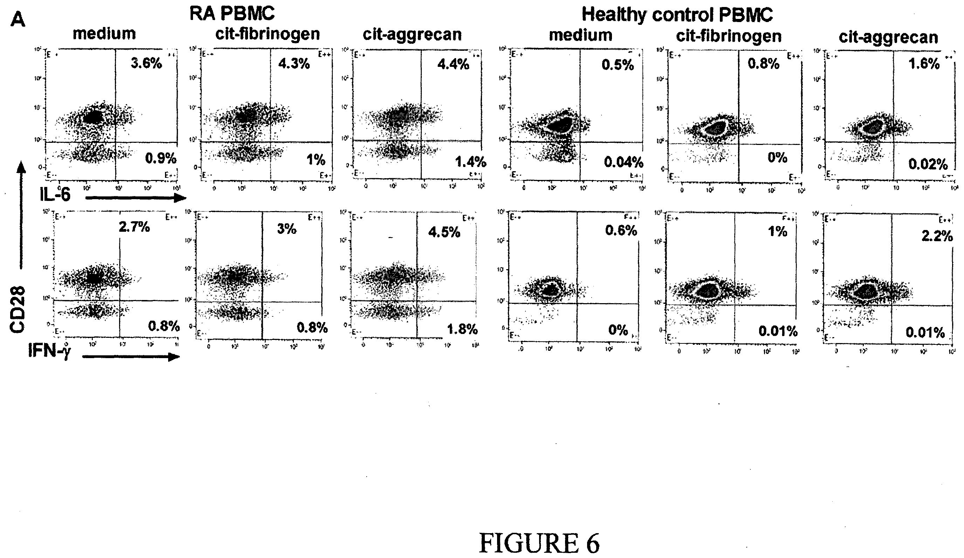

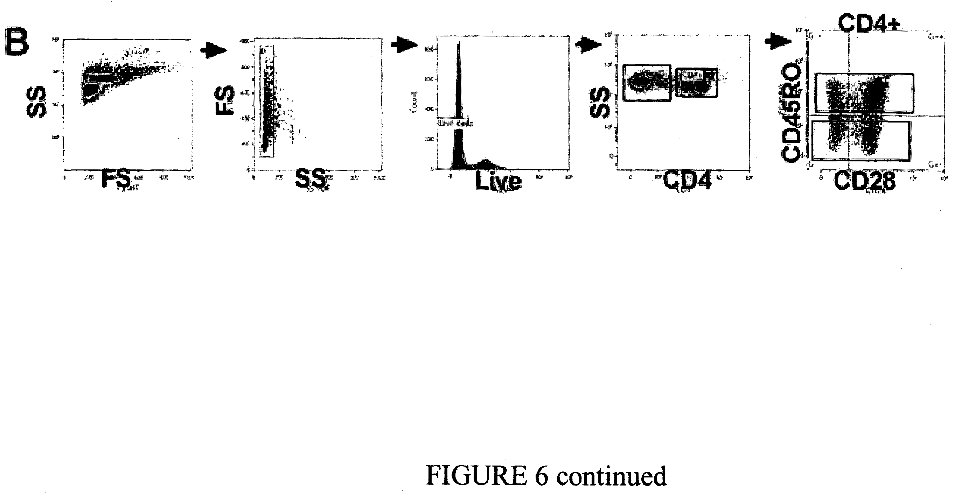

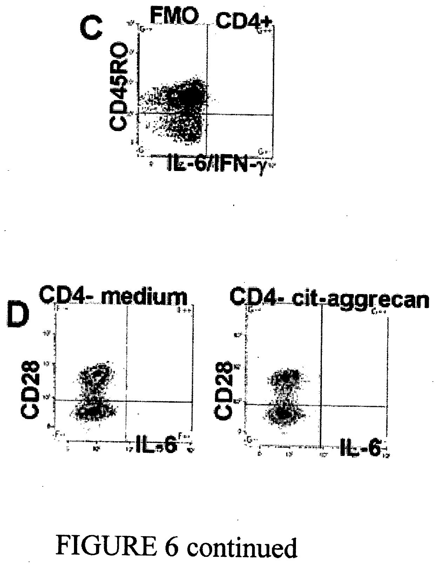

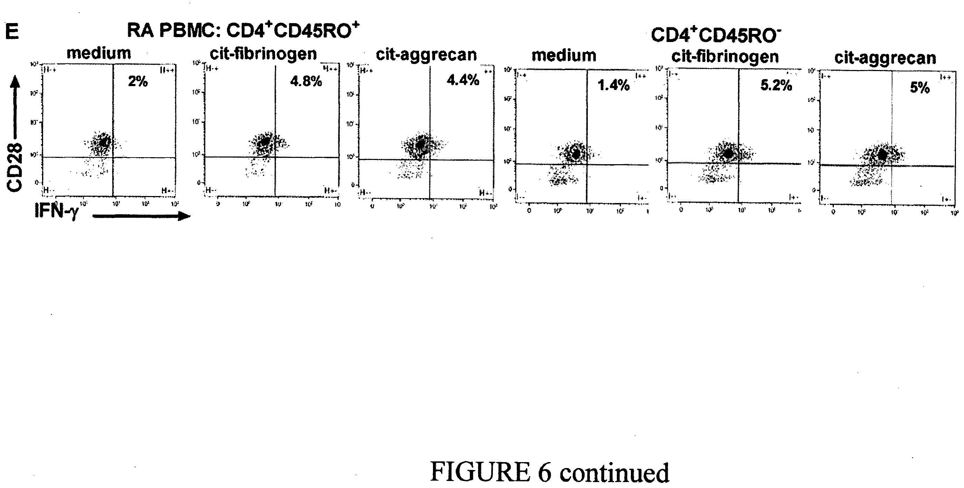

Figure 6 is a graphical representation showing cytokine secretion by CD4⁺ T cell subsets. PBMC from HLA-DR SE⁺ RA patients and healthy controls were incubated with 0 or 30 µg/ml citrullinated fibrinogen or citrullinated aggrecan, stained with mAb directed against CD4, CD28, IFN-γ and IL-6 (A), or CD4, CD45RO, IL-6 and IFN-γ (E) then analyzed by flow cytometry. B: The gating strategy is outlined. C: Fluorescence minus one (FMO) plot showing background staining for intracellular cytokine staining, gated on CD4⁺ T cells. D: IL-6 staining of gated CD4⁻ non-T cells. The proportions in (E) of IL-6⁺CD4⁺CD45RO⁺ T cells were 2.2%, 5.3% and 7.1%, and of IL-6⁺CD4⁺CD45RO⁻ T cells were 0.8%, 2.1% and 4% in response to incubation with no peptide, citrullinated fibrinogen and citrullinated aggrecan respectively. Two individual RA patients and 1 healthy control are shown. Data are representative of 2 healthy donors and 5 RA patients.

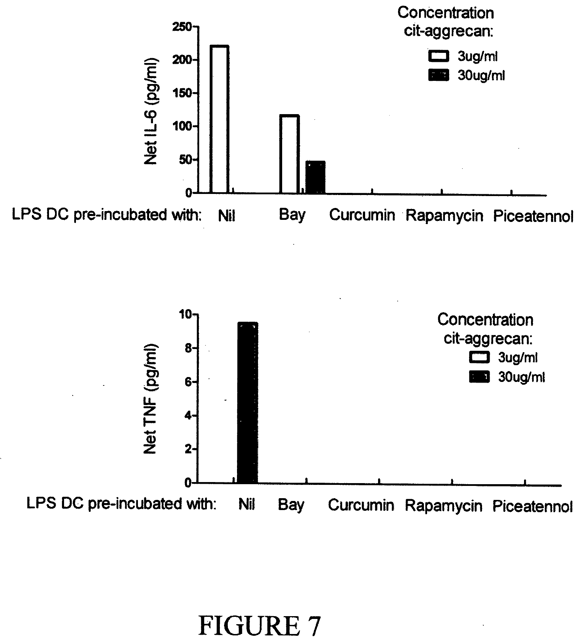

Figure 7 a graphical representation showing that inhibition of antigen-presenting cells from an RA patient with inhibitors of NF-κB, mTOR or Syk suppresses their capacity to induce T cell cytokine production in response to citrullinated aggrecan peptide.

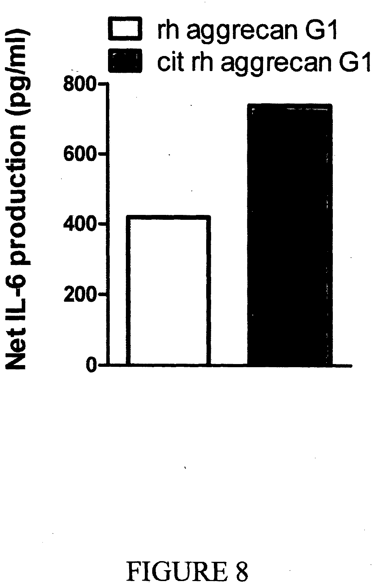

Figure 8 is graphical representation showing a cytokine response to the citrullinated aggrecan G1 epitope in an RA patient.

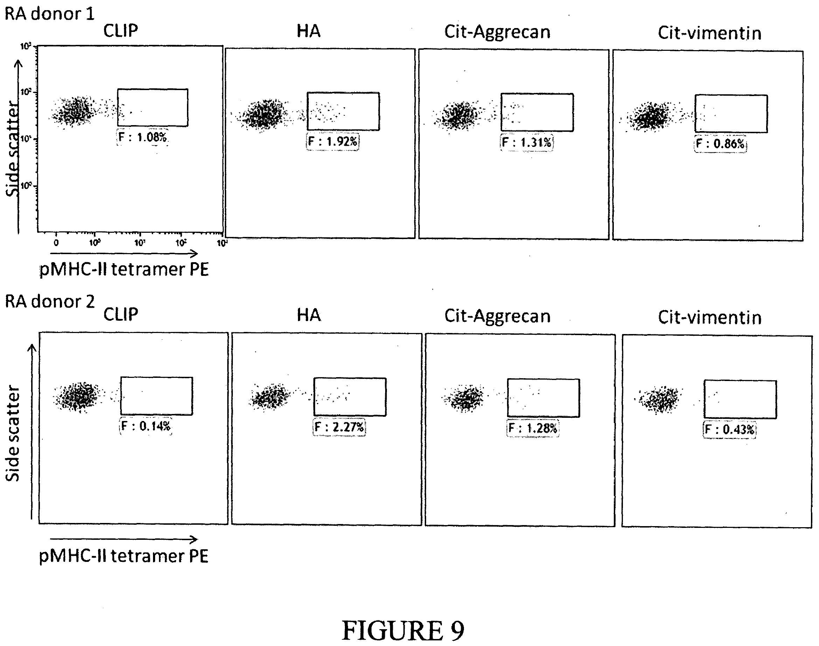

Figure 9 is graphical representation showing that citrullinated aggrecan-specific T cells are present in peripheral blood of rheumatoid arthritis patients as determined by tetramer staining.

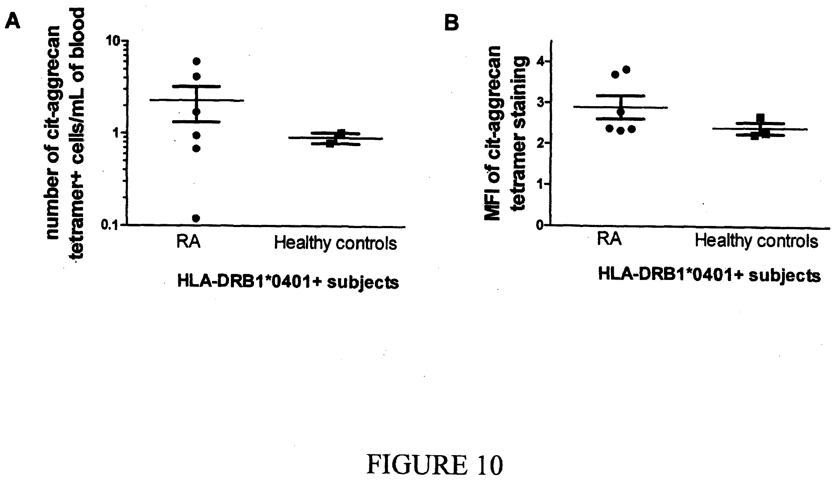

Figure 10 is graphical representation showing number and fluorescence intensity of cit-aggrecan-specific T cells in peripheral blood.

**TABLE A**

| **BRIEF DESCRIPTION OF THE SEQUENCES** | | |
|---|---|---|
| **SEQUENCE ID NUMBER** | **SEQUENCE** | **LENGTH** |
| SEQ ID NO: 1 | Nucleotide sequence corresponding to human aggrecan (ACAN), transcript variant 1, as set forth in GenBank Accession No. NM_001135. | 7296 nts |
| SEQ ID NO: 2 | Polypeptide encoded by SEQ ID NO: 1. | 2431 aa |
| SEQ ID NO: 3 | Nucleotide sequence corresponding to human aggrecan (ACAN), transcript variant 2, as set forth in GenBank Accession No. NM_013227. | 7593 nts |
| SEQ ID NO: 4 | Polypeptide encoded by SEQ ID NO: 3. | 2530 aa |
| SEQ ID NO: 5 | Peptide P22/25-36/39 corresponding to the G1 A loop of aggrecan, as disclosed in Buzás *et al.** | 15 aa |
| SEQ ID NO: 6 | Peptide P49-63 corresponding to the G1 A loop of aggrecan, as disclosed in Buzás *et al.** | 15 aa |
| SEQ ID NO: 7 | Peptide P70-84 corresponding to the G1 A loop of aggrecan, as disclosed in Buzás *et al.** | 15 aa |
| SEQ ID NO: 8 | Peptide P136-150 corresponding to the G1 A loop of aggrecan, as disclosed in Buzás *et al.** | 16 aa |
| SEQ ID NO: 9 | Peptide P268-282 corresponding to the G1 B' loop of aggrecan, as disclosed in Buzás *et al.** | 15 aa |
| SEQ ID NO: 10 | Peptide P13-27 corresponding to the G1 A loop of aggrecan, as disclosed in Buzas *et al.** | 15 aa |
| SEQ ID NO: 11 | Peptide P97-112 corresponding to the G1 A loop of aggrecan, as disclosed in Buzás *et al.** | 15 aa |
| SEQ ID NO: 12 | Peptide P155-169 corresponding to the G1 B loop of aggrecan, as disclosed in Buzás *et al.** | 15 aa |
| SEQ ID NO: 13 | Peptide P322-336 corresponding to the G1 B' loop of aggrecan, as disclosed in Buzás *et al.** | 15 aa |
| SEQ ID NO: 14 | Peptide P1530-1543 corresponding to a chondroitin sulfate (CS) region of aggrecan, as disclosed in Buzás *et al.** | 14 aa |
| SEQ ID NO: 15 | Peptide P2074-2086 corresponding to a CS region of aggrecan, as disclosed in Buzás *et al.***.* | 13 aa |
| SEQ ID NO: 16 | Peptide P2205-2219 corresponding to the G3 domain of aggrecan, as disclosed in Buzás *et al.** | 15 aa |
| SEQ ID NO: 17 | Peptide P2373-2387 corresponding to the G3 domain of aggrecan, as disclosed in Buzás *et al.** | 15 aa |
| SEQ ID NO: 18 | Peptide P2382-2396 corresponding to the G3 domain of aggrecan, as disclosed in Buzás *et al.** | 15 aa |
| SEQ ID NO: 19 | Peptide P570-582 corresponding to the G2 domain of aggrecan, as disclosed in Buzás *et al.** | 13 aa |
| SEQ ID NO: 20 | Peptide P694-705 corresponding to the keratan sulfate (KS) domain of aggrecan, as disclosed in Buzás *et al.** | 12 aa |
| SEQ ID NO: 21 | Peptide P846-859 corresponding to a CS region of aggrecan, as disclosed in Buzás *et al.** | 14 aa |
| SEQ ID NO: 22 | Peptide P968-978 corresponding to a CS region of aggrecan, as disclosed in Buzás *et al.** | 11 aa |
| SEQ ID NO: 23 | Peptide P1055-1066 corresponding to a CS region of aggrecan, as disclosed in Buzás *et al.** | 12 aa |
| SEQ ID NO: 24 | Peptide P1651-1664 corresponding to a CS region of aggrecan, as disclosed in Buzás *et al.** | 14 aa |
| SEQ ID NO: 25 | Peptide P1770-1783 corresponding to a CS region of aggrecan, as disclosed in Buzás *et al.** | 14 aa |
| SEQ ID NO: 26 | Peptide P1822-1835 corresponding to a CS region of aggrecan, as disclosed in Buzás *et al.** | 14 aa |
| SEQ ID NO: 27 | Peptide P18462-1858 corresponding to a CS region of aggrecan, as disclosed in Buzás *et al.** | 14 aa |
| SEQ ID NO: 28 | Peptide P1903-1916 corresponding to a CS region of aggrecan, as disclosed in Buzás *et al.** | 14 aa |
| SEQ ID NO: 29 | Peptide P1989-2003 corresponding to a CS region of aggrecan, as disclosed in Buzás *et al.** | 15 aa |
| SEQ ID NO: 30 | Peptide P2217-2231 corresponding to the G3 domain of aggrecan, as disclosed in Buzás *et al.** | 15 aa |
| SEQ ID NO: 31 | Peptide P2363-2378 corresponding to the G3 domain of aggrecan, as disclosed in Buzás *et al.** | 16 aa |
| SEQ ID NO: 32 | Peptide P84-103 corresponding to the G1 A loop of aggrecan | 20 aa |
| SEQ ID NO: 33 | Peptide P84-103 corresponding to the G1 A loop of aggrecan, with R93Cit substitution | 20 aa |
| SEQ ID NO: 34 | Peptide P84-103 corresponding to the G1 A loop of aggrecan, with R95Cit substitution | 20 aa |
| SEQ ID NO: 35 | Peptide P84-103 corresponding to the G1 A loop of aggrecan, with R93Cit and R95Cit substitutions | 20 aa |
| SEQ ID NO: 36 | Nucleotide sequence corresponding to human aggrecan (ACAN), transcript variant 1, mature polypeptide as set forth in GenBank Accession No. NM_001135. | 7239 nts |
| SEQ ID NO: 37 | Mature polypeptide encoded by SEQ ID NO: 36. | 2412 aa |
| SEQ ID NO: 38 | Nucleotide sequence corresponding to human aggrecan (ACAN), transcript variant 1, mature polypeptide as set forth in GenBank Accession No. NM_001135. | 7536 nts |
| SEQ ID NO: 39 | Mature polypeptide encoded by SEQ ID NO: 38. | 2511 aa |
| SEQ ID NO: 40 | Nucleotide sequence corresponding to human aggrecan (ACAN), transcript variant 1, G1 domain | 906 nts |
| SEQ ID NO: 41 | Aggrecan G1 domain encoded by SEQ ID NO: 40. | 302 aa |
| SEQ ID NO: 42 | Nucleotide sequence corresponding to human aggrecan (ACAN), transcript variant 1, G2 domain | 591 nts |
| SEQ ID NO: 43 | Aggrecan G2 domain encoded by SEQ ID NO: 42. | 197 aa |
| SEQ ID NO: 44 | Nucleotide sequence corresponding to human aggrecan (ACAN), transcript variant 1, G3 domain | 390 nts |
| SEQ ID NO: 45 | Aggrecan G3 domain encoded by SEQ ID NO: 44 | 130 aa |
| SEQ ID NO: 46 | Shared Epitope Motif 1 | 5 aa |
| SEQ ID NO: 47 | Shared Epitope Motif 2 | 5 aa |
| SEQ ID NO: 48 | Shared Epitope Motif 3 | 5 aa |
| SEQ ID NO: 49 to SEQ ID NO: 530 | Overlapping peptides spanning human aggrecan (ACAN) variant 1 with 15 aa overlap to adjacent peptide(s) | 20 aa |
| SEQ ID NO: 531 | Overlapping peptide at carboxyl terminal end of human aggrecan (ACAN) variant 1 with 15 aa overlap to adjacent peptide | 21 aa |

| | | |
|---|---|---|
| * Buzás et al., 2005. Cellular Immunology 235: 98-108. | | |

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

The term "about" is used herein to refer to conditions (*e.g*., amounts, concentrations, time *etc.*) that vary by as much as 15%, and suitably by as much as 10%, 9%, 8%, 7%, 6%, 5%, 4%, 3%, 2% or 1% to a specified condition.

The terms "administration concurrently" or "administering concurrently" or "co-administering" and the like refer to the administration of a single composition containing two or more actives, or the administration of each active as separate compositions and/or delivered by separate routes either contemporaneously or simultaneously or sequentially within a short enough period of time that the effective result is equivalent to that obtained when all such actives are administered as a single composition. By "simultaneously" is meant that the active agents are administered at substantially the same time, and desirably together in the same formulation. By "contemporaneously" it is meant that the active agents are administered closely in time, *e.g*., one agent is administered within from about one minute to within about one day before or after another. Any contemporaneous time is useful. However, it will often be the case that when not administered simultaneously, the agents will be administered within about one minute to within about eight hours and preferably within less than about one to about four hours. When administered contemporaneously, the agents are suitably administered at the same site on the subject. The term "same site" includes the exact location, but can be within about 0.5 to about 15 centimeters, preferably from within about 0.5 to about 5 centimeters. The term "separately" as used herein means that the agents are administered at an interval, for example at an interval of about a day to several weeks or months. The active agents may be administered in either order. The term "sequentially" as used herein means that the agents are administered in sequence, for example at an interval or intervals of minutes, hours, days or weeks. If appropriate the active agents may be administered in a regular repeating cycle.

The term "anergy" as used herein refers to a suppressed response, or a state of non-responsiveness, to a specified antigen or group of antigens by an immune system. For example, T lymphocytes and B lymphocytes are anergic when they cannot respond to their specific antigen under optimal conditions of stimulation.

By "antigen" is meant all, or part of, a protein, peptide, or other molecule or macromolecule capable of eliciting an immune response in a vertebrate animal, especially a mammal. Such antigens are also reactive with antibodies from animals immunized with that protein, peptide, or other molecule or macromolecule.

By "antigen-binding molecule" is meant a molecule that has binding affinity for a target antigen. It will be understood that this term extends to immunoglobulins, immunoglobulin fragments and non-immunoglobulin derived protein frameworks that exhibit antigen-binding activity.

By "autologous" is meant something (*e.g.*, cells, tissues *etc.*) derived from the same organism.

The term "allogeneic" as used herein refers to cells, tissues, organisms etc. that are of different genetic constitution.

By "alloantigen" is meant an antigen found only in some members of a species, such as blood group antigens. By contrast a "xenoantigen" refers to an antigen that is present in members of one species but not members of another. Correspondingly, an "allograft" is a graft between members of the same species and a "xenograft" is a graft between members of a different species.

The term "biological sample" as used herein refers to a sample that may be extracted, untreated, treated, diluted or concentrated from a subject. The biological sample may include a biological fluid such as whole blood, serum, plasma, saliva, urine, sweat, ascitic fluid, peritoneal fluid, synovial fluid, amniotic fluid, cerebrospinal fluid, tissue biopsy, and the like. In certain embodiments, the biological sample is selected from synovial fluid and blood, including peripheral blood.

"Clinical improvement" refers to prevention of further progress of RA or joint damage or any improvement in RA or joint damage as a result of treatment, as determined by various testing, including radiographic testing. Thus, clinical improvement may, for example, be determined by assessing the number of tender or swollen joints, performing the Disease Activity Score (DAS) or American College of Rheumatology (ACR) score, performing a global clinical assessment of the subject, assessing erythrocyte sedimentation rate, or assessing the amount of C-reactive protein level.

Throughout this specification, unless the context requires otherwise, the words "comprise," "comprises" and "comprising" will be understood to imply the inclusion of a stated step or element or group of steps or elements but not the exclusion of any other step or element or group of steps or elements. Thus, use of the term "comprising" and the like indicates that the listed elements are required or mandatory, but that other elements are optional and may or may not be present. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

By "corresponds to" or "corresponding to" is meant an antigen that encodes an amino acid sequence that displays substantial similarity to an amino acid sequence in a target antigen. In general the antigen will display at least about 30, 40, 50, 55, 60, 65, 70, 75, 80, 85, 90, 91, 92, 93, 94, 95, 96, 97, 98, 99 % similarity to at least a portion of the target antigen.

"Citrulline" and "Cit" refers to 2-amino-5-(carbamoylamino)pentanoic acid and is an α-amino acid with formula: H₂NC(O)NH(CH₂)₃CH(NH₂)CO₂H.

The expression "effective amount" refers to an amount of an agent or medicament, either in a single dose or as part of a series, which is effective for treating or preventing RA or joint damage. This would include an amount that is effective in achieving a reduction in RA or joint damage as compared to baseline prior to administration of such amount as determined, *e.g*., by radiographic or other testing. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

As used herein, "gene therapy" refers to the insertion, *ex vivo* or *in vivo,* of a gene or genes into individual cells or groups of cells (such as tissues or organs), whereby expression of the gene or genes in the cells or groups of cells provides a therapeutic effect. Such "therapeutic genes" are generally delivered using a vector. Cells targeted by gene therapy can be either somatic cells or germ cells or cell lines. Gene therapy includes and encompasses the use of vectors to deliver, either *ex vivo* or *in vivo,* a gene that requires overexpression or ectopic expression in a cell or group of cells. The vector can facilitate integration of the new gene in the nucleus or can lead to episomal expression of that gene.

"Immune effector cells" refers to cells capable of binding an antigen and which mediate an immune response selective for the antigen. These cells include, but are not limited to, T cells (T lymphocytes), B cells (B lymphocytes), monocytes, macrophages, natural killer (NK) cells and cytotoxic T lymphocytes (CTLs), for example CTL lines, CTL clones, and CTLs from tumor, inflammatory, or other infiltrates
Reference herein to "immuno-interactive" includes reference to any interaction, reaction, or other form of association between molecules and in particular where one of the molecules is, or mimics, a component of the immune system.

"Joint damage" is used herein in the broadest sense and refers to damage or partial or complete destruction to any part of one or more joints, including the connective tissue and cartilage, where damage includes structural and/or functional damage of any cause, and may or may not cause joint pain/arthralgia. It includes, without limitation, joint damage associated with or resulting from inflammatory joint disease as well as non-inflammatory joint disease. This damage may be caused by any condition, such as an autoimmune disease, especially arthritis, and most especially RA. Exemplary conditions of this type include acute and chronic arthritis, RA including juvenile-onset RA, juvenile idiopathic arthritis (JIA), or juvenile RA (JRA), and stages such as rheumatoid synovitis, gout or gouty arthritis, acute immunological arthritis, chronic inflammatory arthritis, degenerative arthritis, type II collagen-induced arthritis, infectious arthritis, septic arthritis, Lyme arthritis, proliferative arthritis, psoriatic arthritis, Still's disease, vertebral arthritis, osteoarthritis, arthritis chronica progrediente, arthritis deformans, polyarthritis chronica primaria, reactive arthritis, menopausal arthritis, estrogen-depletion arthritis, and ankylosing spondylitis/rheumatoid spondylitis), rheumatic autoimmune disease other than RA, and significant systemic involvement secondary to RA (including but not limited to vasculitis, pulmonary fibrosis, or Felty's syndrome). For purposes herein, joints are points of contact between elements of a skeleton (of a vertebrate such as an animal) with the parts that surround and support it and include, but are not limited to, for example, hips, joints between the vertebrae of the spine, joints between the spine and pelvis (sacroiliac joints), joints where the tendons and ligaments attach to bones, joints between the ribs and spine, shoulders, knees, feet, elbows, hands, fingers, ankles and toes, but especially joints in the hands and feet.

Reference herein to a "level or functional activity" in the context of a gene expression product (*e.g*., a protein or a transcript) produced by a specified cell is to be taken in its broadest sense and includes a level or functional activity of the expression product that is produced in a single cell or in a plurality or population of cells. In the latter case, therefore, it will be understood that the phrase will encompass a mean level or functional activity of the protein produced by a plurality or population of cells.

By "pharmaceutically-acceptable carrier" is meant a solid or liquid filler, diluent or encapsulating substance that may be safely used in topical or systemic administration.

The terms "polyarthritis" and "synovitis" are used interchangeably herein to refer to inflammation, *i.e.*, swelling, tenderness, or warmth, at one or more joints of a subject.

"Polypeptide," "peptide" and "protein" are used interchangeably herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers.

"Prevention" or "prophylaxis," as used herein, refers to prophylactic or preventative measures. Those in need of prevention or prophylaxis include those in whom the RA or joint damage is to be prevented, and in some embodiments, may be predisposed or susceptible to the RA or joint damage e.g. individuals with a family history of RA, or individuals bearing the HLA-SE and anti-citrullinated peptide antibodies but not meeting ACR/EULAR 2010 criteria for RA. Prevention or prophylaxis is successful herein if the development of RA or joint damage is completely or partially prevented or slowed down, as compared to the development of RA or joint or structural damage in the absence of tolerogenesis.

By "regulatory lymphocyte" is meant a lymphocyte that is involved in regulating or suppressing responses and actions of other cells, especially of other immune cells such as B lymphocytes, T helper and innate (including NKT and gamma-delta T) lymphocytes.

As used herein, "rheumatoid arthritis" or "RA refers to a recognized disease state that may be diagnosed according to the 2000 revised American Rheumatoid Association criteria for the classification of RA, or any similar criteria. The term includes not only active and early RA, but also incipient RA, as defined below. Physiological indicators of RA include symmetric joint swelling, which is characteristic though not invariable in RA. Fusiform swelling of the proximal interphalangeal (PIP) joints of the hands as well as metacarpophalangeal (MCP), wrists, elbows, knees, ankles, and metatarsophalangeal (MTP) joints are commonly affected and swelling is easily detected. Pain on passive motion is the most sensitive test for joint inflammation, and inflammation and structural deformity often limits the range of motion for the affected joint. Typical visible changes include ulnar deviation of the fingers at the MCP joints, hyperextension, or hyperflexion of the MCP and PIP joints, flexion contractures of the elbows, and subluxation of the carpal bones and toes. RA includes, for example, juvenile-onset RA, juvenile idiopathic arthritis (JIA), or juvenile RA (JRA). A patient with "active rheumatoid arthritis" means a patient with active and not latent symptoms of RA. Subjects with "early rheumatoid arthritis" are those subjects who are diagnosed as having 'definite' RA for no longer than four years, no longer than three years, no longer than two years, no longer than one year, no longer than six months, wherein definite RA is diagnosed when the subject's clinical parameters provide a score of ≥ 6/10 according to the revised 2010 American College of Rheumatology (ACR) / European League Against Rheumatism (EULAR) criteria for classification of RA (Aletaha et al., 2010. Arthritis & Rheumatism 62(9):2569-2581). Patients with "incipient RA" have early polyarthritis (synovitis) that does not fully meet ACR/EULAR criteria for a diagnosis of definite RA (*e.g.*, a score of < 6/10). In specific examples, the polyarthritis associates with the presence of RA-specific prognostic biomarkers such as anti-cyclic citrullinated peptide (CCP) antibodies and SE⁺.

"Shared epitope" or "SE" or "rheumatoid epitope," as used herein, means the sequence motifs in residues 70 to 74 of the third hypervariable region of the HLA-DRB1 chain encoded by the HLA-DRB1^{∗}0401, *0404/0408, *0405, *0409, *0410, *0413, *0416, *0101, *0102, *0104, *1001, *1402, and *1406 alleles in the predisposition to RA. Specifically, the sequence motifs are characterized by the amino acid coding sequence QKRAA (SEQ ID NO: 46) or QRRAA (SEQ ID NO: 47) or RRRAA (SEQ ID NO: 48) in the third hypervariable region, encompassing amino acid residues 70 to 74 of the HLA-DRB1 chain of the major histocompatibility complex class II molecule. Because DNA typing examines the alleles at a given locus, the name of the locus precedes the designation of the specific allele (with the two terms separated by an asterisk); for example, HLA-DRB1^{∗}0401 refers to the 0401 allele of the HLA-DRB 1 locus. One particular HLA-DR specificity is encoded by several HLA-DRB 1 alleles in conjunction with the product of the HLA-DRA1 locus; for example, more than 11 HLA-DRB1 alleles (HLA-DRB1^{∗}0401 to *0411) can encode the B chain of the HLA-DR4 specificity. For purposes herein, responsiveness to treatment of RA with an aggrecan-specific tolerogenic therapy is positively correlated with the incidence or presence of this genetic biomarker in patients with alleles for SE that are homozygous or heterozygous.

The terms "subject," "patient" or "individual," which are used interchangeably herein, refer to any subject, particularly a vertebrate subject, and even more particularly a mammalian subject, for whom therapy or prophylaxis is desired. Suitable vertebrate animals that fall within the scope of the invention include, but are not restricted to, any member of the subphylum Chordata including humans as well as non-human primates, rodents (*e.g*., mice rats, guinea pigs), lagomorphs (*e.g.*, rabbits, hares), bovines (*e.g*., cattle), ovines (*e.g.*, sheep), caprines (*e.g.*, goats), porcines (*e.g.*, pigs), equines (*e.g.*, horses), canines (*e.g.*, dogs), felines (*e.g.*, cats), avians (*e.g.*, chickens, turkeys, ducks, geese, companion birds such as canaries, budgerigars *etc.*), marine mammals (*e.g.*, dolphins, whales), reptiles (snakes, frogs, lizards *etc.*), and fish. In specific embodiments, the "subject," "patient" or "individual" is a human in need of treatment or prophylaxis of joint damage, including in subjects with early RA, or in subjects at risk of developing RA (*e.g*., incipient RA). In specific embodiments, the terms "subject," "patient" or "individual" refer to any single human subject, including a patient, eligible for treatment who is experiencing or has experienced one or more signs, symptoms, or other indicators of RA or joint damage, whether, for example, newly diagnosed or previously diagnosed and now experiencing a recurrence or relapse, or is at risk for RA or joint damage, no matter the cause. Intended to be included as a "subject," "patient" or "individual" are any subjects involved in clinical research trials not showing any clinical sign of disease, or subjects involved in epidemiological studies, or subjects once used as controls. The "subject," "patient" or "individual" may have been previously treated with a medicament for RA or joint damage, or not so treated.

By "suppression," "suppressing" and the like is meant any attenuation or regulation of an immune response, including B-lymphocyte and T lymphocyte immune responses, to an antigen or group of antigens. In some embodiments, the attenuation is mediated at least in part by suppressor T lymphocytes (*e.g*., CD4⁺CD25⁺ regulatory T lymphocytes).

As used herein, the term "surfactant" refers to any agent, which preferentially absorbs to an interface between two immiscible phases, such as the interface between water and an organic polymer solution, a water/air interface or organic solvent/air interface. Surfactants generally possess a hydrophilic moiety and a lipophilic moiety; such that, upon absorbing to microparticles, they tend to present moieties to the external environment that do not attract similarly coated particles, thus reducing particle agglomeration. Surfactants may also promote absorption of a therapeutic or diagnostic agent and increase bioavailability of the agent.

As used herein, a particle "incorporating a surfactant" refers to a particle with a surfactant on at least the surface of the particle. The surfactant may be incorporated throughout the particle and on the surface during particle formation, or may be coated on the particle after particle formation. The surfactant can be coated on the particle surface by adsorption, ionic or covalent attachment, or physically "entrapped" by the surrounding matrix. The surfactant can be, for example, incorporated into controlled release particles, such as polymeric microspheres.

A "symptom" of RA or joint damage is any morbid phenomenon or departure from the normal in structure, function, or sensation, experienced by the subject and indicative of RA or joint damage, such as those noted above, including tender or swollen joints.

"Total modified Sharp score" means a score obtained for assessment of radiographs using the method according to Sharp, as modified by Genant (1983, Am. J. Med., 30:35-47). The primary assessment will be the change in the total Sharp-Genant score from screening. The Sharp-Genant score combines an erosion score and a joint space narrowing score of both hands and feet. Joint damage is measured in this test scoring by a mean change of less than the score at baseline (when patient is screened or tested before first administration of the tolerogenic composition as disclosed herein).

As used herein, the term "transduction" or "transduced" to the introduction of foreign or exogenous nucleic acid, usually DNA, into a cell and includes "stable transduction" and "transient transduction." "Stable transduction" or "stably transduced" refers to the introduction and integration of foreign or exogenous nucleic acid into the genome of the transduced cell. The term "stable transductant" refers to a cell that has stably integrated foreign DNA into the genomic DNA. Conversely, the term "transient transduction" or "transiently transduced" refers to the introduction of foreign or exogenous nucleic acid into a cell where the foreign or exogenous DNA fails to integrate into the genome of the transduced cell. The foreign or exogenous DNA persists in the nucleus of the transducted cell for several days. During this time the foreign or exogenous DNA is subject to the regulatory controls that govern the expression of endogenous genes in the chromosomes. The term "transient transductant" refers to cells that have taken up foreign or exogenous DNA but have failed to integrate this DNA.

"Treatment" of a subject herein refers to therapeutic treatment. Those in need of treatment include those already with RA or joint damage as well as those in whom the progress of RA or joint damage is to be prevented. Hence, the subject may have been diagnosed as having the RA or joint damage or may have RA or joint symptoms or damage that is likely to progress in the absence of treatment. Alternatively the subject may be symptom-free but has risk factors for development of RA *e.g*., positive family history and/or presence of autoantibodies such as ACPA or rheumatoid factor or evidence of an inflammatory phenotype in peripheral blood. Treatment is successful herein if the RA or joint damage is alleviated or healed, or progression of RA or joint damage, including its signs and symptoms and/or structural damage, is halted or slowed down as compared to the condition of the subject prior to administration. Successful treatment further includes complete or partial prevention of the development of joint or structural damage. For purposes herein, slowing down or reducing RA or joint damage or the progression of joint damage is the same as arrest, decrease, or reversal of the RA or joint damage.

The terms "wild-type" and "normal" are used interchangeably to refer to the phenotype that is characteristic of most of the members of the species occurring naturally and contrast for example with the phenotype of a mutant.

As used herein, underscoring or italicizing the name of a gene shall indicate the gene, in contrast to its protein product, which is indicated by the name of the gene in the absence of any underscoring or italicizing. For example, "*ACAN*" shall mean the *ACAN* gene (*i.e.*, the gene encoding aggrecan), whereas "ACAN" shall indicate the protein product or products generated from transcription and translation and alternative splicing of the "*ACAN*" gene.

### 2. Abbreviations

The following abbreviations are used throughout the application:
aa = amino acid(s)
*ACAN* = aggrecan gene
ACAN = aggrecan polypeptide
ACPA = anti-citrullinated peptide antibodies
agg-tolAPC = aggrecan-specific antigen-presenting cells
APC = antigen-presenting cell(s)
*cit-agg* = citrullinated aggrecan
d = Day
GM-CSF = granulocyte macrophage colony-stimulating factor
h = Hour
IL-2 = interleukin 2
IL-3 = interleukin 3
IL-6 = interleukin 6
IL-10 = interleukin 10
IL-12 = interleukin 12
IL-17 = interleukin 17
kb = kilobase(s) or kilobase pair(s)
kDa = kilodalton(s)
nt = Nucleotide
nts = Nucleotides
PB = peripheral blood
PBMC = peripheral blood mononuclear cells
RA = rheumatoid arthritis
s = seconds
SE = shared epitope
TCR = T cell receptor
tolAPC = tolerogenic antigen-presenting cells
TNF =tumor necrosis factor

### 3. Modes for carrying out the invention

The present invention is based in part on the finding that patients with early RA are more likely to make a T lymphocyte response, including a pro-inflammatory T lymphocyte response, to no autoantigen or to citrullinated aggrecan alone, while patients with longstanding RA are more likely to make a T lymphocyte response, including a pro-inflammatory T lymphocyte response, to multiple autoantigens. These results suggest that T lymphocyte responses to citrullinated aggrecan, including pro-inflammatory T lymphocyte responses, precede heterogeneous maturation of the T lymphocyte response to other citrullinated self-epitopes as RA progresses. It was also found that patients with RA of any disease duration were more likely to respond to citrullinated aggrecan than to other citrullinated self-epitopes. Based on these findings, the present inventors propose that antigens that correspond in whole, or in part, to an aggrecan polypeptide, including citrullinated forms thereof, will allow earlier therapeutic and/or prophylactic intervention of joint damage in affected or predisposed subjects including subjects with early RA and subjects at risk of developing RA, suitably before the disease progresses to the chronic and debilitating form of RA, and that therapeutic application of citrullinated aggrecan antigens will provide broader coverage of the RA population as a whole than of other citrullinated antigens.

For illustration only, the present invention thus provides methods for treating or preventing joint damage in a subject through antigen-specific suppression of an immune response (also referred to herein as an "antigen-specific tolerogenic response") to an aggrecan polypeptide.

### 3.1 Aggrecan-specific immune response and affected subjects

The aggrecan polypeptide is typically citrullinated in which at least one arginine residue in the aggrecan sequence is converted to citrulline. If desired, the presence of citrullinated aggrecan polypeptide may be determined using assays that directly detect citrullinated aggrecan, illustrative examples of which include mass spectroscopic analysis, as described for example by Goëb et al. (2009, Arthritis Research & Therapy 11:R38), Stensland et al. (2009, Rapid Commun Mass Spectrom. 23(17):2754-2762), Hermansson et al. (2010, Proteomics Clin Appl. 4(5):511-518), van Beers et al. (2010, Arthritis Res Ther. 12(6):R219), and De Ceuleneer et al. (2011, Rapid Commun Mass Spectrom. 25(11):1536-1542). Alternatively, immunoassays may be employed, which use for example anti-modified citrulline antibodies for detecting citrulline residues in an antigen of interest and optionally antigen-specific antibodies for detecting the antigen of interest itself (*i.e*., whether citrullinated or not). Non-limiting immunoassays of this type are described by Tilleman et al. (2008, Rheumatology 47:597-604), Tabushi et al. (2008, Annals of Clinical Biochemistry 45:413-417) and in US Patent Application 2011/0244492. In other examples, the presence of citrullinated aggrecan may be determined indirectly by detecting ACPA specific for citrullinated aggrecan. Representative assays of this type may be set up by using one or more peptides corresponding to a citrullinated aggrecan polypeptide (*cit*-agg-specific peptides) or a protein corresponding to citrullinated aggrecan polypeptide as antigen, and detecting the binding of *cit*-agg-specific ACPA comprised in a sample to the peptide antigen by appropriate means. ACPA may be detected by homogeneous assay formats (*e.g*., by agglutination of latex particles coated with *cit*-agg peptide), by a heterogeneous immunoassay (*e.g.*, based on directly or indirectly coating *cit*-agg peptide to a solid phase, incubating the solid phase with a sample known or suspected to comprise *cit*-agg-specific ACPA under conditions allowing for binding of ACPA antibodies to peptide antigen, and directly or indirectly detecting the ACPA bound) or using a double-antigen bridge assay, in which *cit*-agg peptide is used both at the solid-phase side as well as at the detection side of this immunoassay. Non-limiting antigen-specific ACPA assays of this type are described for example in Wegner *et al.* (2010, *supra*) and bibliographic references listed therein.

In specific embodiments, the subject is identified as having an RA stratification selected from early RA or incipient RA, as defined herein, and in illustrative examples of this type, the methods further comprise identifying that the subject has the RA stratification prior to eliciting the antigen-specific tolerogenic response. The RA stratification may be determined using any suitable clinical parameters known to practitioners in the art, non-limiting examples of which include to: 1) age; 2) gender; 3) distribution of involved joints; 4) duration of morning stiffness; 5) number of tender joints (*e.g*., positive squeeze across metacarpophalangeal or metatarsophalangeal joints); 6) number of swollen joints; 7) joint pain; 8) previous episodes; 9) family history of RA; 10) the presence or absence of systemic flu-like features and fatigue; 11) symmetrical joint involvement in hands and/or feet; 12) erythrocyte sedimentation rate; 13) level of C-reactive protein; 14) level of high sensitivity C-reactive protein; 15) level of rheumatoid factor (RF); 16) presence, absence or level of ACPA antibody; 17) presence, absence or level of antibodies to mutated citrullinated vimentin (anti-MCV antibody); 18) presence or absence of SE; 19) presence, absence or level of the J protein, Hdj2 (*e.g.*, in synovial fluid; 20) rheumatoid nodules; or 21) radiographic changes. In specific embodiments, the presence or absence of RA is assessed using the 2010 ACR/EULAR classification criteria for RA as disclosed by Aletaha *et al.* (2010, *supra*), which are summarized in the Table 1.

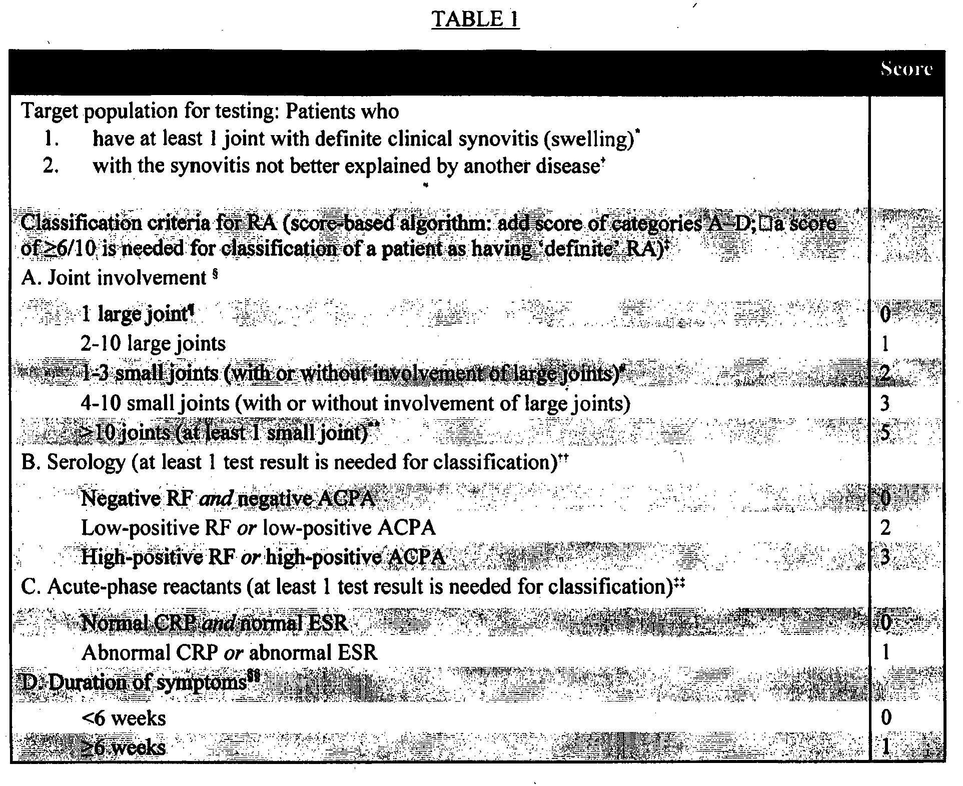

The symbols *, †, ‡, §, #, **, ††, ‡‡ and §§ have the same meaning as in Aletaha *et al.* (2010, *supra*).

In illustrative examples, the subject is identified as having early RA when the subject is diagnosed as having 'definite' RA for no longer than four years, no longer than three years, no longer than two years, no longer than one year, no longer than six months, wherein definite RA is identified when the subject's assessed clinical parameters provide a score of ≥ 6/10 according to the 2010 ACR/EULAR criteria. In other illustrative examples, the subject is identified as having incipient RA when the subject has polyarthritis (synovitis) but does not fully meet ACR/EULAR criteria for a diagnosis of definite RA (*e.g*., a score of < 6/10). In specific examples, the polyarthritis associates with the presence of RA-specific prognostic biomarkers such as ACPA antibodies and shared epitope positive (SE⁺). Subjects with incipient RA include ACPA antibody-positive patients who present with polyarthritis, and do not yet have a diagnosis of definite RA, but are at high risk for going on to develop definite RA according to the 2010 ACR/EULAR criteria (*e.g*., 95% probability).

In specific embodiments, the subject is SE⁺. Shared epitope positivity can be tested using any suitable assay that identifies the sequence motifs in residues 70 to 74 of the third hypervariable region of the HLA-DRB1 chain of the major histocompatibility complex class II molecule, as predisposing the subject to RA. For instance, the sequence motifs may be characterized by the amino acid coding sequence QKRAA (SEQ ID NO: 46) or QRRAA (SEQ ID NO: 47) or RRRAA (SEQ ID NO: 48), which are encoded for example by the HLA-DRB 1^{∗}0401, *0404/0408, *0405, *0409, *0410, *0413, *0416, *0101, *0102, *0104, *1001, *1402, and *1406 alleles. The assays may involve genotyping using for example sequence analysis, polymerase chain reaction (PCR) typing or oligonucleotide probe hybridization (*e.g*., reverse hybridization) and immunoassays as known in the art. In these embodiments, the methods may further comprise identifying that the subject is SE⁺ prior to eliciting the antigen-specific tolerogenic response.

The present inventors have also found that the T lymphocyte (*e.g*., CD4⁺ T lymphocyte) response to citrullinated self-epitopes, including citrullinated aggrecan, in subjects with early RA or at risk of developing RA, includes the production (*e.g*., secretion) of at least 1, 2, 3 or cytokine(s). In specific embodiments, the cytokine(s) is (are) selected from IL-6, IFN-γ, TNF and IL-10. The T lymphocyte response largely includes a pro-inflammatory or autoreactive T lymphocyte response which is characterized by the production (*e.g*., secretion) of at least 1, 2 or 3 pro-inflammatory (*e.g.*, T*ₕ*1 or T*ₕ*17) cytokine(s). In specific embodiments, the pro-inflammatory cytokine(s) is (are) selected from IL-6, IFN-γ and TNF. Thus, in some embodiments, the methods of the present invention may further comprise identifying that the subject produces a pro-inflammatory T lymphocyte response (*e.g.*, the production of at least 1, 2 or 3 pro-inflammatory cytokine selected from IL-6, IFN-γ and TNF) prior to eliciting the antigen-specific tolerogenic response. However, the present inventors have also observed that subjects with early RA or at risk of developing RA are more likely to produce IL-6 to no epitope or to citrullinated aggrecan alone, and consequently the T lymphocyte response in those subjects may be characterized essentially by the production of IL-6. Accordingly, in specific embodiments, the methods of the present invention may further comprise identifying that the subject produces IL-6 prior to eliciting the antigen-specific tolerogenic response.

In order to identify whether a subject is making the T lymphocyte response, the methods of the invention may employ assays for detection or assessment of released cytokine(s). The cytokine(s) may be assayed using any suitable technique. Standard assays for determining cytokine levels include functional activity protocols such as but not limited to: (a) cytokine-induced proliferation of indicator cell lines; (b) cytokine-induced apoptosis; (c) cytokine-induced protection from viral infection; and (d) cytokine-induced cytokine production. Such assays are well known to the skilled person and can be found, for example, at "Cytokine Bioassays" (www.ebioscience.com/ebioscience/appls/BAC.htm). Alternatively, the cytokine levels may be determined using conventional cytokine release assays, intracellular cytokine secretion assays, intracellular cytokine secretion assays, detection of activation of signal transduction pathways downstream of the T cell receptor and microarray detection of mRNA for species of proteins that indicate a T cell response such as cytokine mRNA, ELISAs, bead arrays or multiplex assays, which are well known to the skilled person. Non-limiting bioassays for IL-6, IFN-γ, TNF and IL-10 are disclosed in the Examples.

The present inventors have also determined that the T lymphocyte response in subjects with early RA or at risk of developing RA is produced at least in part by CD4⁺ CD28 T lymphocytes and/or CD4⁺ CD28⁺ T lymphocytes. Such T lymphocytes can be routinely detected using standard immunoassays including, for example, flow cytometry (*e.g*., using a fluorescence activated cell sorter (FACS)), immunohistochemistry, immunomagnetic separation and the like. Non-limiting immunoassays of this type are disclosed in the Examples. The T lymphocytes for analysis may be obtained from any suitable biological sample from the subject, representative samples of which include blood (*e.g*., peripheral blood) and synovial fluid.

### 3.2 Immune modulators for producing an aggrecan-specific tolerogenic response

The antigen-specific tolerogenic response may be elicited using any suitable strategy, illustrative examples of which include: (1) increasing the number of tolerogenic antigen-presenting cells in the subject, which present a peptide (*e.g.*, an autoantigen) corresponding to a portion of the aggrecan polypeptide (also referred to herein as "aggrecan-specific tolerogenic antigen-presenting cells" or "agg-tolAPC"), wherein the portion is associated with the pro-inflammatory or autoreactive T lymphocyte response to the aggrecan polypeptide (*e.g.*, a *cit*-agg polypeptide); (2) inducing anergy or apoptosis in autoreactive effector lymphocytes in the subject, which are reactive against the aggrecan polypeptide (*e.g*., a *cit*-agg polypeptide) or portion thereof; and (3) increasing the number of regulatory or suppressor T lymphocytes in the subject, which suppress or otherwise reduce the autoreactive T lymphocyte response.

Aggrecan-specific tolAPC may be produced by contacting antigen-presenting cells (*e.g.*, dendritic cells, macrophages, Langerhans cells *etc.*) with an inhibitor of the NF-κB pathway in an amount sufficient to inhibit the NF-κB pathway of the antigen-presenting cells and with an antigenic molecule selected from an antigen that corresponds in whole, or in part, to an aggrecan polypeptide (*e.g*., a *cit*-aggrecan polypeptide) or a nucleic acid molecule from which the antigen is expressible, in an amount sufficient for the antigen-presenting cells to present the antigen or a processed form thereof on their surface. Non-limiting methods for producing tolerogenic APC *in vivo* and/or *in vitro* are described in U.S. Pat. Appl. Pubs. 20030153518, 20060182726, 20100151000 and European Patent Application 1462111. In other embodiments, aggrecan-specific tolAPC are produced by contacting the antigen-presenting cells with an mTOR inhibitor or a Syk pathway inhibitor in an amount sufficient to inhibit mTOR or the Syk pathway of the antigen-presenting cells, together with an antigenic molecule as described above.

Antigen-presenting cells include both professional and facultative types of antigen-presenting cells. Professional antigen-presenting cells include, but are not limited to, macrophages, monocytes, B lymphocytes, cells of myeloid lineage, including monocytic-granulocytic-DC precursors, marginal zone Küpffer cells, microglia, T cells, Langerhans cells and dendritic cells including interdigitating dendritic cells and follicular dendritic cells. Examples of facultative antigen-presenting cells include but are not limited to activated T cells, astrocytes, follicular cells, endothelium and fibroblasts. In some embodiments, the antigen-presenting cell is selected from monocytes, macrophages, B-lymphocytes, cells of myeloid lineage, dendritic cells or Langerhans cells.

### 3.2.1 Aggrecan antigen

The aggrecan antigen may comprise, consist or consist essentially of an amino acid sequence corresponding to a putatively full-length aggrecan polypeptide (*e.g*., as set forth in SEQ ID NO: 2 or 4, including citrullinated forms thereof). Alternatively, the antigen may comprise, consist or consist essentially of an amino acid sequence corresponding to a mature aggrecan polypeptide (*e.g*., as set forth in SEQ ID NO: 37 or 39, including citrullinated forms thereof) or to a domain thereof such as but not limited to the G1 domain (*e.g*., as set forth in SEQ ID NO: 41, including citrullinated forms thereof), the G2 domain (*e.g*., as set forth in SEQ ID NO: 43, including citrullinated forms thereof) or the G3 domain (*e.g*., as set forth in SEQ ID NO: 43, including citrullinated forms thereof). In other embodiments, the antigen may comprise, consist or consist essentially of an amino acid sequence corresponding to a T cell epitope (*e.g.*, an autoantigen) of an aggrecan polypeptide (*e.g.*, as set forth in any one of SEQ ID NO: 5-35, including citrullinated forms thereof). In specific embodiments, the antigen comprises, consists or consists essentially of an amino acid sequence as set forth in any one of SEQ ID NO: 32-35. In non-limiting examples, the antigen is HLA DR restricted and the subject is suitably positive for an HLA DR allele. In these examples, HLA DR-binding peptides (*e.g*., HLA-DRB-binding peptides) may be predicted using any suitable approach including the use of predictive algorithms as described for example by James et al. (2009, J Immunol 183:3249-3258; and 2010, Arthritis Rheum. 62(10):2909-2918), Knapp et al. (2011, BMC Bioinformatics 12:241), Honeyman et al. (1997, Ann. Med 29:401-404) and Hu et al. (2010, Nucleic Acids Research 38:Web Server issue W474-W479).

In some embodiments, the antigen is in the form of one or more peptides corresponding in whole or in part to an aggrecan polypeptide, including citrullinated forms thereof. Usually, such peptides are at least 6, 7, 8, 9, 10, 11, 12, 13, 14, 15, 20, 25, 30 amino acid residues in length and suitably no more than about 500, 200, 100, 80, 60, 50, 40 amino acid residues in length. In some embodiments in which two or more peptides are used, the peptides can be in the form of a plurality of contiguous overlapping peptides whose sequences span at least a portion of an aggrecan polypeptide, including citrullinated forms thereof. Suitably, the peptide sequences are derived from at least about 30, 40, 50, 60, 70, 80, 90, 91, 92, 93, 94, 95, 96, 97, 98, 99% of the sequence corresponding to the aggrecan polypeptide, including citrullinated forms thereof. In some embodiments, each peptide of the plurality of contiguous overlapping peptide fragments can be 30-90 amino acids in length, *e.g.*, 30, 35, 40, 45, 50, 55, 60, 65, 70, 73, 75, 80, 81, 85, 86 and 90 amino acids in length. In various embodiments, the amino acid sequences of contiguous overlapping peptide fragments in the plurality overlap by about 10 to about 15 amino acids, *e.g.*, 10, 11, 12, 13, 14 and 15 amino acids. Suitably, the length of the peptides is selected for presentation to cytolytic T lymphocytes (*e.g*., peptides of about 8 to about 10 amino acids in length), or for presentation to T helper lymphocytes (*e.g*., peptides of about 12 to about 20 amino acids in length). Exemplary methods for producing such peptide antigens are described, for example, by Astori et al. (2000, J. Immunol. 165:3497-3505; and references cited therein) and in U.S. Pat. Appl. Pub. No. 2004/0241178. If desired, the aggrecan antigen may be modified, for example, by lipid modification to modify its physico-chemical properties. Non-limiting examples of overlapping peptides spanning substantially the entire length of an aggrecan polypeptide, are set forth in SEQ ID NO: 49-531.

The aggrecan antigen(s) may be isolated from a natural source (*e.g*., from the subject). Alternatively, they may be prepared in recombinant form using standard protocols as for example described in: Sambrook, et al., MOLECULAR CLONING. A LABORATORY MANUAL (Cold Spring Harbor Press, 1989), in particular Sections 16 and 17; Ausubel et al., CURRENT PROTOCOLS IN MOLECULAR BIOLOGY (John Wiley & Sons, Inc. 1994-1998), in particular Chapters 10 and 16; and Coligan et al., CURRENT PROTOCOLS IN PROTEIN SCIENCE (John Wiley & Sons, Inc. 1995-1997), in particular Chapters 1, 5 and 6. In representative examples, the aggrecan antigen(s) may be prepared by a procedure including the steps of (a) providing an expression vector from which a nucleotide sequence encoding the aggrecan antigen is expressible; (b) introducing the vector into a suitable host cell; (c) culturing the host cell to express recombinant polypeptide from the vector; and (d) isolating the recombinant antigen. In specific embodiments, the aggrecan antigen(s) correspond to the sequence of an aggrecan polypeptide, whether citrullinated or not, which is produced by the species of animal to which the subject belongs.

Alternatively, the aggrecan antigen(s) is (are) synthesized using solution synthesis or solid phase synthesis as described, for example, by Atherton and Sheppard (Solid Phase Peptide Synthesis: A Practical Approach, IRL Press at Oxford University Press, Oxford, England, 1989) or by Roberge et al. (1995, Science 269:202).

In some embodiments, the aggrecan antigen comprises at least one citrulline residue. Citrullinated antigens can, for example, be obtained from natural, recombinant or synthetic forms of aggrecan antigen, through the action of peptidylarginine deiminase (PAD); they can also be obtained by peptide synthesis, by directly incorporating one or more citrulline residues into a synthesized aggrecan peptide. These methods are well known to the skilled practitioner. Illustrative methods of using PAD enzymes for making citrullinated antigens are described in U.S. Pat. Appl. Pub. No. 2011/0028399. Exemplary methods of synthesizing citrullinated antigens are described in Perez et al., 2006. Chem Biol Drug Des 68:194-200)

In other embodiments, the aggrecan antigen(s) are provided in nucleic acid form, generally by operably linking a nucleotide sequence that encodes the antigen(s) to a promoter, which may be constitutive or inducible, and which is operable in antigen-presenting cells of interest, to form a nucleic acid construct. Delivery of the nucleic acid constructs into antigen-presenting cells or their precursors may be achieved either by directly exposing a patient to the nucleic acid construct or by first transforming antigen-presenting cells or their precursors with the nucleic acid construct *in vitro,* and then transplanting the transformed antigen-presenting cells or precursors into the patient.

The nucleic acid construct may be introduced into the antigen-presenting cell by any suitable means including for example by contacting the antigen-presenting cell with the nucleic acid construct, electroporation, transformation, transduction, conjugation or triparental mating, transfection, infection membrane fusion with cationic lipids, high-velocity bombardment with DNA-coated microprojectiles, incubation with calcium phosphate-DNA precipitate, direct microinjection into single cells, and the like. Other methods also are available and are known to those skilled in the art. In specific embodiments, the nucleic acid constructs are introduced by means of cationic lipids, *e.g*., liposomes. Such liposomes are commercially available (*e.g.*, Lipofectin®, Lipofectamine™, and the like, supplied by Life Technologies, Gibco BRL, Gaithersburg, Md.).

The antigen-encoding nucleotide sequence of the construct may comprise a naturally occurring sequence or a variant thereof, which has been engineered using recombinant techniques. In one example of a variant, the antigen-encoding nucleotide sequence is codon optimized to permit enhanced expression of the antigen in an antigen-presenting cell of interest. Illustrative methods for codon optimization are described for example in U.S. Patent No. 5,795,737, WO 99/02694 and WO 00/42215.

The delivery of aggrecan antigen to an antigen-presenting cell or its precursor can be enhanced by methods known to practitioners in the art. For example, several different strategies have been developed for delivery of exogenous antigen to the endogenous processing pathway of antigen-presenting cells, especially dendritic cells. These methods include insertion of antigen into pH-sensitive liposomes (Zhou and Huang, 1994. Immunomethods 4:229-235), osmotic lysis of pinosomes after pinocytic uptake of soluble antigen (Moore et al., 1988. Cell 54:777-785), coupling of antigens to potent adjuvants (Aichele et al., 1990. J. Exp. Med. 171:1815-1820; Gao et al., 1991. J. Immunol. 147:3268-3273; Schulz et al., 1991. Proc. Natl. Acad Sci. USA 88:991-993; Kuzu et al., 1993. Euro. J. Immunol. 23:1397-1400; and Jondal et al., 1996. Immunity 5:295-302), exosomes (Zitvogel et al., 1998. Nat Med 4:594-600; 2002, Nat Rev Immunol. 2:569-79), and apoptotic cell delivery of antigen (Albert et al., 1998. Nature 392:86-89**;** Albert et al., 1998, Nature Med. 4:1321-1324; and in International Publications WO 99/42564 and WO 01/85207). Recombinant bacteria (*e.g*., *E. coli*) or transfected host mammalian cells may be pulsed onto dendritic cells (as particulate antigen, or apoptotic bodies respectively) for antigen delivery. Recombinant chimeric virus-like particles (VLPs) have also been used as vehicles for delivery of exogenous heterologous antigen to the MHC class I processing pathway of a dendritic cell line (Bachmann et al., 1996. Eur. J. Immunol. 26(11):2595-2600).

Alternatively, or in addition, an aggrecan antigen may be linked to, or otherwise associated with, a cytolysin to enhance the transfer of the antigen into the cytosol of an antigen-presenting cell of the invention for delivery to the MHC class I pathway. Exemplary cytolysins include saponin compounds such as saponin-containing Immune Stimulating Complexes (ISCOMs) (see *e.g.*, Cox and Coulter, 1997. Vaccine 15(3):248-256 and U.S. Patent No. 6,352,697), phospholipases (see, *e.g.*, Camilli et al., 1991. J. Exp. Med. 173:751-754), pore-forming toxins (*e.g.*, an alpha-toxin), natural cytolysins of gram-positive bacteria, such as listeriolysin O (LLO, *e.g.*, Mengaud et al., 1988. Infect. Immun. 56:766-772 and Portnoy et al., 1992. Infect. Immun. 60:2710-2717), streptolysin O (SLO, *e.g.*, Palmer et al., 1998. Biochemistry 37(8):2378-2383) and perfringolysin O (PFO, *e.g.*, Rossjohn et al., Cell 89(5), 685-692). Where the antigen-presenting cell is phagosomal, acid activated cytolysins may be advantageously used. For example, listeriolysin exhibits greater pore-forming ability at mildly acidic pH (the pH conditions within the phagosome), thereby facilitating delivery of vacuole (including phagosome and endosome) contents to the cytoplasm (see, *e.g.,* Portnoy et al., 1992. Infect. Immun. 60:2710-2717).

The cytolysin may be provided together with an aggrecan antigen in the form of a single composition or may be provided as a separate composition, for contacting the antigen-presenting cells. In one embodiment, the cytolysin is fused or otherwise linked to the antigen, wherein the fusion or linkage permits the delivery of the antigen to the cytosol of the target cell. In another embodiment, the cytolysin and antigen are provided in the form of a delivery vehicle such as, but not limited to, a liposome or a microbial delivery vehicle selected from virus, bacterium, or yeast. Suitably, when the delivery vehicle is a microbial delivery vehicle, the delivery vehicle is non-virulent. In a preferred embodiment of this type, the delivery vehicle is a non-virulent bacterium, as for example described by Portnoy et al. in U.S. Patent No. 6,287,556, comprising a first polynucleotide encoding a non-secreted functional cytolysin operably linked to a regulatory polynucleotide which expresses the cytolysin in the bacterium, and a second polynucleotide encoding one or more pre-selected antigens. Non-secreted cytolysins may be provided by various mechanisms, *e.g*., absence of a functional signal sequence, a secretion incompetent microbe, such as microbes having genetic lesions (*e.g.*, a functional signal sequence mutation), or poisoned microbes, etc. A wide variety of nonvirulent, non-pathogenic bacteria may be used; preferred microbes are relatively well-characterized strains, particularly laboratory strains of *E. coli,* such as MC4100, MC1061, DH5α, etc. Other bacteria that can be engineered for the invention include well-characterized, nonvirulent, non-pathogenic strains of *Listeria monocytogenes, Shigella flexneri,* mycobacterium, *Salmonella, Bacillus subtilis,* etc. In a particular embodiment, the bacteria are attenuated to be non-replicative, non-integrative into the host cell genome, and/or non-motile inter- or intra-cellularly.

The delivery vehicles described above can be used to deliver one or more aggrecan antigens to virtually any antigen-presenting cell capable of endocytosis of the subject vehicle, including phagocytic and non-phagocytic antigen-presenting cells. In embodiments when the delivery vehicle is a microbe, the subject methods generally require microbial uptake by the target cell and subsequent lysis within the antigen-presenting cell vacuole (including phagosomes and endosomes).

### 3.2.2 Altered Peptide Ligands

In some embodiments, an altered peptide ligand (APL) is used for stimulating aggrecan-specific tolerogenesis. The APL suitably comprises, consists or consists essentially of an amino acid sequence corresponding to a portion (*e.g*., an autoantigen) of an aggrecan polypeptide (*e.g.*, a cit-aggrecan polypeptide), which is suitably associated with rheumatoid arthritis (RA), and which binds to an antigen receptor (*e.g*., T cell receptor) on pro-inflammatory or autoreactive T lymphocytes. The APL amino acid sequence is generally distinguished from the amino acid sequence of the aggrecan polypeptide portion by at least one amino acid substitution, deletion or addition, and suitably interferes with normal signaling through the antigen receptor.

APL can be designed based on natural aggrecan peptide epitopes identified using any method known in the art. For example, methods involving isolating and assaying MHC molecules from antigen presenting cells can be used to identify peptides bound to the MHC molecules (Chicz and Urban, 1994. Immunol. Today 1-5:155-160. Bacteriophage "phage display" libraries can also be constructed. Using the "phage method" (Scott and Smith, 1990. Science 249:386-390; Cwirla et al., 1990. Proc. Natl. Acad. Sci. USA 87:6378-6382; Devlin et al., 1990. Science 249:404-406), very large libraries can be constructed (10⁶-10⁸ chemical entities). Other methods to identify peptide epitopes which can be used involve primarily chemical methods, of which the Geysen method (Geysen *et al.,* and the method of Fodor et al., 1991. Science 251:767-773) are examples. Furka et al., 1988. 14th International Congress of Biochemistry, Volume 5. Abstract FR:013; Furka, 1991. Int. J. Peptide Protein Res. 37:487-493). Houghton (U.S. Pat. No. 4,631,211) and Rutter et al.(U. S. Pat. No. 5,101,175) describe methods to produce a mixture of peptides that can be tested as agonists or antagonists. Other methods which can be employed involve use of synthetic libraries (Needels et al., 1993. Proc. Natl. Acad. Sci. USA 90:10700-4; Ohlmeyer et al., 1993. Proc. Natl. Acad. Sci. USA 90:10922-10926; Lam *et al*., International Patent Publication No. WO 92/00252), and the like can be used to screen for receptor ligands. Techniques based on cDNA subtraction or differential display have been described amply in the literature and can also be used see, for example, Hedrick et al., 1984. Nature 308:149; and Lian and Pardee, 1992. Science 257:967. The expressed sequence tag (EST) approach is a valuable tool for gene discovery (Adams et al., 1991. Science 252:1651), as are Northern blotting, RNase protection, and reverse transcriptase-polymerase chain reaction (RT-PCR) analysis (Alwine et al., 1977. Proc. Natl. Acad. Sci. USA 74:5350; Zinn et al., 1983. Cell 34:865; Veres et al., 1987. Science 237:415). Another technique which can be used is the "pepscan" technique (Van der Zee, 1989. Eur. J. Immunol. 19:43-47) in which several dozens of peptides are simultaneously synthesized on polyethylene rods arrayed in a 96-well microtiter plate pattern, similar to an indexed library in that the position of each pin defines the synthesis history on it. Peptides are then chemically cleaved from the solid support and supplied to irradiated syngeneic thymocytes for antigen presentation. A cloned CTL line is then tested for reactivity in a proliferation assay monitored by ³H-thymidine incorporation.

SPHERE is described in WO 97/35035. This approach utilizes combinatorial peptide libraries synthesized on polystyrene beads wherein each bead contains a pure population of a unique peptide that can be chemically released from the beads in discrete aliquots. Released peptide from pooled bead arrays are screened using methods to detect T cell activation, including, for example, .sup.3H-thymidine incorporation (for CD4⁺ or CD8⁺ T cells), ⁵¹Cr-release assay (for CTLs) or IL-2 production (for CD4⁺ T cells) to identify peptide pools capable of activating a T cell of interest. By utilizing an iterative peptide pool/releasing strategy, it is possible to screen more than 10⁷ peptides in just a few days. Analysis of residual peptide on the corresponding positive beads (>100 pmoles) allows rapid and unambiguous identification of the epitope sequence.

A brief overview of an assay to identify aggrecan peptides binding to CTLs is as follows: roughly speaking, ten 96-well plates with 1000 beads per well will accommodate 10⁶ beads; ten 96-well plates with 100 beads per well will accommodate 10⁵ beads. In order to minimize both the number of CTL cells required per screen and the amount of manual manipulations, the eluted peptides can be further pooled to yield wells with any desired complexity. For example, based on experiments with soluble libraries, it is possible to screen 10⁷ peptides in 96-well plates (10,000 peptides per well) with as few as 2×10⁶ CTL cells. After cleaving a percentage of the peptides from the beads, incubating them with gamma-irradiated foster APCs and the cloned CTL line(s), positive wells determined by ³H-thymidine incorporation are further examined. Alternatively, as pointed out above, cytokine production or cytolytic ⁵¹Cr-release assays may be used. Coulie et al., 1992. Int. J. Cancer 50:289-291. Beads from each positive well will be separated and assayed individually as before, utilizing an additional percentage of the peptide from each bead. Positive individual beads will then be decoded, identifying the reactive amino acid sequence. Analysis of all positives will give a partial profile of conservatively substituted epitopes that stimulate the CTL clone tested. At this point, the peptide can be resynthesized and retested. Also, a second library (of minimal complexity) can be synthesized with representations of all conservative substitutions in order to enumerate the complete spectrum of derivatives tolerated by a particular CTL. By screening multiple CTLs (of the same MHC restriction) simultaneously, the search for crossreacting epitopes is greatly facilitated.

The described method for the identification of CD8⁺ MHC Class I-restricted CTL epitopes can be applied to the identification of CD4⁺ MHC Class II-restricted CD4⁺ T cell epitopes. In this case, MHC Class II allele-specific libraries are synthesized such that haplotype-specific anchor residues are represented at the appropriate positions. MHC Class II agretopic motifs have been identified for the common alleles (*see*, for example, Rammensee, 1995. Curr. Opin. Immunol. 7:85-96; Altuvia et al., 1994. Mol. Immunol. 24:375-379; Reay et al., 1994. J. Immunol. 152:3946-3957; Verreck et al., 1994. Eur. J. Immunol. 24:375-379; Sinigaglia and Hammer, 1994. Curr. Opin. Immunol. 6:52-56; Rotzschke and Falk, 1994. Curr. Opin. Immunol. 6:45-51). The overall length of the peptides will generally be 12-20 amino acid residues, and previously described methods may be employed to limit library complexity. Aggrecan APLs may be synthesized using solution synthesis or solid phase synthesis as described, for example, by Atherton and Sheppard (1989, *supra*) or by Roberge *et al.*(1995, *supra*).

In specific embodiments, the APL has at least one activity selected from: (i) antagonizing the response of the T lymphocytes to the aggrecan polypeptide (*e.g*., a *cit*-aggrecan polypeptide), (ii) inducing anergy in aggrecan- or citrullinated aggrecan-specific T lymphocytes, (iii) inducing apoptosis in aggrecan- or citrullinated aggrecan-specific T lymphocytes, (iv) stimulating or inducing a aggrecan- or citrullinated aggrecan-specific Th2 immune response, (v) suppressing development of a aggrecan- or citrullinated aggrecan-specific Th1 immune response including suppressing the production of pro-inflammatory cytokines, (vi) stimulating activation of aggrecan- or citrullinated aggrecan-specific regulatory lymphocytes (*e.g*., T regulatory lymphocytes (Treg)), or (vii) preventing or inhibiting the activation of aggrecan- or citrullinated aggrecan-specific antigen-presenting cells by an inflammatory stimulus.

### 3.2.3 NF-κB inhibitors

The NF-κB inhibitor includes any molecule or compound that reduces the level or functional activity of NF-κB in immune cells, especially antigen-presenting cells. NF-κB inhibitors can take various forms, non-limiting examples of which include small molecules, nucleic acids, peptides, polypeptides, peptidomimetics *etc.*

In some embodiments, the NF-κB inhibitor decreases the level or functional activity of a member of the NF-κB pathway, which is suitably selected from BTK, LYN, BCR Igα, BCR Igβ, Syk, Blnk, PLCγ2, PKCβ, DAG, CARMA1, BCL10, MALT1, PI3K, PIP3, AKT, p38 MAPK, ERK, COT, IKKα, IKKβ, IKKγ, NIK, RelA/p65, P105/p50, c-Rel, RelB, p52, NIK, Leul3, CD81, CD19, CD21 and its ligands in the complement and coagulation cascade, TRAF6, ubiquitin ligase, Tab2, TAK1, NEMO, NOD2, RIP2, Lck, fyn, Zap70, LAT, GRB2, SOS, CD3 zeta, Slp-76, GADS, ITK, PLCγ1, PKCθ, ICOS, CD28, SHP2, SAP, SLAM and 2B4. In illustrative examples of this type, the NF-κB inhibitor decreases the level or functional activity of any one or more of RelA/p65, P105/p50, c-Rel, RelB or p52. Suitably, in these embodiments, the NF-κB inhibitor blocks, inhibits or otherwise antagonizes at least one function or activity of the member. In other embodiments, the NF-κB inhibitor increases the level or functional activity of a member of the NF-κB pathway, which is suitably selected from SHP1, SHIP, PIR-B, CD22, CD72, FcgRIIB, IκB, P100, CTLA4, PD-1, Cb1, KIR3DL1, KIR3DL2, KIR2DL and Csk. In these embodiments, the NF-κB inhibitor increases, stimulates or otherwise agonizes at least one function or activity of the member.

A plethora of NF-κB inhibitors is described in the literature and representative examples are listed in the following tables:

**TABLE 2 ANTI-OXIDANTS THAT INHIBIT ACTIVATION OF NF-κB**

| **Molecule** | **Reference** |
|---|---|
| alpha-lipoic acid | Sen et al., 1998 Jun 18; Biochem Biophys Res Commun; 247(2):223-8; |
| | Suzuki et al., 1992 Dec 30; Biochem Biophys Res Commun.; 189(3): 1709-15 |
| alpha-tocopherol | Islam et al., 1998 Nov 24; Circulation; 98(21):2255-61 |
| Aged garlic extract (allicin) | Ide & Lau, 2001 Mar; J Nutr; 131(3s):1020S-6S. Lang et al., 2004 Oct; Clin Nutr; 23(5): 1199-208. |
| 2-Amino-1-methyl-6-phenylimidazo[4,5-b]pyridine (PhIP) | Yun et al:, 2005 Jan 5; Toxicology; 217(1):31-8. Epub 2005 Sep 15. |
| N-acetyldopamine dimers (from P. cicadae) | Xu et al., 2006 Aug 16; Bioorg Med Chem. |
| Allopurinol | Gomez-Cabrera et al., 2006 Aug; Br J Nutr; 96 Suppl 1:S31-3 |
| Anetholdithiolthione | Sen et al., 1996 Jan 5; Biochem Biophys Res Commun; 218(1):148-53 |
| Apocynin | Barbieri et al., 2004 Jul 15; Free Radic Biol Med; 37(2):156-65. |
| Apple juice/extracts | Shi & Jiang, 2002; T Environ Pathol Toxicol Oncol; 21(3):233-42. |
| | Davis et al., 2006 May; Exp Biol Med (Maywood); 231(5):594-8 |
| Aretemsia p7F (5,6,3',5'-tetramethoxy 7,4'-hydroxyflavone) | Lee et al., 2004 Dec; Ann N Y Acad Sci; 1030:555-68 |
| Astaxanthin | Lee et al., 2003 Aug 31; Mol Cells; 16(1):97-105. |
| Benidipine | Matsubara & Hazegawa, Eur J Pharmacol. 2004 Sep 13;498(1-3):303-14 |
| bis-eugenol | Murakami et al., Biochem Pharmacol. 2003 Sep 15;66(6):1061-6 |
| Bruguiera gymnorrhiza compounds | Homhual et al., Planta Med. 2006 Feb;72(3):255-60. |
| Butylated hydroxyanisole (BHA) | Israël et al., J Immunol. 1992 Nov 15;149(10):3386-93. |
| | Schulze-Osthoff et al., EMBO J. 1993 Aug;12(8):3095-104. |
| Cepharanthine | Okamoto et al., J Biol Chem. 1994 Mar 18;269(11):8582-9. |
| Caffeic Acid Phenethyl Ester (3,4-dihydroxycinnamic acid, CAPE) | Natarajan et al., Proc Natl Acad Sci U S A. 1996 Aug 20;93(17):9090-5 |
| Carnosol | Lo et al., Carcinogenesis. 2002 Jun;23(6):983-91 |
| | Huang et al., Biochem Pharmacol. 2005 Jan 15;69(2):221-32. Epub 2004 Nov 23. |
| beta-Carotene | Bai et al., Exp Mol Med. 2005 Aug 31 ;37(4):323-34 |
| Carvedilol | Yang et al., Cardiovasc Res. 2003 Sep 1;59(3):776-87 |
| Catechol Derivatives | Suzuki & Packer, Biochem Mol Biol Int. 1994 Feb;32(2):299-305 |
| Chlorogenic acid | Feng et al., J Biol Chem. 2005 Jul 29;280(30):27888-95. Epub 2005 Jun 8. |
| Cocoa polyphenols | Lee et al., J Nutr. 2006 May; 136(5): 1150-5. |
| Curcumin (Diferulolylmethane) | Singh & Aggarwal, J Biol Chem. 1995 Oct 20;270(42):24995-5000 |
| Dehydroepiandrosterone (DHEA) and DHEA-sulfate (DHEAS) | Iwasaki et al., J Clin Endocrinol Metab. 2004 Jul;89(7):3449-54 |
| | Liu et al., Cancer Res. 2005 Mar 15;65(6):2269-76 |
| Dibenzylbutyrolactone lignans | Cho et al., Int Immunopharmacol. 2002 Jan;2(1):105-16 |
| Diethyldithiocarbamate (DDC) | Schreck et al., J Exp Med. 1992 May 1;175(5):1181-94. |
| Diferoxamine | Sappey et al., AIDS Res Hum Retroviruses. 1995 Sep; 11(9):1049-61; |
| | Schreck et al., Free Radic Res Commun. 1992;17(4):221-37 |
| Dihydroisoeugenol | Murakami et al., Arch Biochem Biophys. 2005 Feb 15;434(2):326-32 |
| Dihydrolipoic Acid | Suzuki et al., Biochem Biophys Res Commun. 1992 Dec 30;189(3):1709-15; |
| | Suzuki et al., Biochem Mol Biol Int. 1995 Jun;36(2):241-6 |
| Dilazep + fenofibric acid | Sonoki et al., Eur J Pharmacol. 2003 Aug 15;475(1-3):139-47; |
| | Yang et al., Naunyn Schmiedebergs Arch Pharmacol. 2005 May;371(5):401-7. Epub 2005 May 25 |
| Dimethyldithiocarbamates (DMDTC) | Pyatt et al., Toxicology. 1998 Jul 3;128(2):83-90. |
| Dimethylsulfoxide (DMSO) | Kelly et al., Infect Immun. 1994 Aug;62(8):3122-8. |
| Disulfiram | Schreck et al., Free Radic Res Commun. 1992; 17(4):221-37. |
| Ebselen | Schreck et al., Free Radic Res Commun. 1992; 17(4):221-37 |
| Edaravone | Kokura et al., Cancer Lett. 2005 Nov 18;229(2):223-33. Epub 2005 Aug 10 |
| EPC-K1 (phosphodiester compound of vitamin E and vitamin C) | Hirano et al., Immunopharmacology. 1998 Mar;39(1):31-8 |
| Epigallocatechin-3-gallate (EGCG; green tea polyphenols) | Lin & Lin, Mol Pharmacol. 1997 Sep;52(3):465-72; Yang et al., J Nutr. 1998 Dec;128(12):2334-40. |
| Ergothioneine | Rahman et al., Biochem Biophys Res Commun. 2003 Mar 21;302(4):860-4 |
| Ethyl Pyruvate (Glutathione depletion) | Song et al., J Pharmacol Exp Ther. 2004 Jan;308(1):307-16. Epub 2003 Oct 20.; |
| | Tsung et al., Transplantation. 2005 Jan 27;79(2): 196-204 |
| Ethylene Glycol Tetraacetic Acid (EGTA) | Janssen et al., Methods Enzymol. 1999;300:363-74 |
| Flavonoids (Crataegus; Boerhaavia diffusa root; xanthohumol) | Zhang et al., J Neurochem. 2004 Jul;90(1):211-9; |
| | Chen et al., Mol Pharmacol. 2004 Sep;66(3):683-93.; |
| | Pandey et al., Int Immunopharmacol. 2005 Mar;5(3):541-53; |
| | Albini et al., FASEB J. 2006 Mar;20(3):527-9. Epub 2005 Dec 30; |
| | Colgate et al., Cancer Lett. 2006 Mar 22; [Epub ahead of print] |
| Folic acid | Au-Yeung et al., Can J Physiol Pharmacol. 2006 Jan;84(1):141-7 |
| Gamma-glutamylcysteine synthetase (gamma-GCS) | Manna et al., Oncogene. 1999 Jul 29;18(30):4371-82 |
| Ganoderma lucidum polysaccharides | Zhang et al., Life Sci. 2003 Sep 19;73(18):2307-19. |
| Garcinol (from extract of Garcinia indica fruit rind) | Liao et al., Mol Carcinog. 2004 Nov;41(3):140-9 |
| Ginkgo biloba extract | Chen et al., Arterioscler Thromb Vasc Biol. 2003 Sep 1;23(9):1559-66. Epub 2003 Jul 31 |
| Glutathione | Cho et al., Biochem Biophys Res Commun. 1998 Dec 9;253(1):104-8; |
| | Schreck et al., Free Radic Res Commun. 1992;17(4):221-37 |
| Hematein | Choi et al., J Cardiovasc Pharmacol. 2003 Aug;42(2):287-95 |
| Hydroquinone | Pyatt et al., Toxicol Appl Pharmacol. 1998 Apr;149(2):178-84.; |
| | Yang et al., Zhongguo Shi Yan Xue Ye Xue Za Zhi. 2006 Aug;14(4):804-7 |
| 23-hydroxyursolic acid | Shin et al., Planta Med. 2004 Sep;70(9):803-7 |
| IRFI 042 (Vitamin E-like compound) | Altavilla et al., Free Radic Biol Med. 2001 May 15;30(10):1055-66 |
| Iron tetrakis | Kang et al., Toxicol Appl Pharmacol. 2003 Sep 1;191(2):147-55. |
| Isovitexin | Lin et al., Planta Med. 2005 Aug;71(8):748-53 |
| Kangen-karyu extract | Satoh et al., J Pharm Pharmacol. 2005 Oct;57(10):1335-43 |
| L-cysteine | Mihm et al., AIDS. 1991 May;5(5):497-503 |
| Lacidipine | Cominacini et al., J Hypertens. 1997 Dec;15(12 Pt 2):1633-40 |
| Lazaroids | Marubayashi et al., Transplant Proc. 2002 Nov;34(7):2662-3 |
| Ligonberries | Wang et al., J Agric Food Chem. 2005 Apr 20;53(8):3156-66 |
| Lupeol | Saleem et al., Oncogene. 2004 Jul 1;23(30):5203-14 |
| Magnolol | Chen et al., Br J Pharmacol. 2002 Jan;135(1):37-47 |
| Maltol | Yang et al., J Biochem Mol Biol. 2006 Mar 31;39(2):145-9 |
| Manganese superoxide dismutase (Mn-SOD) | Manna et al., J Biol Chem. 1998 May 22;273(21): 13245-54 |
| Extract of the stem bark of Mangifera indica L. | Leiro et al., Int Immunopharmacol. 2004 Aug;4(8):991-1003; |
| | Garrido et al., Phytother Res. 2005 Mar;19(3):211-5 |
| Melatonin | Gilad et al., FASEB J. 1998 Jun;12(9):685-93; |
| | Mohan et al., Biochem Mol Biol Int. 1995 Dec;37(6):1063-70; |
| | Li et al., Mediators Inflamm. 2005 Aug 31;2005(4):185-93 |
| Mulberry anthocyanins | Chen et al., Cancer Lett. 2006 Apr 28;235(2):248-59. Epub 2005 Jun 22 |
| N-acetyl-L-cysteine (NAC) | Schreck et al., EMBO J. 1991 Aug;10(8):2247-58 |
| Nacyselyn (NAL) | Antonicelli et al., Free Radic Biol Med. 2002 Mar 15;32(6):492-502 |
| Nordihydroguaiaritic acid (NDGA) | Brennan & O'Neill, Biochem Pharmacol. 1998 Apr 1;55(7):965-73; |
| | Israël et al., J Immunol. 1992 Nov 15;149(10):3386-93; Schulze-Osthoff et al., EMBO J. 1993 Aug;12(8):3095-104; |
| | Staal et al., AIDS Res Hum Retroviruses. 1993 Apr;9(4):299-306 |
| Ochnaflavone | Suh et al., Arch Biochem Biophys. 2006 Mar 15;447(2):136-46. Epub 2006 Feb 10 |
| Orthophenanthroline | Schreck et al., Free Radic Res Commun. 1992;17(4):221-37 |
| Phenolic antioxidants (Hydroquinone and tert-butyl hydroquinone) | Ma *et al.*, 2003 |
| alpha-phenyl-n-tert-butyl-nitrone (PBN) | Kotake et al., Biochim Biophys Acta. 1998 Nov 19;1448(1):77-84; |
| | Lin et al., Neurosci Lett. 2006 Sep 11;405(1-2):52-6. Epub 2006 Jul 28 |
| Phenylarsine oxide (PAO, tyrosine phosphatase inhibitor) | Arbault et al., Biomed Pharmacother. 1997;51(10):430-8 |
| Phyllanthus urinaria | Chularojmontri et al., Biol Pharm Bull. 2005 Jul;28(7):1165-71 |
| Pyrrolinedithiocarbamate (PDTC) | Schreck et al., J Exp Med. 1992 May 1;175(5):1181-94 |
| Quercetin (low concentrations) | Musonda & Chipman, Carcinogenesis. 1998 Sep;19(9):1583-9; |
| | Shih et al., Eur J Pharmacol. 2004 Aug 2;496(1-3):41-8 |
| Red Wine | Blanco-Colio et al., Circulation. 2000 Aug 29;102(9):1020-6; |
| | Cui & He, Zhonghua Yu Fang Yi Xue Za Zhi. 2004 Mar;38(2):103-6 |
| Ref-1 (redox factor 1) | Ozaki et al., FASEB J. 2002 Jun;16(8):889-90. Epub 2002 Apr 23 |
| Rg(3), a ginseng derivative | Keum et al., Mutat Res. 2003 Feb-Mar;523-524:75-85 |
| Rotenone | Schulze-Osthoff et al., EMBO J. 1993 Aug;12(8):3095-104 |
| Roxithromycin | Ueno et al., Clin Cancer Res. 2005 Aug 1;11(15):5645-50 |
| S-allyl-cysteine (SAC, garlic compound) | Geng et al., Free Radic Biol Med. 1997;23(2):345-50 |
| Sauchinone | Lee et al., Br J Pharmacol. 2003 May;139(1):11-20.; |
| | Hwang et al., Planta Med. 2003 Dec;69(12):1096-101 |
| Spironolactone | Han et al., J Am Soc Nephrol. 2006 May;17(5):1362-72. Epub 2006 Mar 29 |
| Strawberry extracts | Wang et al., J Agric Food Chem. 2005 May 18;53(10):4187-93 |
| Taxifolin | Wang et al., J Biomed Sci. 2006 Jan;13(1):127-41. Epub 2005 Nov 9 |
| Tempol | Cuzzocrea et al., Free Radic Res. 2004 Aug;38(8):813-9 |
| Tepoxaline (5-(4-chlorophenyl)-N-hydroxy-(4-methoxyphenyl)-N-methyl-1H-pyrazole-3-propanamide) | Kazmi et al., J Cell Biochem. 1995 Feb;57(2):299-310.; |
| | Ritchie et al., Int J Immunopharmacol. 1995 Oct; 17(10):805-12 |
| Vitamin C | Staal et al., AIDS Res Hum Retroviruses. 1993 Apr;9(4):299-306; |
| | Son et al., Arch Pharm Res. 2004 Oct;27(10):1073-9 |
| Vitamin B6 | Yanaka et al., Int J Mol Med. 2005 Dec;16(6): 1071-5 |
| Vitamin E derivatives | Suzuki & Packer, Biochem Biophys Res Commun. 1993 May 28;193(1):277-83 |
| a-torphryl succinate | Staal et al., AIDS Res Hum Retroviruses. 1993 Apr;9(4):299-306; |
| | Suzuki & Packer, Biochem Mol Biol Int. 1993 Nov;31(4):693-700 |
| a-torphryl acetate | Suzuki & Packer, Biochem Biophys Res Commun. 1993 May 28;193(1):277-83 |
| PMC (2,2,5,7,8-pentamethyl-6-hydroxychromane) | Suzuki & Packer, Biochem Biophys Res Commun. 1993 May 28;193(1):277-83 |
| Yakuchinone A and B | Chun et al., J Environ Pathol Toxicol Oncol. 2002;21(2):131-9 |

**TABLE 3A PROTEASOME AND PROTEASES INHIBITORS OF REL/NF-KB**

| **Molecule** | **References** |
|---|---|
| **Proteasome inhibitors** | |
| Peptide Aldehydes: | Palombella et al., Cell. 1994 Sep 9;78(5):773-85; |
| | Grisham et al., Methods Enzymol. 1999;300:345-63; |
| | Jobin et al., Gut. 1998 Jun;42(6):779-87 |
| ALLnL (N-acetyl-leucinyl-leucynil-norleucynal, MG101) | |
| LLM (N-acetyl-leucinyl-leucynil-methional) | |
| Z-LLnV (carbobenzoxyl-leucinyl-leucynil-norvalinal,MG115) | |
| Z-LLL (carbobenzoxyl-leucinyl-leucynil-leucynal, MG132) | |
| Lactacystine, b-lactone | Fenteany & Schreiber, J Biol Chem. 1998 Apr 10;273(15):8545-8; |
| | Grisham et al., Methods Enzymol. 1999;300:345-63 |
| Boronic Acid Peptide | Grisham et al., Methods Enzymol. 1999;300:345-63; |
| | Iqbal et al., J Med Chem. 1995 Jun 23;38(13):2276-7 |
| Ubiquitin Ligase Inhibitors | Yaron et al., EMBO J. 1997 Nov 3;16(21):6486-94 |
| PS-341 (Bortezomib) | Adams, Cancer Cell. 2004 May;5(5):417-21 |
| Salinosporamide A (1, NPI-0052) | Macherla et al., J Med Chem. 2005 Jun 2;48(11):3684-7 |
| Cyclosporin A | Frantz et al., EMBO J. 1994 Feb 15;13(4):861-70; |
| | Kunz et al., Biochem Biophys Res Commun. 1995 Nov 13;216(2):438-46; |
| | Marienfeld et al., Eur J Immunol. 1997 Jul;27(7):1601-9; |
| | McCaffrey et al., Nucleic Acids Res. 1994 Jun 11;22(11):2134-42; |
| | Meyer et al., FEBS Lett. 1997 Aug 18;413(2):354-8; |
| | Wechsler et al., J Immunol. 1994 Sep 15;153(6):2515-23 |
| FK506 (Tacrolimus) | Okamoto et al., J Biol Chem. 1994 Mar 18;269(11):8582-9; |
| | Venkataraman et al., J Exp Med. 1995 Mar 1;181(3):1091-9 |
| Deoxyspergualin | Tepper et al., J Immunol. 1995 Sep 1;155(5):2427-36 |
| Disulfiram | Lovborg et al., Int J Cancer. 2006 Mar 15;118(6):1577-80 |

| **Protease inhibitors** | |
|---|---|
| APNE (N-acetyl-DL-phenylalanine-b-naphthylester) | Higuchi et al., Blood. 1995 Sep 15;86(6):2248-56 |
| BTEE (N-benzoyl L-tyrosine-ethylester) | Rossi et al., J Biol Chem. 1998 Jun 26;273(26):16446-52 |
| DCIC (3,4-dichloroisocoumarin) | D'Acquisto et al., FEBS Lett. 1998 Nov 27;440(1-2):76-80 |
| DFP (diisopropyl fluorophosphate) | |
| TPCK (N-a-tosyl-L-phenylalanine chloromethyl ketone) | |
| TLCK (N-a-tosyl-L-lysine chloromethyl ketone) | |

**TABLE 3B IκBα PHOSPHORYLATION AND/OR DEGRADATION INHIBITORS**

| **Molecule** | **Point of Inhibition** | **References** |
|---|---|---|
| BAY 11-7082 | IκBα phosphorylation | Pierce et al. J. Biol Chem 1997; 272, 21096-21103 |
| | | BioMol, Plymouth Meeting, PA |
| BAY 11-7085 | IκBα phosphorylation | Pierce et al. J. Biol Chem 1997; 272, 21096-21103 |
| | | BioMol, Plymouth Meeting, PA |
| Desloratadine | Histamine H1 receptor | Wu et al., Int Arch Allergy Immunol. 2004 Dec;135(4):313-8. Epub 2004 Nov 24 |
| Salmeterol, fluticasone propionate | beta2 agonists | Baouz et al., Int Immunol. 2005 Nov;17(11):1473-81. Epub 2005 Oct 6 |
| CPU0213 | Endothelin receptor antagonist | He et al., Acta Pharmacol Sin. 2006 Sep;27(9):1213-21 |
| Erbin overexpression | NOD2 inhibitor | McDonald et al., J Biol Chem. 2005 Dec 2;280(48):40301-9. Epub 2005 Oct 3 |
| Protein-bound polysaccharide from basidiomycetes | LPS-CD 14 interaction | Asai et al., FEMS Immunol Med Microbiol. 2005 Jan 1;43(1):91-8. |
| Calagualine (fern derivative) | upstream of IKK (TRAF2-NIK) | Manna et al., Cancer Lett. 2003 Feb 20;190(2):171-82 |
| NS3/4A (HCV protease) | upstream of IKK | Karayiannis, J Hepatol. 2005 Oct;43(4):743-5 |
| golli BG21 (product of myelin protein) | upstream of IKK (PKC) | Feng et al., J Neuroimmunol. 2004 Jul;152(1-2):57-66 |
| NPM-ALK oncoprotein | Traf2 inhibition | Horie et al., Cancer Cell. 2004 Apr;5(4):353-64 |
| NS5A (Hepatitis C virus) | Traf2 inhibition | Park et al., J Biol Chem. 2002 Apr 12;277(15):13122-8. Epub 2002 Jan 30 |
| LY29 and LY30 | PI3 Kinase inhibitors | Choi et al., FEBS Lett. 2004 Feb 13;559(1-3):141-4 |
| Evodiamine (Evodiae Fructus component) | AKT-IKK interaction | Takada et al., J Biol Chem. 2005 Apr 29;280(17):17203-12. Epub 2005 Feb 14 |
| Rituximab (anti-CD20 antibody) | up-regulates Raf-1 kinase inhibitor | Jazirehi et al., Cancer Res. 2005 Jan 1;65(1):264-76 |
| Kinase suppressor of ras (KSR2) | MEKK3 inhibitor | Channavajhala et al., Biochem Biophys Res Commun. 2005 Sep 9;334(4):1214-8 |
| M2L (Vaccinia virus) | ERK2 inhibitor | Gedey et al., J Virol. 2006 Sep;80(17):8676-85 |
| Pefabloc (serine protease inhibitor) | upstream of IKK | Tando et al., Digestion. 2002;66(4):237-45 |
| Rocaglamides (Aglaia derivatives) | upstream of IKK | Baumann et al., J Biol Chem. 2002 Nov 22;277(47):44791-800. Epub 2002 Sep 16 |
| Betaine | NIK/IKK | Hu et al., J Biol Chem. 2004 Aug 20;279(34):35975-83. Epub 2004 Jun 18 |
| TNAP | NIK | Go et al., J Gerontol A Biol Sci. Med Sci. 2005 Oct;60(10):1252-64 |
| Geldanamycin | IKK complex formation | Chen et al., Mol Cell. 2002 Feb;9(2):401-10 |
| Grape seed proanthocyanidins | IKKa activity | Mantena & Katiyar, Free Radic Biol Med. 2006 May 1;40(9):1603-14. Epub 2006 Jan 26 |
| MC 160 (Molluscum contagiosum virus) | IKKa activity | Nichols & Shisler, J Virol. 2006 Jan;80(2):578-86 |
| NS5B (Hepatitis C protein) | IKKa activity | Choi et al., Mol Cell Biol. 2006 Apr;26(8):3048-59 |
| Pomegranate fruit extract | IKKa activity | Afaq et al., Photochem Photobiol. 2005 Jan-Feb;81(1):38-45; |
| | | Khan et al., Carcinogenesis. 2006 Aug 18; [Epub ahead of print] |
| Tetrandine (plant alkaloid) | IKKa activity | Ho et al., Br J Pharmacol. 2004 Dec;143(7):919-27. Epub 2004 Oct 25 |
| BMS-345541 (4(2'-Aminoethyl)amino-1,8-dimethylimidazo(1,2-a) quinoxaline) | IKKa and IKKb kinase activity | Burke et al., J Biol Chem. 2003 Jan 17;278(3):1450-6. Epub 2002 Oct 25; |
| | | Yang *et al.*, 2006 |
| 2-amino-3-cyano-4-aryl-6-(2-hydroxy-phenyl)pyridine derivatives | IKKb activity | Murata et al., Bioorg Med Chem Lett. 2003 Mar 10;13(5):913-8, |
| | | Murata et al., Bioorg Med Chem Lett. 2004 Aug 2;14(15):4013-7, |
| | | Murata et al., Bioorg Med Chem Lett. 2004 Aug 2;14(15):4019-22 |
| Acrolein | IKKb activity | Vallacchi et al., Antioxid Redox Signal. 2005 Jan-Feb;7(1-2):25-31 |
| Anandamide | IKKb activity | Sancho et al., Mol Pharmacol. 2003 Feb;63(2):429-38 |
| AS602868 | IKKb activity | Frelin et al., Oncogene. 2003 Nov 6;22(50):8187-94 |
| Cobrotoxin | IKKb activity and p50 DNA binding | Park et al., Biochemistry 2005 Jun 14;44(23):8326-36 |
| Core protein (Hepatitis C) | IKKb activity | Joo et al., J Virol. 2005 Jun;79(12):7648-57; |
| | | Shrivastava et al., J Virol. 1998 Dec;72(12):9722-8 |
| Dihydroxyphenylethanol | IKKb activity | Guichard et al., Carcinogenesis. 2006 Sep;27(9):1812-27. Epub 2006 Mar 7 |
| Herbimycin A | IKKb activity | Iwasaki et al., FEBS Lett. 1992 Feb 24;298(2-3):240-4; |
| | | Mahon & O'Neill, Biochem Soc Trans. 1995 Feb;23(1):111S; |
| | | Ogino et al., Mol Pharmacol. 2004 Jun;65(6):1344-51 |
| Inhibitor 22 | IKKb activity | Baxter et al., Bioorg Med Chem Lett. 2004 Jun 7;14(11):2817-22 |
| Isorhapontigenin | IKKb activity | Li et al., Free Radic Biol Med. 2005 Jan 15;38(2):243-57 |
| Manumycin A | IKKb activity | Bernier et al., J Biol Chem. 2006 Feb 3;281(5):2551-61. Epub 2005 Nov 30; Frassanito et al., Clin Exp Med. 2005 Mar;4(4):174-82 |
| MLB120 (small molecule) | IKKb activity | Nagashima et al., Blood. 2006 Jun 1;107(11):4266-73. Epub 2006 Jan 26 |
| Novel Inhibitor | IKKb activity | Kamon et al., Biochem Biophys Res Commun. 2004 Oct 8;323(1):242-8 |
| vIRF3 (KSHV) | IKKb activity | Seo et al., Oncogene. 2004 Aug 12;23(36):6146-55 |
| Nitric oxide | IKKb activity/IkB phosphorylation | Katsuyama et al., Arterioscler Thromb Vasc Biol. 1998 Nov; 18(11:1796-802; |
| | | Matthews et al., Nucleic Acids Res 1996 Jun 15;24(12):2236-42; |
| | | Spieker & Liao, Methods Enzymol. 1999;300:374-88; |
| | | Reynaert et al., Proc Natl Acad Sci USA. 2004 Jun 15;101(24):8945-50. Epub 2004 Jun 7 |
| SC-514 (small molecule) | IKKb activity | Kishore et al., J Biol Chem. 2003 Aug 29;278(35):32861-71. Epub 2003 Jun 17 |
| Thienopyridine | IKKb activity | Morwick et al., J Med Chem. 2006 May 18;49(10):2898-908 |
| Acetyl-boswellic acids | IKK activity | Syrovets et al., J Biol Chem. 2005 Feb 18;280(7):6170-80. Epub 2004 Dec 2; |
| | | Syrovets et al., J Immunol. 2005 Jan 1;174(1):498-506 |
| Amino-pyrimidine derivative | IKK activity | Karin et al., Nat Rev Drug Discov. 2004 Jan;3(1):17-26 |
| Benzoimidazole derivative | IKK activity | Karin et al., Nat Rev Drug Discov. 2004 Jan;3(1):17-26 |
| BMS-345541 | IKK activity | Burke et al., J Biol Chem. 2003 Jan 17;278(3):1450-6. Epub 2002 Oct 25. |
| Beta-carboline | IKK activity | Yoon et al., J Toxicol Environ Health A. 2005 Dec 10;68(23-24):2005-17 |
| CYL-19s and CYL-26z, two synthetic alpha-methylene-gamma-butyrolactone derivatives | IKK activity | Huang et al., Carcinogenesis. 2004 Oct;25(10):1925-34. Epub 2004 Jun 24 |
| ACHP (2-amino-6-[2-(cyclopropylmethoxy)-6-hydroxyphenyl]-4-piperidin-4-yl nicotinonitrile | IKKb activity (ATP analog) | Sanda et al., Leukemia. 2006 Apr;20(4):590-8 |
| Compound A | IKKb activity (ATP analog) | Ziegetbauer et al., Br J Pharmacol, 2005 May;20(4):590-8 |
| Flavopiridol | IKK activity and RelA phosphor. | Takada & Aggarwal, J Biol Chem. 2004 Feb 6;279(6):4750-9. Epub 2003 Nov 20 |
| Cyclopentones | IKKb activity | Bickley et al., Bioorg Med Chem. 2004 Jun 15;12(12):3221-7 |
| Dehydroascorbic acid (Vitamin C) | IKKb activity | Carcamo et al., Mol Cell Biol. 2004 Aug;24(15):6645-52 |
| IMD-0354 | IKKb activity | Tanaka et al., Blood. 2005 Mar 15;105(6):2324-31. Epub 2004 Nov 23, |
| | | Tanaka et al., Cancer Res. 2006 Jan 1;66(1):419-26; |
| | | Inayama et al., Am J Respir Crit Care Med. 2006 May 1;173(9): 1016-22. Epub 2006 Feb 2 |
| Jesterone dimer | IKKb activity; DNA binding | Liang et al., Mol Pharmacol. 2003 Jul;64(1):123-31; |
| | | Liang *et al.,* 2006 |
| PS-1145 (MLN1145) | IKKb activity | Hideshima et al., J Biol Chem. 2002 May 10;277(19):16639-47. Epub 2002 Feb 28 |
| 2-[(aminocarbonyl)amino]-5-acetylenyl-3-thiophenecarboxamides (TPCA-1) | IKKb activity | Bonafoux et al., Bioorg Med Chem Lett. 2005 Jun 2;15(11):2870-5; |
| | | Podolin *et al.,* 2005 |
| 1'-Acetoxychavicol acetate (Languas galanga) | IKK activity | Ichikawa et al., J Immunol. 2005 Jun 1;174(11):7383-92; |
| | | Ito et al., Cancer Res. 2005 May 15;65(10):4417-24 |
| Apigenin (plant flavinoid) | IKK activity | Shukla & Gupta, Clin Cancer Res. 2004 May 1;10(9):3169-78; |
| | | Yoon et al., Mol Pharmacol. 2006 Sep;70(3):1033-44. Epub 2006 Jun 16 |
| Cardamomin | IKK activity | Lee et al., J Pharmacol Exp Ther. 2006 Jan;316(1):271-8. Epub 2005 Sep 23 |
| CDDO-Me (synthetic triterpenoid) | IKK activity | Shishodia et al., Clin Cancer Res. 2006 Mar 15;12(6):1828-38 |
| CHS 828 (anticancer drug) | IKK activity | Olsen et al., Int J Cancer. 2004 Aug 20;111(2):198-205 |
| CML-1 | IKK activity | Mo et al., J Ethnopharmacol. 2006 Jul 11; [Epub ahead of print] |
| Compound 5 (Uredio-thiophenecarboxamide derivative) | IKK activity | Roshak et al., Curr Opin Pharmacol. 2002 Jun;2(3):316-21 |
| Diaylpyridine derivative | IKK activity | Murata et al., Bioorg Med Chem Lett. 2003 Mar 10;13(5):913-8 |
| Diosgenin | IKK activity | Shishodia & Aggarwal, Oncogene. 2006 Mar 9;25(10): 1463-73; Liagre et al., Int J Mol Med. 2005 Dec; 16(6):1095-101 |
| E3-14.7K (Adenovirus) | IKK activity | Li et al., Proc Natl Acad Sci U S A. 1999 Feb 2;96(3):1042-7 |
| E3-10.4K/14.5K (Adenovirus) | IKK activity | Friedman & Horwitz, J Virol. 2002 Jun;76(11):5515-21 |
| E7 (human papillomavirus) . | IKK activity | Spitkovsky et al., J Biol Chem. 2002 Jul 12;277(28):25576-82. Epub 2002 May 1 |
| Furonaphthoquinone | IKK activity | Shin et al., Int Immunopharmacol. 2006 Jun;6(6):916-23. Epub 2006 Feb 3 |
| Guggulsterone | IKK activity | Ichikawa & Aggarwal, Clin Cancer Res. 2006 Jan 15;12(2):662-8 |
| HB-EGF (Heparin-binding epidermal growth factor-like growth factor) | IKK activity | Mehta & Besner, J Immunol. 2003 Dec 1;171(11):6014-22 |
| Falcarindol | IKK activity | Shiao et al., Br J Pharmacol. 2005 Jan;144(1):42-51 |
| Hepatocyte growth factor | IKK activity | Min et al., Circ Res. 2005 Feb 18;96(3):300-7. Epub 2005 Jan 6; |
| | | Gong et al., J Am Soc Nephrol. 2006 Sep; 17(9):2464-73. Epub 2006 Aug 2 |
| Honokiol | IKK activity | Tse et al., Biochem Pharmacol. 2005 Nov 15;70(10):1443-57. Epub 2005 Sep 21 |
| Hypoestoxide | IKK activity | Ojo-Amaize et al., Cell Immunol. 2001 May 1;209(2):149-57 |
| Indolecarboxamide derivative | IKK activity | Karin et al., Nat Rev Drug Discov. 2004 Jan;3(1):17-26 |
| LF15-0195 (analog of 15-deoxyspergualine) | IKK activity | Yang et al., J Leukoc Biol. 2003 Sep;74(3):438-47 |
| gamma-mangostin (from Garcinia mangostana) | IKK activity | Nakatani et al., Mol Pharmacol. 2004 Sep;66(3):667-74 |
| Garcinone B | IKK activity | Yamakuni et al., Neurosci Lett. 2006 Feb 20;394(3):206-10. Epub 2005 Nov 2 |
| (Amino)imidazolylcarboxald ehyde derivative | IKK activity | Karin et al., Nat Rev Drug Discov. 2004 Jan;3(1):17-26. |
| Imidazolylquinoline-carboxaldehyde derivative | IKK activity | Karin et al., Nat Rev Drug Discov. 2004 Jan;3(1):17-26 |
| Kahweol | IKK activity | Kim et al., Cancer Lett. 2004 Sep 30;213(2):147-54 |
| Kava (Piper methysticum) derivatives | IKK activity | Folmer et al., Biochem Pharmacol. 2006 Apr 14;71(8):1206-18. Epub 2006 Feb 7 |
| Lead | IKK activity | Xu et al., Cell Biol Toxicol. 2006 May;22(3):189-98 |
| Mild hypothermia | IKK activity | Han et al., J Cereb Blood Flow Metab. 2003 May;23(5):589-98 |
| ML120B | IKK activity | Catley et al., Mol Pharmacol. 2006 Aug;70(2):697-705. Epub 2006 May 10 |
| MX781 (retinoid antagonist) | IKK activity | Bayon et al., Mol Cell Biol. 2003 Feb;23(3):1061-74 |
| N-acetylcysteine | IKK activity | Oka et al., FEBS Lett. 2000 Apr 28;472(2-3): 196-202 |
| Nitrosylcobalamin (vitamin B12 analog) | IKK activity | Chawla-Sarkar et al., J Biol Chem. 2003 Oct 10;278(41):39461-9. Epub 2003 Jul 24 |
| NSAIDs | IKK activity | Takada et al., Oncogene. 2004 Dec 9;23(57):9247-58 |
| Hepatits C virus NS5B | IKK activity | Choi et al., Mol Cell Biol. 2006 Apr;26(8):3048-59 |
| PAN1 (aka NALP2 or PYPAF2) | IKK activity | Bruey et al., J BiolChem. 2004 Dec 10;279(50):51897-907. Epub 2004 Sep 28 |
| Pectin (citrus) | IKK activity | Chen et al., Biochem Pharmacol. 2006 Oct 16;72(8):1001-9. Epub 2006 Aug 22 |
| Pyrazolo[4,3-c]quinoline derivative | IKK activity | Karin et al., Nat Rev Drug Discov. 2004 Jan;3(1):17-26 |
| Pyridooxazinone derivative | IKK activity | Karin et al., Nat Rev Drug Discov. 2004 Jan;3(1):17-26 |
| N-(4-hydroxyphenyl) retinamide | IKK activity | Shishodia et al., Cancer Res. 2005 Oct 15;65(20):9555-65 |
| Scytonemin | IKK activity | Stevenson et al., Inflamm Res. 2002 Feb;51(2):112-4 |
| Semecarpus anacardiu extract | IKK activity | Singh et al., J Ethnopharmacol. 2006 Jun 2; [Epub ahead of print] |
| SPC-839 | IKK activity | Palanki et al., Bioorg Med Chem Lett. 2002 Sep 16;12(18):2573-7 |
| Sulforaphane and phenylisothiocyanate | IKK activity | Xu et al., Oncogene. 2005 Jun 30;24(28):4486-95 |
| Survanta (Surfactant product) | IKK activity | Raychaudhuri et al., Am J Respir Cell Mol Biol. 2004 Feb;30(2):228-32. Epub 2003 Aug 14 |
| Piceatannol | IKK activity | Islam et al., Microbiol Immunol. 2004;48(10):729-36 |
| Plumbagin (5-hydroxy-2-methyl-1,4-naphthoquinone) | IKK activity | Sandur et al., J Biol Chem. 2006 Jun 23;281(25):17023-33. Epub 2006 Apr 19 |
| IKKb peptide to NEMO binding domain | IKK-NEMO interaction | May et al., Science. 2000 Sep 1;289(5484):1550-4 |
| NEMO CC2-LZ peptide | NEMO oligomerization | Agou *et al*., 2004 |
| AGRO100 (G-quadraplex oligodeoxynucleotide) | NEMO binding | Girvan et al., Mol Cancer Ther. 2006 Jul;5(7):1790-9 |
| PTEN (tumor suppressor) | Activation of IKK | Gustin et al., J Biol Chem. 2001 Jul 20;276(29):27740-4. Epub 2001 May 16 |
| Theaflavin (black tea component) | Activation of IKK | Aneja et al., Crit Care Med. 2004 Oct;32(10):2097-103; |
| | | Ukil et al., Br J Pharmacol. 2006 Sep;149(1):121-31. Epub 2006 Jul 31 |
| Tilianin | Activation of IKK | Nam et al., Atherosclerosis. 2005 May;180(1):27-35. Epub 2005 Jan 19 |
| Withanolides | Activation of IKK | Ichikawa et al., MolCancer Ther. 2006 Jun;5(6):1434-45 |
| Zerumbone | Activation of IKK | Takada et al., Oncogene. 2005 Oct 20;24(46):6957-69 |
| Silibinin | IKKα activity; nuclear translocation | Dhanalakshmi et al., Oncogene. 2002 Mar 7;21(11):1759-67; |
| | | Singh et al., Oncogene. 2005 Feb 10;24(7):1188-202 |
| Sulfasalazine | IKKa and IKKb kinase activity | Wahl et al., J Clin Invest. 1998 Mar 1;101(5):1163-74 |
| | | Weber et al., Gastroenterology. 2000 Nov;119(5):1209-18 |
| Sulfasalazine analogs | IKK kinase activity | Habens et al., Apoptosis. 2005 May;10(3):481-91 |
| Quercetin | IKK activity | Peet & Li, J Biol Chem. 1999 Nov 12;274(46):32655-61 |
| Rosmarinic acid | IKK activity | Lee et al., Br J Pharmacol. 2006 Jun; 148(3):366-75 |
| Staurosporine | IKK activity | Peet & Li, J Biol Chem. 1999 Nov 12;274(46):32655-61 |
| gamma-Tocotrienol | IKK activity | Shah & Sylvester, Exp Biol Med (Maywood) 2005 Apr;230(4):235-41 |
| Wedelolactone | IKK activity | Kobori et al., Cell Death Differ. 2004 Jan;II(1):123-30 |
| Betulinic acid | IKKa activity and p65 phosphorylation | Takada & Aggarwal, J Immunol. 2003 Sep 15;171 (6):3278-86 |
| Ursolic acid | IKKa activity and p65 phosphorylation | Shishodia et al., Cancer Res. 2003 Aug 1;63(15):4375-83 |
| Thalidomide (and thalidomide analogs) | IKK activity | Keifer et al., J Biol Chem. 2001 Jun 22;276(25):22382-7. Epub 2001 Apr 10; |
| | | Ge et al., Blood.u 2006 Aug 29; [Epub ahead of print] |
| Interleukin-10 | Reduced IKKa and IKKb expression | Tabary et al., Am J Pathol. 2003 Jan;162(1):293-302 |
| MC160 (molluscum contagiosum virus) | Reduced IKKa expression | Nichols & Shisler, J Virol. 2006 Jan;80(2):578-86 |
| Monochloramine and , glycine chloramine (NH2Cl) | Oxidizes IkB | Kim et al., Biochim Biophys Acta. 2005 Dec 15;1746(2):13542. Epub 2005 Oct 28; |
| | | Midwinter et al., Biochem J. 2006 May 15;396(1):71-8 |
| Anethole | Phosphorylation | Chainy et al., Oncogene. 2000 Jun 8;19(25):2943-50 |
| Anti-thrombin III | Phosphorylation | Oelschlager et al., Blood. 2002 Jun 1;99(11):4015-20 |
| Artemisia vestita | Phosphorylation | Sun et al., Int J Mol Med. 2006 May;17(5):957-62 |
| Aspirin, sodium salicylate | Phosphorylation, IKKbeta | Frantz & O'Neill, Science. 1995 Dec 22;270(5244):2017-9; |
| | | Kopp & Ghosh, Science. 1994 Aug 12;265(5174):956-9; |
| | | Yin et al., Nature. 1998 Nov 5;396(6706):77-80 |
| Azidothymidine (AZT) | Phosphorylation | Ghosh et al., Blood. 2003 Mar 15;101(6):2321-7. Epub 2002 Oct 24.; |
| | | Kurokawa et al., Blood. 2005 Jul 1;106(1):235-40. Epub 2005 Mar 24 |
| Baoganning | Phosphorylation | Tan et al., Zhongguo Zhong Xi Yi Jie He Za Zhi 2005 Sep;25(9):804-7 |
| BAY-11-7082 (E3((4-methylphenyl)-sulfonyl)-2-propenenitrile) | Phosphorylation | Pierce et al., J Biol Chem. 1997 Aug 22;272(34):21096-103. |
| BAY-117083 (E3((4-t-butylphenyl)-sulfonyl)-2-propenenitrile) | Phosphorylation | Pierce et al., J Biol Chem. 1997 Aug 22;272(34):21096-103 |
| Benzyl isothiocyanate | Phosphorylation | Srivastava & Singh, Carcinogenesis. 2004 Sep;25(9):1701-9. Epub 2004 Apr 29 |
| Black raspberry extracts (cyanidin 3-O-glucoside, cyanidin 3-O-(2(G)-xylosylrutinoside), cyanidin 3-O-rutinoside) | Phosphorylation | Huang et al., Cancer Res. 2002 Dec 1;62(23):6857-63.; |
| | | Hecht et al., Carcinogenesis. 2006 Aug;27(8):1617-26. Epub 2006 Mar 7 |
| Buddlejasaponin IV | Phosphorylation | Won et al., Br J Pharmacol. 2006 May;148(2):216-25 |
| Cacospongionolide B | Phosphorylation | Posadas et al., Br J Pharmacol. 2003 Apr;138(8):1571-9 |
| Calagualine | Phosphorylation | Manna et al., Cancer Lett. 2003 Feb 20;190(2):171-82 |
| Carbon monoxide | Phosphorylation | Sarady et al., Am J Respir Cell Mol Biol. 2002 Dec;27(6):739-45 |
| Carboplatin | Phosphorylation | Singh & Bhat, Biochem Biophys Res Commun. 2004 May 28;318(2):346-53 |
| Cardamonin | Phosphorylation | Israf et al., Mol Immunol. 2007 Feb;44(5):673-9. Epub 2006 Jun 13 |
| Chorionic gonadotropin | Phosphorylation | Manna et al., J Biol Chem. 2000 May 5;275(18):13307-14 |
| Cordycepin | Phosphorylation | Kim et al., Eur J Pharmacol. 2006 Sep 18;545(2-3): 192-9. Epub 2006 Jun 28 |
| Cycloepoxydon; 1-hydroxy-2-hydroxymethyl-3-pent-1-enylbenzene | Phosphorylation | Gehrt et al., J Antibiot (Tokyo), 1998 May;51(5):455-63 |
| Cytomegalovirus | Phosphorylation | Jarvis *et al*., 2006 |
| Decursin | Phosphorylation | Kim et al., Mol Pharmacol. 2006 Jun;69(6):1783-90. Epub 2006 Mar 1 |
| Dexanabinol | Phosphorylation | Juttler et al., Neuropharmacology, 2004 Sep;47(4):580-92 |
| Digitoxin | Phosphorylation | Srivastava et al., Proc Natl Acad Sci U S A. 2004 May 18;101(20):7693-8. Epub 2004 May 10 |
| Diterpenes (synthetic) | Phosphorylation | Chao et al., Chembiochem. 2005 Jan;6(1):133-44 |
| Docosahexaenoic acid | Phosphorylation | Chen et al., Invest Ophthalmol Vis Sci. 2005 Nov;46(11):4342-7 |
| Entamoeba histolytica | Phosphorylation | Kammanadiminti & Chadee, J Biol Chem. 2006 Sep 8;281(36):26112-20. Epub 2006 Jul 13 |
| Extensively oxidized low density lipoprotein (ox-LDL), 4-Hydroxynonenal (HNE) | Phosphorylation | Brand et al., Arterioscler Thromb Vasc Biol. 1997 Oct; 17(10): 1901-9; |
| | | Page et al., J Biol Chem. 1999 Apr 23;274(17):11611-8 |
| FHIT (Fragile histidine triad protein) | Phosphorylation | Nakagawa & Akao, Exp Cell Res. 2006 Aug 1;312(13):2433-42. Epub 2006 Apr 25 |
| Gabexate mesilate | Phosphorylation | Uchiba et al., Crit Care Med. 2003 Apr;31(4): 1147-53 |
| [6]-gingerol; casparol | Phosphorylation | Kim et al., Oncogene. 2005 Apr 7;24(15):2558-67.; |
| | | Aktan et al., Planta Med. 2006 Jun;72(8):727-34. Epub 2006 May 29 |
| Gleevec (Imatanib) | Phosphory lation | Wolf et al., Proc Natl Acad Sci U S A. 2005 Sep 20;102(38):13622-7. Epub 2005 Sep 8 |
| Glossogyne tenuifolia | Phosphorylation | Wu et al., J Biomed Sci. 2004 Mar-Apr;11(2):186-99; |
| | | Ha et al., J Ethnopharmacol. 2006 Aug 11;107(1):116-25. Epub 2006 Apr 3 |
| Guggulsterone | Phosphorylation | Shishodia & Aggarwal, J Biol Chem. 2004 Nov 5;279(45):47148-58. Epub 2004 Aug 17 |
| Hydroquinone | Phosphorylation | Kerzic et al., Toxicology. 2003 May 3;187(2-3):127-37 |
| Ibuprofen | Phosphorylation | Palayoor et al., Oncogene. 1999 Dec 2;18(51):7389-94 |
| Indirubin-3'-oxime | Phosphorylation | Mak et al., Biochem Pharmacol. 2004 Jan 1;67(1):167-74 |
| Interferon-alpha | Phosphorylation | Manna et al., J Immunol. 2000 Nov 1; 165(9):4927-34 |
| Inhaled isobutyl nitrite | Phosphorylation | Ponnappan et al., Int Immunopharmacol. 2004 Aug;4(8):1075-82 |
| Licorce extracts | Phosphorylation | Kim et al., Biochem Biophys Res Commun. 2006 Jul 7;345(3):1215-23. Epub 2006 May 15 |
| Melatonin | Phosphorylation | Alonso et al., J Pineal Res. 2006 Aug;41(1):8-14 |
| Methotrexate | Phosphorylation | Majumdar & Aggarwal, J Immunol. 2001 Sep 1;167(5):2911-20; |
| | | Yozai et al., J Am Soc Nephrol. 2005 Nov;16(11):3326-38. Epub 2005 Sep 21 |
| Monochloramine | Phosphorylation | Omori et al., Free Radic Res-2002 Aug;36(8):845-52 |
| Nafamostat mesilate | Phosphorylation | Noguchi et al., Int Immunopharmacol. 2003 Sep;3(9): 1335-44 |
| Oleandrin | Phosphorylation | Manna et al., Cancer Res. 2000 Jul 15;60(14):3838-47; |
| | | Sreeivasan et al., Biochem Pharmacol. 2003 Dec 1;66(11):2223-39 |
| Omega 3 fatty acids | Phosphorylation | Novak et al., Am J Physiol Lung Cell Mol Physiol. 2003 Jan;284(1):L84-9. Epub 2002 Aug 30 |
| Panduratin A (from Kaempferia pandurata, Zingiberaceae) | Phosphorylation | Yun et al., Planta Med. 2003 Dec;69(12):1102-8 |
| Petrosaspongiolide M | Phosphorylation | Posadas et al., Biochem Pharmacol. 2003 Mar 1;65(5):887-95 |
| Pinosylvin | Phosphorylation | Lee et al., Planta Med. 2006 Jul;72(9):801-6. Epub 2006 Jun 19 |
| Plagius flosculosus extract polyacetylene spiroketal | Phosphorylation | Calzado et al., Biochim Biophys Acta. 2005 Jun 30; 1729(2):88-93 |
| Phytic acid (inositol hexakisphosphate) | Phosphorylation | Ferry et al., Carcinogenesis. 2002 Dec;23(12):2031-41 |
| Pomegranate fruit extract | Phosphorylation | Ahmed et al., J Nutr. 2005 Sep;135(9):2096-102 |
| Prostaglandin Al | Phosphorylation/IKK | Rossi et al., Proc Natl Acad Sci U S A. 1997 Jan 21;94(2):746-50; |
| | | Rossi et al., Nature 2000 Jan 6;403(6765): 103-8 |
| 20(S)-Protopanaxatriol (ginsenoside metabolite) | Phosphorylation | Oh et al., Cancer Lett. 2004 Mar 8;205(1):23-9; |
| | | Lee et al., Planta Med. 2005 Dec;71(12):1167-70 |
| Rengyolone | Phosphorylation | Kim et al., Biochem Pharmacol. 2006 Apr 14;71(8):1198-205. Epub 2006 Feb 2 |
| Rottlerin | Phosphorylation | Kim et al., Biochem Biophys Res Commun. 2005 Nov 11;337(1):110-5 |
| Saikosaponin-d | Phosphorylation | Leung et al., Biochem Biophys Res Commun. 2005 Dec 30;338(4):1920-7. Epub 2005 Nov 11. |
| Saline (low Na+ istonic) | Phosphorylation | Tabary et al., Biochem Biophys Res Commun. 2003 Sep 19;309(2):310-6 |
| Salvia miltiorrhizae watersoluble extract | Phosphorylation | Kim et al., Clin Exp Immunol. 2005 Aug; 141 (2):288-97. |
| Sanguinarine (pseudochelerythrine, 13-methyl-[1,3]-benzodioxolo-[5,6-c]-1,3-dioxolo-4,5 phenanthridinium) | Phosphorylation | Chaturvedi et al., J Biol Chem. 1997 Nov 28;272(48):30129-34 |
| Scoparone | Phosphorylation | Jang et al., Life Sci. 2006 May 15;78(25):2937-43. Epub 2005 Dec 22 |
| Sesaminol glucosides | Phosphorylation | Lee et al., Neurosci Res. 2006 Oct;56(2):204-12. Epub 2006 Jul 13 |
| Silymarin | Phosphorylation | Manna et al., J Immunol. 1999 Dec 15;163(12):6800-9; |
| | | Saliou et al., FEBS Lett. 1998 Nov 27;440(1-2):8-12 |
| SOCS1 | Phosphorylation | Kinjyo et al., Immunity. 2002 Nov;17(5):583-91; |
| | | Nakagawa et al., Immunity. 2002 Nov;17(5):677-87 |
| Statins (several) | Phosphorylation | Hilgendorff et al., Int J Clin Pharmacol Ther. 2003 Sep;41(9):397-401; |
| | | Han *et al.*, 2004; |
| | | Planavila et al., Biochim Biophys Acta. 2005 Feb 21;1687(1-3):76-83 |
| Sulindac | IKK/Phosphorylation | Yamamato et al., J Biol Chem. 1999 Sep 17;274(38):27307-14 |
| THI 52(1-naphthylethyl-6,7-dihydroxy-1,2,3,4-tetrahydroisoquinoline) | Phosphorylation | Kang et al., Biochem Pharmacol. 2003 Feb 1;65(3):457-64 |
| 1,2,4-thiadiazolidine derivatives | Phosphorylation | Manna et al., Int J Cancer. 2005 Feb 10;113(4):549-60 |
| Vesnarinone | Phosphorylation | Manna & Aggarwal, J Immunol. 2000 Jun 1;164(11):5815-25; |
| | | Harada et al., Int J Oncol. 2005 Dec;27(6):1489-97 |
| Xanthoangelol D | Phosphorylation | Sugii et al., Biol Pharm Bull. 2005 Apr;28(4):607-10. |
| YC-1 | Phosphorylation | Huang et al., Mol Cancer Ther. 2005 Oct;4(10):1628-35 |
| YopJ (encoded by Yersinia pseudotuberculosis) | Deubiquintinase for IkBa | Schesser et al., Mol Microbiol. 1998 Jun;28(6):1067-79; |
| | | Zhou et al., J Exp Med. 2005 Nov 21;202(10):1327-32 |
| Acetaminophen | Degradation | Mancini et al., Neurosci Lett. 2003 Dec 19;353(2):79-82 |
| Activated Protein C (APC) | Degradation | Yuksel et al., Thromb Haemost. 2002 Aug;88(2):267-73 |
| Alachlor | Degradation | Shimomura-Shimizu et al., Biochem Biophys Res Commun. 2005 Jul 8;332(3):793-9 |
| a-melanocyte-stimulating hormone (a-MSH) | Degradation | Manna & Aggarwal, J Immunol. 1998 Sep 15;161(6):2873-80 |
| Amentoflavone | Degradation | Banerjee et al., Mol Cell Biochem. 2002 Sep;238(1-2):105-10 |
| Artemisia capillaris Thunb extract | Degradation | Hong et al., Int J Mol Med. 2004 May;13(5):717-20 |
| Artemisia iwayomogi extract | Degradation | Kim et al., Exp Biol Med (Maywood). 2005 Jan;230(1):82-8 |
| L-ascorbic acid | Degradation | Han et al., J Cell Biochem. 2004 Oct 1;93(2):257-70 |
| Antrodia camphorata | Degradation | Hseu et al., Int Immunopharmacol. 2005 Dec;5(13-14):1914-25. Epub 2005 Jul 18 |
| Aucubin | Degradation | Jeong et al., Cytokine. 2002 Jun 7;18(5):252-9. |
| Baicalein | Degradation | Ma et al., Blood. 2005 Apr 15;105(8):3312-8. Epub 2004 Dec 30 |
| beta-lapachone | Degradation | Manna et al., Biochem Pharmacol. 1999 Apr 1;57(7):763-74 |
| Blackberry extract | Degradation | Pergola et al., Nitric Oxide. 2006 Aug;15(1):30-9. Epub 2006 Mar 6 |
| 1-Bromopropane | Degradation | Yoshida et al., Neurotoxicology. 2006 Jun 2; [Epub ahead of print] |
| Buchang-tang | Degradation | Shin et al., J Ethnopharmacol. 2005 Oct 31;102(1):95-101 |
| Capsaicin (8-methyl-N-vanillyl-6-nonenamide) | Degradation | Singh et al., J Immunol. 1996 Nov 15;157(10):4412-20; |
| | | Mori et al., Cancer Res. 2006 Mar 15;66(6):3222-9 |
| Catalposide | Degradation | Kim et al., Inflamm Bowel Dis. 2004 Sep;10(5):564-72 |
| Cyclolinteinone (sponge sesterterpene) | Degradation | D'Acquisto et al., Biochem J. 2000 Mar 15;346 Pt 3:793-8 |
| DA-9601 (Artemisia asiatica extract) | Degradation | Choi et al., World J Gastroenterol. 2006 Aug 14;12(30):4850-8 |
| Diamide (tyrosine phosphatase inhibitor) | Degradation | Toledano & Leonard, Proc Natl Acad Sci U S A. 1991 May 15;88(10):4328-32; |
| | | Singh & Aggarwal, J Biol Chem. 1995 May 5;270(18): 1063 1-9. |
| Dihydroarteanniun | Degradation | Li et al., Int Immunopharmacol. 2006 Aug;6(8):1243-50. Epub 2006 Apr 7 |
| Dobutamine | Degradation | Loop et al., Anesth Analg. 2004 Nov;99(5):1508-15; table of contents. |
| Docosahexaenoic acid | Degradation | Weldon et al., J Nutr Biochem. 2006 Jun 15;[Epub ahead of print] |
| E-73 (cycloheximide analog) | Degradation | Sugimoto et al., Biochem Biophys Res Commun. 2000 Oct 22;277(2):330-3 |
| Ecabet sodium | Degradation | Kim et al., Helicobacter. 2003;8(5):542-53 |
| Electrical stimulation of vagus nerve | Degradation | Guarini et al., Circulation. 2003 Mar 4;107(8):1189-94 |
| Emodin (3-methyl-1,6,8-trihydroxyanthraquinone) | Degradation | Kumar et al., Oncogene. 1998 Aug 20;17(7):913-8; |
| | | Huang et al., Biochem Pharmacol. 2004 Jul 15;68(2):361-71 |
| Ephedrae herba (Mao) | Degradation | Aoki et al., J Pharmacol Sci. 2005 Jul;98(3):327-30. Epub 2005 Jul 9 |
| Equol | Degradation | Kang et al., Biochem Pharmacol. 2005 Dec 19;71(1-2):136-43. Epub 2005 Nov 10 |
| Erbstatin (tyrosine kinase inhibitor) | Degradation | Natarajan et al., Arch Biochem Biophys. 1998 Apr 1;352(1):59-70 |
| Estrogen (E2) | Degradation/and various other steps | Sun et al., Biochem Biophys Res Commun. 1998 Mar 27;244(3):691-5; |
| | | Kalaitzidis & Gilmore, Trends Endocrinol Metab. 2005 Mar; 16(2):46-52; |
| | | Steffan et al., Curr Top Med Chem. 2006;6(2): 103-11. |
| Ethacrynic acid | Degradation (and DNA binding) | Han *et al*., 2004 |
| Fosfomycin | Degradation | Yoneshima et al., Int J Antimicrob Agents. 2003 Jun;21(6):589-92 |
| Fungal gliotoxin | Degradation | Pahl et al., Oncogene. 1999 Nov 22; 18(49):6853-66 |
| Gabexate mesilate | Degradation | Yuksel et al., J Pharmacol Exp Ther. 2003 Apr;305(1):298-305 |
| Gamisanghyulyunbueum | Degradation | Shin et al., Biol Pharm Bull. 2005 Jul;28(7):1177-82 |
| Genistein (tyrosine kinase inhibitor) | Degradation; caspase cleavage of IkBa | Natarajan et al., Arch Biochem Biophys. 1998 Apr 1;352(1):59-70; |
| | | Baxa & Yoshimura, Biochem Pharmacol. 2003 Sep 15;66(6):1009-18. |
| Genipin | Degradation | Koo et al., Eur J Pharmacol. 2004 Jul 14;495(2-3):201-8 |
| Glabridin | Degradation | Kang et al., J Pharmacol Exp Ther. 2005 Mar;312(3):1187-94. Epub 2004 Nov 10 |
| Glimepiride | Degradation | Schiekofer et al., Diabetes Obes Metab. 2003 Jul;5(4):251-61 |
| Glucosamine sulfate | Degradation | Largo et al., Osteoarthritis Cartilage. 2003 Apr;11(4):290-8 |
| gamma-glutamylcysteine synthetase | Degradation | Manna et al., Oncogene. 1999 Jul 29;18(30):4371-82. |
| Glutamine | Degradation | Singleton et al., Shock. 2005 Dec;24(6):583-9 |
| Gumiganghwaltang | Degradation | Kim et al., Biol Pharm Bull. 2005 Feb;28(2):233-7 |
| Heat shock protein-70 | Degradation | Chan et al., Circulation. 2004 Dec 7;110(23):3560-6. Epub 2004 Nov 22.; |
| | | Shi et al., Shock. 2006 Sep;26(3):277-84 |
| Hypochlorite | Degradation | Mohri et al., Invest Ophthalmol Vis Sci. 2002 Oct;43(10):3190-5. |
| IL-13 | Degradation | Manna & Aggarwal, J Immunol. 1998 Sep 15;161(6):2863-72 |
| Intravenous immunoglobulin | Degradation | Ichiyama et al., Inflamm Res. 2004 Jun;53(6):253-6. Epub 2004 May 12 |
| Isomallotochromanol and isomallotochromene | Degradation | Ishii et al., Biochim Biophys Acta. 2003 Mar 17;1620(1-3):108-18 |
| K1L (Vaccinia virus protein) | Degradation | Shisler & Jin, J Virol. 2004 Apr;78(7):3553-60 |
| Kochia scoparia fruit (methanol extract) | Degradation | Shin et al., Biol Pharm Bull. 2004 Apr;27(4):538-43 |
| Leflunomide metabolite (A77 1726) | Degradation | Manna & Aggarwal, J Immunol. 1999 Feb 15;162(4):2095-102 |
| Losartin | Degradation | Chen *et al.*, 2002 |
| Low level laser therapy | Degradation | Rizzi et al., Lasers Surg Med. 2006 Aug;38(7):704-13 |
| LY294002 (PI3-kinase inhibitor) [2-(4-morpholinyl)-8-phenylchromone] | Degradation | Park et al., Cell Biol Toxicol. 2002;18(2):121-30. |
| MC159 (Molluscum contagiosum virus) | Degradation of IkBb | Murao & Shisler, 2005 |
| Melatonin | Degradation | Zhang et al., Eur J Pharmacol. 2004 Oct 6;501(1-3):25-30 |
| 5'-methylthioadenosine | Degradation | Hevia et al., Hepatology. 2004 Apr;39(4):1088-98. |
| Midazolam | Degradation | Kim et al., Anesthesiology. 2006 Jul;105(t):105-10 |
| Momordin I | Degradation | Hwang et al., Biochem Biophys Res Commun. 2005 Nov 25;337(3):815-23. Epub 2005 Sep 28. |
| Morinda officinalis extract | Degradation | Kim et al., J Pharm Pharmacol. 2005 May;57(5):607-15 |
| Mosla dianthera extract | Degradation | Lee et al., Toxicol Appl Pharmacol. 2006 Jun 22; [Epub ahead of print] |
| Murrl gene product | Degradation | Ganesh et al., Nature. 2003 Dec 18;426(6968):853-7. |
| Neurofibromatosis-2 (NF-2; merlin) protein | Degradation | Kim et al., Biochem Biophys Res Commun. 2002 Sep 6;296(5):1295-302 |
| Opuntia ficus indica va saboten extract | Degradation | Lee *et al*., 2006 |
| Penetratin | | Letoya et al., Mol Pharmacol. 2006 Jun;69(6):2027-36. Epub 2006 Feb 27. |
| Pervanadate (tyrosine phosphatase inhibitor) | Degradation | Singh & Aggarwal, J Biol Chem. 1995 May 5;270(18):10631-9; |
| | | Singh et al., J Biol Chem. 1996 Dec 6;271(49):31049-54 |
| Phenylarsine oxide (PAO, tyrosine phosphatase inhibitor) | Degradation | Mahboubi et al., J Pharmacol Exp Ther. 1998 May;285(2):862-8; |
| | | Singh & Aggarwal, J Biol Chem. 1995 May 5;270(18):10631-9 |
| beta-Phenylethyl (PEITC) and 8-methylsulphinyloctyl isothiocyanates (MSO) (watercress) | Degradation | Rose et al., Nitric Oxide. 2005 Jun;12(4):237-43 |
| Phenytoin | Degradation | Kato *et al.*, 2005 |
| Platycodin saponins | Degradation | Ahn et al., Life Sci. 2005 Apr 1;76(20):2315-28 |
| Polymyxin B | Degradation | Jiang et al., Chin Med J (Engl). 2006 Mar 5;119(5):384-90. |
| Poncirus trifoliata fruit extract | Degradation | Shin et al., Toxicol In vitro. 2006 Oct;20(7):1071-6. Epub 2006 Mar 6 |
| Probiotics | Degradation | Petrof et al., Gastroenterology. 2004 Nov;127(5):1474-87. |
| Pituitary adenylate cyclase-activating polypeptide (PACAP) | Degradation | Delgado & Ganea, J Biol Chem. 2001 Jan 5;276(1):369-80 |
| Prostaglandin 15-deoxy-Delta( 12,14)-PGJ(2) | Degradation | Cuzzocrea et al., Br J Pharmacol. 2003 Feb;138(4):678-88; |
| | | Chatterjee et al., Cardiovasc Res. 2004 Feb 15;61(3):630-43 |
| PS-341 | Degradation/proteasome | Hideshima et al., J Biol Chem. 2002 May 10;277(19):16639-47. Epub 2002 Feb 28 |
| Resiniferatoxin | Degradation | Singh et al., J Immunol. 1996 Nov 15;157(10):4412-20 |
| Sabaeksan | Degradation | Choi et al., Exp Mol Pathol. 2005 Jun;78(3):257-62. Epub 2005 Feb 17 |
| SAIF (Saccharomyces boulardii anti-inflammatory factor) | Degradation | Sougioultzis et al., Biochem Biophys Res Commun. 2006 Apr 28;343(1):69-76. Epub 2006 Feb 23. |
| Sesquiterpene lactones (parthenolide; ergolide; guaianolides) | Degradation | Hehner et al., J Biol Chem. 1998 Jan 16;273(3):1288-97; |
| | | Whan Han et al., Br J Pharmacol. 2001 Jun;133(4):503-12.; |
| | | Schorr et al., Phytochemistry. 2002 Aug;60(7):733-40 |
| ST2 (IL-1-like receptor secreted form) | Degradation | Takezako et al., Biochem Biophys Res Commun. 2006 Mar 10;341(2):425-32. Epub 2006 Jan 11 |
| Thiopental | Degradation | Loop et al., Anesthesiology. 2002 May;96(5):1202-13 |
| Tipifarnib | Degradation | Xue et al., J Pharmacol Exp Ther. 2006 Apr;317(1):53-60. Epub 2005 Dec 13 |
| Titanium | Degradation | Yang et al., J Biomed Mater Res A. 2003 Sep 15;66(4):802-10 |
| TNP-470 (angiogenesis inhibitor) | Degradation | Mauriz et al., Free Radic Res. 2003 Aug;37(8):841-8 |
| Stinging nettle (Urtica dioica) plant extracts | Degradation | Riehemann et al., FEBS Lett. 1999 Jan 8;442(1):89-94 |
| Trichomomas vaginalis infection | Degradation | Chang et al., Mol Cells. 2004 Oct 31;18(2):177-85 |
| Triglyceride-rich lipoproteins | Degradation | Kumwenda et al., Shock. 2002 Aug;18(2):182-8 |
| U0126 (MEK inhibitor) | Degradation | Takaya et al., Am J Physiol Renal Physiol. 2003 May;284(5):F1037-45. Epub 2003 Jan 7 |
| Ursodeoxycholic acid | Degradation | Joo et al., Arch Pharm Res. 2004 Sep;27(9):954-60 |
| Xanthium strumarium L. (methanol extract) | Degradation | Kim et al., Biol Pharm Bull. 2005 Jan;28(1):94-100 |
| Zinc | Degradation | Uzzo et al., Carcinogenesis. 2006 Oct;27(10):1980-90. Epub 2006 Apr 10; |
| | | Bao et al., Toxicol Lett. 2006 Oct 25;166(3):222-8. Epub 2006 Jul 18 |
| Molluscum contagiosum virus MC159 protein | IkBbeta degradation | Murao & Shisler, Virology. 2005 Sep 30;340(2):255-64 |
| Vasoactive intestinal peptide | Degradation (and CBP-RelA interaction) | Delgado & Ganea, J Biol Chem. 2001 Jan 5;276(1):369-80; |
| | | Delgado, Biochem Biophys Res Commun. 2002 Oct 11;297(5):1181-5 |
| HIV-1 Vpu protein | TrCP ubiquitin ligase inhibitor | Bour et al., J Biol Chem. 2001 May 11;276(19):15920-8. Epub 2001 Feb 16 |
| Epoxyquinone A monomer | IKKb/DNA binding | Liang et al., Biochem Pharmacol. 2006 Feb 28;71(5):634-45. Epub 2005 Dec 19 |
| Ro106-9920 (small molecule) | IkBa ubiqutination inhibitor | Swinney et al., J Biol Chem. 2002 Jun 28;277(26):23573-81. Epub 2002 Apr 11 |

**TABLE 4 MISCELLANEOUS INHIBITORS OF NF-κB**

| **Inhibitor Molecule** | **Effect or point of inhibition** | **References** |
|---|---|---|
| Conophylline (Ervatamia microphylla) | Down regulated TNF-Receptors | Gohda et al., 2003 Int J Oncol. 23(5):1373-9 |
| MOL 294 (small molecule) | Redox regulated activation of NF-κB | Henderson et al., J Immunol. 2002 Nov 1;169(9):5294-9 |
| PEDF (pigment epithelium derived factor) | ROS generation | Yamagishi et al., J Mol Cell Cardiol. 2004 Aug;37(2):497-506. |
| Perrilyl alcohol | Calcium pathway | Berchtold et al., Cancer Res. 2005 Sep 15;65(18):8558-66. |
| MAST205 | TRAF6 binding | Xiong et al., J Biol Chem. 2004 Oct 15;279(42):43675-83. Epub 2004 Aug 11. |
| Rhein | MEKK activation of NF-κB | Martin et al., Inflammation. 2003 Aug;27(4):233-46; |
| | | Domagala et al., Biorheology. 2006;43(3-4):577-87. |
| 15-deoxy-prostaglandin J(2) | PPARg activation of NF-κB | Boyault et al., FEBS Lett. 2004 Aug 13;572(1-3):33-40. |
| Antrodia camphorata extract | IkBa upregulation | Hsu et al., Cancer Lett. 2005 Apr 18;221(1):77-89. |
| apigenin (4',5,7-trihydroxyflavone) | IkBa upregulation | Shukla & Gupta, Clin Cancer Res. 2004 May 1;10(9):3169-78. |
| beta-amyloid protein | IkBa upregulation | Bales et al., Brain Res Mol Brain Res. 1998 Jun 1;57(1):63-72 |
| human breast milk | IkBa upregulation | Minekawa et al., Am J Physiol Cell Physiol. 2004 Nov;287(5):C1404-11. Epub 2004 Jun 30 |
| Surfactant protein A (SP-A) | IkBa upregulation | Wu et al., Am J Respir Cell Mol Biol. 2004 Dec;31(6):587-94. Epub 2004 Aug 12 |
| DQ 65-79 (aa 65-79 of the alpha helix of the alpha-chain of the class II HLA molecule DQA03011) | IkBa upregulation and IKK inhibition | Jiang et al., J Immunol. 2002 Apr 1;168(7):3323-8. |
| C5a | IkBa upregulation | Riedemann et al., Immunity. 2003 Aug; 19(2):193-202. |
| Glucocorticoids (dexamethasone, prednisone, methylprednisolone) | IkBa upregulation | Auphan et al., Science. 1995 Oct 13;270(5234):286-90; |
| | | Brostjan et al., J Biol Chem. 1996 Aug 9;271(32):19612-6; |
| | | Ray & Prefontaine, Proc Natl Acad Sci U S A. 1994 Jan 18;91(2):752-6; |
| | | Scheinman et al., Mol Cell Biol. 1995 Feb;15(2):943-53. |
| IL-10 | IkBa upregulation | Ehrlich et al., Neuroreport. 1998 Jun 1;9(8): 1723-6; |
| | | Lentsch et al., J Clin Invest. 1997 Nov 15;100(10):2443-8; |
| | | Shames et al., Shock. 1998 Dec;10(6):389-94 |
| IL-13 | IkBa upregulation | Ehrlich et al., Neuroreport. 1998 Jun 1;9(8):1723-6; |
| | | Lentsch et al., J Clin Invest. 1997 Nov 15;100(10):2443-8; |
| | | Manna & Aggarwal, J Immunol. 1998 Sep 15;161(6):2863-72. |
| IL-11 | IKKa; IkBa,IkBb upregulation | Trepicchio & Dorner, Ann N Y Acad Sci. 1998 Sep 29;856:12-21; |
| | | Lgssiar et al., Exp Biol Med (Maywood). 2004 May;229(5):425-36. |
| alpha-pinene | IkBa upregulation | Zhou et al., Acta Pharmacol Sin. 2004 Apr;25(4):480-4. |
| NEF (HIV-1) | IkBa upregulation | Qiao et al., Nat Immunol. 2006 Mar;7(3):302-10. Epub 2006 Jan 22. |
| R-etodolac | IkBa upregulation | Neri et al., Br J Haematol. 2006 Jul; 134(1):37-44. |
| Vitamin D | IkBa upregulation | Cohen-Lahav et al., Nephrol Dial Transplant. 2006 Apr;21(4):889-97. Epub 2006 Feb 2. |
| Fox1j | IkBb upregulation | Lin *et al*., 2004 |
| Dioxin | RelA nuclear transport | Ruby et al., Mol Pharmacol. 2002 Sep;62(3):722-8 |
| Agastache rugosa leaf extract | Nuclear translocation | Oh et al., Arch Pharm Res. 2005 Mar;28(3):305-10. |
| Alginic acid | Nuclear translocation | Jeong et al., Clin Exp Allergy. 2006 Jun;36(6):785-94. |
| Astragaloside IV | Nuclear translocation | Zhang et al., Thromb Haemost. 2003 Nov;90(5):904-14. |
| Atorvastatin | Nuclear translocation | Haloui et al., Eur J Pharmacol. 2003 Aug 8;474(2-3):175-84. |
| Blue honeysuckle extract | Nuclear translocation | Jin et al., Exp Eve Res. 2006 May;82(5):860-7. Epub 2005 Nov 23. |
| BMD (N(1)-Benzyl-4-methylbenzene-1,2-diamine) | Nuclear translocation | Shin et al., Eur J Pharmacol. 2005 Oct 3;521(1-3):1-8. Epub 2005 Sep 23. |
| Buthus martensi Karsch extract | Nuclear translocation | Kim *et al*., 2005 |
| Canine Distemper Virus | Nuclear translocation | Friess et al., J Comp Pathol. 2005 Jan;132(1):82-9. |
| Carbaryl | Nuclear translocation | Shimomura-Shimizu et al., Biochem Biophys Res Commun. 2005 Jul 8;332(3):793-9. |
| Celastrol | Nuclear translocation | Pinna et al., Biochem Biophys Res Commun. 2004 Sep 24;322(3):778-86. |
| Chiisanoside | RelA Nuclear translocation | Won et al., Biol Pharm Bull. 2005 Oct;28(10):1919-24. |
| CP-1158 | Nuclear translocation | Kim et al., Eur J Pharmacol. 2006 Aug 14;543(1-3):158-65. Epub 2006 Jun 2. |
| Dehydroxymethylepoxyquin omicin (DHMEQ) | Nuclear translocation | Chaicharoenpong et al., Bioorg Med Chem. 2002 Dec;10(12):3933-9 |
| 15-deoxyspergualin | Nuclear translocation | Hutchings et al., Transpl Immunol. 2003 Jul-Sep; 11(3-4):335-44. |
| Dipyridamole | Nuclear translocation | Weyrich et al., Circulation. 2005 Feb 8;111(5):633-42. Epub 2005 Jan 24. |
| Disulfiram | Nuclear translocation | Wang et al., Int J Cancer. 2003 Apr 20;104(4):504-11. |
| Diltiazem | Nuclear translocation; induced translocation of p50 dimers | Severa et al., Biochem Pharmacol. 2005 Feb 1;69(3):425-32. Epub 2004 Dec 9. |
| Eriocalyxin B | Nuclear translocation/DNA binding | Wang et al., Cell Death Differ. 2006 Jun 16; [Epub ahead of print]; |
| | | Leung et al., Mol Pharmacol. 2006 Aug 29; [Epub ahead of print] |
| Estrogen enhanced transcript | Nuclear translocation | Jin et al., Cell Immunol. 2003 May;223( 1):26-34. |
| FAK-related nonkinase | Nuclear translocation | Qin & Liu, Acta Pharmacol Sin. 2006 Sep;27(9):1159-64 |
| Gangliosides | Nuclear translocation | Caldwell et al., J Immunol. 2003 Aug 15;171(4):1676-83. |
| Glucorticoid-induced leucine zipper protein (GILZ) | Nuclear translocation | Riccardi et al., Adv Exp Med Biol. 2001;495:31-9. |
| Harpagophytum procumbens (Devil's Claw) extracts | Nuclear translocation | Kaszkin et al., Phytomedicine. 2004 Nov;1 1(7-8):585-95. |
| Heat shock protein 72 | Nuclear translocation | Meldrum et al., Circ Res. 2003 Feb 21;92(3):293-9 |
| Hirsutenone | Nuclear translocation | Kim et al., FEBS Lettu. 2006 Jan 23;580(2):385-92. Epub 2005 Dec 19 |
| Indole-3-carbinol | Nuclear translocation | Rahman & Sarkar, Cancer Res. 2005 Jan 1;65(1):364-71 |
| JM34 (benzamide derivative) | Nuclear translocation | Carbonnelle *et al*., 2005 |
| JSH-23 (4-Methyl- -(3-phenyl-propyl)-benzene-1,2-diamine | Nuclear translocation | Shin et al., FEBS Lett. 2004 Jul 30;571(1-3):50-4 |
| KIOM-79 (combined plant extracts) | Nuclear translocation | Jeon et al., J Ethnopharmacol. 2006 Apr 28; [Epub ahead of print] |
| KL-1156 (6-Hydroxy-7-methoxychroman-2-carboxylic acid phenylamide) | Nuclear translocation | Kim et al., Biochem Biophys Res Commun. 2004 Dec 3;325(1):223-8. |
| Leptomycin B (LMB) | Nuclear translocation | Rodriguez et al., J Biol Chem. 1999 Mar 26;274(13):9108-15. |
| Levamisole | Nuclear translocation | Liu et al., J Surg Res. 2004 Apr; 117(2):223-31. |
| MEB (2-(4-morpholynl) ethyl butyrate hydrochloride) | Nuclear translocation | Soderberg et al., Int Immunopharmacol. 2004 Sep;4(9):1231-9. |
| MNF (IkB-like Myxoma virus) | Nuclear translocation | Camus-Bouclainville et al., J Virol. 2004 Mar;78(5):2510-6. |
| Montelukast | Nuclear translocation | Wu et al., Can J Physiol Pharmacol. 2006 May;84(5):531-7. |
| NLS Cell permeable peptides (SN50) | Nuclear translocation | Lin et al., J Biol Chem. 1995 Jun 16;270(24):14255-8. |
| 2',8"-biapigenin | RelA nuclear translocation | Woo et al., Biol Pharm Bull. 2006 May;29(5):976-80. |
| Nucling | RelA nuclear translocation | Liu et al., Biochem J. 2004 May 15;380(Pt 1):31-41. |
| o,o'-bismyristoyl thiamine disulfide (BMT) | Nuclear translocation | Shoji et al., Biochem Biophys Res Commun. 1998 Aug 28;249(3):745-53. |
| Oregonin | RelA nuclear translocation | Lee et al., Br J Pharmacol. 2005 Oct; 146(3):378-88 |
| 1,2,3,4,6-penta-O-galloyl-beta-d-glucose | RelA nuclear translocation | Kang et al., Eur J Pharmacol. 2005 Nov 7;524(1-3):111-9. Epub 2005 Oct 25 |
| Platycodi radix extract | RelA nuclear translocation | Lee et al., Int J Mol Med. 2004 Jun;13(6):843-7 |
| Phallacidin | Nuclear translocation | Papakonstanti & Strounaras, Mol Biol Cell. 2004 Mar;15(3):1273-86. Epub 2003 Dec 29 |
| Piperine | Nuclear translocation | Pradeep & Kuttan, Int Immunopharmacol. 2004 Dec 20;4(14):1795-803 |
| Pitavastatin | Nuclear translocation | Wang et al., Biol Pharm Bull. 2006 Apr;29(4):634-9 |
| PN-50 | Nuclear translocation | Letoha et al., World J Gastroenterol. 2005 Feb 2 1;11(7):990-9 |
| Probiotics | RelA nuclear translocation | Bai et al., World J Gastroenterol. 2004 Feb 1;10(3):455-7. |
| RelA peptides (P1 and P6) | Nuclear translocation | Takada et al., J Biol Chem. 2004 Apr 9;279(15):15096-104. Epub 2004 Jan 7. |
| Retinoic acid receptor-related orphan receptor-alpha | Nuclear translocation | Migita et al., FEBS Lett. 2004 Jan 16;557(1-3):269-74 |
| Rhubarb aqueous extract | RelA nuclear translocation | Moon et al., Life Sci. 2006 Feb 28;78(14):1550-7. Epub 2005 Nov 2 |
| Rolipram | Nuclear translocation | Sanchez et al., J Neuroimmunol. 2005 Nov;168(1-2):13-20. Epub 2005 Sep 22; |
| | | Ikezoe et al., Cancer Res. 2004 Oct 15;64(20):7426-31. |
| Salvia miltiorrhoza Bunge extract | Nuclear translocation | Ding et al., J Cardiovasc Pharmacol. 2005 Jun;45(6):516-24 |
| SC236 (a selective COX-2 inhibitor) | Nuclear translocation | Wong et al., Oncogene. 2003 Feb 27;22(8):1189-97 |
| Selenomethionine | Nuclear translocation | Cherukuri et al., Cancer Biol Ther. 2005 Feb;4(2):175-80. Epub 2005 Feb 8 |
| ShenQi compound recipe | RelA Nuclear translocation | Zhang et al., Zhong Yao Cai. 2006 Mar;29(3):249-53 |
| Sophorae radix extract | Nuclear translocation | Kwon et al., Clin Chim Acta. 2004 Oct;348(1-2):79-86 |
| Sopoongsan | Nuclear translocation | Na et al., Int Arch Allergy Immunol. 2006;139(1):31-7. Epub 2005 Nov 3 |
| Sphondin (furanocoumarin derivative from Heracleum laciniatum) | Nuclear translocation | Yang et al., Life Sci. 2002 Nov 29;72(2):199-213 |
| TAT-SR-IkBa; MTS-SR-IkBa | Nuclear translocation | Blackwell et al., Arthritis Rheum. 2004 Aug;50(8):2675-84; |
| | | Mora et al., Am J Physiol Lung Cell Mol Physiol. 2005 Oct;289(4):L536-44. Epub 2005 Jun 10 |
| Volatile anesthetic treatment | Nuclear translocation | Lee et al., Anesthesiology. 2004 Dec;101(6):1313-24 |
| Younggaechulgam-tang | Nuclear translocation | Shin et al., Immunopharmacol Immunotoxicol. 2004;26(4):545-58 |
| ZUD protein | Activation of NF-κB; binds p105/RelA | Zhang et al., J Biol Chem. 2004 Apr 23;279(17):17819-25. Epub 2004 Feb 9 |
| ZAS3 protein | RelA nuclear translocation; DNA competition | Hong et al., Proc Natl Acad Sci U S A. 2003 Oct 14;100(21):12301-6. Epub 2003 Oct 6 |
| Clarithromycin | nuclear expression | Ichiyama et al., Antimicrob Agents Chemother. 2001 Jan;45(1):44-7 |
| Fluvastatin | nuclear expression | Azuma et al., Cardiovasc Res. 2004 Dec 1;64(3):412-20 |
| Leflunomide | RelA nuclear expression | Yao et al., Acta Pharmacol Sin. 2004 Jul;25(7):915-20 |
| RASSF 1A gene overexpression | RelA nuclear expression | Deng et al., Zhong Nan Da Xue Xue Bao Yi Xue Ban. 2005 Apr;30(2):193-6 |
| oxidized 1-palmitoyl-2-arachidonoyl-sn-glycero-3-phosphorylcholine (OXPAPC) | RelA expression | Li et al., Zhonghua Yi Xue Za Zhi. 2004 Aug 2;84(15): 1235-9 |
| 3C protease (Poliovirus) | RelA expression (cleavage) | Neznanov et al., J Biol Chem. 2005 Jun 24;280(25):24153-8. Epub 2005 Apr 21. |
| 5F (from Pteri syeminpinnata L) | RelA expression | He et al., Zhong Yao Cai. 2005 Aug;28(8):672-6 |
| AT514 (serratamolide) | RelA expression | Escobar-Diaz et al., Leukemia. 2005 Apr;19(4):572-9 |
| Sorbus commixta cortex (methanol extract) | RelA expression | Sohn et al., Biol Pharm Bull. 2005 Aug;28(8): 1444-9 |
| Cantharidin | NF-κB expression | He et al., Ai Zheng. 2005 Apr;24(4):443-7 |
| Cornus officinalis extract | NF-κB expression | Li et al., Zhongguo Zhong Yao Za Zhi. 2005 Nov;30(21):1667-70 |
| Neomycin | NF-κB expression | Garcia-Trapero et al., Neurol Res. 2004 Dec;26(8):816-24 |
| omapatrilat, enalapril, CGS 25462 | NF-κB expression | Pu et al., J Hypertens. 2005 Feb;23(2):401-9 |
| Onconase (Ranpirnase) | NF-κB expression | Tsai et al., Int J Oncol. 2004 Dec;25(6):1745-52 |
| Paeoniflorin | NF-κB expression | Liu et al., Brain Res. 2006 May 17;1089(1):162-70. Epub 2006 May 5 |
| Rapamycin | NF-κB expression | Lawrence et al., J Vasc Surg. 2004 Aug;40(2):334-8 |
| Sargassum hemiphyllum methanol extract | NF-κB expression | Na et al., J Pharmacol Sci. 2005 Feb;97(2):219-26. Epub 2005 Feb 5 |
| Shenfu | NF-κB expression | Zhang et al., Chin J Traumatol. 2005 Aug 1;8(4):200-4 |
| Tripterygium polyglycosides | NF-κB expression | Zhou et al., Zhongguo Zhong Xi Yi Jie He Za Zhi. 2005 Aug;25(8):723-6 |
| Triflusal | nuclear expression | Acarin et al., Neurosci Lett. 2000 Jul 7;288(1):41-4 |
| HSCO (hepatoma protein) | Accelerates RelA nuclear export | Higashitsuji et al., Cancer Cell. 2002 Oct;2(4):335-46 |
| Andrographolide | Covlalent adduct with Cys-62 of p50 | Xia et al., J Immunol. 2004 Sep 15;173(6):4207-17 |
| Bee venom (melittin) | DNA binding by binding to p50 | Park et al., Arthritis Rheum. 2004 Nov;50(11):3504-15 |
| Ethyl pyruvate | DNA binding by RelA thru Cys-38 | Han et al., J Pharmacol Exp Ther. 2005 Mar;312(3):1097-105. Epub 2004 Nov 3 |
| 1'-acetoxychavicol acetate | DNA binding | Ito et al., Biochem Biophys Res Commun. 2005 Dec 30;338(4):1702-10. Epub 2005 Nov 2 |
| 2-acetylaminofluorene | DNA binding | Kang et al., Cancer Lett. 2004 Jan 8;203(1):91-8; |
| | | Jeon et al., Toxicol Lett. 1999 Feb 22;104(3):195-202 |
| Actinodaphine (from Cinnamomum insularimontanum) | DNA binding | Hsieh et al., Food Chem Toxicol. 2006 Mar;44(3):344-54. Epub 2005 Sep 15 |
| Adiponectin | DNA binding | Ajuwon & Spurlock, Am J Physiol Regul Integr Comp Physiol. 2005 May;288(5):R1220-5. Epub 2004 Dec 16 |
| ADP ribosylation inhibitors (nicotinamide, 3-aminobenzamide) | DNA binding | Le Page et al., Biochem Biophys Res Commun. 1998 Feb 13;243(2):451-7 |
| AIM2 (Absent In Melanoma protein) overexpression | DNA binding | Chen et al., Mol Cancer Ther. 2006 Jan;5(1):1-7 |
| Moderate alcohol intake | DNA binding | Mandrekar et al., Alcohol Clin Exp Res. 2006 Jan;30(1):135-9 |
| 7 -amino-4-methylcoumarin | DNA binding | Kurokawa et al., Eur J Pharmacol. 2003 Aug 8;474(2-3):283-93 |
| Amrinone | DNA binding | Chanani et al., Circulation. 2002 Sep 24;106(12 Suppl 1):I284-9. |
| Angiopoietin-1 | DNA binding | Jeon et al., Circ Res. 2003 Apr 4;92(6):586-8 |
| Anthocyanins (soybean) | DNA binding | Kim et al., FEBS Lett. 2006 Feb 20;580(5):1391-7. Epub 2006 Jan 26 |
| Arnica montana extract (sequiterpene lactones) | DNA binding | Kos et al., Planta Med. 2005 Nov;71(11):1044-52 |
| Artemisinin | DNA binding | Aldieri et al., FEBS Lett. 2003 Sep 25;552(2-3):141-4; |
| | | Wang et al., Antimicrob Agents Chemother. 2006 Jul;50(7):2420-7 |
| Atrial Natriuretic Peptide (ANP) | DNA binding; IkBa upregulation | Gerbes et al., Hepatology. 1998 Nov;28(5):1309-17; |
| | | Kiemer et al., Biochem Biophys Res Commun. 2002 Aug 2;295(5):1068-76. |
| Atrovastat (HMG-CoA reductase inhibitor) | DNA binding | Bustos et al., J Am Coll Cardiol. 1998 Dec;32(7):2057-64; |
| | | Hernandez-Presa et al., Am J Pathol. 1998 Dec;153(6):1825-37 |
| AvrA protein (Salmonella) | DNA binding | Collier-Hyams et al., J Immunol. 2002 Sep 15;169(6):2846-50 |
| Baicalein (5,6,7-trihydroxyflavone) | DNA binding | Suk et al., J Pharmacol Exp Ther. 2003 May;305(2):638-45. Epub 2003 Jan 21 |
| Bambara groundnut (Vignea subterranean) | DNA binding | Na et al., Biofactors. 2004;21(1-4): 149-53 |
| Benfotiamine (thiamine derivative) | DNA binding | Hammes et al., Nat Med. 2003 Mar;9(3):294-9. Epub 2003 Feb 18 |
| beta-catenin | DNA binding | Deng et al., Cancer Cell. 2002 Oct;2(4):323-34 |
| beta-lapachone (a 1,2-naphthoquinone) | DNA binding | Tzeng et al., Am J Respir Crit Care Med. 2003 Jul 1;168(1):85-91. Epub 2003 Apr 30 |
| Biliverdin | DNA binding | Yamashita et al., FASEB J. 2004 Apr; 18(6):765-7. Epub 2004 Feb 20 |
| Bisphenol A | DNA binding | Kim & Jeong, Cancer Lett. 2003 Jun 30;196(1):69-76 |
| Bovine serum albumin | DNA binding | Zhang & Frei, Cardiovasc Res. 2002 Sep;55(4):820-9 |
| Brazilian green propolis | DNA binding | Bae et al., Eur J Pharmacol. 2005 Apr 25;513(3):237-42. Epub 2005 Apr 15; |
| | | Paulino et al., Planta Med. 2006 Aug;72(10):899-906. Epub 2006 Aug 10 |
| Bromelain | DNA binding | Hou et al., J Agric Food Chem. 2006 Mar 22;54(6):2193-8 |
| Calcium/calmodulin-dependent kinase kinase (CaMKK) (and increased intracellular calcium by ionomycin, UTP and thapsigargin) | DNA binding | Chen et al., J Biol Chem. 2002 Jul 5;277(27):24169-79. Epub 2002 Apr 25 |
| Calcitriol (1a,25-dihydroxyvitamin D3) | DNA binding | Harant et al., Eur J Biochem. 1997 Nov 15;250(1):63-71 |
| Campthothecin | DNA binding | Hentze et al., Hepatology. 2004 May;39(5):1311-20 |
| Cancer bush (Sutherlandia frutescens) | DNA binding | Na et al., Biofactors. 2004;21(1-4):149-53 |
| Caprofen | DNA binding | Bryant et al., Am J Vet Res. 2003 Feb;64(2):211-5 |
| Capsiate | DNA binding | Sancho et al., Eur J Immunol. 2002 Jun;32(6):1753-63 |
| Carbocisteine | DNA binding | Yasuda et al., Eur Respir J. 2006 Jul;28(1):51-8. Epub 2006 Mar 1 |
| Catalposide (stem bark) | DNA binding | Oh et al., Planta Med. 2002 Aug;68(8):685-9 |
| Cat's claw bark (Uncaria tomentosa; Rubiaceae); Maca | DNA binding | Aguilar et al., J Ethnopharmacol. 2002 Jul;81 (2):271-6; |
| | | Valerio & Gonzales, Toxicol Rev. 2005;24(1):1 1-35 |
| CD43 overexpression | DNA binding (RelA) | Laos et al., Int J Oncol. 2006 Mar;28(3):695-704 |
| Celecoxib and germcitabine | DNA binding | El-Rayes et al., Mol Cancer Ther. 2004 Nov;3(11):1421-6 |
| Cheongyeolsaseuptang | DNA binding | Kim et al., J Ethnopharmacol. 2005 Feb 10;97(1):83-8. Epub 2004 Dec 10 |
| Chitosan | DNA binding | Seo et al., Biol Pharm Bull. 2003 May;26(5):717-21 |
| Cinnamaldehyde, 2-methoxycinnamaldehyde, 2-hydroxycinnamaldehyde | DNA binding | Reddy et al., Planta Med. 2004 Sep;70(9): 823-7; |
| | | Lee et al., Biochem Pharmacol. 2005 Mar 1;69(5):791-9. Epub 2005 Jan 16 |
| Chicory root (guaianolide 8-deoxylactucin) | DNA binding | Cavin et al., Biochem Biophys Res Commun. 2005 Feb 18;327(3):742-9 |
| Chlorophyllin | DNA binding | Yun et al., Int Immunopharmacol. 2005 Dec;5(13-14):1926-35. Epub 2005 Jul 6. |
| Chondrotin sulfate proteoglycan degradation product | DNA binding | Rolls et al., FASEB J. 2006 Mar;20(3):547-9. Epub 2006 Jan 5 |
| Clarithromycin | DNA binding | Miyanohara et al., Laryngoscope. 2000 Jan;1 10(1):126-31 |
| Cloricromene | DNA binding | Ianaro et al., Naunyn Schmiedebergs Arch Pharmacol. 2004 Aug;370(2):140-5. Epub 2004 Jul 30 |
| Cocaethylene | DNA binding | Tacker et al., Clin Chem. 2006 Oct;52(10):1926-33. Epub 2006 Aug 17 |
| Commerical peritoneal dialysis solution | DNA binding | Douvdevani et al., Kidney Int. 1995 Jun;47(6):1537-45 |
| Compound K (from Panax ginseng) | DNA binding | Park et al., Biol Pharm Bull. 2005 Apr;28(4):652-6. |
| Cortex cinnamomi extract | DNA binding | Kwon et al., World J Gastroenterol. 2006 Jul 21;12(27):4331-7 |
| CP Compound (6-Hydroxy-7-methoxychroman-2-carboxylic acid phenylamide) | DNA binding | Rak Min et al., Life Sci. 2005 Nov 4;77(25):3242-57. Epub 2005 Jun 22 |
| Cryptotanshinone | DNA binding | Zhou et al., Biochim Biophys Acta. 2006 Jan;1760(1):1-9. Epub 2005 Oct 3. |
| Cyanoguanidine CHS 828 | DNA binding | Johanson et al., Neuroendocrinology. 2005;82(3-4):171-6. Epub 2006 Feb 24 |
| Cytochalasin D | DNA binding | Kim et al., J Biol Chem. 2003 Oct 24;278(43):42448-56. Epub 2003 Aug 7 |
| DA-9201 (from black rice) | DNA binding | Lee et al., Arch Pharm Res. 2005 Dec;28(12):1350-7. |
| Danshenshu | DNA binding | Jiang et al., Zhonghua Shao Shang Za Zhi. 2001 Feb;17(1):36-8 |
| (kB site) Decoy oligonucleotides | DNA binding | Kupatt et al., Gene Ther. 2002 Apr;9(8):518-26; |
| | | Morishita et al., Nat Med. 1997 Aug;3(8):894-9 |
| Diamide | DNA binding | Toledano & Leonard, Proc Natl Acad Sci U S A. 1991 May 15;88(10):4328-32 |
| Diarylheptanoid 7-(4'-hydroxy-3'-methoxyphenyl)-1-phenylhept-4-en-3-one | DNA binding | Yadav et al., J Pharmacol Exp Ther. 2003 Jun;305(3):925-31. Epub 2003 Mar 6 |
| alpha-difluoromethylornithine (polyamine depletion) | DNA binding | Facchini et al., J Cell Physiol. 2005 Sep;204(3):956-63 |
| DIM/13C | DNA binding | Li et al., Front Biosci. 2005 Jan 1;10:236-43. Print 2005 Jan 1 |
| Diterpenoids from Isodon rubescens or Liverwort Jungermannia | DNA binding | Leung et al., Mol Pharmacol. 2005 Aug;68(2):286-97. Epub 2005 May 4; |
| | | Kondoh et al., Planta Med. 2005 Nov;71(11):1005-9 |
| DTD (4,10-dichloropyrido[5,6:4,5]thieno[3,2-d':3,2-d]-1,2,3-ditriazine) | DNA binding | Rioja et al., Naunyn Schmiedebergs Arch Pharmacol. 2002 May;365(5):357-64. Epub 2002 Mar 19. |
| E1B (Adenovirus) | DNA binding | Limbourg et al., J Biol Chem. 1996 Aug 23;271(34):20392-8 |
| E3330 (quinone derivative) | DNA binding | Hiramoto et al., J Immunol. 1998 Jan 15;160(2):810-9; |
| | | Kimura et al., Biochem Biophys Res Commun. 1997 Feb 24;231(3):557-60 |
| ent-kaurane diterpenoids (Croton tonkinensis leaves) | DNA binding | Giang et al., J Nat Prod. 2003 Sep;66(9):1217-20 |
| Epinastine hydrochloride | DNA binding | Kanai et al., Int Arch Allergy Immunol. 2006;140(1):43-52. Epub 2006 Mar 13 |
| Epoxyquinol A (fungal metabolite) | DNA binding | Li et al., Org Lett. 2002 Sep 19;4(19):3267-70 |
| Erythromycin | DNA binding/transactivation | Ren et al., J Orthop Res. 2004 Jan;22(1):21-9; |
| | | Desaki et al., Antimicrob Agents Chemother. 2004 May;48(5):1581-5 |
| Evans Blue | DNA binding | Sharma et al., Bioorg Med Chem Lett. 2004 Dec 20;14(24):6123-7 |
| Evodiamine | DNA binding | Choi et al., Arch Pharm Res. 2006 Apr;29(4):293-7 |
| Fenoldopam | DNA binding | Aravindan et al., J Cardiothorac Vasc Anesth. 2006 Apr;20(2):179-86. Epub 2006 Jan 6 |
| Fexofenadine hydrochloride | DNA binding | Asano et al., Clin Exp Allergy. 2004 Dec;34(12):1890-8 |
| Fibrates | DNA binding | Hirano et al., Int Immunopharmacol. 2003 Feb;3(2):225-32 |
| Fish oil feeding | DNA binding | Fan et al., J Immunol. 2004 Nov 15;173(10):6151-60 |
| FK778 | DNA binding | Zeyda et al., Transplant Proc. 2005 May;37(4):1968-9 |
| FLN29 overexpression | DNA binding | Mashima et al., J Biol Chem. 2005 Dec 16;280(50):41289-97. Epub 2005 Oct 12 |
| FLICE-Like Inhibitory Protein (FLIP) | DNA binding | Bannerman et al., Am J Pathol. 2004 Oct;165(4):1423-31 |
| Flunixin meglumine | DNA binding | Bryant et al., Am J Vet Res. 2003 Feb;64(2):211-5 |
| Flurbiprofen | DNA binding | Fratelli et al., Antioxid Redox Signal. 2003 Apr;5(2):229-35 |
| Fomes fomentarius methanol extracts | DNA binding | Park et al., Biol Pharm Bull. 2004 Oct;27(10):1588-93 |
| Fucoidan | DNA binding | Haneji et al., Nutr Cancer. 2005;52(2):189-201 |
| G-120 (Ulmus davidiana Nakai glycoprotein) | DNA binding; IkB increases | Son et al., Mol Cells. 2004 Oct 31;18(2):163-70.; |
| | | Lee et al., Food Chem Toxicol. 2005 Jun;43(6):961-8 |
| Gallic acid | DNA binding | Kim et al., Toxicol Sci. 2006 May;91(1):123-31. Epub 2005 Dec 1 |
| Ganoderma lucidum (fungal dried spores or fruting body) | DNA binding | Sliva et al., Biochem Biophys Res Commun. 2002 Nov 8;298(4):603-12. |
| Garcinol (fruit rind of Garcinia spp) | DNA binding | Hong et al., Carcinogenesis. 2006 Feb;27(2):278-86. Epub 2005 Aug 10 |
| Gax (homeobox protein) | DNA binding | Patel et al., Cancer Res. 2005 Feb 15;65(4):1414-24 |
| Geranylgeraniol | DNA binding | Espindola et al., Carcinogenesis. 2005 Jun;26(6):1091-9. Epub 2005 Feb 17 |
| Ghrelin | DNA binding | Li et al., Circulation. 2004 May 11;109(18):2221-6. Epub 2004 Apr 26 |
| Gigantol (Cymbidium georingii) | DNA binding | Won et al., Planta Med. 2006 Aug 21; [Epub ahead of print] |
| Ginkgolide B | DNA binding | Nie et al., Yao Xue Xue Bao. 2004 Jun;39(6):415-8. |
| Glycyrrhizin | DNA binding | Wang et al., Liver. 1998 Jun;18(3): 180-5; |
| | | Yuan et al., World J Gastroenterol. 2006 Jul 28;12(28):4578-81 |
| H4/N5 (IkB-like proteins of Microplitis demolitor bracovirus) | DNA binding | Thoetkiattikul et al., Proc Natl Acad Sci U S A. 2005 Aug 9;102(32):11426-31. Epub 2005 Aug 1 |
| Halofuginone | DNA binding | Leiba et al., J Leukoc Biol. 2006 Aug;80(2):399-406. Epub 2006 Jun 12 |
| Heat (fever-like) | DNA binding | Salanova et al., FASEB J. 2005 May;19(7):816-8. Epub 2005 Mar 8 |
| Helenalin (sesquiterpene lactone) | DNA binding | Kim et al., Eur J Pharmacol. 2005 Mar 28;511(2-3):89-97 |
| Hematein (plant compound) | DNA binding | Oh et al., Atherosclerosis. 2001 Nov;159(1):17-26 |
| Herbal compound 861 | DNA binding | You et al., Zhonghua Gan Zang Bing Za Zhi. 2001 Apr;9(2):73-4 |
| Hesperetin | DNA binding | Kim et al., Aging Cell. 2006 Oct;5(5):401-11. Epub 2006 Aug 25 |
| HIV-1 Resistance Factor | DNA binding | Lesner et al., J Immunol. 2005 Aug 15;175(4):2548-54 |
| Hydroxyethyl starch | DNA binding | Tian et al., Ann Clin Lab Sci. 2003 Fall;33(4):451-8; |
| | | Feng et al., J Surg Res. 2006 Sep;135(1):129-36. Epub 2006 Apr 17 |
| Hydroxyethylpuerarin | DNA binding | Lou et al., Chin J Physiol. 2004 Dec 31;47(4):197-201 |
| Hypercapnic acidosis | DNA binding | Chonghaile et al., Curr Opin Crit Care. 2005 Feb;11(1):56-62 |
| Hypericin | DNA binding | Bork et al., Planta Med. 1999 May;65(4):297-300 |
| Hyperosmolarity | DNA binding | Lang et al., Am J Physiol Cell Physiol. 2003 Jan;284(1):C200-8 |
| Hypothermia | DNA binding | Hassoun et al., J Surg Res. 2003 Nov;115(1):121-6 |
| Hydroquinone (HQ) | DNA binding | Pyatt et al., Toxicol Appl Pharmacol. 1998 Apr;149(2):178-84 |
| ICP27 (HSV-1) | DNA binding | Melchjorsen et al., J Gen Virol. 2006 May;87(Pt 5):1099-108 |
| Interleukin 4 (IL-4) | DNA binding | Manna & Aggarwal, J Biol Chem. 1998 Dec 11;273(50):33333-41 |
| IkB-like protein A238L (encoded by ASFV) | DNA binding | Powell et al., J Virol. 1996 Dec;70(12):8527-33; Revilla et al., J Biol Chem. 1998 Feb 27;273(9):5405-11 |
| Insulin-like growth factor binding protein-3 | DNA binding | Williams et al., Cell Death Differ. 2006 Apr 28; [Epub ahead of print] |
| JSH-21 (N1-Benzyl-4-methylbenzene-1,2-diamine) | DNA binding | Min et al., Arch Pharm Res. 2004 Oct;27(10):1053-9 |
| Kamebakaurin | DNA binding | Lee et al., J Biol Chem. 2002 May 24;277(21):18411-20. Epub 2002 Mar 4 |
| Kaposi's sarcoma-associated herpesvirus Kl protein | DNA binding | Lee et al., J Virol. 2002 Dec;76(23):12185-99 |
| Ketamine | DNA binding | Sun et al., Inflamm Res. 2004 Jul;53(7):304-8. Epub 2004 Jun 25 |
| KT-90 (morphine synthetic derivative) | DNA binding | Sueoka et al., Biochem Biophys Res Commun. 1998 Nov 27;252(3):566-70 |
| Linoleic acid | DNA binding | Zhao et al., Arch Anim Nutr. 2005 Dec;59(6):429-38 |
| Lithospermi radix | DNA binding | Chung et al., J Ethnopharmacol. 2005 Dec 1;102(3):412-7. Epub 2005 Jul 28 |
| Lovastatin | DNA binding | Sun & Fernandes, Cell Immunol. 2003 May;223(1):52-62 |
| Macrolide antibiotics | DNA binding | Nguyen et al., Curr Opin Pulm Med. 2002 Nov;8(6):521-8 |
| Mediterranean plant extracts | DNA binding | Stalinska et al., J Physiol Pharmacol. 2005 Mar;56 Suppl 1:157-69 |
| Mercaptopyrazine | DNA binding | Lim et al., Biochem Pharmacol. 2004 Aug 15;68(4):719-28 |
| 2-methoxyestradiol | DNA binding; Transactivation | Shimada et al., Mol Carcinog. 2004 Jan;39(1):1-9; |
| | | Takada et al., Acta Med Okayama. 2004 Aug;58(4):181-7 |
| 6-(Methylsulfinyl)hexyl isothiocyanate (Wasabi) | DNA binding; Transactivation | Uto et al., Biochem Pharmacol. 2005 Dec 5;70(12):1772-84. Epub 2005 Oct 27 |
| Metals (chromium, cadmium, gold, lead, mercury, zinc, arsenic) | DNA binding | Shumilla et al., Arch Biochem Biophys. 1998 Jan 15;349(2):356-62; |
| | | Yang *et al*., 1995; |
| | | Zuscik et al., J Orthop Res. 2002 Jul;20(4):811-8 |
| Mevinolin, 5'-methylthioadenosine (MTA) | DNA binding | Law et al., Mol Cell Biol. 1992 Jan;12(1):103-11 |
| Monomethylfumarate | DNA binding | Litjens et al., Eur J Immunol. 2004 Feb;34(2):565-75 |
| Moxifloxacin | DNA binding | Werber et al., J Antimicrob Chemother. 2005 Mar;55(3):293-300. Epub 2005 Jan 19; |
| | | Shalit et al., J Antimicrob Chemother. 2006 Feb;57(2):230-5. Epub 2005 Dec 13 |
| Myricetin | DNA binding | Kang et al., Arch Pharm Res. 2005 Mar;28(3):274-9. |
| NDPP1 (CARD protein) | DNA binding | Zhang & Fu, Int J Oncol. 2002 May;20(5):1035-40 |
| N-ethyl-maleimide (NEM) | DNA binding | Toledano & Leonard, Proc Natl Acad Sci U S A. 1991 May 15;88(10):4328-32 |
| Naringen | DNA binding | Kanno et al., Life Sci. 2006 Jan 11;78(7):673-81. Epub 2005 Aug 31 |
| Nicorandil | DNA binding | Katamura et al., Shock. 2005 Aug;24(2): 103-8 |
| Nicotine | DNA binding | Sugano et al., Biochem Biophys Res Commun. 1998 Nov 9;252(1):25-8 |
| Nitric oxide-donating aspirin | DNA binding | Kashfi & Rigas, Biochem Soc Trans. 2005 Aug;33(Pt 4):701-4. |
| Nilvadipine | DNA binding | Iwasaki et al., Clin Chim Acta. 2004 Dec;350(1-2):151-7 |
| N itrosoglutathione | DNA binding | Kuo et al., J Trauma. 2004 Nov;57(5):1025-31; |
| | | Khan et al., J Cereb Blood Flow Metab. 2005 Feb;25(2): 177-92 |
| NS1 (Influenza A) | DNA binding | Wang et al., J Virol. 2000 Dec;74(24): 11566-73 |
| NS3/4A (Hepatitis C virus) | DNA binding | Karayiannis, J Hepatol. 2005 Oct;43(4):743-5 |
| Extracts of Ochna macrocalyx bark | DNA binding | Tang et al., Planta Med. 2003 Mar;69(3):247-53 |
| Leucine-rich effector proteins of Salmonella & Shigella (SspH1 and IpaH9.8) | DNA binding | Haraga & Miller, Infect Immun. 2003 Jul;71(7):4052-8 |
| Omega-3 fatty acids | DNA binding | Sethi, Redox Rep. 2002;7(6):369-78 |
| Oridonin (diterpenoid from Rabdosia rubescens) | DNA binding | Ikezoe et al., Mol Cancer Ther. 2005 Apr;4(4):578-86 |
| p8 | DNA binding | Vasseur et al., J Biol Chem. 2004 Feb 20;279(8):7199-207. Epub 2003 Dec 1 |
| 1,2,3,4,6-penta-O-galloyl-beta-D-glucose | DNA binding | Oh et al., Cancer Lett. 2001 Dec 10;174(1):17-24 |
| p202a (interferon inducible protein) | DNA binding by p65 and p50/p65; increases p50 | Ma et α/., J Biol Chem. 2003 Jun 20;278(25):23008-19. Epub 2003 Apr 3 |
| p21 (recombinant) | DNA binding | Khanna et al., J Immunol. 2005 Jun 15;174(12):7610-7 |
| PC-SPES (8 herb mixture) | DNA binding | Ikezoe et al., Mol Pharmacol. 2003 Dec;64(6):1521-9; |
| | | Ikezoe et alInt J Oncol. 2006 Aug;29(2):453-61 |
| Panepoxydone | DNA binding | Erkel et al., Biochem Biophys Res Commun. 1996 Sep 4;226(1):214-21 |
| Peptide nucleic acid-DNA decoys | DNA binding | Penolazzi et al., Int J Mol Med. 2004 Aug; 14(2): 145-52 |
| Pentoxifylline (1-(5'-oxohexyl) 3,7-dimetylxanthine, PTX) | DNA binding | Biswas et al., J Acauir Immune Defic Syndr. 1993 Jul;6(7):778-86; |
| | | Wang et al., Immunity. 1997 Feb;6(2):1 65-74; |
| | | Ji et al., Ann Clin Lab Sci. 2004 Autumn;34(4):427-36 |
| Peptide YY | DNA binding | Vona-Davis et al., J Am Coll Surg. 2004 Jul;199(1):87-95 |
| Pepluanone | DNA binding | Corea et al., J Med Chem. 2005 Nov 3;48(22):7055-62 |
| Perindopril | DNA binding | Li et al., World J Gastroenterol. 2005 Aug 21;11(31):4807-11 |
| 6(5H)-phenanthridinone and benzamide | DNA binding | Chiarugi, Br J Pharmacol. 2002 Nov;137(6):761-70 |
| Phyllanthus amarus extracts | DNA binding | Kiemer et al., J Hepatol. 2003 Mar;3 8(3):289-97 |
| PLAS1 (protein inhibitor of activatated STAT1) | RelA DNA binding | Liu et al., Mol Cell Biol. 2005 Feb;25(3):1113-23 |
| Pioglitazone (PPARgamma ligand) | DNA binding | Takagi et al., Redox Rep. 2002;7(5):283-9 |
| Pirfenidone | DNA binding | Tsuchiya et al., J Hepatol. 2004 Jan;40(1):94-101; |
| | | Nakanishi et al., J Hepatol. 2004 Nov;41(5):730-6 |
| Polyozellin | DNA binding | Jin et al., Planta Med. 2006 Jul;72(9):857-9. Epub 2006 Jun 19. |
| Prenylbisabolane 3 (from Croton eluteria Bennett) | DNA binding | Campagnuoloe et al., Bioorg Med Chem. 2005 Jul 1;13(13):4238-42 |
| Pro-opiomelanocortin | DNA binding | Liu et al., Mol Pharmacol. 2006 Feb;69(2):440-51. Epub 2005 Nov 3 |
| Prostaglandin E2 | DNA binding and RelA nuclear translocation | Min et al., J Rheumatol. 2002 Jul;29(7):1366-76.; |
| | | Gomez et al., J Immunol. 2005 Nov 15;175(10):6924-30 |
| Protein-bound polysaccharide (PSK) | DNA binding | Zhang et al., Oncogene. 2003 Apr 10;22(14):2088-96 |
| PYPAF1 protein | DNA binding | Jeru et al., Arthritis Rheum. 2006 Feb;54(2):508-14 |
| Pyridine N-oxide derivatives | DNA binding | Stevens et al., Biochem Pharmacol. 2006 Apr 14;71(8):1122-35. Epub 2006 Jan 24 |
| Pyrithione | DNA binding | Kim et al., Biochem Biophys Res Commun. 1999 Jun 16;259(3):505-9 |
| Pyrrole-imidazole polyamides | DNA binding | Wurtz et al., Biochemistry. 2002 Jun 18;4 1(24):7604-9. |
| Quinadril (ACE inhibitor) | DNA binding | Bustos et al., J Am Coll Cardiol. 1998 Dec;32(7):2057-64; Hernandez-Presa et al., Am J Pathol. 1998 Dec; 153(6):1825-37 |
| Quinic acid | DNA binding | Akesson et al., Int Immunopharmacol. 2005 Jan;5(1):219-29 |
| Raf Kinase Inhibitor Protein (RKIP) | DNA binding | Keller, Anticancer Drugs. 2004 Aug;15(7):663-9 |
| Rapomycin | DNA binding | Dichtl et al., Atherosclerosis. 2006 Jun;186(2):321-30. Epub 2005 Sep 23. |
| Raloxifene | RelA DNA binding | Olivier et al., Mol Pharmacol. 2006 May;69(5):1615-23. Epub 2006 Feb 23 |
| Raxofelast | DNA binding | Altavilla et al., Free Radic Res. 2003 Apr;37(4):425-35 |
| Rebamipide | DNA binding | Hahm et al., Aliment Pharmacol Ther. 2003 Jul;18 Suppl 1:24-38 |
| Rhus verniciflua Stokes fruits 36 kDa glycoprotein | DNA binding | Ko et al., Toxicol In vitro. 2005 Apr;19(3):353-63. Epub 2004 Dec 24 |
| Ribavirin | DNA binding | Fiedler et al., J Virol. 1996 Dec;70(12):9079-82. |
| Rifamides | DNA binding | Pahlevan et al., J Antimicrob Chemother. 2002 Mar;49(3):531-4 |
| Ritonavir | DNA binding | Ikezoe et al., Cancer Res. 2004 Oct 15;64(20):7426-31 |
| Rosiglitazone | DNA binding | Gruden et al., J Am Soc Nephrol. 2005 Mar;16(3):688-96. Epub 2005 Jan 26 |
| Roxithromycin | DNA binding | Kim et al., Pharmacology. 2004 Sep;72(1):6-11 |
| Sanggenon C | DNA binding | Li et al., Acta Pharmacol Sin. 2002 Feb;23(2):138-42 |
| Santonin diacetoxy acetal derivative | DNA binding | Kim et al., J Biol Chem. 2006 May 12;281(19):13117-25. Epub 2006 Mar 22 |
| Secretory leukoprotease inhibitor (SLPI) | DNA binding | Jin et al., Cell. 1997 Feb 7;88(3):417-26 |
| | | Greene et al., Infect Immun. 2004 Jun;72(6):3684-7; |
| | | Taggart et al., J Exp Med. 2005 Dec 19;202(12):1659-68. Epub 2005 Dec 13. |
| Serotonin derivative (N-(p-coumaroyl) serotonin, SC) | DNA binding | Kawashima et al., J Interferon Cytokine Res. 1998 Jun; 18(6):423-8 |
| Sesamin (from sesame oil) | DNA binding | Jeng et al., Immunol Lett. 2005 Feb 15;97(1):101-6. |
| Shen-Fu | DNA binding | Qian et al., Am J Chin Med. 2006;34(4):613-21. |
| Siah2 | DNA binding | Habelhah et al., EMBO J. 2002 Nov 1;21(21):5756-65 |
| Silibinin | DNA binding | Schumann et al., J Hepatol. 2003 Sep;39(3):333-40. |
| Simvastatin | DNA binding | Li et al., J Pharmacol Exp Ther. 2002 Aug;302(2):601-5.; |
| | | Kalyanasundaram et al., J Vasc Surg. 2006 Jan;43(1):117-24. |
| Sinomenine | DNA binding | Chen et al., Zhongguo Zhong Yao Za Zhi. 2004 Sep;29(9):900-3. |
| SIRT1 Deacetylase overexpression | DNA binding | Chen et al., J Biol Chem. 2005 Dec 2;280(48):40364-74. Epub 2005 Sep 23. |
| Siva-1 | DNA binding | Gudi et al., Oncogene. 2006 Jun 8;25(24):3458-62. Epub 2006 Feb 20. |
| SM-7368 (small molecule) | DNA binding | Lee et al., Biochem Biophys Res Commun. 2005 Oct 21;336(2):716-22. |
| Solana nigrum L. 150 kDa glycoprotein | DNA binding | Heo et al., Toxicol In vitro. 2004 Dec;18(6):755-63.; |
| | | Lee & Lim, Toxicol In vitro. 2006 Oct;20(7):1088-97. Epub 2006 Mar 9. |
| Sulfasalazine | DNA binding | Egan & Sandborn, Gastroenterology. 1998 Nov;115(5):1295-6. |
| SUN C8079 | DNA binding | Matsumori et al., Eur J Heart Fail. 2004 Mar 1;6(2):137-44. |
| Surfactant protein A | DNA binding | Alcorn & Wright, J Biol Chem. 2004 Jul 16;279(29):30871-9. Epub 2004 May 3. |
| Sword brake fern extract | DNA binding | Wu et al., J Ethnopharmacol. 2005 Apr 8;98(1-2):73-81. |
| T-614 (a methanesulfoanilide anti-arthritis inhibitor) | DNA binding | Aikawa et al., Inflamm Res. 2002 Apr;5 1 (4): 188-94 |
| Tanacetum larvatum extract | DNA binding | Petrovic et al., J Ethnopharmacol. 2003 Jul;87(1):109-13 |
| Tansinones (Salvia miltiorrhiza Bunge, Labiatae roots) | DNA binding | Choi et al., Arch Pharm Res. 2004 Dec;27(12):1233-7. |
| Taurine + niacine | DNA binding | Giri, Adv Exp Med Biol. 2003;526:381-94.; |
| | | Kim & Kim, Biochem Pharmacol. 2005 Nov 1;70(9):1352-60. |
| Tetramethylpyrazine | DNA binding | Cheng et al., Planta Med. 2006 Aug;72(10):888-93. Epub 2006 Aug 10. |
| Tobacoo smoke | DNA binding | Zhong et al., Am J Respir Crit Care Med. 2006 Aug 15;174(4):428-36. Epub 2006 May 18 |
| Tom1 (target of Myb-1) overexpression | DNA binding | Yamakami & Yokosawa, Biol Pharm Bull. 2004 Apr;27(4):564-6. |
| Thiazolidinedione MCC-555 | DNA binding | Kurebayashi et al., Atherosclerosis. 2005 Sep;182(1):71-7. Epub 2005 Mar 4. |
| Transdominant p50 | DNA binding | Logeat et al., EMBO J. 1991 Jul;10(7):1827-32. |
| Trichostatin A | RelA DNA binding | Hu & Colburn, 2005 |
| Triclosan plus cetylpyridinium chloride | DNA binding | Kim et al., J Periodontol. 2005 Oct;76(10):1735-42. |
| Triptolide (PG490, extract of Chinese herb) | DNA binding | Qiu et al., J Biol Chem. 1999 May 7;274(19): 13443-50; |
| | | Kim et al., Eur J Pharmacol. 2004 Jun 21;494(1):1-9.; |
| | | Yinjun et al., Leuk Res. 2005 Jan;29(1):99-105. |
| Tyrphostin AG-126 | DNA binding | Moore *et al*., 2003 |
| Ursolic acid | DNA binding | Hsu et al., Life Sci. 2004 Sep 24;75(19):2303-16. |
| Uteroglobin | DNA binding | Mandal et al., J Exp Med. 2004 May 17;199(10):1317-30. |
| V,C proteins (Sendai virus) | DNA binding | Komatsu et al., Virology. 2004 Jul 20;325(1):137-48. |
| Vascular endothelial growth factor (VEGF) | DNA binding | Oyama et al., J Immunol. 1998 Feb 1;160(3):1224-32.; |
| | | Gabrilovich et al., Blood. 1998 Dec 1;92(11):4150-66 |
| Verapamil | DNA binding | Li et al., Inflamm Res. 2006 Mar;55(3):108-13. |
| Withaferin A | DNA binding | Mohan et al., Angiogenesis 2004;7(2): 115-22. |
| Wogonin (5,7-dihydroxy-8-methoxyflavone) | DNA binding | Lee et al., FASEB J. 2003 Oct;17(13):1943-4. Epub 2003 Aug 1; |
| | | Piao et al., Arch Pharm Res. 2004 Sep;27(9):930-6. |
| Xanthohumol (a hops prenylflavonoid) | DNA binding | Colgate et al., Cancer Lett. 2006 Mar 22; [Epub ahead of print] |
| Xylitol | DNA binding | Han et al., Clin Diagn Lab Immunol. 2005 Nov;12(11);1285-91 |
| Yan-gan-wan | DNA binding | Yang et al., Hepatol Res. 2005 Aug;32(4):202-212. Epub 2005 Aug 16 |
| Yin-Chen-Hao | DNA binding | Cai et al., J Pharm Pharmacol. 2006 May;58(5):677-84. |
| Yucca schidigera extract | DNA binding | Marzocco et al., Life Sci. 2004 Aug 6;75(12):1491-501.; |
| | | Cheeke et al., J Inflamm (Lond). 2006 Mar 29;3:6. |
| Overexpressed ZIP1 | DNA binding | Khadeer et al., Bone. 2005 Sep;37(3):296-304. |
| Plant compound A (a phenyl aziridine precursor) | DNA binding and transactivation | De Bosscher et al., Proc Natl Acad Sci U S A. 2005 Nov 1;102(44): 15827-32. Epub 2005 Oct 21. |
| 8-acetoxy-5-hydroxyumbelliprenin (from Asafetida) | Transactivation | Appendino et al., J Nat Prod. 2006 Jul;69(7):1101-4. |
| AMP-activated protein kinase | Transactivation | Cacicedo et al., Biochem Biophys Res Commun. 2004 Nov 26;324(4):1204-9. |
| APC0576 | Transactivation | Yuzawa et al., Transplantation. 2003 May 15;75(9):1463-8. |
| Artemisia sylvatica sesquiterpene lactones | Transactivation (reporter assays) | Jin et al., Phytochemistry. 2004 Aug;65(15):2247-53. |
| Artemisolide | Transactivation | Reddy et al., Arch Pharm Res. 2006 Jul;29(7):591-7. |
| BSASM (plant extract mixture) | Transactivation (reporter assays) | Lee et al., J Ethnopharmacol. 2005 Jan 4;96(1-2):211-9. |
| Bifodobacteria | Transactivation | Riedel et al., World J Gastroenterol. 2006 Jun 21;12(23):3729-35. |
| Bupleurum fruticosum phenylpropanoids | Transactivation | Bremner et al., Planta Med. 2004 Oct;70(10):914-8. |
| Blueberry and berry mix (Optiberry) | Transactivation | Atalay et al., FEBS Lett. 2003 Jun 5;544(1-3):252-7 |
| BZLF1 (EBV protein) | Transactivation | Morrison et al., Virology. 2004 Oct 25;328(2):219-32 |
| Chromene derivatives | Transactivation | Cheng et al., Bioorg Med Chem Lett. 2003 Nov 3;13(21):3647-50. |
| D609 (phosphatidylcholine-phospholipase C inhibitor) | Transactivation | Bergmann et al., J Biol Chem. 1998 Mar 20;273(12):6607-10 |
| Dehydroevodiamine | Transactivation | Noh et al., Life Sci. 2006 Jul 10;79(7):695-701. Epub 2006 Mar 6 |
| 4'-demethyl-6-methoxypodophyllotoxin (lignan of Linum tauricum Willd. ssp. tauricum) | Transactivation | Vailev et al., Neoplasma. 2005;52(5):425-9. |
| Ethyl 2-[(3-methyl-2,5-dioxo(3-pyrrolinyl)) amino]-4-(trifluoromethyl) pyrimidine-5-carboxylate | Transactivation | Palanki et al., Bioorg Med Chem Lett. 2002 Sep 16;12(18):2573-7 |
| Cycloprodigiosin hycrochloride | Transactivation | Kamata et al., FEBS Lett. 2001 Oct 19;507(1):74-80. |
| Dimethylfumarate (DMF) | Nuclear translocation | Loewe et al., J Immunol. 2002 May 1;168(9):4781-7. |
| E1A (Adenovirus) | Transactivation | Cook et al., Proc Natl Acad Sci U S A. 2002 Jul 23;99(15):9966-71. Epub 2002 Jul 15. |
| Eckol/Dieckol (seaweed E stolonifera) | Transactivation | Joe et al., Biol Pharm Bull. 2006 Aug;29(8):1735-9. |
| Fructus Benincasae Recens extract | Transactivation | Kwon et al., Immunopharmacol Immunotoxicol. 2003 Nov;25(4):615-25. |
| Glucocorticoids (dexametasone, prednisone, methylprednisolone) | Transactivation and increases IkBa levels | Auphan et al., Science. 1995 Oct 13;270(5234):286-90; |
| | | Brostjan et al., J Biol Chem. 1996 Aug 9;271(32):19612-6; |
| | | Ray & Prefontaine, Proc Natl Acad Sci U S A. 1994 Jan 18;91(2):752-6; |
| | | Scheinman et al., Mol Cell Biol. 1995 Feb;15(2):943-53 |
| Gypenoside XLIX (from Gynostemma pentaphyllum) | Transactivation (PPAR-alpha-dependent) | Huang et al., J Biomed Sci. 2006 Jul;13(4):535-48. Epub 2006 Mar 10. |
| Histidine | Transactivation | Son et al., FEBS Lett. 2005 Aug 29;579(21):4671-7. |
| HIV-1 protease inhibitors (nelfinavir, ritonavir, or saquinavir) | Transactivation | Equils et al., Antimicrob Apents Chemother. 2004 Oct;48(10):3905-11. |
| Kwei Ling Ko (Tortoise shell-Rhizome jelly) | Transactivation | Yip et al., Phytomedicine. 2005 Nov; 12(10):748-59. |
| Ligusticum chuanxiong Hort root | Transactivation | Liu et al., Planta Med. 2005 Sep; 71(9):808-13. |
| Low gravity | Transactivation | Boonyaratanakornkit et al., FASEB J. 2005 Dec;19(14):2020-2. Epub 2005 Oct 6 |
| Nobiletin | Transactivation | Murakami et al., J Nutr. 2005 Dec; 135(12 Suppl):2987S-2992S |
| NRF (NF-κB repression factor) | Transactivation | Jianfeng et al., Mol Cells. 2003 Dec 31;16(3):397-401 |
| Paeonol (from Mountain Cortx) | Transactivation | Ishiguro et al., Toxicol Appl Pharmacol. 2006 Jul 14; [Epub ahead of print] |
| Phenethylisothiocyanate | Transactivation | Gerhauser et al., Mutat Res. 2003 Feb-Mar;523-524: 163-72. |
| 4-phenylcoumarins (from Marila pluricostata) | Transactivation | Bedoya et al., Bioorg Med Chem Lett. 2005 Oct 15;15(20):4447-50. |
| Phomol | Transactivation | Weber et al., J Antibiot (Tokyo). 2004 Sep;57(9):559-63. |
| PIAS3 | Transactivation | Jang et al., J Biol Chem. 2004 Jun 4;279(23):24873-80. Epub 2004 Mar 26. |
| Pranlukast | Transactivation | Ichiyama et al., Clin Exp Allergy. 2003 Jun;33(6):802-7; |
| | | Ishinaga et al., Pharmacology. 2005 Feb;73(2):89-96. Epub 2004 Oct 5. |
| Psychosine | Transactivation | Haq et al., J Neurochem. 2003 Sep;86(6): 1428-40. |
| Quinazolines | Transactivation | Tobe et al., Bioorg Med Chem. 2003 Sep 1;11(18):3869-78. |
| Resveratrol | RelA nuclear localization and transactivation | Manna et al., J Immunol. 2000 Jun 15;164(12):6509-19; |
| | | Pendurthi et al., Thromb Haemost. 2002 Jan;87(1):155-62. |
| RO31-8220 (PKC inhibitor) | Transactivation | Bergmann et al., J Biol Chem. 1998 Mar 20;273(12):6607-10. |
| Saucerneol D and saucerneol E | Transactivation | Hwang et al., Phytochemistry. 2003 Oct;64(3):765-71 |
| SB203580 (p38 MAPK inhibitor) | Transactivation | Bergmann et al., J Biol Chem. 1998 Mar 20;273(12):6607-10. |
| SH protein (Mumps virus) | Transactivation | Wilson et al., J Virol. 2006 Feb;80(4):1700-9. |
| Tranilast [N-(3,4-dimethoxycinnamoyl)anthranilic acid] | Transactivation | Spiecker et al., Mol Pharmacol. 2002 Oct;62(4):856-63. |
| 3,4,5-trimethoxy-4'-fluorochalcone | Transactivation | Rojas et al., Naunyn Schmiedebergs Arch Pharmacol. 2003 Sep;368(3):225-33. Epub 2003 Aug 2. |
| Uncaria tomentosum plant extract | Transactivation | Akesson et al., Int Immunopharmacol. 2003 Dec;3(13-14):1889-900. |
| LY294,002 | Transactivation | Sizemore et al., Mol Cell Biol. 1999 Jul;19(7):4798-805. |
| Mesalamine | RelA phosphorylation & transactivation | Egan et al., J Biol Chem. 1999 Sep 1 0;274(37):26448-53. |
| Mesuol | RelA phosphorylation & transactivation | Marquez et al., Antiviral Res. 2005 Jun;66(2-3):137-45. Epub 2005 Apr 20 |
| PTX-B (pertussis toxin binding protein) | RelA phosphorylation and transactivation | Iordanskiy et al., Virology. 2002 Oct 10;302(1):195-206 |
| 9-aminoacridine (9AA) derivatives (including the antimalaria drug quinacrine) | RelA phosphorylation and transactivation | Gurova et al., Proc Natl Acad Sci U S A. 2005 Nov 29;102(48): 17448-53. Epub 2005 Nov 15. |
| Adenosine and cyclic AMP | Transactivation | Majumdar & Aggarwal, Oncogene. 2003 Feb 27;22(8):1206-18.; Minguet et al., Eur J Immunol. 2005 Jan;35(1):31-41 |
| 17-allylamino-17-demethoxygeldanamycin | Transactivation | Rakitina et al., Cancer Res. 2003 Dec 15;63(24):8600-5 |
| 6-aminoquinazoline derivatives | Transactivation | Tobe et al., Bioorg Med Chem. 2003 Sep 1;11(18):3869-78 |
| Luteolin | p65 Transactivation | Kim et al., Biochem Pharmacol. 2003 Sep 15;66(6):955-63 |
| Manassantins A and B | p65 Transactivation | Lee et al., Biochem Pharmacol. 2003 Nov 15;66(10):1925-33; |
| | | Son et al., Mol Cells. 2005 Aug 31;20(1):105-11. |
| Paromyxovirus SH gene products | Transactivation | Wilson et al., J Virol. 2006 Feb;80(4):1700-9 |
| Qingkailing and Shuanghuanglian (Chinese medicinal preparations) | Transactivation | Chen et al., Life Sci. 2002 May 3;70(24):2897-913. |
| Smilax bockii warb extract (flavenoids) | Transactivation | Xu et al., Arch Pharm Res. 2005 Apr;28(4):395-9. |
| Tetracyclic A | Transactivation (ROS production) | Turbyville et al., Mol Cancer Ther. 2005 Oct;4(10): 1569-76. |
| Tetrathiomolybdate | Transactivation | Pan et al., Cancer Res. 2002 Sep 1;62(17):4854-9 |
| Trilinolein | Transactivation | Liu et al., Eur J Pharmacol. 2004 Jan 19;484(1):1-8. |
| Troglitazone | Transactivation | Ruan et al., J Biol Chem. 2003 Jul 25;278(30):28181-92. Epub 2003 May 5. |
| Valerenic acid/acetylvalerenolic acid | Transactivation | Jacobo-Herrera et al., Phytother Res. 2006 Oct;20(10):917-9. |
| Witheringia solanacea leaf extracts | Transactivation | Jacobo-Herrera et al., J Nat Prod. 2006 Mar;69(3):328-31 |
| Wortmannin (fungal metabolite) | Transactivation | Reddy et al., J Biol Chem. 1997 Nov 14;272(46):29167-73.; |
| | | Manna & Aggarwal, FEBS Lett. 2000 May 4;473(1):113-8. |
| Xia-Bai-San | Transactivation | Yeh et al., Int Immunopharmacol. 2006 Sep;6(9):1506-14. Epub 2006 Jun 2. |
| Alpha-zearalenol | Transactivation | Li et al., Biomed Environ Sci. 2005 Oct;18(5):314-20. |
| Antithrombin | RelA-p300 interaction | Uchiba et al., Thromb Haemost. 2004 Dec;92(6):1420-7. |
| Extract of the stem bark of Mangifera indica L. | NF-κB mRNA expression | Leiro et al., Int Immunopharmacol. 2004 Aug;4(8):991-1003 |
| Rifampicin | Glucocorticoid receptor modulation | Yerramesetti et al., J Clin Immunol. 2002 Jan;22(1):37-47. |
| Mangiferin | Inhibition of RelA and RelB expression | Leiro et al., Int Immunopharmacol. 2004 Jun;4(6):763-78 |

In specific embodiments, the NF-κB inhibitor is an inhibitor of IκB phosphorylation, such as BAY 11-7082 and BAY 11-7085 (BioMol, Plymouth Meeting, PA), curcumin and curcumin derivatives or analogs. Numerous curcumin derivatives and analogs are known in the art and may be used in the present invention (see, *e*.*g*., WO 2007/051314; US 2006/0276536). Such derivatives may have increased solubility or potency. Examples of curcumin derivatives include dimethoxy curcumin (Jeong et al., 2009. J. Clin. Biochem. Nutr. 44:79-84), 3,5-bis(2-fluorobenzylidene)-4-piperidone (EF24) (U.S. Pat. Appl. Pub. 20110059157), bis(arylmethylidene)acetone (WO 2007/000998), desmethoxy curcumin and bisdesmethoxy curcumin (WO 2006/117077). Other curcumin analogs that may be used include dihydrocurcumin, tetrahydrocurcumin, hexahydrocurcumin, dihydroxytetrahydrocurcumin, Yakuchinone A and Yakuchinone B, and their salts, oxidants, reductants, glycosides and esters thereof (U.S. Pat. Appl. Pub. 20030147979; U.S. Pat. No. 5,891,924). Further examples of curcumin analogs include but are not limited to (a) ferulic acid, (*e*.*g*., 4-hydroxy-3-methoxycinnamic acid; 3,4-methylenedioxy cinnamic acid; and 3,4-dimethoxycinnamic acid); (b) aromatic ketones (*e*.*g*., 4-(4-hydroxy-3-methoxyphenyl)-3-buten-2-one; zingerone; 4-(3,4-methylenedioxyphenyl)-2-butanone; 4-(p-hydroxyphenyl)-3-buten-2-one; 4-hydroxyvalerophenone; 4-liydroxybenzylactone; 4-hydroxybenzophenone; 1,5-bis(4-dimethylaminophenyl)-1,4-pentadien-3-one); (c) aromatic diketones (*e*.*g*., 6-hydroxydibenzoylmethane) (d) caffeic acid compounds (*e*.*g*., 3, 4-dihydroxycinnamic acid); (e) cinnamic acid; (i) aromatic carboxylic acids (*e*.*g*., 3,4-dihydroxyhydrocinnainic acid; 2-hydroxycinnamic acid; 3-hydroxycinnamic acid and 4-liydroxycinnamic acid); (g) aromatic ketocarboxylic acids (*e*.*g*., 4-hydroxyphenylpyruvic acid); and (h) aromatic alcohols (*e*.*g*., *4-*hydroxyphenethyl alcohol). These analogs and other representative analogs that can be used in the present invention are further described in WO 95/18606 and WO 01/040188. Other curcumin derivatives and analogs, including dimers, dextran and dendrimer conjugates, may also be used (see, *e.g.,* U.S. Pat. Appl. Pub. 20100240905; Shi, W., et al., 2007. Org. Lett. 9(26):5461-5464).

The curcumin or curcumin derivative or analog may be provided as a conjugate such as a prodrug. Examples of curcumin prodrugs are known (see, *e.g.,* Lu, P., et al., 2005. J Huazhong Univ Sci Technolog Med. Sci. 25(6):668-670, 678, Kapoor, N., et al. 2007. Cancer Lett. 248(2):245-250), and methods of making prodrugs are known (see, *e.g.,* WO 2006/076734; U.S. Pat. No. 5,952,294; Balant, L. P., et al., 1990. Eur. J. Drug Metab. Pharmacokinet. 15:143-153; Bundgaard, H., et al., 1991. Drugs of the Future 16:443-458). See also U.S. Published Patent Application U.S. 2007/0270464.

Desirably, the NF-κB inhibitor is non-toxic to the host with minimal or negligible side effects. Suitably, the inhibitor of NF-κB blocks the alternate NF-κB pathway, or both the classical and the alternate NF-κB pathways.

In some embodiments, the NF-κB inhibitor is in nucleic acid form, generally by operable linkage of a nucleotide sequence that encodes the inhibitor to a promoter, which may be constitutive or inducible, and which is operable in antigen-presenting cells of interest, to form a nucleic acid construct. Delivery of the nucleic acid constructs into antigen-presenting cells or their precursors may be achieved either by directly exposing a patient to the nucleic acid construct or by first transforming antigen-presenting cells or their precursors with the nucleic acid construct *in vitro,* and then transplanting the transformed antigen-presenting cells or precursors into the patient.

Considerations for introducing nucleic acid constructs into antigen-presenting cells and for producing variant nucleotide sequences as discussed in Section 3.2.1 for antigen-encoding nucleic acid constructs apply equally to nucleic acid constructs from which NF-κB inhibitor peptides/polypeptides are expressible.

### 3.2.4 mTOR inhibitors

Inhibition of the mTOR pathway is known to stimulate tolerogenicity in antigen-presenting cells (*e*.*g*., dendritic cells, B cells *etc*.) as decribed for example by Delgoffe and Powell (2009. Immunology 127(4):459-465) and Fischer et al. (2009, Handb Exp Pharmacol. 188:215-232). Accordingly, the present invention contemplates the use of inhibitors of the mTOR pathway, together with an antigenic molecule selected from an antigen that corresponds in whole, or in part, to an aggrecan polypeptide (*e*.*g*., a *cit*-aggrecan polypeptide) or a nucleic acid molecule from which the antigen is expressible, for suppressing the immune response to an aggrecan polypeptide, in the treatment or prevention of joint damage in a subject.

As used herein, mTOR inhibitors include any molecule or compound that reduces the level or functional activity of mTOR in immune cells, especially antigen-presenting cells. mTOR inhibitors can take various forms, non-limiting examples of which include small molecules, nucleic acids, peptides, polypeptides, peptidomimetics *etc.*

In some embodiments, the mTOR inhibitor is selected from rapamycin, which is a known macrolide antibiotic produced by *Streptomyces hygroscopicus,* and rapamycin derivatives, *e*.*g*., rapamycin substituted in position 40 and/or 16 and/or 32, for example a compound of formula (I): 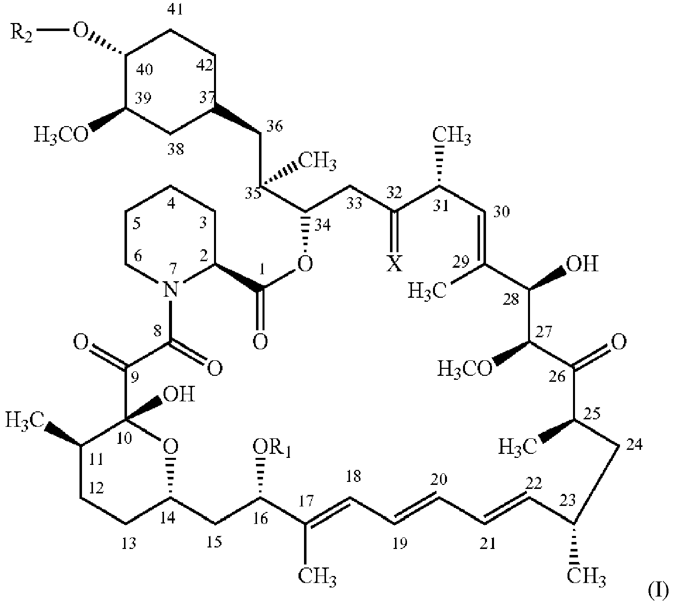
wherein R₁ is CH₃ or C₃₋₆alkynyl,
R₂ is H, -CH₂-CH₂-OH, or -CH₂-CH₂-O-CH₂-CH₃.

3-hydroxy-2-(hydroxymethyl)-2-methyl-propanoyl or tetrazolyl, e.g. tetrazol-1-yl, and X is =O, (H, H) or (H, OH), provided that R₂ is other than H when X is =O and R₁ is CH₃.

When R₂ in a compound of formula I is -CH₂-CH₂-OH, a compound of formula I includes a physiologically hydrolysable ether thereof, for instance -CH₂-CH₂-O-C₁₋₈)alkyl.

Representative examples of compounds of formula I include e.g. 40-O-(2-hydroxy)ethyl-rapamycin (also known as everolimus), 32-deoxorapamycin, 16-O-substituted rapamycins such as 16-pent-2-ynyloxy-32-deoxorapamycin, 16-pent-2-ynyloxy-32 (S or R)-dihydrorapamycin, 16-pent-2-ynyloxy-32 (S or R)-dihydro-40-0-(2-hydroxyethyl)-rapamycin, 40-[3-hydroxy-2-(hydroxy-methyl)-2-methylpropanoate]-rapamycin (also known as CCI779), 40-epi-(tetrazolyl)-rapamycin (also known as ABT578), or 40-O-ethoxyethyl-rapamycin (also known as biolimus 9).

mTOR inhibitors also include the so-called rapalogs, *e.g.,* as disclosed in WO9802441, WO0114387 and WO0364383 such as AP23573, *e*.*g*., 40-O-(dimethylphosphinoyl)-rapamycin, compounds as disclosed disclosed in WO2005047295 in Example 1, also designated as biolimus A9 and compounds disclosed under the name TAFA-93. Other mTOR inhibitors are *e*.*g*., disclosed in WO2004101583, WO9205179, WO9402136, WO9402385, WO9613273. their entirety.

In some embodiments, mTOR inhibitors include rapamycin, a compound of formula I, *e*.*g*., and including a rapalog, TAFA-93, more preferably rapamycin, a compound of formula I or a rapalog,

In specific embodiments, the mTOR inhibitor is 40-O-(2-hydroxyethyl)-rapamycin disclosed in Example 8 in WO9409010.

In other embodiments, the mTOR inhibitor is 32-deoxorapamycin or 16-pent-2-ynyloxy-32 (S)-dihydro-rapamycin as disclosed in WO9641807, *e*.*g*., or a compound as disclosed in WO9516691.

Exemplary mTOR inhibitors include: rapamycin, and/or 40-O-(2-hydroxyethyl)-rapamycin, and/or 32-deoxorapamycin, and/or 16-pent-2-ynyloxy-32-deoxorapamycin, and/or 16-pent-2-ynyloxy-32 (S or R)-dihydro-rapamycin, and/or 16-pent-2-ynyloxy-32 (S or R)-dihydro-40-0-(2-hydroxyethyl)-rapamycin, and/or 40-[3-hydroxy-2-(hydroxy-methyl)-2-methylpropanoate]-rapamycin (also known as CCI779) and/or 40-epi-(tetrazolyl)-rapamycin (also known as ABT578), and/or AP23573, and/or biolimus A9, *e*.*g*., and/or compounds disclosed under the name TAFA-93, such as 40-O-(2-hydroxyethyl)-rapamycin, and/or 32-deoxorapamycin, and/or CCI779, and/or ABT578, and/or AP23573.

In other embodimenbts, mTOR inhibitors are selected from pyrazolopyrimidine derivatives, including but not limited to compounds having a structure of the following formula (II) and formula (III).

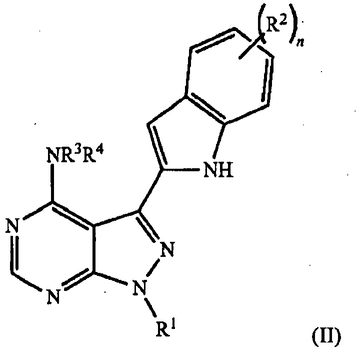

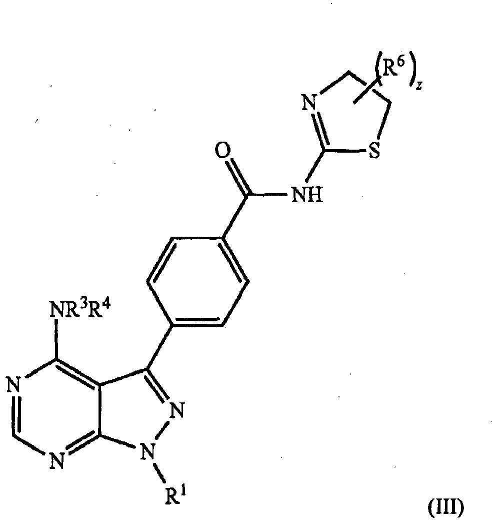

In some embodiments of the compounds of formulae (II) or (III), R¹, R³, and R⁴ are independently hydrogen, halogen, CN, -CF₃, -OH, -NH₂, -SO₂, -COOH, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In some embodiments, at least one of R³ or R⁴ is hydrogen. In some embodiments, R¹, R³, and R⁴ are independently hydrogen or substituted or unsubstituted C₁-C₁₀ alkyl (e.g. C₁-C₅ alkyl or C₁-C₃ alkyl). R', R³, and R⁴ may also independently be hydrogen or unsubstituted C₁-C₁₀ alkyl (e.g. C₁-C₅ alkyl or C₁-C₃ alkyl).

In some embodiments of formulae (II) or (III), R¹, R³, and R⁴ are independently hydrogen, halogen, -CN, -CF₃, -OH, -NH₂, -SO₂, -COOH, R⁷-substituted or unsubstituted alkyl, R⁷-substituted or unsubstituted heteroalkyl, R⁷-substituted or unsubstituted cycloalkyl, R⁷-substituted or unsubstituted heterocycloalkyl, R⁷-substituted or unsubstituted aryl, or R⁷-substituted or unsubstituted heteroaryl. R⁷ is independently oxo, halogen, -CN, -CF₃, -OH, -NH₂, -SO₂, -COOH, R⁸-substituted or unsubstituted alkyl, R⁸-substituted or unsubstituted heteroalkyl, R⁸-substituted or unsubstituted cycloalkyl, R⁸-substituted or unsubstituted heterocycloalkyl, R⁸-substituted or unsubstituted aryl, or R⁸-substituted or unsubstituted heteroaryl. R⁸ is independently halogen, oxo, -CN, -CF₃, -OH, -NH₂, -SO₂, -COOH, unsubstituted alkyl, unsubstituted heteroalkyl, unsubstituted cycloalkyl, unsubstituted heterocycloalkyl, unsubstituted aryl or unsubstituted heteroaryl. In some embodiments, R¹ is substituted or unsubstituted alkyl or substituted or unsubstituted heterocycloalkyl (e.g. morpholino). In some embodiments, R¹ is substituted with -C(O)R^{8A}, wherein R^{8A} is unsubstituted alkyl.

In some embodiments of formula (II), R² is independently hydrogen, halogen, - CN, -CF₃, -OR⁵, -NH₂, -SO₂, -COOH, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In some embodiments, R² is independently hydrogen, -OR⁵, -CN, -NH₂, -SH, -CN; -CF₃, NO₂, or substituted or unsubstituted alkyl. R⁵ is independently hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In some embodiments, R⁵ is hydrogen or substituted or unsubstituted alkyl (e.g. unsubstituted C₁-C₅ alkyl).

R² may independently be hydrogen, halogen, -OR⁵, -CN, -NH₂, -SH, -CN, -CF₃, -NO₂, R⁹-substituted or unsubstituted alkyl, R⁹-substituted or unsubstituted heteroalkyl, R⁹-substituted or unsubstituted cycloalkyl, R⁹-substituted or unsubstituted heterocycloalkyl, R⁹-substituted or unsubstituted aryl, or R⁹-substituted or unsubstituted heteroaryl. R⁹ is independently halogen, oxo, - CN, -CF₃, -OH, -NH₂, -SO₂, -COOH, R¹⁰-substituted or unsubstituted alkyl, R¹⁰-substituted or unsubstituted heteroalkyl, R¹⁰-substituted or unsubstituted cycloalkyl, R¹⁰-substituted or unsubstituted heterocycloalkyl, R¹⁰-substituted or unsubstituted aryl, or R¹⁰-substituted or unsubstituted heteroaryl. R¹⁰ is independently halogen, oxo, -CN, -CF₃, -OH, -NH₂, -SO₂, -COOH, unsubstituted alkyl, unsubstituted heteroalkyl, unsubstituted cycloalkyl, unsubstituted heterocycloalkyl, unsubstituted aryl or unsubstituted heteroaryl. In other embodiments, R² is -OR⁵. In some related embodiments, R⁵ is hydrogen or unsubstituted C₁-C₅ alkyl (e.g. hydrogen).

In some embodiments of formulae (II) or (III), R⁵ is independently hydrogen, R¹¹-substituted or unsubstituted alkyl, R¹¹-substituted or unsubstituted heteroalkyl, R¹¹-substituted or unsubstituted cycloalkyl, R¹¹-substituted or unsubstituted heterocycloalkyl, R¹¹-substituted or unsubstituted aryl, or R¹¹-substituted or unsubstituted heteroaryl. R¹¹ is independently halogen, oxo, - CN, -CF₃, -OH, -NH₂, -SO₂, -COOH, R¹²-substituted or unsubstituted alkyl, R¹²-substituted or unsubstituted heteroalkyl, R¹²-substituted or unsubstituted cycloalkyl, R¹²-substituted or unsubstituted heterocycloalkyl, R¹²-substituted or unsubstituted aryl, or R¹²-substituted or unsubstituted heteroaryl. R¹² is independently halogen, oxo, -CN, -CF₃, -OH, -NH₂, -SO₂, -COOH, unsubstituted alkyl, unsubstituted heteroalkyl, unsubstituted cycloalkyl, unsubstituted heterocycloalkyl, unsubstituted aryl or unsubstituted heteroaryl.

In some embodiments of formula (III), R⁶ is independently hydrogen, halogen, - CN, -CF₃, -OR⁵, -NH₂, -SO₂, -COOH, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. R⁶ may also independently be hydrogen, -OR⁵, -CN, halogen, -NH₂, -SH, -CN, -CF₃, -NO₂, halogen, or substituted or unsubstituted alkyl (e.g. unsubstituted C₁-C₅ alkyl). R⁵ is as defined above in the description of Formula (I). In some embodiments, R⁶ is independently hydrogen, -OR⁵, -CN, -NH₂, -SH, -CN, - CF₃, -NO₂, R¹³-substituted or unsubstituted alkyl, R¹³-substituted or unsubstituted heteroalkyl, R¹³-substituted or unsubstituted cycloalkyl, R¹³-substituted or unsubstituted heterocycloalkyl, R¹³-substituted or unsubstituted aryl, or R¹³-substituted or unsubstituted heteroaryl. R¹³ is independently halogen, oxo, -CN, -CF₃, -OH, -NH₂, -SO₂, -COOH, R¹⁴-substituted or unsubstituted alkyl, R¹³-substituted or unsubstituted heteroalkyl, R¹⁴-substituted or unsubstituted cycloalkyl, R¹⁴-substituted or unsubstituted heterocycloalkyl, R¹⁴-substituted or unsubstituted aryl, or R¹⁴-substituted or unsubstituted heteroaryl. R¹⁴ is independently halogen, oxo, -CN, -CF₃, -OH, -NH₂, -SO₂, -COOH, unsubstituted alkyl, unsubstituted heteroalkyl, unsubstituted cycloalkyl, unsubstituted heterocycloalkyl, unsubstituted aryl or unsubstituted heteroaryl. R⁶ may also independently be hydrogen, -OR⁵, -CN, halogen, -NH₂, -SH, -CN, -CF₃, -NO₂, halogen, or unsubstituted C₁-C₅ alkyl. In some embodiments, R⁶ is hydrogen.

**In** some embodiments of formulae (II) or (III), R³ and R⁴ are hydrogen. In some embodiments of formula (II), n is 1 or 2. In some related embodiments of formula (II), n is 1. In other related embodiments, R² is -OR⁵ and n is 1. In still other related embodiments, R⁵ is hydrogen. In some embodiments of formula (III), z is an integer from 1 to 2. In some embodiments, z is 1.

In some embodiments, R¹, R², R³, R⁴, R⁵, R⁶, R⁷, R⁸, R⁹, R¹⁰, R¹¹, R¹², R¹³ and/or R¹⁴ are size-limited substituents. In some embodiments, R¹, R², R³, R⁴, R⁵, R⁶, R⁷, R⁸, R⁹, R¹⁰, R¹¹, R¹², R¹³ and/or R¹⁴ are C₁-C₁₀, C₁-C₅ alkyl or C₁-C₃ alkyl, for example methyl, ethyl, propyl, isopropyl, butyl and the like, optionally substituted as described herein. In some embodiments, R¹, R², R³, R⁴, R⁵, R⁶, R⁷, R⁸, R⁹, R¹⁰, R¹¹, R¹², R¹³ and/or R¹⁴ are 2-10 membered, 2-5 membered, or 2-3-membered heteroalkyl, optionally substituted as described herein. In some embodiments, R¹, R², R³, R⁴, R⁵, R⁶, R⁷, R⁸, R⁹, R¹⁰, R¹¹, R¹², R¹³ and/or R¹⁴ are C₃-C₁₀, C₃-C₈, C₃-C₆ or C₃-C₅ cycloalkyl, including but not limited to cyclopropyl, cyclobutyl, cyclopentyl, cyclohexyl, cycloheptyl, cyclooctyl and the like, optionally substituted as described herein. In some embodiments, R¹, R², R³, R⁴, R⁵, R⁶, R⁷, R⁸, R⁹, R¹⁰, R¹¹, R¹², R¹³ and/or R¹⁴ are 3-membered, 4-membered, 5-membered, 6-membered, 7-membered, 8-membered, 9-membered or 10-membered heterocycloalkyl, including but not limited to aziridine, oxirane, thiirane, azetidine, oxetane, thietane, pyrrolidine, dihydrofuran, tetrahydropyran, dihydrothiophene, tetrahydrothiophene, piperidine, dihydropyran, tetrahydropyran, dihydrothiopyran, tetrahydrothiopyran, optionally substituted as described herein. In some embodiments, R¹, R², R³, R⁴, R⁵, R⁶, R⁷, R⁸, R⁹, R¹⁰, R¹¹, R¹², R¹³ and/or R¹⁴ are C₆-C₁₀ aryl, including but not limited to phenyl or naphthyl, optionally substituted as described herein. In some embodiments, R¹, R², R³, R⁴, R⁵, R⁶, R⁷, R⁸, R⁹, R¹⁰, R¹¹, R¹², R¹³ and/or R¹⁴ are 5-10-membered, 5-6-membered heteroaryl as described herein, optionally substituted as described herein.

In other embodiments, the biologically active agent (e.g. the compounds of formulae (II) or (III)) is selected from the following compounds:

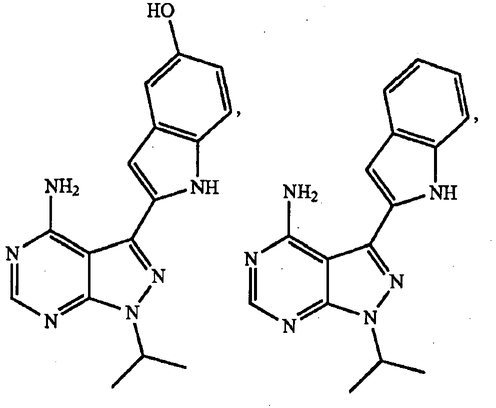

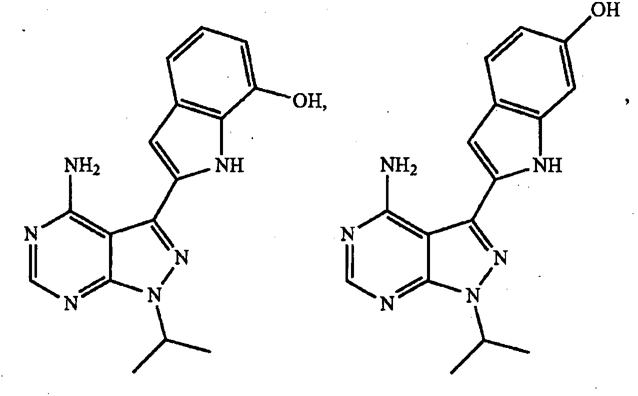

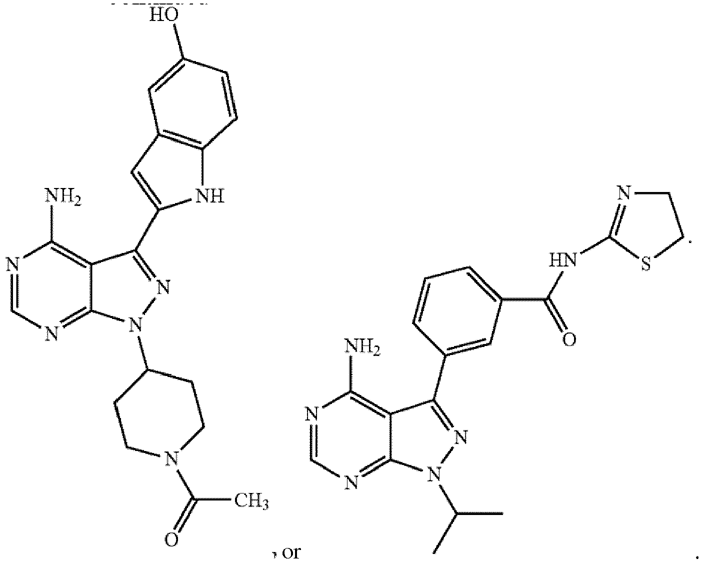

### 3.2.5 Syk inhibitors

Alternatively, or in addition, tolerogenicity in antigen-presenting cells (*e*.*g*., dendritic cells, B cells *etc*.) can be stimulated by inhibiting the Syk pathway, as decribed for example by Colonna et al. (2010. J Immunol. 185(3):1532-43), Matsubara et al. (2006. Am J Respir Cell Mol Biol. 34(4):426-433) and Nakashima et al. (2004. Eur J Pharmacol. 505(1-3):223-228). Thus, the present invention also contemplates the use of Syk inhibitors, in combination with an antigenic molecule selected from an antigen that corresponds in whole, or in part, to an aggrecan polypeptide (*e*.*g*., a *cit*-aggrecan polypeptide) or a nucleic acid molecule from which the antigen is expressible, for suppressing the immune response to an aggrecan polypeptide, and for treating or preventing joint damage in a subject.

As used herein, Syk pathway inhibitors include any molecule or compound that reduces signaling through the Syk pathway or that reduces the level or functional activity of Syk in immune cells, especially antigen-presenting cells. Syk inhibitors can take various forms, non-limiting examples of which include small molecules, nucleic acids, peptides, polypeptides, peptidomimetics *etc.*

In some embodiments, the Syk inhibitor is selected from compounds disclosed in U.S. Pat. No. 6,432,963. Exemplary compounds for example are encompassed by the definition set out between column 3, line 45 to column 6, line 22, and in particular a compound selected from the group consisting of 2-(2-aminoethylamino)-4-(3-methylanilino)pyrimidine-5-carboxamide, 2-(2-aminoethylamino)-4-(3-trifluoromethylanilino)pyrimidine-5-carboxamid-e, 2-(4-aminobutylamino)-4-(3-trifluoromethylanilino)pyrimidine-5-carboxam-ide, 2-(2-aminoethylamino)-4-(3-bromoanilino)pyrimidine-5-carboxamide, 2-(2-aminoethylamino)-4-(3-nitroanilino)pyrimidine-5-carboxamide, 2-(2-aminoethylamino)-4-(3,5-dimethylanilino)pyrimidine-5-carboxamide, 2-(2-aminoethylamino)-4-(2-naphthylamino)pyrimidine-5-carboxamide, 2-(cis-2-aminocyclohexylamino)-4-(3-methylanilino)pyrimidine-5-carboxamid-e, 2-(cis-2-aminocyclohexylamino)-4-(3-bromo-anilino)pyrimidine-5-carboxam-ide, 2-(cis-2-aminocyclohexylamino)-4-(3,5-dichloroanilino)pyrimidine-5-carboxamide and 2-(cis-2-aminocyclohexylamino)-4-(3,4,5-trimethoxyanilino)pyrimidine-5-carboxamide or a salt thereof. Methods for the synthesis of such compounds are set forth between column 6, line 43 to column 13, line 17.

Syk inhibitors, as employed in the present invention, also include compounds disclosed in U.S. Patent Application Publication No. US2004/0029902; including compounds encompassed by the definition set out between paragraphs [0109] and [0218], and in particular a compound selected from the group consisting of N2,N4-[(2,2-Dimethyl-4H-benzo[1,4]oxazin-3-one)-6-yl]-5-fluoro-2,4-pyrimi-dinediamine, N4-(3,4-Dichlorophenyl)-5-fluoro-N2-(indazoline-6-yl)-2,4-pyrimidinediami-ne, N4-(3,4-Ethylenedioxyphenyl)-5-fluoro-N2-(1-methyl-indazoline-5-yl)-2,4-pyrimidinediamine, N2,N4-Bis(3-hydroxyphenyl)-5-fluoro-2,4-pyrimidinediamine, N2,N4-Bis(3,4-ethylenedioxyphenyl)-5-fluoro-2,4-pyrimidinediamine, N4-(1,4-Benzoxazin-6-yl)-5-fluoro-N2-[3-(N-methylamino)carbonylme-thyleneoxyphenyl]-2,4-pyrimidinediamine, N2,N4-Bis(3-aminophenyl)-5-fluoro-2,4-pyrimidinediamine, N4-(3,4-Ethylenedioxyphenyl)-5-fluoro-N2-[3-(N-methylamino)-carbonylmethy-leneoxyphenyl]-2,4-pyrimidinediamine, 5-Fluoro-N4-(3-hydroxyphenyl)-N2-[3-(N-methylamino)carbonylmethyleneoxyph-enyl]-2,4-pyrimidinediamine, N4-(3-Hydroxyphenyl)-5-trifluoromethyl-N2-[3-(N-methylamino)carbonylmethy-leneoxyphenyl]-2,4-pyrimidinediamine, 5-Fluoro-N4-[(1H)-indol-6-yl]-N2-[3-(N-methylamino)carbonylmethyleneoxyph-enyl]-2,4-pyrimidinediamine, 5-Fluoro-N4-(3-hydroxyphenyl)-N2-[3-(N-methylamino)carbonylmethyleneoxyph-enyl]-2,4-pyrimidinediamine, 5-Fluoro-N2-(3-methylaminocarbonylmethyleneoxyphenyl)-N4-[2-H-pyrido[3,2-b]-1,4-oxazin-3(4H)-one-6-yl]-2,4-pyrimidinediamine, N4-(3,4-Ethylenedioxyphenyl)-5-fluoro-N2-[3-(2-hydroxyethyl-amino)carbonylmethyleneoxyphenyl]-2,4-pyrimidinediamine, 5-Fluoro-N4-(3-hydroxyphenyl)-N2-[3-(N-methylamino)carbonylmethyleneoxyph-enyl]-2,4-pyrimidinediamine, N2,N4-Bis(indol-6-yl)-5-fluoro-2,4-pyrimidinediamine, 5-Fluoro-N2-[2-(2-hydroxy-1,1-dimethylethylamino)carbonylbenzofuran-5-yl]--N4-(3-hydroxyphenyl)-2,4-pyrimidinediamine, N2-[3-(N2,3-Dihydroxypropylamino)carbonylmethyleneoxyphenyl]-N4-(3,4-ethy-lenedioxyphenyl)-5-fluoro-2,4-pyrimidinediamine, N2-(3,5-Dimethoxyphenyl)-N4-(3,4-ethylenedioxyphenyl)-5-fluoro-2,4-pyrimi-dinediamine, N4-(3,4-Ethylenedioxyphenyl)-5-fluoro-N2-[3-(1,3-oxazol-5-yl)phenyl]-2,4-pyrimidinediamine, N4-(3,4-Ethylenedioxyphenyl)-5-fluoro-N2-[3-(N-methylamino)-carbonylmethy- leneoxyphenyl]-2,4-pyrimidinediamine, 5-Fluoro-N2-(3-hydroxyphenyl)-N4-[4-(3-phenyl-1,2-4-oxadiazol-5-yl)methyl-eneoxyphenyl]-2,4-pyrimidinediamine, N4-(3,4-Ethylenedioxyphenyl)-5-fluoro-N2-(indazolin-6-yl)-2,4-pyrimidined-iamine, 5-Fluoro-N4-(3-hydroxyphenyl)-N2-(indazolin-6-yl)-2,4-pyrimidinediamine, N4-(3,4-Ethylenedioxyphenyl)-5-fluoro-N2-(1-methyl-indazoline-5-y-l)-2,4-pyrimidinediamine, 5-Fluoro-N4-(3-hydroxyphenyl)-N2-(1-methy-indazoline-5-yl)-2,4-pyrimidine-diamine, N4-(3,4-Ethylenedioxyphenyl)-5-fluoro-N2-[4-(3-phenyl-1,2,4-oxadiazol-5-y-l)methyleneoxyphenyl]-2,4-pyrimidinediamine, N4-(3,5-Dimethyl-4-hydroxyphenyl)-5-fluoro-N2-[3-[2-(N-morpholino)ethylen-eoxy]phenyl]-2,4-pyrimidinediamine, N4-(3,5-Dimethyl-4-hydroxypheny))-5-fluoro-N2-[3-[2-(N-morpholino)ethylox-y]phenyl]-2,4-pyrimidinediamine, N4-(3-Chloro-4-hydroxy-5-methylphenyl)-5-fluoro-N2-[3-[2-(N-morpholino)ethyloxy]phenyl]-2,4-pyrimidinediamine, N2-(3-tert-Butylcarbonylaminophenyl)-N4-(3-hydroxyphenyl)-5-fluoro-2,4-py-rimidinediamine, N4-(3-tert-Butylphenyl)-N2-[3-(N-methylamino)carbonylmethyleneoxyphenyl]-5-fluoro-2,4-pyrimidinediamine, N4-(3-tert-Butylphenyl)-N2-[3-(N2,3-dihydroxypropylamino)carbonylmethylen- eoxyphenyl]-5-fluoro-2,4-pyrimidinediamine, N2-[3-(N2,3-Dihydroxypropylamino)carbonylmethyleneoxyphenyl]-5-fluoro-N4-(3-isopropylphenyl)-2,4-pyrimidinediamine, N4-[4-(Cyanomethyleneoxy)phenyl]-5-fluoro-N2-(3-hydroxyphenyl)-2,4-pyrimi- dinediamine, N4-(3,5-Dimethyl-4-hydroxyphenyl)-5-fluoro-N2-[3-(N-piperazino)carbonylme- thyleneoxyphenyl]-2,4-pyrimidinediamine, N4-(3,5-Dimethyl-4-hydroxyphenyl)-5-fluoro-N2-[3-[2-(N-piperazino)ethoxy]-phenyl]-2,4-pyrimidine-diamine bis hydrogenchloride salt, N4-(3,4-Ethylenedioxyphenyl)-5-fluoro-N2-[4-(2-hydroxyethyloxy)phenyl]-2,-4-pyrimidinediamine, N4-(1,4-Benzoxazine-3-on-6-yl)-5-fluoro-N2-(3-hydroxyphenyl)-2,4-pyrimidinediamine, (+/-)-5-Fluoro-N2-[(N-methylacetamido-2)-3-phenoxy]-N4-(2-methyl-1,4-benz- oxazin-6-yl)-2,4-pyrimidinediamine, N2-(1,4-Benzoxazin-3-on-6-yl)-5-fluoro-N4-(3-hydroxyphenyl)-2,4-pyrimidin- ediamine, N4-(3-Chloro-4-trifluoromethoxyphenyl)-5-fluoro-N2-[3-(N-methylamino)carbonylmethyleneoxyphenyl]-2,4-pyrimidinediamine, 5-Fluoro-N4-(3-hydroxy-4-methylphenyl)-N2-[3-[(N-methylamino)carbonylmeth-yleneoxy]phenyl]-2,4-pyrimidinediamine, 5-Fluoro-N4-(3-hydroxyphenyl)-N2-[4-methyl-3-[(N-methylamino)carbonylmeth-yleneoxy]phenyl]-2,4-pyrimidinediamine, 5-Fluoro-N4-(3-hydroxy-4-methoxyphenyl)-N2-[3-(N-methylamino)carbonylmeth- yleneoxyphenyl]-2,4-pyrimidinediamine, N4-(3-Chloro-4-methylphenyl)-5-fluoro-N2-[3-(N-methylamino)-carbonylmethy-leneoxyphenyl]-2,4-pyrimidinediamine, N4-(3-Chloro-4-methoxyphenyl)-5-fluoro-N2-[3-[(N-methylamino)carbonylmethyleneoxy]phenyl]-2,4-pyrimidinediamine, 5-Fluoro-N4-1(1H)-indol-5-yl]-N2-[3-[(N-methylamino)carbonylmethyleneoxy]- phenyl]-2,4-pyrimidinediamine, 5-Fluoro-N4-(3-hydroxyphenyl)-N2-[1-(methoxycarbonyl)methyl-indazoline-5-yl]-2,4-pyrimidinediamine, 5-Fluoro-N4-(3-hydroxyphenyl)-N2-[1-(3-hydroxypropyl)indazoline-6-yl]-2,4--pyrimidinediamine, N4-(3,4-Ethylenedioxyphenyl)-5-fluoro-N2-[1-(3-hydroxypropyl)indazoline-5--yl]-2,4-pyrimidinediamine, 5-Fluoro-N4-(3-hydroxyphenyl)-N2-[1-(3-hydroxypropyl)indazoline-5-yl]-2,4--pyrimidinediamine, 5-Fluoro-N2-[1-(3-hydroxypropyl)indazoline-5-yl]-N4-(4-isopropoxyphenyl)-2,4-pyrimidinediamine, N4-(3,4-Ethylenedioxyphenyl)-5-fluoro-N2-[1-[2(N-methylaminocarbonyl)ethy-1]-indazoline-5-yl]-2,4-pyrimidinediamine, 5-Fluoro-N4-(4-isopropoxyphenyl)-N2-[1-[2(N-methylaminocarbonyl)ethyl]-in-dazoline-5-yl]-2,4-pyrimidinediamine, N4-[(2,2-dimethyl-4H-benzo[1,4]oxazin-3-one)-6-yl]-5-fluoro-N2-[3-(methyl-aminocarbonylmethylene-oxy)phenyl]-2,4-pyrimidinediamine, N4-[(2,2-Dimethyl-4H-benzo[1,4]oxazin-3-one)-6-yl]-5-fluoro-N2-(1-methyli-ndazolin-5-yl)-2,4-pyrimidinediamine, N4-[(2,2-Difluoro-4H-benzo[1,4]oxazin-3-one)-6-yl]-5-fluoro-N2-[3-(methylaminocarbonylmethyleneoxy)phenyl]-2,4-pyrimidinediamine, N4-1(2,2-Dimethyl-4H-5-pyridol-1,4]oxazin-3-one)-6-yl]-5-fluoro-N2-[3-(methyl-aminocarbonyl-methyleneoxy)phenyl]-2,4-pyrimidinediamine, 5-Fluoro-N2-(3-methylaminocarbonylmethyleneoxyphenyl)-N4-[2H-pyrido[3,2-b-]-1,4-oxazin-3(4H)-one-6-yl]-2,4-pyrimidinediamine, N4-(4-Amino-3,4-dihydro-2H-1-benzopyran-6-yl)-5-fluoro-N2-[3-(N-methylami-no)carbonylmethyleneoxyphenyl]-2,4-pyrimidinediamine, N4-(3-Chloro-4-hydroxy-5-methylphenyl)-5-fluoro-N2-[3-[2-(N-piperazino)et-hoxy]phenyl]-2,4-pyrimidinediamine, and N4-(3-Methylcarbonyloximephenyl)-5-fluoro-N2-[3-(N-methylamino)carbonylme-thyleneoxyphenyl]-2,4-pyrimidinediamine or a salt thereof. Such compounds can be synthesized for example in by methods set out between paragraphs [0218] and [0260] of U.S. Pat. Appl. Pub. No. 2004/0029902.

In other embodiments, Syk inhibitors are sepected from compounds described in U.S. Pat. Appl. Pub. No. 2010/0316649. Representative compounds of this type are represented by the following formulae Iz and Ib.

Compounds according to formula Iz are as set out below: 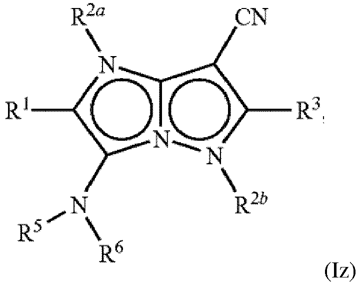 wherein
R¹ is selected from aryl, substituted aryl, heteroaryl, substituted heteroaryl, cycloalkyl, substituted cycloalkyl, cycloalkenyl, substituted cycloalkenyl, heterocyclyl, substituted heterocyclyl, aralkyl, heteroaralkyl, hydrogen, alkyl, substituted alkyl, alkenyl, substituted alkenyl, alkynyl, substituted alkynyl, acyl, acylamino, and acyloxy;
R^{2a} and R^{2b} are independently selected from hydrogen, alkyl, substituted alkyl, acyl, acylamino, acyloxy, -SO-alkyl, -SO-aryl, -SO-heteroaryl, -SO₂-alkyl, -SO₂-aryl, -SO₂-heteroaryl, aryl, substituted aryl, heteroaryl, heterocyclyl, aralkyl, and heteroaralkyl; and wherein either R^{2a} or R^{2b} is present;
R³ is selected from hydrogen, alkyl, substituted alkyl, alkenyl, substituted alkenyl, halo, nitro, cyano, hydroxy, alkoxy, carboxyl, acyl, acylamino, aminoacyl, acyloxy, oxyacyl, amino, substituted amino, aryl, substituted aryl, heteroaryl, and substituted heteroaryl;
R⁵ is selected from hydrogen, alkyl, and substituted alkyl; and
R⁶ is selected from hydrogen, alkyl, substituted alkyl, alkenyl, substituted alkenyl, alkynyl, substituted alkynyl, acyl, acylamino, acyloxy, cycloalkyl, substituted cycloalkyl, cycloalkenyl, substituted cycloalkenyl, aralkyl, heteroaralkyl, aryl, substituted aryl, heteroaryl, substituted heteroaryl, heterocyclyl, and substituted heterocyclyl;
or a salt or stereoisomer thereof.

Compounds according to formula Ib are shown below: 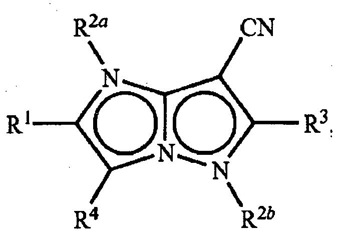 wherein
R¹ is selected from aryl, substituted aryl, heteroaryl, substituted heteroaryl, cycloalkyl, substituted cycloalkyl, cycloalkenyl, substituted cycloalkenyl, heterocyclyl, substituted heterocyclyl, aralkyl, heteroaralkyl, hydrogen, alkyl, substituted alkyl, alkenyl, substituted alkenyl, alkynyl, substituted alkynyl, acyl, acylamino, and acyloxy;
R^{2a} and R^{2b} are independently selected from hydrogen, alkyl, substituted alkyl, acyl, acylamino, acyloxy, -SO-alkyl, -SO-aryl, -SO-heteroaryl, -SO₂-alkyl, -SO₂-aryl, -SO₂-heteroaryl, aryl, substituted aryl, heteroaryl, heterocyclyl, aralkyl, and heteroaralkyl, and wherein either R^{2a} or R^{2b} is present;
R³ is selected from hydrogen, alkyl, substituted alkyl, alkenyl, substituted alkenyl, halo, nitro, cyano, hydroxy, alkoxy, carboxyl, acyl, acylamino, aminoacyl, acyloxy, oxyacyl, amino, substituted amino, aryl, substituted aryl, heteroaryl, and substituted heteroaryl;
R⁴ is selected from hydrogen, alkyl, substituted alkyl, amino, or -NR⁵R⁶;
R⁵ is selected from hydrogen, alkyl, and substituted alkyl; and
R⁶ is selected from aryl, substituted aryl, heteroaryl, substituted heteroaryl, cycloalkyl, substituted cycloalkyl, cycloalkenyl, substituted cycloalkenyl, heterocyclyl, substituted heterocyclyl, aralkyl, heteroaralkyl, hydrogen, alkyl, substituted alkyl, alkenyl, substituted alkenyl, alkynyl, substituted alkynyl, acyl, acylamino, and acyloxy;
or a salt or stereoisomer thereof.

Illustrative examples of compounds according to formula Iz may be selected from: 3-(3,4-Dimethoxyphenylamino)-2-(2,4-dihydro-2-oxo-1H-benzo[d][1,3]oxazin-7-yl)-1H-imidazo[1,2-b]pyrazole-7-carbonitrile; N-(4-(3-(3,4-Dimethoxyphenylamino)-7-cyano-1H-imidazo[1,2-b]pyrazol-2-yl)- phenyl)acetamide; N-(4-(3-(3-(Trifluoromethyl)phenylamino)-7-cyano-1H-imidazo[1,2-b]pyrazol--2-yl)phenyl)acetamide; 3-(3,4-Dimethoxyphenylamino)-2-(5-methoxy-1H-indol-3-yl)-1H-imidazo[1,2-b-]pyrazole-7-carbonitrile; 3-(3,4-Dimethoxyphenylamino)-2-(1H-indol-5-yl)-1H-imidazo[1,2-b]pyrazole-7-carbonitrile; 3-(3,4-Dimethoxyphenylamino)-2-(1H-indol-6-yl)-1H-imidazo[1,2-b]pyrazole-7-carbonitrile; 3-(3,4-Dichlorophenylamino)-2-(2,4-dihydro-2-oxo-1H-benzo[d][1,3]oxazin-7--yl)-1H-imidazo[1,2-b]pyrazole-7-carbonitrile; 3-(3,4-Dimethoxyphenylamino)-2-(4-phenoxyphenyl)-1H-imidazo[1,2-b]pyrazol-e-7-carbonitrile; 3-(3-Cyanophenylamino)-2-(3,4-dihydro-3-oxo-2H-benzo[b][1,4]oxazin-6-yl)-1H-imidazo[1,2-b]pyrazole-7-carbonitrile; Methyl 3-(7-cyano-2-(3,4-dihydro-3-oxo-2H-benzo[b][1,4]oxazin-6-yl)-1H-imidazo[1-,2-b]pyrazol-3-ylamino)benzoate; 3-(2,4,4-Trimethylpentan-2-ylamino)-2-(3,4,5-trimethoxyphenyl)-1H-imidazo-[1,2-b]pyrazole-7-carbonitrile; N-(7-Cyano-2-(3,4,5-trimethoxyphenyl)-1H-imidazo[1,2-b]pyrazol-3-yl)-2,2,-2-trifluoroacetamide; Methyl 4-(7-cyano-2-(2,4-dihydro-2-oxo-1H-benzo[d][1,3]oxazin-7-yl)-1H-imidazo[1-,2-b]pyrazol-3-ylamino)benzoate; 3-(3-Methoxyphenylamino)-2-(3,4,5-trimethoxyphenyl)-1H-imidazo[1,2-b]pyra-zole-7-carbonitrile; 3-(3-Methoxyphenylamino)-2-(2,4-dihydro-2-oxo-1H-benzo[d][1,3]oxazin-7-yl-)-1H-imidazo[1,2-b]pyrazole-7-carbonitrile; 3-(3,4-Dimethoxyphenylamino)-2-(2,4-dihydro-2-oxo-1H-benzo[d][1,3]oxazin-7-yl)-6-methyl-1H-imidazo[1,2-b]pyrazole-7-carbonitrile; 3-Amino-2-(3,4,5-trimethoxyphenyl)-1H-imidazo[1,2-b]pyrazole-7-carbonitri-le; N-(7-Cyano-2-(3,4,5-trimethoxyphenyl)-1H-imidazo[1,2-b]pyrazol-3-yl)-3--fluoro-4-(trifluoromethyl)benzamide; N-(7-Cyano-2-(3,4,5-trimethoxyphenyl)-1H-imidazo[1,2-b]pyrazo1-3-yl)benza-mide; N-(7-Cyano-2-(3,4,5-trimethoxyphenyl)-1H-imidazo[1,2-b]pyrazol-3-yl)--4-fluorobenz amide; N-(7-Cyano-2-(3,4,5-trimethoxyphenyl)-1H-imidazo[1,2-b]pyrazol-3-yl)-3-me-thoxybenzamide; 3-(3,4-Dichlorophenylamino)-2-(3,4,5-trimethoxyphenyl)-1H-imidazo[1,2-b]p-yrazole-7-carbonitrile; 3-(4-Bromophenylamino)-2-(3,4,5-trimethoxyphenyl)-1H-imidazo[1,2-b]pyrazo-le-7-carbonitrile; 3-(3,4-Dimethoxyphenylamino)-2-(6-methoxy-1H-indol-3-yl)-1H-imidazo[1,2-b-]pyrazole-7-carbonitrile; 3-(4-Morpholinophenylamino)-2-(3,4,5-trimethoxyphenyl)-1H-imidazo[1,2-b]p- yrazole-7-carbonitrile; 3-(3,4,5-Trimethoxyphenylamino)-2-(1H-indol-6-yl)-1H-imidazo[1,2-b]pyrazo-le-7-carbonitrile; 3-(4-Morpholinophenylamino)-2-(1H-indol-6-yl)-1H-imidazo[1,2-b]pyrazole-7--carbonitrile; 3-(3,4-Dimethoxyphenylamino)-2-(3,4,5-trimethoxyphenyl)-1H-imidazo[1,2-b]-pyrazole-7-carbonitrile; 3-(3,4,5-Trimethoxyphenylamino)-2-(3,4,5-trimethoxyphenyl)-1H-imidazo[1,2--b]pyrazole-7-carbonitrile; N-(3-(7-Cyano-2-(3,4,5-trimethoxyphenyl)-1H-imidazo[1,2-b]pyrazol-3-ylamino)phenyl)acetamide; N-(3-(7-Cyano-2-(3,4-dihydro-3-oxo-2H-benzo[b][1,4]oxazin-6-yl)-1H-imidazo[1,2-b]pyrazol-3-ylamino)phenyl)acetamide; N-(3-(7-Cyano-2-(3,4-dimethoxyphenyl)-1H-imidazo[1,2-b]pyrazol-3-ylamino)-phenyl)acetamide; N-(3-(7-Cyano-2-(4-(methylthio)phenyl)-1H-imidazo[1,2-b]pyrazol-3-ylamino-)phenyl)acetamide; N-(4-(7-Cyano-3-(3-acetamidophenylamino)-1H-imidazo[1,2-b]pyrazol-2-yl)ph- enyl)acetamide; 3-(3-Methoxybenzylamino)-2-(3,4-dihydro-3-oxo-2H-benzo[b][1,4]oxazin-6-yl-)-1H-imidazo[1,2-b]pyrazole-7-carbonitrile; 3-(3,4-Dimethoxyphenylamino)-2-(2,4-difluorophenyl)-1H-imidazo[1,2-b]pyra-zole-7-carbonitrile; 3-(3,4-Dimethoxyphenylamino)-2-(3,4-difluorophenyl)-1H-imidazo[1,2-b]pyra-zole-7-carbonitrile; 3-(3,4-Dimethoxyphenylamino)-2-(2,3-dihydrobenzo[b][1,4]dioxin-7-yl)-1H-i-midazo[1,2-b]pyrazole-7-carbonitrile; N-(7-Cyano-2-(3,4,5-trimethoxyphenyl)-1H-imidazo[1,2-b]pyrazol-3-yl)-5-ph- enyl-1,3,4-oxadiazole-2-carboxamide; 3-(3,4-Dimethoxyphenethylamino)-2-(3,4-dihydro-3-oxo-2H-benzo[b][1,4]oxaz-in-6-yl)-1H-imidazo[1,2-b]pyrazole-7-carbonitrile; 3-(3,4-Dimethoxyphenethylamino)-2-(3,4,5-trimethoxyphenyl)-1H-imidazo[1,2--b]pyrazole-7-carbonitrile; N-(7-Cyano-2-(3,4,5-trimethoxyphenyl)-1H-imidazo[1,2-b]pyrazol-3-yl)-2-(3- ,4-dimethoxyphenyl)acetamide; 3-(4-Morpholinophenylamino)-2-(3,4-dihydro-3-oxo-2H-benzo[b][1,4]oxazin-6--yl)-1H-imidazo[1,2-b]pyrazole-7-carbonitrile; 3-(3-Methoxyphenylamino)-2-(1H-indol-6-yl)-1H-imidazo[1,2-b]pyrazole-7-ca-rbonitrile; 3-(4-Bromophenylamino)-2-(3,4-dihydro-3-oxo-2H-benzo[b][1,4]oxazin-6-yl)-1H-imidazo[1,2-b]pyrazole-7-carbonitrile; 3-(3-Methoxybenzylamino)-2-(3,4,5-trimethoxyphenyl)-1H-imidazo[1,2-b]pyra-zole-7-carbonitrile; N-(3-(7-Cyano-2-(1H-indol-6-yl)-1H-imidazo[1,2-b]pyrazol-3-ylamino)phenyl-)acetamide; 3-(3,4-Dimethoxyphenylamino)-2-(4-(methylsulfonyl)phenyl)-1H-imidazo[1,2-b]pyrazole-7-carbonitrile; 3-(2,3-Dihydrobenzo[b][1,4]dioxin-6-ylamino)-2-(3,4,5-trimethoxyphenyl)-1-H-imidazo[1,2'-b]pyrazole-7-carbonitrile; 3-(2,3-Dihydrobenzo[b][1,4]dioxin-6-ylamino)-2-(3,4-dihydro-3-oxo-2H-benz-o[b][1,4]oxazin-6-yl)-1H-imidazo[1,2-b]pyrazole-7-carbonitrile; 3-(3,4-Dimethoxyphenylamino)-2-(benzo[d][1,3]dioxol-6-yl)-1H-imidazo[1,2-b]pyrazole-7-carbonitrile; 3-(3,4-Dimethoxyphenylamino)-2-(3-fluoro-4-methoxyphenyl)-1H-imidazo[1,2-b]pyrazole-7-carbonitrile; 2-Bromo-N-(7-cyano-2-(3,4,5-trimethoxyphenyl)-1H-imidazo[1,2-b]pyrazol-3-yl)acetamide; N-(7-Cyano-2-(3,4,5-trimethoxyphenyl)-1H-imidazo[1,2-b]pyrazol-3-yl)-2-phenoxyacetamide; 3-(3,4-Dimethoxyphenylamino)-2-benzyl-1H-imidazo[1,2-b]pyrazole-7-carbonitrile; 2-(3,4-Dichlorophenyl)-N-(7-cyano-2-(3,4,5-trimethoxyphenyl)-1H-imi-dazo[1,2-b]pyrazol-3-yl)acetamide; 3-(3,4-Dimethoxyphenylamino)-2-(3,4,5-trifluorophenyl)-1H-imidazo[1,2-b]p-yrazole-7-carbonitrile; 3-(3,4-Dimethoxyphenylamino)-2-(3-chloro-4,5-dimethoxyphenyl)-1H-imidazo[-1,2-b]pyrazole-7-carbonitrile; 3-(3,4-Dimethoxyphenylamino)-2-(4-fluoro-3-methoxyphenyl)-1H-imidazo[1,2-b]pyrazole-7-carbonitrile; 3-(2,3-Dihydrobenzo[b][1,4]dioxin-6-ylamino)-2-(1H-indol-6-yl)-1H-imidazo-[1,2-b]pyrazole-7-carbonitrile; N-(4-(7-Cyano-2-(3,4,5-trimethoxyphenyl)-1H-imidazo[1,2-b]pyrazol-3-ylami-no)phenyl)acetamide; N-(4-(7-Cyano-2-(6-methoxy-1H-indol-3-yl)-1H-imidazo[1,2-b]pyrazol-3-ylam- ino)phenyl)acetamide; N-(4-(7-Cyano-2-(2,3-dihydrobenzo[b][1,4]dioxin-7-yl)-1H-imidazo[1,2-b]py-razol-3-ylamino)phenyl)acetamide; 3-(3,4-Difluorophenylamino)-2-(3,4,5-trimethoxyphenyl)-1H-imidazo[1,2-b]p-yrazole-7-carbonitrile; tert-Butyl 4-(7-cyano-2-(3,4,5-trimethoxyphenyl)-1H-imidazo[1,2-b]pyrazol-3-ylamino)-benzylcarbamate; 3-(4-(Aminomethyl)phenylamino)-2-(3,4,5-trimethoxyphenyl)-1H-imidazo[1,2-b]pyrazole-7-carbonitrile; Methyl 3-(7-cyano-2-(3,4,5-trimethoxyphenyl)-1H-imidazo[1,2-b]pyrazol-3-ylamino)- benzoate; N-(4-(7-Cyano-2-(3,4-dihydro-3-oxo-2H-benzo[b][1,4]oxazin-6-yl)-1H-imidazo[1,2-b]pyrazol-3-ylamino)phenyl)acetamide; N-(4-(7-Cyano-2-(3,4-dimethoxyphenyl)-1H-imidazo[1,2-b]pyrazol-3-ylamino)-phenyl)acetamide; 3-(2,3-Dihydrobenzo[b][1,4]dioxin-6-ylamino)-2-(3-hydroxy-4,5-dimethoxyph-enyl)-1H-imidazo[1,2-b]pyrazole-7-carbonitrile; Methyl 4-(7-cyano-2-(3-hydroxy-4,5-dimethoxyphenyl)-1H-imidazo[1,2-b]pyrazol-3-y-lamino)benzoate; N-(4-(7-Cyano-2-(3,4,5-trimethoxyphenyl)-1H-imidazo[1,2-b]pyrazol-3-ylami-no)benzyl)acetamide; tert-Butyl-4-((7-cyano-2-(3,4,5-trimethoxyphenyl)-1H-imidazo[1,2-b]pyrazo-1-3-ylamino)methyl)piperidine-1-carboxylate; 3-((Piperidin-4-yl)methylamino)-2-(3,4,5-trimethoxyphenyl)-1H-imidazo[1,2-b]pyrazole-7-carbonitrile; 3-(3-Fluoro-4-(4-(pyrrolidin-1-yl)piperidin-1-yl)phenylamino)-2-(3,4,5-trimethoxyphenyl)-1H-imidazo[1,2-b]pyrazole-7-carbonitrile; (S)-Methyl 2-(7-cyano-2-(3,4,5-trimethoxyphenyl)-1H-imidazo[1,2-b]pyrazol-3-ylamino)--3-phenylpropanoate; Methyl 3-(7-cyano-2-(3,4-dimethoxyphenyl)-1H-imidazo[1,2-b]pyrazol-3-ylamino)ben-zoate; Methyl 3-(2-(3-chloro-4,5-dimethoxyphenyl)-7-cyano-1H-imidazo[1,2-b]pyrazol-3-yl-amino)benzoate; 3-(3,4,5-Trimethoxyphenylamino)-2-(4-morpholinophenyl)-1H-imidazo[1,2-b]p-yrazole-7-carbonitrile; 3-(4-Bromophenylamino)-2-(4-morpholinophenyl)-1H-imidazo[1,2-b]pyrazole-7--carbonitrile; 4-(3-(3,4,5-Trimethoxyphenylamino)-7-cyano-1H-imidazo[1,2-b]pyrazol-2-yl)-benzamide; 3-Amino-2-(3,4,5-trimethoxyphenyl)-5-(4-methoxyphenyl)-5H-imida-zo[1,2-b]pyrazole-7-carbonitrile; N-(4-(7-Cyano-2-(3,4,5-trimethoxyphenyl)-5H-imidazo[1,2-b]pyrazol-3-ylami-no)benzyl)nicotinamide; Methyl 3-(2-(4-((methoxycarbonyl)methoxy)-3-methoxyphenyl)-7-cyano-1H-imidazo[1,-2-b]pyrazol-3-ylamino)benzoate; 3-(2,3-Dihydrobenzo[b][1,4]dioxin-6-ylamino)-2-(4-((methoxycarbonyl)methoxy)-3-methoxyphenyl)-1H-imidazo[1,2-b]pyrazole-7-carbonitrile; 2-(5-(7-Cyano-3-(3-(methoxycarbonyl)phenylamino)-1H-imidazo[1,2-b]pyrazol--2-yl)-2-methoxyphenoxy)acetic acid; 3-(4-Fluoro-3-methoxyphenylamino)-2-(3,4,5-trimethoxyphenyl)-1H-imidazo[1-,2-b]pyrazole-7-carbonitrile; 6-(4-Chlorophenyl)-2-(3,4-dimethoxyphenyl)-5H-imidazo[1,2-b]pyrazole-7-carbonitrile; N-(4-(7-Cyano-2-(3,4,5-trimethoxyphenyl)-5H-imidazo[1,2-b]pyrazol-3-ylami-no)benzyl)-3,4-dimethoxybenzamide; N-(4-(7-Cyano-2-(3,4,5-trimethoxyphenyl)-5H-imidazo[1,2-b]pyrazol-3-ylami-no)benzyl)-3-(4-hydroxyphenyl)propanamide; N-(4-(7-Cyano-2-(3,4,5-trimethoxyphenyl)-5H-imidazo[1,2-b]pyrazol-3-ylami-no)benzyl)-3-(piperidin-1-yl)propanamide; N-(4-(7-Cyano-2-(3,4,5-trimethoxyphenyl)-5H-imidazo[1,2-b]pyrazol-3-ylami-no)benzyl)-4-cyanobenzamide; N-(4-(7-Cyano-2-(3,4,5-trimethoxyphenyl)-5H-imidazo[1,2-b]pyrazol-3-ylami-no)benzyl)-1-methylpiperidine-4-carboxamide; N-(4-(7-Cyano-2-(3,4,5-trimethoxyphenyl)-5H-imidazo[1,2-b]pyrazol-3-ylami-no)benzyl)-1H-indazole-3-carboxamide; N-(4-(7-Cyano-2-(3,4,5-trimethoxyphenyl)-5H-imidazo[1,2-b]pyrazol-3-ylami-no)benzyl)-1,6-dihydro-6-oxopyridine-3-carboxamide; 3-((1-Nicotinoylpiperidin-4-yl)methylamino)-2-(3,4,5-trimethoxyphenyl)-5H-imidazo[1,2-b]pyrazole-7-carbonitrile; 4-(3-(2,3-Dihydrobenzo[b][1,4]dioxin-6-ylamino)-7-cyano-1H-imidazo[1,2-b]-pyrazol-2-yl)benzamide; 4-(3-(4-Bromophenylamino)-7-cyano-1H-imidazo[1,2-b]pyrazol-2-yl)benzamide-; Methyl 2-(4-(7-cyano-3-(3,4-dimethoxyphenylamino)-1H-imidazo[1,2-b]pyraz-ol-2-yl)-2-methoxyphenoxy)acetate; 3-(3,4-Dimethoxyphenylamino)-2-(3-hydroxy-4,5-dimethoxyphenyl)-1H-imidazo-[1,2-b]pyrazole-7-carbonitrile; 2-(4-(2-Hydroxyethoxy)-3-methoxyphenyl)-3-(3,4-dimethoxyphenylamino)-1H-i-midazo[1,2-b]pyrazole-7-carbonitrile; 3-(4-Fluoro-3-methoxyphenylamino)-2-(3,4-dimethoxyphenyl)-5H-imidazo[1,2-b]pyrazole-7-carbonitrile; 4-(3-(3,4-Dimethoxyphenylamino)-7-cyano-1H-imidazo[1,2-b]pyrazol-2-yl)ben-zamide; 3-(3,4-Dimethoxyphenylamino)-2-(4-morpholinophenyl)-1H-imidazo[1,2--b]pyrazole-7-carbonitrile; 3-(3,4-Dimethoxyphenylamino)-2-(3-morpholinophenyl)-1H-imidazo[1,2-b]pyra-zole-7-carbonitrile; 3-(3,4-Dimethoxyphenylamino)-2-(3-(cyclopentyloxy)-4-methoxyphenyl)-1H-im-idazo[1,2-b]pyrazole-7-carbonitrile; 3-(3,4-Dimethoxyphenylamino)-2-(4-(2-pyrrolidin-1-yl)ethoxy)-1H-imidazo[1-,2-b]pyrazole-7-carbonitrile; 2-(4-(2-Methoxyethoxy)-3-methoxyphenyl)-3-(3,4-dimethoxyphenylamino)-1H-i-midazo[1,2-b]pyrazole-7-carbonitrile; 3-(4-Fluoro-3-methoxyphenylamino)-2-(3,4-dihydro-3-oxo-2H-benzo[b][1,4]ox-azin-6-yl)-1H-imidazo[1,2-b]pyrazole-7-carbonitrile; 3-(3-(Trifluoromethoxy)phenylamino)-2-(3,4,5-trimethoxyphenyl)-1H-imidazo-[1,2-b]pyrazole-7-carbonitrile; 3-(3-Chloro-4-methoxyphenylamino)-2-(3,4,5-trimethoxyphenyl)-1H-imidazo[1-,2-b]pyrazole-7-carbonitrile; 3-(3,4-Dimethoxyphenylamino)-2-(3-hydroxyphenyl)-1H-imidazo[1,2-b]pyrazol-e-7-carbonitrile; Methyl 2-(4-(7-cyano-3-(3,4-dimethoxyphenylamino)-1H-imidazo[1,2-b]pyrazol-2-yl)-phenoxy)acetate; N-(3-(7-Cyano-3-(3,4-dimethoxyphenylamino)-1H-imidazo[1,2-b]pyrazol-2-yl)-phenyl)methanesulfonamide; 3-(3-(Cyclopentyloxy)-4-methoxyphenylamino)-2-(3,4,5-trimethoxyphenyl)-1H--imidazo[1,2-b]pyrazole-7-carbonitrile; 2-(4-(2-Hydroxyethoxy)-3-methoxyphenyl)-3-(4-fluoro-3-methoxyphenylamino)--1H-imidazo[1,2-b]pyrazole-7-carbonitrile; 4-(3-(4-Fluoro-3-methoxyphenylamino)-7-cyano-1H-imidazo[1,2-b]pyrazol-2-y-l)benzamide; 3-(4-Fluoro-3-methoxyphenylamino)-2-(3-hydroxy-4,5-dimethoxyphenyl)-1H-im- idazo[1,2-b]pyrazole-7-carbonitrile; 3-(4-Fluoro-3-methoxyphenylamino)-2-(3-(cyclopentyloxy)-4-methoxyphenyl)-1H-imidazo[1,2-b]pyrazole-7-carbonitrile; 3-(4-Fluoro-3-methylphenylamino)-2-(3,4,5-trimethoxyphenyl)-1H-imidazo[1,-2-b]pyrazole-7-carbonitrile; 3-(3-Fluoro-4-methylphenylamino)-2-(3,4,5-trimethoxyphenyl)-1H-imidazo[1,-2-b]pyrazole-7-carbonitrile; 2-(4-(2-Methoxyethoxy)-3-methoxyphenyl)-3-(4-fluoro-3-methoxyphenylamino)--1H-imidazo[1,2-b]pyrazole-7-carbonitrile; Methyl 2-(4-(7-cyano-3-(4-fluoro-3-methoxyphenylamino)-1H-imidazo[1,2-b]pyrazol-2-yl)-2-methoxyphenoxy)acetate; 2-(4-(2-Morpholinoethoxy)phenyl)-3-(3,4-dimethoxyphenylamino)-1H-imidazo[-1,2-b]pyrazole-7-carbonitrile; 2-(5-(7-Cyano-3-(3,4-dimethoxyphenylamino)-1H-imidazo[1,2-b]pyrazol-2-yl)--2-methoxyphenoxy)acetamide; 3-(3-Isopropoxy-4-methoxyphenylamino)-2-(3,4,5-trimethoxyphenyl)-1H-imida-zo[1,2-b]pyrazole-7-carbonitrile; 3-(3-Fluoro-4-methoxyphenylamino)-2-(3,4,5-trimethoxyphenyl)-1H-imidazo[1-,2-b]pyrazole-7-carbonitrile; 3-(4-(Cyclopentyloxy)-3-methoxyphenylamino)-2-(3,4,5-trimethoxyphenyl)-1H--imidazo[1,2-b]pyrazole-7-carbonitrile; 3-(4-(2-(Pyrrolidin-1-yl)ethoxy)-3-methoxyphenylamino)-2-(3,4,5-trimethoxyphenyl)-1H-imidazo[1,2-b]pyrazole-7-carbonitrile; 3-(4-Fluoro-3-(trifluoromethyl)phenylamino)-2-(3,4,5-trimethoxyphenyl)-1H--imidazo[1,2-b]pyrazole-7-carbonitrile; 3-(4-(Trifluoromethoxy)phenylamino)-2-(3,4,5-trimethoxyphenyl)-1H-imidazo-[1,2-b]pyrazole-7-carbonitrile; 3-(4-Chloro-3-methoxyphenylamino)-2-(3,4,5-trimethoxyphenyl)-1H-imidazo[1-,2-b]pyrazole-7-carbonitrile; 3-(4-Fluoro-3-isopropoxyphenylamino)-2-(3,4,5-trimethoxyphenyl)-1H-imidaz-o[1,2-b]pyrazole-7-carbonitrile; 3-(3-Fluoro-4-(pyrrolidin-1-yl)phenylamino)-2-(3,4,5-trimethoxyphenyl)-1H--imidazo[1,2-b]pyrazole-7-carbonitrile; 2-(4-(7-Cyano-3-(3,4-dimethoxyphenylamino)-1H-imidazo[1,2-b]pyrazol-2-yl)--2-methoxyphenoxy)acetamide; 3-(3,4-Dimethoxyphenylamino)-2-(4-methoxy-3,5-dimethylphenyl)-1H-imidazo[- 1,2-b]pyrazole-7-carbonitrile; 3-(3,4-Dihydro-3-oxo-2H-benzo[b][1,4]oxazin-6-ylamino)-2-(3,4,5-trimethox-yphenyl)-1H-imidazo[1,2-b]pyrazole-7-carbonitrile; 2-(5-(7-Cyano-3-(4-fluoro-3-methoxyphenylamino)-1H-imidazo[1,2-b]pyrazol-2-yl)-2-methoxyphenoxy)acetamide; 2-(4-(7-Cyano-3-(4-fluoro-3-methoxyphenylamino)-1H-imidazo[1,2-b]pyrazol-2-yl)-2-methoxyphenoxy)acetamide; 2-(4-(7-Cyano-3-(3,4-dimethoxyphenylamino)-1H-imidazo[1,2-b]pyrazol-2-yl)--2-methoxyphenoxy)-N-cyclopropylacetamide; 3-(3-Chloro-4-isopropoxyphenylamino)-2-(3,4,5-trimethoxyphenyl)-1H-imidaz- o[1,2-b]pyrazole-7-carbonitrile; 3-(3,5-Dimethoxyphenylamino)-2-(3,4,5-trimethoxyphenyl)-1H-imidazo[1,2-b]-pyrazole-7-carbonitrile; 3-(3,5-Difluoro-4-methoxyphenylamino)-2-(3,4,5-trimethoxyphenyl)-1H-imida-zo[1,2-b]pyrazole-7-carbonitrile; 3-(3-Ethoxy-4-fluorophenylamino)-2-(3,4,5-trimethoxyphenyl)-1H-imidazo[1,-2-b]pyrazole-7-carbonitrile; 3-(3-(Cyclopentyloxy)-4-fluorophenylamino)-2-(3,4,5-trimethoxyphenyl)-1H-imidazo[1,2-b]pyrazole-7-carbonitrile; N-(3-(7-Cyano-2-(3,4,5-trimethoxyphenyl)-1 H-imidazo[1,2-b]pyrazol-3-ylami-no)benzyl)nicotinamide; N-(3-(7-Cyano-2-(3,4,5-trimethoxyphenyl)-1H-imidazo[1,2-b]pyrazol-3-ylami-no)benzyl)picolinamide; N-(3-(7-Cyano-2-(3,4,5-trimethoxyphenyl)-1H-imidazo[1,2-b]pyrazol-3-ylami-no)benzyl)isonicotinamide; N-(4-(7-Cyano-2-(3,4,5-trimethoxyphenyl)-1H-imidazo[1,2-b]pyrazol-3-ylami-no)benzyl)picolinamide; N-(4-(7-Cyano-2-(3,4,5-trimethoxyphenyl)-1H-imidazo[1,2-b]pyrazol-3-ylami-no)benzyl)isonicotinamide; N-(4-(7-Cyano-2-(3,4,5-trimethoxyphenyl)-1H-imidazo[1,2-b]pyrazol-3-ylami- no)benzyl)-6-cyanopyridine-3-carboxamide; N-(4-(7-Cyano-2-(3,4,5-trimethoxyphenyl)-1H-imidazo[1,2-b]pyrazol-3-ylami- no)benzyl)-2-methylpyridine-3-carboxamide; N-(4-(7-Cyano-2-(3,4,5-trimethoxyphenyl)-1H-imidazo[1,2-b]pyrazol-3-ylami-no)benzyl)-2-methoxypyridine-3-carboxamide; N-(4-(7-Cyano-2-(3,4,5-trimethoxyphenyl)-1H-imidazo[1,2-b]pyrazol-3-ylami-no)benzyl)-6-methylpyridine-3-carboxamide; N-(4-(7-Cyano-2-(3,4,5-trimethoxyphenyl)-1H-imidazo[1,2-b]pyrazol-3-ylamino)benzyl)-4-(trifluoromethyl)pyridine-3-carboxamide; N-(4-(7-Cyano-2-(3,4,5-trimethoxyphenyl)-1H-imidazo[1,2-b]pyrazol-3-ylami-no)benzyl)-6-(trifluoromethyl)pyridine-3-carboxamide; and 3-(3-Fluoro-4-(methylthio)phenylamino)-2-(3)4,5-trimethoxyphenyl)-1H-imid-azo[1,2-b]pyrazole-7-carbonitrile.

In still other embodiments, the Syk inhibitors may be selected from aminopyrimidine compounds as disclosed for example in U.S. Pat. Appl. Pub. No. 2011/0245205, which is hereby incoportaed by reference in its entirety. Non-limiting inhibitor compounds of this type are represented by the formula (IV) or a pharmaceutically acceptable salt thereof: 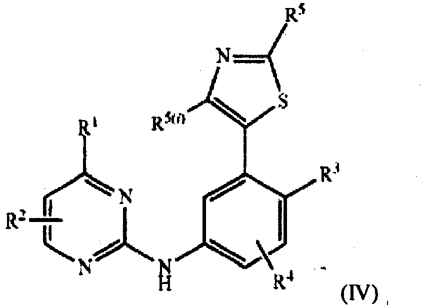 wherein:
R¹ is selected from the group consisting of (a) hydrogen, (b) halogen, (c) CN, (d) C₁₋₆ alkyl optionally substituted with one or more groups independently selected from the group consisting of OR^{a}, C₃₋₆cycloalkyl, and halogen, (e) C₂₋₆ alkenyl optionally substituted with OC₁₋₆alkyl, (f) C₂₋₆ alkynyl, (g) C₃₋₆ cycloalkyl, (h) OH, (i) -O-C₁₋₆ alkyl optionally substituted with one or more groups independently selected from (i) aryl, (ii) 5- or 6-membered heteroaryl optionally substituted with one or more groups independently selected from C₁₋₆ alkyl, (iii) 4- to 8-membered heterocyclyl optionally substituted with one or more groups independently selected from oxo, halogen, C₁₋₆ alkyl, (iv) -CO₂R^{a}, (v) -CONR^{b}R^{c}, (vi) -NR^{b}R^{c}, and (vii) -OR^{a}, (j) -A-X, wherein A is a bond or O, X is selected from the group consisting of (i) 4- to 8-membered heterocyclyl optionally substituted with one or more groups independently selected from halogen, C₁₋₆ alkyl, -C₁₋₆ haloalkyl, -C₁₋₆ hydroxyalkyl, COR^{a}, CO₂R^{a}, (ii) C₃₋₆ cycloalkyl optionally substituted with one or more groups independently selected from C₁₋₆ alkyl, -OR^{a}, -CO₂R^{a}, -NR^{b}R^{c}, (iii) heteroaryl optionally substituted with a benzyl which is optionally substituted with OR^{a}, (k) O-CH₂C.ident.C-pyrimidinyl, (l) -S(O)ₙ-C₁₋₆ alkyl, (m) -COR^{a}, (n) -CO₂R^{a}, (o) -CONR^{b}R^{c}, (p) -NR^{b}R^{c};
R² is selected from the group consisting of (a) H, (b) halogen, (c) C₁₋₆ alkyl, (d) O-C₁₋₆ alkyl, (e) C₁₋₆ haloalkyl and (f) O-C₁₋₆ haloalkyl; or R¹ and R² on adjacent carbon atoms together represent (CH₂)₃-4;
R³ is H, halogen, OR^{a}, or C₁₋₄allyl;
R⁴ is selected from the group consisting of (a) H, (b)halogen, (c) C₁₋₆ alkyl optionally substituted with one or more groups independently selected from (i) halogen, (ii) OR^{a}, (iii) OC(O)R^{a}, (iv) NR^{b}R^{c}, (v) NHC(O)R^{a}, and (vi) NHC(O)NHR^{b}, (d) C₂₋₆ alkenyl, (e) C₂₋₆ alkynyl, (f) C₃₋₆ cycloalkyl (g) OR^{a}, (h) NO₂, (i) NR^{b}R^{c}, (j) NHC(O)R^{a}, (k) NHC(O)NHR^{b}, (l) NHC(O)NHC(O)NR^{b}R^{c};
R⁵ is selected from the group consisting of (a) H, (b) halogen, (c) C₁₋₈alkyl, C₂₋₆alkenyl, C₂₋₆ alkynyl, each of which is optionally substituted with one or more groups independently selected from R^{y}, (d) C₃₋₁₂ carbocycle, or a carbon-linked 3- to 12-membered heterocyclyl each optionally substituted with one or more groups independently selected from R^{z}, (e) heteroaryl optionally substituted with C₁₋₃ alkyl (optionally substituted with one or more OH or CN or heterocycle); (f) -C(O)R^{a}, (g) -C(O)₂R^{a}, and (h) -C(O)NR^{b}R^{c};
R⁵⁽ⁱ⁾ is selected from the group consisting of H and C₁₋₃ alkyl;
R^{a} is selected from the group consisting of (a) H, (b) C₁₋₆ alkyl optionally substituted with one or more groups independently selected from (i) halogen, (ii) CN, (iii) OH, (iv) OC₁₋₄alkyl, (v) heterocyclyl optionally substituted with oxo, (vi) C(O)C₁₋₆alkyl optionally substituted with OH, (vii) CO₂H, (viii) CO₂C₁₋₆alkyl, (ix) CONR^{b}(i)R^{c}(i), (x) SO₂C₁₋₆alkyl, (xi) -NR^{b}(i)R^{c}(i), (xii) NR^{b}(i) C(O)NR^{b}(i)R^{c}(i); (xiii) phenyl, and (xiv) heteroaryl optionally substituted with OH, (c) C₂₋₆alkenyl, (d) C₃₋₆ cycloalkyl optionally substituted with one or more groups independently selected from (i) OH, (ii) CO₂H, (iii) CO₂C₁₋₆alkyl, (iv) CONR^{b}(i)R^{c}(i) (e) phenyl optionally substituted with one or more groups independently selected from (i) C₂₋₆alkynyl, (ii) CN, (iii) halogen, (iv) OH, (vi) OC(O)C₁₋₆alkyl, (vii) CO₂H, (viii) CO₂C₁₋₆alkyl, (f) heteroaryl optionally substituted with one or more groups independently selected from C₁₋₆alkyl, C₁₋₆haloalkyl, (CH₂)₁₋₈CO₂H, OH, halogen, phenyl optionally substituted with CO₂H, and (g) heterocyclyl optionally substituted with oxo;
R^{b} and R^{c} are independently selected from the group consisting of (a) H, (b) C₁₋₆ alkyl optionally substituted with one or more groups independently selected from (i) OR^{a}, (ii) halogen, (iii) heterocyclyl optionally substituted with oxo, OH, C₁₋₆ alkyl (optionally substituted with OH), (iv) C₃₋₆ cycloalkyl optionally substituted with one or two groups selected from C₁₋₄alkyl, CH₂OH, CONR^{b}(i)R^{c}(i), and CO₂R^{a}, (v) heteroaryl optionally substituted with C₁₋₆alkyl optionally substituted with OH, CO₂H or heteroaryl optionally substituted with a heteroaryl, (vi) SO₂NR^{b}(i)R^{c}(i), (vii) SO₂C₁₋₄alkyl, (viii) CONR^{b}(i)R^{c}(i), (ix) NR^{b}(i)R^{c}(i), (x) CO₂R^{a}, (xi) aryl optionally substituted with one or more groups selected from halogen, OR^{a}, C₁₋₆alkyl (optionally substituted with halogen, heterocycle (optionally substituted with oxo), or OR^{a}), SO₂NH₂, and heteroaryl optionally substituted with CH₂OH (xii) SO₃H, (xiii) NR^{b}(i) CONR^{b}(i)R^{c}(i), (xiv) CN, and (xv) NHC(O)R^{a}, (c) C₃₋₆ alkenyl optionally substituted with F; (d) C₃₋₆ cycloalkyl (optionally fused to a benzene ring) optionally substituted with one or more groups independently selected from (i) C₁₋₄alkyl, (ii) OR^{a}, (iii) CH₂OH, (iv) CO₂R^{a}, and (v) CONR^{b}(i)R^{c}(i), (e) aryl optionally substituted with one or two groups independently selected from (i) C₁₋₆alkyl (optionally substituted with OR^{a}), (ii) CN, (iii) OR^{a}, (iv) halogen, and (v) OCOC₁₋₄alkyl; (f) heteroaryl optionally substituted with one or more groups independently selected from (i) OR^{a}, (ii) CO₂R^{a} and (iii) C₁₋₆ alkyl optionally substituted with OH, (g) heterocyclyl optionally substituted with one or more groups independently selected from (i) oxo, (ii) OH and (iii) C₁₋₆ alkyl; or
R^{b}, R^{c} and the nitrogen atom to which they are attached together form a 5-, 6- or 7-membered heterocycle having 0 or 1 additional heteroatom selected from 0, P(O)(C₁₋₆alkyl), S(O)ₙ and N-R^{x}, and optionally substituted with one or more groups independently selected from (a) oxo, (b) thioxo, (c) C₁₋₆ alkyl optionally substituted with one or more groups independently selected from (i) OR^{a}, (ii) CO₂R^{a}, (iii) OP(O)(C₁₋₆alkyl)₂, (iv) aryl, and (v) halogen, (d) OR^{a}, (e) C(O)R^{a}, (f) C(O)₂R^{a}, (g) CONR^{b}(i)R^{c}(i), (h) P(O)(OH)₂, (i) SO₂R^{a}, and (j) CN, or R^{b}, R^{c} and the nitrogen atom to which they are attached together form 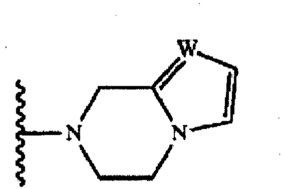
wherein W is CH or N; 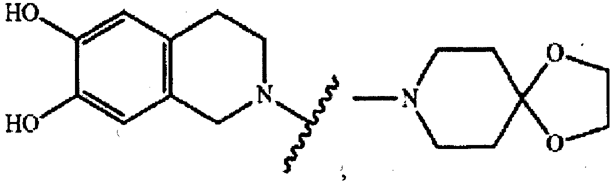
R^{b(i)} and R^{c(i)} are independently selected from the group consisting of (a) H and (b) C₁₋₆ alkyl optionally substituted with OH, CO₂H or CO₂C₁₋₆alkyl; or
R^{b(i)}, R^{c(i)} and the nitrogen atom to which they are attached together form a 5- or 6-membered heterocycle having 0 or 1 additional heteroatom selected from O, S and N-R^{x}, and optionally substituted with one or more groups independently selected from oxo;
R^{u} is selected from the group consisting of (a) C₁₋₆ alkyl optionally substituted with one to three groups selected from halogen, OH, SO₂R^{a}, CONR^{b}R^{c}, NR^{b}R^{c}, phenyl, heterocyclyl and heteroaryl, (b) C₃-8 cycloalkyl optionally substituted with OH, CO₂R^{a}, -CONH₂, (c) heterocycle optionally substituted with oxo, (d) aryl optionally substituted with C₂₋₆alkynyl, CN, halogen, OR^{a}, (e) heteroaryl optionally substituted with OH;
R^{x} is selected from the group consisting of (a) H, (b) C₁₋₆ alkyl optionally substituted with heterocycle, (c) phenyl optionally substituted with OH or OC₁₋₄alkyl, (d) -C(O)-C₁₋₆ alkyl, (e) -C(O)₂-C₁₋₆ alkyl, (f) -C(O)NH₂, -C(O)NH-C₁₋₆ alkyl, -C(O)N(C₁₋₆ alkyl)₂, (g) - C(O)₂NHC(O)NH₂, -C(O)₂NHC(O)NH-C₁₋₆ alkyl, -C(O)₂NHC(O)N(C₁₋₆ alkyl)₂, (h) -SO₂-C₁₋₆ alkyl (optionally substituted with halogen), -SO₂-heteroaryl (optionally substituted with alkyl), (i) - S(O)₂NH₂, -S(O)₂NH-C₁₋₆ alkyl, -S(O)₂N(C₁₋₆ alkyl)₂, (j) -SO₂NHC(O)₂-C₁₋₆ alkyl;
R^{y} is selected from the group consisting of (a) aryl optionally substituted with one or more groups independently selected from (i) halogen, (ii) C₁₋₆allyl optionally substituted with OH or CO₂R^{a}, (iii) C₂₋₆alkenyl optionally substituted with CO₂R^{a}, (iv) phenyl optionally substituted with CO₂R^{a}, (v) COR^{a}, (vi) CO₂R^{a}, (vii) CONR^{b}R^{c}, (viii) OR^{a}, (ix) S(O)ₙR^{a}, (x) SO₂NR^{b}R^{c}, (xi) SO₂NHC(O)R^{a}, (xii) NO₂, and (xiii) NHC(O)R^{a}, (b) heteroaryl optionally substituted with one or more groups independently selected from (i) halogen, (ii) C₁₋₆ alkyl optionally substituted with CO₂R^{a}, (iii) C₃₋₆ cycloalkyl, (iv) aryl optionally substituted with CO₂R^{a}, (v) CONR^{b}R^{c}, (vi) OR^{a}, (vii) SO₂R^{a}, and (viii) CO₂R^{a}, (c) C₃₋₈ cycloalkyl optionally substituted with one or more groups independently selected from (i) C₁₋₆ alkyl, (ii) CO₂R^{a}, and (iii) NR^{b}R^{c}, (d) C₆₋₈cycloalkenyl (optionally substituted with CO₂R^{a}), (e) halogen, (f) CN, (g) -C(O)R^{a}, (h) -C(O)₂R^{a}, (i) C(O)CO₂R^{a}, (j) -C(O)NR^{b}R^{c}, (k) - C(O)NHC(O)NR^{b}R^{c}, (l) -OR^{a}, (m) -OC(O)R^{a}, (n) -NR^{b}R^{c}, (o) -NHC(O)R^{u}, (p) -NHC(O)NR^{b}R^{c}, (q) -NHC(O)NHC(O)NH₂, (r) -NHSO.sub.mR^{a} (s) -NHSO₂NR^{b}R^{c}, (t) SOₙR^{a}, (u) -SO₂NR^{b}R^{c}, (v) - SO₂NHC(O)R^{a}, (w) -SO₂NHC(O)₂R^{a}, (x) SO₃H, (y) -P(O)(OR^{a})₂, and (z) CONHOH; (aa) heterocyclyl optionally substituted with one or more groups independently selected from oxo, thioxo, C₁₋₆alkyl, and CO₂R^{a};
R^{z} is selected from the group consisting of (a) a group selected from R^{y}, (b) C₁₋₆ alkyl optionally substituted with one or more groups independently selected from halogen, NR^{b}R^{c}, OR^{a}, CN, phenyl (optionally substituted with C₁₋₆alkanoic acid), CONR^{b}R^{c}, and -CO₂R^{a}, (c) oxo, and (d) NOR^{a}, m is 1 or 2, n is 0, 1 or 2.

Representative compounds according to formula IV include but are not limited to: (1R,4S)-4-[5-(3-cyclopropyl-5-{[4-(trifluoromethyl)pyrimidin-2-yl]amino}p-henyl)-1,3-thiazol-2-yl]-4-hydroxy-2,2-dimethylcyclohexanecarboxylic acid; (1S,4R)-4-[5-(3-cyclopropyl-5-{[4-(trifluoromethyl)pyrimidin-2-yl]amino}p-henyl)-1,3-thiazol-2-yl]-4-hydroxy-2,2-dimethylcyclohexanecarboxylic acid; (1S,4R)-4-hydroxy-2,2-dimethyl-4-{5-[3-methyl-5-(4-methylpyrimidin-2-yla-mino)-phenyl]-1,3-thiazol-2-yl}-cyclohexanecarboxylic acid; (1S,4R)-4-[5-(3-{[4-(difluoromethyl)pyrimidin-2-yl]amino}-5-methylphenyl)--1,3-thiazol-2-yl]-4-hydroxy-2,2-dimethylcyclohexanecarboxylic acid; trans-4-hydroxy-4-[5-(3-methyl-5-{[4-(trifluoromethyl)pyrimidin-2-yl]amin-o}phenyl)-1,3-thiazol-2-yl]cyclohexanecarboxylic acid; cis-4-hydroxy-4-[5-(3-methyl-5-{[4-(trifluoromethyl)pyrimidin-2-yl]amino}-phenyl)-],3-thiazol-2-yl]cyclohexanecarboxylic acid; 5-hydroxy-5-[5-(3-methyl-5-{[4-(trifluoromethyl)pyrimidin-2-yl]amino}phen-yl)-1,3-thiazol-2-yl]azepan-2-one; cis-4-[(hydroxyacetyl)amino]-1-[5-(3-methyl-5-{[4-(trifluoromethyl)pyrimi- din-2-yl]amino} phenyl)-1,3-thiazol-2-yl]cyclohexanecarboxamide; and (1S,4R)-4-hydroxy-2,2-dimethyl-4-[5-(3-methyl-5-{[4-(trifluoromethyl)pyri-midin-2-yl] amino}phenyl)-1,3-thiazol-2-yl]-N-[3-(2-oxopyrrolidin-1-yl)propyl]cyclohe-xanecarboxamide; (1S,4R)-4-hydroxy-2,2-dimethyl-4-[5-(3-methyl-5-{[4-(trifluoromethyl)pyrimidin-2-yl]amino}-phenyl)-1,3-thiazol-2-yl]cy-clohexanecarboxylic acid; (1R,48)-4-hydroxy-2,2-dimethyl-4-[5-(3-methyl-5-{[4-(trifluoromethyl)pyri-midin-2-yl]amino}-phenyl)-1,3-thiazol-2-yl]cyclohexanecarboxylic acid; (1S,4S)-4-hydroxy-2,2-dimethyl-4-[5-(3-methyl-5-{[4-(trifluoromethyl)pyri- midin-2-yl]amino}-phenyl)-1,3-thiazol-2-yl]cyclohexanecarboxylic acid; (1R,4R)-4-hydroxy-2,2-dimethyl-4-[5-(3-methyl-5-{[4-(trifluoromethyl)pyri-midin-2-yl]amino}-phenyl)-1,3-thiazol-2-yl]cyclohexanecarboxylic acid; (1RAS)-4-hydroxy-2,2-dimethyl-4-[5-(3-methyl-5-{[4-(trifluoromethyl)pyrim-idin-2-yl]amino}phenyl)-1,3-thiazol-2-yl]cyclohexanecarboxamide; (1S,4R)-4-hydroxy-2,2-dimethyl-4-[5-(3-methyl-5-{[4-(trifluoromethyl)pyri-midin-2-yl]amino}phenyl)-1,3-thiazol-2-yl]cyclohexanecarboxamide; (1S,4R) 4-{5-[3-({4-[(1R)-1-fluoroethyl]pyrimidin-2-yl}amino)-5-methylphenyl]-1,3--thiazol-2-yl}-4-hydroxy-2,2-dimethylcyclohexanecarboxylic acid; (1S,4R)4-{5-[3-({4-[(1S)-1-fluoroethyl]pyrimidin-2-yl}amino)-5-methylphenyl]-1,3--thiazol-2-yl}-4-hydroxy-2,2-dimethylcyclohexanecarboxylic acid; or a pharmaceutically acceptable salt thereof.

### 3.2.6 Immunomodulating particle embodiments

In some embodiments, at least one aggrecan antigen as described for example in Section 3.2.1 together with one or more inhibitors as described above (e.g., at least one NF-κB inhibitor as described for example in Section 3.2.3, and/or at least one mTOR inhibitor as described for example in Section 3.2.4, and/or at least one Syk inhibitor as described for example in Section 3.2.5), or alternatively at least one or aggrecan APL as descreibed for example in Section 3.2.2 (collectively referred to herein as "the bioactive agents") are provided in particulate form, which facilitate *in vivo* or *in vitro* delivery of the antigen and inhibitor to antigen-presenting cells. The inhibitor(s) and the antigen(s) or the APLs may be contained in or otherwise associated with the same particle or different particles. A variety of particles may be used in the practice of the present invention, including but not limited to, liposomes, micelles, lipidic particles, ceramic/inorganic particles and are typically selected from nanoparticles and microparticles. The particles are suitably sized for being taken up (*e.g.,* by phagocytosis or endocytosis) by antigen-presenting cells. In illustrative examples, the particles have a dimension of less than about 100 µm, more suitably in the range of less than or equal to about 500 nm, although the particles may be as large as about 10 µm, and as small as a few nm.

Liposomes consist basically of a phospholipid bilayer forming a shell around an aqueous core. Advantages include the lipophilicity of the outer layers which "mimic" the outer membrane layers of cells and that they are taken up relatively easily by a variety of cells. Polymeric vehicles typically consist of micro/nanospheres and micro/nanocapsules formed of biocompatible polymers, which are either biodegradable (for example, polylactic acid) or non-biodegradable (for example, ethylenevinyl acetate). Some of the advantages of the polymeric devices are ease of manufacture and high loading capacity, range of size from nanometer to micron diameter, as well as controlled release and degradation profile.

In some embodiments, the particles comprise an antigen-binding molecule on their surface, which is immuno-interactive with a marker that is expressed at higher levels on antigen-presenting cells (*e.g*., dendritic cells) than on non-antigen-presenting cells. Illustrative markers of this type include MGL, DCL-1, DEC-205, macrophage mannose R, DC-SIGN or other DC or myeloid specific (lectin) receptors, as for example disclosed by Hawiger et al. (2001, J Exp Med 194:769), Kato et al. 2003, J Biol Chem 278:34035), Benito et al. (2004, J Am Chem Soc 126:10355), Schjetne, et al. (2002, Int Immunol 14:1423) and van Vliet et al., 2006, Nat Immunol 7(11):1200-1208; Immunobiology 211:577-585).

The immunomodulating particles of the present invention can be prepared from a combination of the bioactive agent(s), and a surfactant, excipient or polymeric material. In some embodiments, the particles are biodegradable and biocompatible, and optionally are capable of biodegrading at a controlled rate for delivery of a therapeutic or diagnostic agent. The particles can be made of a variety of materials. Both inorganic and organic materials can be used. Polymeric and non-polymeric materials, such as fatty acids, may be used. Other suitable materials include, but are not limited to, gelatine, polyethylene glycol, trehalulose, dextran and chitosan. Particles with degradation and release times ranging from seconds to months can be designed and fabricated, based on factors such as the particle material.

### 3.2.6.1 Polymeric Particles

Polymeric particles may be formed from any biocompatible and desirably biodegradable polymer, copolymer, or blend. The polymers may be tailored to optimize different characteristics of the particle including: i) interactions between the bioactive agents to be delivered and the polymer to provide stabilization of the bioactive agents and retention of activity upon delivery; ii) rate of polymer degradation and, thereby, rate of agent release profiles; iii) surface characteristics and targeting capabilities *via* chemical modification; and iv) particle porosity.

Surface eroding polymers such as polyanhydrides may be used to form the particles. For example, polyanhydrides such as poly[(p-carboxyphenoxy)-hexane anhydride] (PCPH) may be used. Biodegradable polyanhydrides are described in U.S. Pat. No. 4,857,311.

In other embodiments, bulk-eroding polymers such as those based on polyesters including poly(hydroxy acids) or poly(esters) can be used. For example, polyglycolic acid (PGA), polylactic acid (PLA), or copolymers thereof may be used to form the particles. The polyester may also have a charged or functionalizable group, such as an amino acid. In illustrative examples, particles with controlled release properties can be formed of poly(D,L-lactic acid) and/or poly(D,L-lactic-co-glycolic acid) ("PLGA"), which incorporate a surfactant such as DPPC.

Other polymers include poly(alkylcyanoacrylates), polyamides, polycarbonates, polyalkylenes such as polyethylene, polypropylene, poly(ethylene glycol), poly(ethylene oxide), poly(ethylene terephthalate), poly vinyl compounds such as polyvinyl alcohols, polyvinyl ethers, and polyvinyl esters, polymers of acrylic and methacrylic acids, celluloses and other polysaccharides, and peptides or proteins, or copolymers or blends thereof. Polymers may be selected with or modified to have the appropriate stability and degradation rates *in vivo* for different controlled drug delivery applications.

In some embodiments, particles are formed from functionalized polyester graft copolymers, as described in Hrkach et al. (1995, Macromolecules, 28:4736-4739; and "Poly(L-Lactic acid-co-amino acid) Graft Copolymers: A Class of Functional Biodegradable Biomaterials" in Hydrogels and Biodegradable Polymers for Bioapplications, ACS Symposium Series No. 627, Raphael M. Ottenbrite et al., Eds., American Chemical Society, Chapter 8, pp. 93-101, 1996.)

Materials other than biodegradable polymers may be used to form the particles. Suitable materials include various non-biodegradable polymers and various excipients. The particles also may be formed of the bioactive agent(s) and surfactant alone.

Polymeric particles may be prepared using single and double emulsion solvent evaporation, spray drying, solvent extraction, solvent evaporation, phase separation, simple and complex coacervation, interfacial polymerization, and other methods well known to those of ordinary skill in the art. Particles may be made using methods for making microspheres or microcapsules known in the art, provided that the conditions are optimized for forming particles with the desired diameter.

Methods developed for making microspheres for delivery of encapsulated agents are described in the literature, for example, as described in Doubrow, M., Ed., "Microcapsules and Nanoparticles in Medicine and Pharmacy," CRC Press, Boca Raton, 1992. Methods also are described in Mathiowitz and Langer (1987, J. Controlled Release 5:13-22); Mathiowitz et al. (1987, Reactive Polymers 6:275-283); and Mathiowitz et al. (1988, J. Appl. Polymer Sci. 3:, 755-774) as well as in U.S. Pat. No. 5,213,812, U.S. Pat. No. 5,417,986, U.S. Pat. No. 5,360,610, and U.S. Pat. No. 5,384,133. The selection of the method depends on the polymer selection, the size, external morphology, and crystallinity that is desired, as described, for example, by Mathiowitz et al. (1990, Scanning Microscopy 4:329-340; 1992, J. Appl. Polymer Sci. 45:125-134); and Benita et al. (1984, J. Pharm. Sci. 73:1721-1724).

In solvent evaporation, described for example, in Mathiowitz et al., (1990), Benita; and U.S. Pat. No. 4,272,398 to Jaffe, the polymer is dissolved in a volatile organic solvent, such as methylene chloride. Several different polymer concentrations can be used, for example, between 0.05 and 2.0 g/mL. The bioactive agent(s), either in soluble form or dispersed as fine particles, is (are) added to the polymer solution, and the mixture is suspended in an aqueous phase that contains a surface-active agent such as poly(vinyl alcohol). The aqueous phase may be, for example, a concentration of 1% poly(vinyl alcohol) w/v in distilled water. The resulting emulsion is stirred until most of the organic solvent evaporates, leaving solid microspheres, which may be washed with water and dried overnight in a lyophilizer. Microspheres with different sizes (between 1 and 1000 µm) and morphologies can be obtained by this method.

Solvent removal was primarily designed for use with less stable polymers, such as the polyanhydrides. In this method, the agent is dispersed or dissolved in a solution of a selected polymer in a volatile organic solvent like methylene chloride. The mixture is then suspended in oil, such as silicon oil, by stirring, to form an emulsion. Within 24 hours, the solvent diffuses into the oil phase and the emulsion droplets harden into solid polymer microspheres. Unlike the hot-melt microencapsulation method described for example in Mathiowitz et al. (1987, Reactive Polymers 6:275), this method can be used to make microspheres from polymers with high melting points and a wide range of molecular weights. Microspheres having a diameter for example between one and 300 microns can be obtained with this procedure.

With some polymeric systems, polymeric particles prepared using a single or double emulsion technique, vary in size depending on the size of the droplets. If droplets in water-in-oil emulsions are not of a suitably small size to form particles with the desired size range, smaller droplets can be prepared, for example, by sonication or homogenation of the emulsion, or by the addition of surfactants.

If the particles prepared by any of the above methods have a size range outside of the desired range, particles can be sized, for example, using a sieve, and further separated according to density using techniques known to those of skill in the art.

The polymeric particles can be prepared by spray drying. Methods of spray drying, such as that disclosed in WO 96/09814 by Sutton and Johnson, disclose the preparation of smooth, spherical microparticles of a water-soluble material with at least 90% of the particles possessing a mean size between 1 and 10 µm.

### 3.2.6.2 Liposomes

Liposomes can be produced by standard methods such as those reported by Kim et al. (1983, Biochim. Biophys. Acta 728:339-348); Liu et al. (1992, Biochim. Biophys. Acta 1104:95-101); Lee et al. (1992, Biochim. Biophys. Acta. 1103:185-197), Brey et al. (U.S. Pat. Appl. Pub. 20020041861), Hass et al. (U.S. Pat. Appl. Pub. 20050232984), Kisak et al. (U.S. Pat. Appl. Pub. 20050260260) and Smyth-Templeton et al. (U.S. Pat. Appl. Pub. 20060204566). Additionally, reference may be made to Copeland et al. (2005, Immunol. Cell Biol. 83:95-105) who review lipid based particulate formulations for the delivery of antigen, and to Bramwell et al. (2005, Crit Rev Ther Drug Carrier Syst. 22(2):151-214; 2006, J Pharm Pharmacol. 58(6):717-728) who review particulate delivery systems for vaccines, including methods for the preparation of protein-loaded liposomes. Many liposome formulations using a variety of different lipid components have been used in various *in vitro* cell culture and animal experiments. Parameters have been identified that determine liposomal properties and are reported in the literature, for example, by Lee et al. (1992, Biochim. Biophys. Acta. 1103:185-197); Liu et al. (1992, Biochim. Biophys. Acta. 1104:95-101); and Wang et al. (1989, Biochem. 28:9508-9510).

Briefly, the lipids of choice (and any organic-soluble bioactive), dissolved in an organic solvent, are mixed and dried onto the bottom of a glass tube under vacuum. The lipid film is rehydrated using an aqueous buffered solution containing any water-soluble bioactives to be encapsulated by gentle swirling. The hydrated lipid vesicles can then be further processed by extrusion, submitted to a series of freeze-thawing cycles or dehydrated and then rehydrated to promote encapsulation of bioactives. Liposomes can then be washed by centrifugation or loaded onto a size-exclusion column to remove unentrapped bioactive from the liposome formulation and stored at 4° C. The basic method for liposome preparation is described in more detail in Thierry et al. (1992, Nuc. Acids Res. 20:5691-5698).

A particle carrying a payload of bioactive agent(s) can be made using the procedure as described in: Pautot et al. (2003, Proc. Natl. Acad. Sci. USA 100(19):10718-21). Using the Pautot *et al.* technique, streptavidin-coated lipids (DPPC, DSPC, and similar lipids) can be used to manufacture liposomes. The drug encapsulation technique described by Needham et al. (2001, Advanced Drug Delivery Reviews 53(3):285-305) can be used to load these vesicles with one or more active agents.

The liposomes can be prepared by exposing chloroformic solution of various lipid mixtures to high vacuum and subsequently hydrating the resulting lipid films (DSPC/CHOL) with pH 4 buffers, and extruding them through polycarbonated filters, after a freezing and thawing procedure. It is possible to use DPPC supplemented with DSPC or cholesterol to increase encapsulation efficiency or increase stability, etc. A transmembrane pH gradient is created by adjusting the pH of the extravesicular medium to 7.5 by addition of an alkalinization agent. A bioactive agent (*e.g.,* small molecule NF-κB inhibitors, mTOR inhibitors or Syk inhibitors, which are, for example, weak bases) can be subsequently entrapped by addition of a solution of the bioactive agent in small aliquots to the vesicle solution, at an elevated temperature, to allow accumulation of the bioactive agent inside the liposomes.

Other lipid-based particles suitable for the delivery of the bioactive agents of the present invention such as niosomes are described by Copeland et al. (2005, Immunol. Cell Biol. 83:95-105).

### 3.2.6.3 Ceramic Particles

Ceramic particles may also be used to deliver the bioactive agents of the invention. These particles are typically prepared using processes similar to the well known sol-gel process and usually require simple and room temperature conditions as described for example in Brinker et al. ("Sol-Gel Science: The Physics and Chemistry of Sol-Gel Processing;" Academic Press: San Diego, 1990, p-60), and Avnir et al. (1994, Chem. Mater. 6, 1605). Ceramic particles can be prepared with desired size, shape and porosity, and are extremely stable. They also effectively protect doped molecules (polypeptides, drugs etc.) against denaturation induced by extreme pH and temperature (Jain et al., 1998. J. Am. Chem. Soc. 120:11092-11095). In addition, their surfaces can be easily functionalized with different groups (Lal et al., 2000. Chem. Mater. 12:2632-2639; Badley et al., 1990, Langmuir 6:792-801), and therefore they can be attached to a variety of monoclonal antibodies and other ligands in order to target them to desired sites *in vivo.*

Various ceramic particles have been described for delivery *in vivo* of active agent-containing payloads. For example, British Patent 1 590 574 discloses incorporation of biologically active components in a sol-gel matrix. International Publication WO 97/45367 discloses controllably dissolvable silica xerogels prepared *via* a sol-gel process, into which a biologically active agent is incorporated by impregnation into pre-sintered particles (1 to 500 µm) or disks. International Publication WO 0050349 discloses controllably biodegradable silica fibers prepared *via* a sol-gel process, into which a biologically active agent is incorporated during synthesis of the fiber. U.S. Pat. Appl. Pub. 20040180096 describes ceramic nanoparticles in which a bioactive substance is entrapped. The ceramic nanoparticles are made by formation of a micellar composition of the dye. The ceramic material is added to the micellar composition and the ceramic nanoparticles are precipitated by alkaline hydrolysis. U.S. Pat. Appl. Pub. 20050123611 discloses controlled release ceramic particles comprising an active material substantially homogeneously dispersed throughout the particles. These particles are prepared by mixing a surfactant with an apolar solvent to prepare a reverse micelle solution; (b) dissolving a gel precursor, a catalyst, a condensing agent and a soluble active material in a polar solvent to prepare a precursor solution; (c) combining the reverse micelle solution and the precursor solution to provide an emulsion and (d) condensing the precursor in the emulsion. U.S. Pat. Appl. Pub. 20060210634 discloses adsorbing bioactive substances onto ceramic particles comprising a metal oxide (*e.g*., titanium oxide, zirconium oxide, scandium oxide, cerium oxide and yttrium oxide) by evaporation. Kortesuo et al. (2000, Int J Pharm. 200(2):223-229) disclose a spray drying method to produce spherical silica gel particles with a narrow particle size range for controlled delivery of drugs such as toremifene citrate and dexmedetomidine HCl. Wang et al. (2006, Int J Pharm. 308(1-2):160-167) describe the combination of adsorption by porous CaCO₃ microparticles and encapsulation by polyelectrolyte multilayer films for delivery of bioactive substances.

### 3.2.6.4 Ballistic particles

The bioactive agents of the present invention may be attached to (*e.g.,* by coating or conjugation) or otherwise associated with particles suitable for use in needleless or "ballistic" (biolistic) delivery. Illustrative particles for ballistic delivery are described, for example, in: WO 02/101412; WO 02/100380; WO 02/43774; WO 02/19989; WO 01/93829; WO 01/83528; WO 00/63385; WO 00/26385; WO 00/19982; WO 99/01 168; WO 98/10750; and WO 97/48485. It shall be understood, however, that such particles are not limited to their use with a ballistic delivery device and can otherwise be administered by any alternative technique (*e.g*., injection or microneedle delivery) through which particles are deliverable to immune cells.

The bioactive agents can be coated or chemically coupled to carrier particles (*e.g*., core carriers) using a variety of techniques known in the art. Carrier particles are selected from materials that have a suitable density in the range of particle sizes typically used for intracellular delivery. The optimum carrier particle size will, of course, depend on the diameter of the target cells. Illustrative particles have a size ranging from about 0.01 to about 250 µm, from about 10 to about 150 µm, and from about 20 to about 60 µm; and a particle density ranging from about 0.1 to about 25 g/cm³, and a bulk density of about 0.5 to about 3.0 g/cm³, or greater. Non-limiting particles of this type include metal particles such as, tungsten, gold, platinum and iridium carrier particles. Tungsten particles are readily available in average sizes of 0.5 to 2.0 µm in diameter. Gold particles or microcrystalline gold (*e.g.,* gold powder A1570, available from Engelhard Corp., East Newark, N.J.) may also be used. Gold particles provide uniformity in size (available from Alpha Chemicals in particle sizes of 1-3 µm, or available from Degussa, South Plainfield, N.J. in a range of particle sizes including 0.95 µm) and low toxicity. Microcrystalline gold provides a diverse particle size distribution, typically in the range of 0.1-5 µm. The irregular surface area of microcrystalline gold provides for highly efficient coating with the active agents of the present invention.

Many methods are known and have been described for adsorbing, coupling or otherwise attaching bioactive molecules (*e.g*., hydrophilic molecules such as proteins and nucleic acids) onto particles such as gold or tungsten particles. In illustrative examples, such methods combine a predetermined amount of gold or tungsten with the bioactive molecules, CaCl₂ and spermidine. In other examples, ethanol is used to precipitate the bioactive molecules onto gold or tungsten particles (see, for example, Jumar et al., 2004, Phys Med. Biol. 49:3603-3612). The resulting solution is suitably vortexed continually during the coating procedure to ensure uniformity of the reaction mixture. After attachment of the bioactive molecules, the particles can be transferred for example to suitable membranes and allowed to dry prior to use, coated onto surfaces of a sample module or cassette, or loaded into a delivery cassette for use in particular particle-mediated delivery instruments.

The formulated compositions may suitably be prepared as particles using standard techniques, such as by simple evaporation (air drying), vacuum drying, spray drying, freeze drying (lyophilization), spray-freeze drying, spray coating, precipitation, supercritical fluid particle formation, and the like. If desired, the resultant particles can be dandified using the techniques described in International Publication WO 97/48485.

### 3.2.6.5 Surfactants

Surfactants, which can be incorporated into particles, include phosphoglycerides. Exemplary phosphoglycerides include phosphatidylcholines, such as the naturally occurring surfactant, L-α-phosphatidylcholine dipalmitoyl ("DPPC"). The surfactants advantageously improve surface properties by, for example, reducing particle-particle interactions, and can render the surface of the particles less adhesive. The use of surfactants endogenous to the lung may avoid the need for the use of non-physiologic surfactants.

Providing a surfactant on the surfaces of the particles can reduce the tendency of the particles to agglomerate due to interactions such as electrostatic interactions, Van der Waals forces, and capillary action. The presence of the surfactant on the particle surface can provide increased surface rugosity (roughness), thereby improving aerosolization by reducing the surface area available for intimate particle-particle interaction.

Surfactants known in the art can be used including any naturally occurring surfactant. Other exemplary surfactants include diphosphatidyl glycerol (DPPG); hexadecanol; fatty alcohols such as polyethylene glycol (PEG); polyoxyethylene-9-lauryl ether; a surface active fatty acid, such as palmitic acid or oleic acid; sorbitan trioleate (Span 85); glycocholate; surfactin; a poloxamer; a sorbitan fatty acid ester such as sorbitan trioleate; tyloxapol and a phospholipid.

The particles may be used as vehicles for delivery of inhibitor and aggrecan antigen to antigen-presenting cells *ex vivo.* However, in advantageous embodiments, the particles are used to deliver the bioactive compounds to antigen-presenting cells *in vivo,* as disclosed for example in U.S. Pat. Appl. Pub. 20100151000.

### 3.2.7 Ex vivo antigen-presenting cell embodiments

Inhibitor and aggrecan antigen can be delivered *ex vivo* into antigen-presenting cells in various forms, and may be soluble or particulate in nature. The antigen-presenting cells or their precursors may be obtained from the subject to be treated (*i.e*., autologous antigen-presenting cells or precursors). Alternatively, the antigen-presenting cells or their precursors are obtained or derived from a donor that is MHC-matched or mismatched with the subject (*i.e*., an allogeneic antigen-presenting cell). Suitably, in these embodiments, the donor is histocompatible with the subject.

In the above embodiments, the antigen-presenting cells are contacted with one or more inhibitors as described for example in Sections 3.2.3, 3.2.4 and 3.2.5, or with a polynucleotide from which the inhibitor is expressible, for a time and under conditions sufficient to inhibit, reduce or otherwise impair NF-κB activity and/or mTOR activity and/or Syk activity in the antigen-presenting cell. The amount of soluble or particulate inhibitor to be placed in contact with antigen-presenting cells or their precursors can be determined empirically by routine methods known to persons of skill in the art. In some advantageous embodiments the antigen-presenting cells are incubated in the presence of inhibitor for at least about 2, 3, 4, 5, 6, 7, 8, 12, 24, 36, 48, 60, 72, 84, 96 hours, or for less than about 96, 84, 72, 60, 48, 36, 24, 12, 8, 7, 6, 5, 4, 3 or 2 hours. In specific embodiments, antigen-presenting cells are incubated with inhibitor (*e.g*., a NF-κB inhibitor or mTOR inhibitor or Syk inhibitor) for about 48 to about 60 h at 35° C -38° C or for as much time as required to inhibit, reduce or otherwise impair the activity of NF-κB, mTOR or Syk. In illustrative examples of this type, the antigen-presenting cells may be incubated in the presence of an activator of antigen-presenting cells such as lipopolysaccharide (LPS) and optionally additional inhibitors (*e.g*., additional NF-κB inhibitors such as Vitamin D and dexamethasone).

The antigen-presenting cells or their precursors are also contacted with aggrecan antigen as described for example in Section 3.2.1, or with aggrecan APL as described for example in Section 3.2.2, or with a polynucleotide from which the antigen or APL is expressible, for a time and under conditions sufficient for the antigen or a processed form thereof to be presented by the antigen-presenting cells. Suitably, the inhibitor and/or the antigen or a polynucleotide from which they are expressible are in soluble form or in particulate form as described for example in Section 3.2.6. The amount of soluble or particulate antigen or APL to be placed in contact with antigen-presenting cells or their precursors can be determined empirically by routine methods known to persons of skill in the art. In some advantageous embodiments the antigen-presenting cells are incubated in the presence of aggrecan antigen for at least about 2, 3, 4, 5, 6, 7, 8, 12, 24, 36, 48, 60, 72, 84, 96 hours, or for less than about 96, 84, 72, 60, 48, 36, 24, 12, 8, 7, 6, 5, 4, 3 or 2 hours or even for less that about 60, 50, 40, 30, 20, 15, 10, 9, 8, 7, 6, 5, 4, 3 or 2 minutes. The time and dose of polypeptide or peptides necessary for the cells to optionally process and present aggrecan antigen may be determined using pulse-chase protocols in which exposure to aggrecan antigen is followed by a washout period and exposure to a read-out system *e.g*., ability to elicit suppression or inhibition of an aggrecan antigen-specific effector lymphocyte response. Once the optimal time and dose necessary for cells to express the aggrecan antigen or processed form thereof on their surface is determined, a protocol may be used to prepare cells and aggrecan antigen for inducing tolerogenic responses. Those of skill in the art will recognize in this regard that the length of time necessary for an antigen-presenting cell to present an antigen on its surface may vary depending on the antigen or form of antigen employed, its dose, and the antigen-presenting cell employed, as well as the conditions under which antigen loading is undertaken. These parameters can be determined by the skilled artisan using routine procedures.

In some embodiments, antigen-presenting cells are incubated with antigen or APL for about 1 to 6 h at 37° C, although it is also possible to expose antigen-presenting cells to antigen for the duration of incubation with growth factors and inhibitor. Usually, for purified antigens and peptides, 0.1-10 µg/mL is suitable for producing antigen-specific antigen-presenting cells. In illustrative examples, presentation of peptide antigen can be achieved using much shorter periods of incubation (*e.g*., about 5, 10, 15, 20, 30, 40, 50 minutes) using antigen at a concentration of about 10-20 µg/mL.

### 3.2.7.1 Sources of antigen-presenting cells and their precursors

Antigen-presenting cells or their precursors can be isolated by methods known to those of skill in the art. The source of such cells will differ depending upon the antigen-presenting cell required for modulating a specified immune response. In this context, the antigen-presenting cell can be selected from dendritic cells, macrophages, monocytes and other cells of myeloid lineage.

Typically, precursors of antigen-presenting cells can be isolated from any tissue, but are most easily isolated from blood, cord blood or bone marrow (Sorg et al., 2001. Exp Hematol 29:1289-1294; Zheng et al., 2000. J Hematother Stem Cell Res 9:453-464). It is also possible to obtain suitable precursors from diseased tissues such as rheumatoid synovial tissue or fluid following biopsy or joint tap (Thomas et al., 1994a, J Immunol 153:4016-4028; Thomas et al., 1994b, Arthritis Rheum 37(4)). Other examples include, but are not limited to liver, spleen, heart, kidney, gut and tonsil (Lu et al., 1994. J Exp Med 179:1823-1834; McIlroy et al., 2001. Blood 97:3470-3477; Vremec et al., 2000. JImmunol 159:565-573; Hart and Fabre, 1981. J Exp Med 154(2):347-361; Hart and McKenzie, 1988. J Exp Med 168(1):157-170; Pavli et al., 1990. Immunology 70(1):40-47).

Leukocytes isolated directly from tissue provide a major source of antigen-presenting cell precursors. Typically, these precursors can only differentiate into antigen-presenting cells by culturing in the presence or absence of various growth factors. According to the practice of the present invention, the antigen-presenting cells may be so differentiated from crude mixtures or from partially or substantially purified preparations of precursors. Leukocytes can be conveniently purified from blood or bone marrow by density gradient centrifugation using, for example, Ficoll Hypaque which eliminates neutrophils and red cells (peripheral blood mononuclear cells or PBMCs), or by ammonium chloride lysis of red cells (leukocytes or white blood cells). Many precursors of antigen-presenting cells are present in peripheral blood as non-proliferating monocytes, which can be differentiated into specific antigen-presenting cells, including macrophages and dendritic cells, by culturing in the presence of specific cytokines.

Tissue-derived precursors such as precursors of tissue dendritic cells or of Langerhans cells are typically obtained by mincing tissue (*e.g*., basal layer of epidermis) and digesting it with collagenase or dispase followed by density gradient separation, or selection of precursors based on their expression of cell surface markers. For example, Langerhans cell precursors express CD1 molecules as well as HLA-DR and can be purified on this basis.

In some embodiments, the antigen-presenting cell precursor is a precursor of macrophages. Generally these precursors can be obtained from monocytes of any source and can be differentiated into macrophages by prolonged incubation in the presence of medium and macrophage colony stimulating factor (M-CSF) (Erickson-Miller et al., 1990. Int J Cell Cloning 8:346-356; Metcalf and Burgess, 1982. J Cell Physiol 111:275-283).

In other embodiments, the antigen presenting cell precursor is a precursor of Langerhans cells. Usually, Langerhans cells can be generated from human monocytes or CD34⁺ bone marrow precursors in the presence of granulocyte/macrophage colony-stimulating factor (GM-CSF), IL-4/TNFα and TGFβ (Geissmann et al., 1998. J Exp Med 187:961-966; Strobl et al., 1997a. Blood 90:1425-1434; Strobl et al., 1997b. dv Exp Med Biol 417:161-165; Strobl et al., 1996. J Immunol 157:1499-1507).

In still other embodiments, the antigen-presenting cell precursor is a precursor of dendritic cells. Several potential dendritic cell precursors can be obtained from peripheral blood, cord blood or bone marrow. These include monocytes, CD34⁺ stem cells, granulocytes, CD33⁺CD11c⁺ DC precursors, and committed myeloid progenitors -described below.

### Monocytes:

Monocytes can be purified by adherence to plastic for 1-2 h in the presence of tissue culture medium (e.g., RPMI) and serum *(e.g.,* human or fetal calf serum), or in serum-free medium (Anton et al., 1998. Scand J Immunol 47:116-121; Araki et al., 2001. Br J Haematol 114:681-689; Mackensen et al., 2000. Int J Cancer 86:385-392; Nestle et al., 1998. Nat Med 4:328-332; Romani et al., 1996. J Immunol Meth 196:137-151; Thurner et al., 1999. J Immunol Methods 223:1-15). Monocytes can also be elutriated from peripheral blood (Garderet et al., 2001. J Hematother Stem Cell Res 10:553-567). Monocytes can also be purified by immunoaffinity techniques, including immunomagnetic selection, flow cytometric sorting or panning (Araki *et al*., 2001, *supra*; Battye and Shortman, 1991. Curr. Opin. Immunol. 3:238-241), with anti-CD14 antibodies to obtain CD14hi cells. The numbers (and therefore yield) of circulating monocytes can be enhanced by the in vivo use of various cytokines including GM-CSF(Groopman et al., 1987. N Engl J Med 317:593-598; Hill et al., 1995. J Leukoc Biol 58:634-642). Monocytes can be differentiated into dendritic cells by prolonged incubation in the presence of GM-CSF and IL-4 (Romani et al., 1994. J Exp Med 180, 83-93; Romani *et al.,* 1996, *supra*). A combination of GM-CSF and IL-4 at a concentration of each at between about 200 to about 2000 U/mL, more preferably between about 500 to about 1000 U/mL and even more preferably between about 800 U/mL (GM-CSF) and 1000 U/mL (IL-4) produces significant quantities of immature dendritic cells, *i.e*., antigen-capturing phagocytic dendritic cells. Other cytokines which promote differentiation of monocytes into antigen-capturing phagocytic dendritic cells include, for example, IL-13.

### CD34+ stem cells:

Dendritic cells can also be generated from CD34⁺ bone marrow derived precursors in the presence of GM-CSF, TNFα ± stem cell factor (SCF, c-kitL), or GM-CSF, IL-4 ± flt3L (Bai et al., 2002. Int J Oncol 20:247-53; Chen et al., 2001. Clin Immunol 98:280-292; Loudovaris et al., 2001. J Hematother Stem Cell Res 10:569-578). CD34⁺ cells can be derived from a bone marrow aspirate or from blood and can be enriched as for monocytes using, for example, immunomagnetic selection or immunocolumns (Davis et al., 1994. J Immunol Meth 175:247-257). The proportion of CD34⁺ cells in blood can be enhanced by the in vivo use of various cytokines including (most commonly) G-CSF, but also flt3L and progeriipoietin (Fleming et al., 2001. Exp Hematol 29:943-951; Pulendran et al., 2000. J Immunol 165:566-572; Robinson et al., 2000. J Hematother Stem Cell Res 9:711-720).

### Other myeloid progenitors:

DC can be generated from committed early myeloid progenitors in a similar fashion to CD34+ stem cells, in the presence of GM-CSF and IL-4/TNF. Such myeloid precursors infiltrate many tissues in inflammation, including rheumatoid arthritis synovial fluid (Santiago-Schwarz et al., 2001. J Immunol. 167:1758-1768). Expansion of total body myeloid cells including circulating dendritic cell precursors and monocytes, can be achieved with certain cytokines, including flt-3 ligand, granulocyte colony-stimulating factor (G-CSF) or progenipoietin (pro-GP) (Fleming *et al*., 2001, *supra*; Pulendran *et al*., 2000, *supra;* Robinson *et al*., 2000, *supra*). Administration of such cytokines for several days to a human or other mammal would enable much larger numbers of precursors to be derived from peripheral blood or bone marrow for *in vitro* manipulation. Dendritic cells can also be generated from peripheral blood neutrophil precursors in the presence of GM-CSF, IL-4 and TNFα (Kelly et al., 2001. Cell Mol Biol (Noisy-le-grand) 47, 43-54; Oehler et al., 1998. J Exp Med. 187:1019-1028). It should be noted that dendritic cells can also be generated, using similar methods, from acute myeloid leukemia cells (Oehler et al., 2000. Ann Hematol. 79, 355-62).

### Tissue DC precursors and other sources of APC precursors:

Other methods for DC generation exist from, for example, thymic precursors in the presence of IL-3 +/- GM-CSF, and liver DC precursors in the presence of GM-CSF and a collagen matrix. Transformed or immortalized dendritic cell lines may be produced using oncogenes such as v-myc as for example described by (Paglia *et al.,* 1993, *supra)* or by myb (Banyer and Hapel, 1999, *supra*; Gonda *et al.,* 1993, supra).

### Circulating DC precursors:

These have been described in human and mouse peripheral blood. One can also take advantage of particular cell surface markers for identifying suitable dendritic cell precursors. Specifically, various populations of dendritic cell precursors can be identified in blood by the expression of CD11c and the absence or low expression of CD14, CD19, CD56 and CD3 (O'Doherty et al., 1994. Immunology 82, 487-493; O'Doherty et al., 1993. J Exp Med 178:1067-1078). These cells can also be identified by the cell surface markers CD13 and CD33 (Thomas et al., 1993b. J Immunol 151(12):6840-6852). A second subset, which lacks CD14, CD19, CD56 and CD3, known as plasmacytoid dendritic cell precursors, does not express CD11c, but does express CD123 (IL-3R chain) and HLA-DR (Farkas et al., 2001. Am J Pathol 159. 237-243; Grouard et al., 1997. J Exp Med 185:1101-1111; Rissoan et al., 1999. Science 283:1183-1 186). Most circulating CD11c⁺ dendritic cell precursors are HLA-DR⁺, however some precursors may be HLA-DR-. The lack of MHC class II expression has been clearly demonstrated for peripheral blood dendritic cell precursors (del Hoyo et al., 2002. Nature 415:1043-1047).

Optionally, CD33⁺CD14^{-/lo} or CD11c⁺HLA-DR⁺, lineage marker-negative dendritic cell precursors described above can be differentiated into more mature antigen-presenting cells by incubation for 18-36 h in culture medium or in monocyte conditioned medium (Thomas *et al*., 1993b, *supra*; Thomas and Lipsky, 1994. J Immunol 153:4016-4028) (O'Doherty *et al*., 1993, *supra).* Alternatively, following incubation of peripheral blood non-T cells or unpurified PBMC, the mature peripheral blood dendritic cells are characterized by low density and so can be purified on density gradients, including metrizamide and Nycodenz (Freudenthal and Steinman, 1990. Proc Natl Acad Sci USA 87:7698-7702; Vremec and Shortman, 1997. J Immunol 159:565-573), or by specific monoclonal antibodies, such as but not limited to the CMRF-44 mAb (Fearnley et al., 1999. Blood 93:728-736; Vuckovic et al., 1998. Exp Hematol 26:1255-1264). Plasmacytoid dendritic cells can be purified directly from peripheral blood on the basis of cell surface markers, and then incubated in the presence of IL-3 (Grouard *et al*., 1997, *supra*; Rissoan *et al*., 1999, *supra*). Alternatively, plasmacytoid DC can be derived from density gradients or CMRF-44 selection of incubated peripheral blood cells as above.

In general, for dendritic cells generated from any precursor, when incubated in the presence of activation factors such as monocyte-derived cytokines, lipopolysaccharide and DNA containing CpG repeats, cytokines such as TNFα, IL-6, IFN-α, IL-1β, necrotic cells, re-adherence, whole bacteria, membrane components, RNA or polyIC, immature dendritic cells will become activated (Clark, 2002. J Leukoc Biol 71:388-400; Hacker et al., 2002. Immunology 105:245-251; Kaisho and Akira, 2002. Biochim Biophys Acta 1589:1-13; Koski et al., 2001. Crit Rev Immunol 21:179-189). This process of dendritic cell activation is inhibited in the presence of NF-κB inhibitors (O'Sullivan and Thomas, 2002. J Immunol 168:5491-5498).

### 3.2.8 In vivo antigen-presenting cell embodiments

In other embodiments, the number of aggrecan-specific tolerogenic antigen-presenting cells in the subject is increased by co-administering to the subject an NF-κB inhibitor and/or a mTOR inhibitor, and/or a Syk inhibitor (sometimes collectively referred to herein as "inhibitors)"), together with an aggrecan antigen that corresponds in whole, or in part, to an aggrecan polypeptide, including citrullinated forms thereof. The inhibitor(s) and/or the antigen may be in nucleic acid form (*i*.*e*., in which they are produced from a nucleic acid construct) or in non-nucleic acid form. The inhibitor(s) and the antigen may be co-administered in soluble or in particulate form (*e.g.*, the antigen and the inhibitor are both in soluble form, or one of the antigen or inhibitor is in soluble form and the other is in particulate form, or the antigen and the inhibitor are both in particulate form). In specific embodiments, both the inhibitor(s) and the antigen are co-administered in particulate form. Desirably, the inhibitor(s) and the antigen are contained in the same particle. Once administered, the particles are taken up by antigen-presenting cells in the subject (*e.g*., by phagocytosis or endocytosis), and the inhibitor(s)/antigen payload is released into the interior of the cell.

### 3.2.9 Ex vivo regulatory lymphocyte embodiments

Aggrecan-specific antigen-presenting cells obtained or prepared *ex vivo* according to Section 3.2.7 are useful for modulating other immune cells, including T lymphocytes and B lymphocytes, and particularly for producing T lymphocytes and B lymphocytes that exhibit tolerance or anergy to aggrecan antigen (including citrullinated forms thereof). The efficiency of inducing lymphocytes, especially T lymphocytes, to exhibit tolerance/anergy for aggrecan antigen can be determined by assaying immune responses to that antigen including, but not limited to, assaying T lymphocyte cytolytic activity *in vitro* using for example the aggrecan-specific antigen-presenting cells as targets of aggrecan-specific cytolytic T lymphocytes (CTL); assaying aggrecan-specific T lymphocyte proliferation and cytokine response; and assaying T regulatory suppressive function (see, *e.g.,* Vollenweider and Groseurth, 1992, J. Immunol. Meth. 149:133-135), measuring B cell response to the antigen using, for example, ELISPOT assays, and ELISA assays; interrogating cytokine profiles; or measuring delayed-type hypersensitivity (DTH) responses by test of skin reactivity to the aggrecan antigen (see, *e.g.,* Chang et al. (1993, Cancer Res. 53:1043-1050). Other methods known to practitioners in the art, which can detect the presence of antigen on the surface of antigen-presenting cells after exposure to the antigen, are also contemplated by the present invention.

The tolerance/anergy-eliciting antigen-presenting cells have the capacity to efficiently present the aggrecan antigen, or processed form thereof, on one or both of MHC class I molecules and MHC class II molecules. Accordingly, both CD4⁺ T helper lymphocytes and CTL may be rendered tolerant/anergic by the agg-tolAPC of the invention. Moreover, the agg-tolAPC of the present invention can be charged with multiple aggrecan antigens on multiple MHCs to yield polyclonal or oligoclonal tolerance/anergy of T lymphocytes.

Thus, the present invention also provides aggrecan-specific tolerant/anergic or regulatory B or T lymphocytes, especially T lymphocytes, which fail to respond in an antigen-specific fashion to representation of the aggrecan antigen or which actively regulate prior immune responses or subsequent priming to the aggrecan antigen. The regulation is generally long lived and is maintained, for example, for at least about 3 months, and suitably years.

In specific embodiments, aggrecan-specific regulatory T lymphocytes are produced by contacting an aggrecan-specific antigen-presenting cell as defined above with a population of T lymphocytes, which may be obtained from any suitable source such as spleen or tonsil/lymph nodes but is preferably obtained from peripheral blood. The T lymphocytes can be used as crude preparations or as partially purified or substantially purified preparations, which are suitably obtained using standard techniques as, for example, described in "Immunochemical Techniques, Part G: Separation and Characterization of Lymphoid Cells" (Meth. in Enzymol. 108, Edited by Di Sabato et al., 1984, Academic Press). This includes rosetting with sheep red blood cells, passage across columns of nylon wool or plastic adherence to deplete adherent cells, immunomagnetic or flow cytometric selection using appropriate monoclonal antibodies as described (Cavanagh et al., 1998. Blood 92(5)mhibitor1598-1607; Thomas et al., 1993. J Immunol 150inhibitor821-834).

The preparation of T lymphocytes is contacted with the aggrecan-specific antigen-presenting cells as described for example in Section 3.2.7 for an adequate period of time for inducing regulatory function in the T lymphocytes towards the antigen or antigens presented by those antigen-presenting cells. This period will usually be at least about 1 day, and up to about 5 days. Generally, the proliferation of regulatory T lymphocytes produced after this procedure is short-lived, depending on the IL-2 concentration in the culture. The regulatory lymphocytes so prepared will typically produce IL-10 and/or other regulatory cytokines in an antigen-specific manner.

In some embodiments, a population of aggrecan-specific antigen-presenting cell precursors is cultured in the presence of a heterogeneous population of T lymphocytes, which is suitably obtained from peripheral blood, and at least one inhibitor, as descibed for example in Sections 3.2.3, 3.2.4 and 3.2.5, together with an aggrecan antigen, or with a polynucleotide from which the aggrecan antigen is expressible. These cells are cultured for a period of time and under conditions sufficient for: (1) the precursors to differentiate into antigen-presenting cells; (2) the level and/or functional activity of NF-κB and/or mTOR and/or Syk in those antigen-presenting cells to be abrogated or otherwise reduced; (3) the aggrecan antigen, or processed form thereof, to be presented by the antigen-presenting cells; and (4) the antigen-presenting cells to induce a subpopulation of the T lymphocytes to exhibit regulatory function towards the aggrecan antigen, wherein the subpopulation is characterized by supression of an aggrecan-specific T cell response. This can occur using Ficoll-purified PBMC plus aggrecan antigen plus inhibitor (*e.g*., NF-κB and/or mTOR and/or Syk inhibitor(s)) since such a preparation contains both antigen-presenting cell precursors (*e.g*., dendritic cell precursors) and T lymphocytes.

Suitably, the aggrecan-specific antigen-presenting cells so produced induce one or more types of antigen-specific regulatory lymphocytes, especially regulatory T lymphocytes (also referred to herein as "Treg"). Several populations or subsets of Treg have been described (Shevach, 2006. Immunity 25:195-201; Lee et al. 2011, Adv Immunol. 112:25-71; Sakaguchi, 2011. Methods Mol Biol.707:3-17), including, for example, a naturally-ocurring, distinct population of CD4⁺CD25⁺Foxp3⁺ Treg known as natural Treg (nTreg) develop in the thymus and are present in healthy individuals from birth. The specificity of the T cell receptor (TCR) of nTreg is mainly self-reactive. Additionally, a population of CD4⁺CD25⁺Foxp3⁺ Treg can be induced *in vivo* in the periphery under various conditions, such as during certain defined conditions of antigen presentation and cytokine stimulation, and can induce tolerance (reviewed in Roncarolo et al., 2007. Nat Rev Immunol. 7: 585-598). Additional subsets of inducible Treg have been reported, including T*ₕ*3 cells and Trl cells, and CD4⁺CD25⁺ Treg. T*ₕ*3 cells produce TGF β and variable amounts of IL-4 and IL-10 (Chen et al., 1994. Science 265(5176):1237-1240). Tr1 cells secrete IL-10 (Groux et al., 1997. Nature 389(6652):737-742). CD8⁺ Treg have also been reported, which express low levels of Foxp3 (*see,* for example, Smith et al., 2008. Trends Immunol. 29(7):337-342; Guillonneau et al., 2010. Curr Opin Organ Transplant 15:751-756; Filaci et al., 2011. Autoimmunity 44(1):51-57; Picarda et al., 2011. Immunotherapy 3(4 Suppl): 35-37).

Numerous methods for isolating and expanding Treg are known in the art and include for example those disclosed by Brusko et al. (2008. Immunological Reviews 223:371-390), Gregori et al. (2011. Methods Mol Biol.677:31-46), Gregori et al. (2007. Methods Mol Biol. 380:83-105), Ménoret et al., 2011. Methods Mol Biol. 677: 63-83), Wang, L. (2010. Methods Mol Biol. 595:403-412) and Daniel et al. (2011. Methods Mol Biol. 707:173-185).

Thus, the present invention provides means to generate large quantities of antigen-specific lymphocytes by stimulating lymphocytes with aggrecan-specific antigen-presenting cells of the invention *e.g*., for at least about 3 days, suitably at least about 5 days.

Aggrecan-specific tolerance can also be achieved by expressing in B lymphocytes (also referred to herein as B cells) a nucleic acid molecule that encodes an aggrecan antigen, wherein the expression of the nucleic acid molecule leads to presentation of the antigen or processed form thereof on the surface of the B lymphocytes.

In specific embodiments, the nucleic acid molecule further encodes an immunoglobulin (*e.g.*, IgG) or a fragment of an immunoglobulin (*e.g.*, Fv, Fab, Fab' and F(ab')₂ immunoglobulin fragments, immunoglobulin heavy chain *etc.*) fused directly or indirectly to the aggrecan antigen (*e.g*., adjacent to the C-terminus of the antigen). In illustrative examples of this type, immunoglobulins (*e.g*., IgG) are used as carriers for presentation of an aggrecan antigen to the immune system, wherein the aggrecan antigen is fused at the N-terminus of the immunoglobulin heavy chain scaffold to form a fusion protein. The fusion protein is produced in 'activated' B cells by transducing (*e.g.*, by retroviral infection) hematopoietic cells (*e*.*g*., bone marrow-derived cells) or lymphoid cells (*e.g.*, B cells including B cell blasts that have been stimulated with lipopolysaccharide) with a nucleic acid molecule from which the fusion protein is producible to thereby produce tolerogenic APC. The aggrecan antigen or processed form thereof is presented by host major histocompatibility complex (MHC) on these B cells leading to tolerance because of the presentation and long-term *in vivo* expression of immunoglobulin (*e.g.*, IgG) fusion proteins. Suitably, these B cells express MHC class II and co-stimulatory molecules (*e.g*., B7.1 and B7.2), which helps to recruit and trigger regulatory T cells that express CD25 *via* binding to CTLA-4. Immunoglobulin fusions of this type are described for example in U.S. Pat. Appl. Pub. No. 2002/0048562.

In illustrative examples of this type, antigen-specific tolerance is induced using antigen-Ig fusion protein delivered *via* a retroviral vector in B cells as described for example by Scott and colleagues (Zambidis et al., 1996. Proc Natl Acad Sci U S A 93(10):5019-5024; Kang et al., 1999. Proc Natl Acad Sci U S A 96(15):8609-8614; Agarwal et al., 2000. Clin Invest 106(2):245-252; El-Amine et al., 2002. Int Immunol 14(7):761-766; Melo et al., 2002. J Immunol 168(9):4788-4795; Song et al., 2004. Gene Ther 11(20): 1487-1496; Lei et al., 2005. Blood 105(12):4865-4870; Xu et al., 2006. Mol Ther 13(1):42-48; Satpute et al., 2007. Arthritis Rheum 56(5):1490-1496; Scott, 2010. Haemophilia 16(102):89-94).

This B cell-mediated gene therapy approach has been used successful in disease models such as experimental autoimmune uveitis (EAU) (Agarwal *et al*., 2000, *supra),* EAE [induced either by myelin basic protein (MBP) or by myelin oligodendrocyte glycoprotein (MOG)] (Melo *et al*., 2002, *supra*), and the non-obese diabetic (NOD) mouse model of diabetes (Melo *et al*., 2002, *supra;* Song *et al*., 2004, *supra*). This approach has also been successful in inducing tolerance to factor VIII inhibitors in hemophilia A (Lei *et al*., 2005, *supra*) and (in combination with bone marrow (BM) transplantation) in the treatment of EAE (Xu *et al*., 2006, *supra*). B cell-mediated gene therapy has also been used successfully in the adjuvant-induced arthritis (AA) model (Satpute *et al*., 2007, *supra*).

The fusion protein can be delivered by any suitable means including administering a nucleic acid molecule from which the fusion protein is producible to a subject in need thereof. In specific embodiments, the subject is administered hematopoietic or lymphoid cells, which are transduced with the nucleic acid molecule.

In some embodiments, the B lymphocytes expressing the aggrecan antigen-encoding nucleic acid molecule are regulatory B lymphocytes (also referred to herein as "Breg" cells) an include but are not limited to a CD1d^{highCD5+} B cell subset that regulates T cell mediated inflammatory and immune responses through secretion of IL-10, as disclosed for example by Tedder in U.S. Pat. Appl. Pub. No. 2011/013566.

### 3.2.10 MHC-peptide complex embodiments

The present invention also contemplates the use of major histocompatibility complex (MHC)-peptide complexes for eliciting aggrecan-specific tolerogenesis. These complexes generally comprise an aggrecan peptide (*e.g.*, a *cit*-aggrecan peptide) and an isolated (*e.g.*, soluble) MHC component having an antigen-binding site, wherein the antigen is associated with the antigen-binding site. MHC-peptide complexes effectively substitute for the antigen-presenting cell and cause non-responsiveness in autoreactive antigen-specific T-lymphocytes and other cells of the immune system. The MHC component can be either a Class I or a Class II molecule. If desired, the transmembrane and/or intracellular domains of MHC molecules can also be included. The association between the peptide antigen and the antigen binding sites of the MHC protein can be by covalent or by non-covalent bonding.

The MHC molecules may be purified using any suitable protocol, illustrative examples of which involve solubilization of cells (e.g., lymphocytes) by treatment with papain, by treatment with 3M KC1, or by treatment with detergent. In an illustrative protocol, detergent extraction of MHC (e.g., Class II) molecules from lymphocytes is used followed by affinity purification. Detergent can then be removed by dialysis or selective binding beads, *e.g.,* Bio Beads. Alternatively, the amino acid sequence of each of a number of MHC class I and II molecules are known, and their corresponding coding sequences have been cloned, thus permitting recombinant production of the MHC proteins.

The aggrecan antigen is generally a peptide (e.g., about 8 to about 15 amino acids in length), which is predicted to by the MHC molecule using standard algorithms known in the art. Alternatively, peptides (e.g., about 8 to about 15 amino acids in length) corresponding to overlapping portions of the aggrecan amino acid sequence can be used. The peptide(s) may be non-citrullinated or citrullinated.

The elements of the complex can be associated by standard means known in the art. The antigenic peptides can be associated non-covalently with the pocket portion of the MHC protein by, for example, mixing the two components. They can also be covalently bound using standard procedures by, for example, photo affinity labeling, (see e.g., Hall et al., 1985. Biochemistry 24:5702-5711).

Complexes comprising transmembrane-containing MHC molecules are conveniently administered after being incorporated in lipid monolayers or bilayers. Typically liposomes are used for this purpose but any form of lipid membrane, such as planar lipid membranes or the cell membrane of a cell (e.g., a red blood cell) may be used. The complexes are also conveniently incorporated into micelles.

Liposomes can be prepared according to standard methods, as described for example in Section 3.2.6.2. However, if the transmembrane region is deleted, the complex can be administered in a manner conventionally used for peptide-containing pharmaceuticals.

Micelles are commonly used in the art to increase solubility of molecules having nonpolar regions and may be used advantageously to incorporate the MHC-peptide complexes using methods well known in the art (see, e.g., Remington's Pharmaceutical Sciences, Mack Publishing Company, Philadelphia, Pa., 17th ed. (1985)). Generally, complex-containing micelles are prepared using standard surfactants or detergents.

Exemplary methods of producing MHC-peptide complexes are described for example in U.S. Pat. Appl. Pub. No. 2003/0068363.

### 3.2.11 Chimeric constructs based on the Ligand Epitope Antigen Presenting System

The present invention also contemplates the use of the Ligand Epitope Antigen Presenting System (L.E.A.P.S.) technology for eliciting aggrecan-specific tolerogenesis. This technology converts a small peptide containing an autoantigen into a tolerogen by attaching it to an immune or T cell binding ligand (I/TCBL) and presenting it to an immune cell (Cihakova et al., 2008. Int Immunopharmacol 8:624; Rosenthal et al., 1998. Vaccine 17:535-542; Goel et al., 2003. Vaccine 21:4410; Goel et al., 2005. Front Biosci 966; Zimmerman et al., 1996. Vac Res 5:91-102; Zimmerman et al., 1996. Vac Res 5:103-13; Zimmerman et al., 1985. DNA. Med Virol 15:215-22. Charoenvit et al., 2004. Antimicrob Agents Chemother 48:2455; Charoenvit et al., 2004. Vaccine 10:2368; and Zimmerman et al., 2001. Vaccine 19(32):4750-4759). The I/TCBLs are generally derived from immune system molecules known or suspected to bind to T cells, examples of which include portions of MHC Classes I and II or accessory molecules such as β2-microglobulin, portions of LFA-3, portions of the Fc region of the heavy chain of immunoglobulins, and Ia+ molecules. In specific embodiments, β2 microglobulin (J) is used as the I/TCBL and linked to an autoantigen of interest. Fusion constructs of this type have been used successfully to arrest disease progression in a collagen-induced arthritis model, as described for example by Zimmerman et al. (2010, Int Immunopharmacol 10:412-421) with increased IL-12p70 and IL-10 as well as reduced IL-17 and TNFα in the serum of treated animals.

Exemplary methods for making L.E.A.P.S. constructs are disclosed for example in U.S. Pat. No. 5,652,342 and U.S. Pat. Appl. No. 2011/0098444.

### 3.3 Cell based therapy or prophylaxis

Aggrecan-specific antigen-presenting cells as described for example in Section 3.2.7, or aggrecan-specific T or B regulatory lymphocytes or tolerogenic B lymphocytes as described for example in Section 3.2.9 can be administered to a patient, either by themselves or in combination, for suppressing an immune response to an aggrecan antigen, including citrullinated forms thereof. These cell based compositions are useful for treating or preventing joint damage in affected or predisposed subjects including subjects with early RA and subjects at risk of developing RA, suitably before the disease progresses to the chronic and debilitating form of RA. The cells of the invention can be introduced into a patient by any means (*e.g*., injection), which produces an antigen-specific tolerogenic response to aggrecan antigen. The cells may be derived from the patient (*i*.*e*., autologous cells) or from an individual or individuals who are MHC matched or mismatched (*i*.*e*., allogeneic) with the patient. Typically, autologous cells are injected back into the patient from whom the source cells were obtained. The injection site may be subcutaneous, intraperitoneal, intramuscular, intradermal, intravenous or intralymphoid. The cells may be administered to a patient already suffering from joint damage (including subjects with early RA and subjects at risk of developing RA) or who is predisposed to joint damage in sufficient number to result in a clinical improvement in the subject or to prevent joint damage or the symptoms of joint damage in a subject at risk of developing joint damage (including subjects at risk of developing RA). The number of cells injected into the patient in need of the treatment or prophylaxis may vary depending on *inter alia,* the aggrecan antigen or antigens and size of the individual. This number may range for example between about 10³ and 10¹¹, and usually between about 10⁵ and 10⁷ cells (*e.g.*, in the form blood, PMBC or purified dendritic cells, T lymphocytes, hematopoietic or lymphoid cells, B lymphocytes etc.). Single or multiple (2, 3, 4 or 5) administrations of the cells can be carried out with cell numbers and pattern being selected by the treating physician. The cells should be administered in a pharmaceutically acceptable carrier, which is non-toxic to the cells and the individual. Such carrier may be the growth medium in which the cells were grown, or any suitable buffering medium such as phosphate buffered saline. The cells may be administered alone or as an adjunct therapy in conjunction with other therapeutics known in the art for the treatment or prevention of unwanted immune responses for example but not limited to glucocorticoids, methotrexate, D-penicillamine, hydroxychloroquine, gold salts, sulfasalazine, TNFα or IL-1 inhibitors, and/or other forms of specific immunotherapy.

### 3.4 Pharmaceutical compositions

In accordance with the present invention, aggrecan antigens described for example in Section 3.2.1, aggrecan APL as described for example in Section 3.2.2, NF-κB inhibitors as described for example in Section 3.2.3, mTOR inhibitors as described for example in Section 3.2.4, Syk inhibitors as described for example in Section 3.2.5, immunomodulating particles described for example in Section 3.2.6, antigen-presenting cells as described for example in Section 3.2.7, regulatory T or B lymphocytes or tolerogenic B lymphocytes as described for example in Section 3.2.9, MHC-peptide complexes as described for example in Section 3.2.10, and chimeric constructs described for example in Section 3.2.11 (also collectively referred to herein as "immune modulators") are useful in compositions and methods for suppressing an immune response to an aggrecan antigen, including citrullinated forms thereof, and are especially useful for treating or preventing joint damage in affected or predisposed subjects including subjects with early RA and subjects at risk of developing RA.

The immune modulator-containing compositions of the present invention are typically in the form of pharmaceutical compositions, which may comprise a pharmaceutically acceptable carrier or diluent. Depending on the specific conditions being treated, the immune modulator may be formulated and administered systemically, topically or locally. Techniques for formulation and administration may be found in "Remington's Pharmaceutical Sciences," Mack Publishing Co., Easton, Pa., latest edition. Suitable routes may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. For injection, the immune modulators of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art. Intra-muscular and subcutaneous injection is appropriate, for example, for administration of immunogenic compositions, vaccines and DNA vaccines.

The immune modulators can be formulated readily using pharmaceutically acceptable carriers well known in the art into dosages suitable for oral administration. Such carriers enable the compounds of the invention to be formulated in dosage forms such as tablets, pills, capsules, liquids, gels, syrups, slurries, suspensions and the like, for oral ingestion by a patient to be treated. These carriers may be selected from sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, or polyvinylpyrrolidone (PVP). If desired, disintegrating agents may be added, such as the cross-linked polyvinyl pyrrolidone, agar, or alginic acid or a salt thereof such as sodium alginate. Such compositions may be prepared by any of the methods of pharmacy but all methods include the step of bringing into association one or more immune modulators as described above with the carrier, which constitutes one or more necessary ingredients. In general, the pharmaceutical compositions of the present invention may be manufactured in a manner that is itself known, e.g., by means of conventional mixing, dissolving, granulating, dragee-making, levigating, emulsifying, encapsulating, entrapping or lyophilizing processes.

Dragee cores are provided with suitable coatings. For this purpose, concentrated sugar solutions may be used, which may optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments may be added to the tablets or dragee coatings for identification or to characterize different combinations of active compound doses.

Pharmaceutical which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

Dosage forms of the drugs of the invention may also include injecting or implanting controlled releasing devices designed specifically for this purpose or other forms of implants modified to act additionally in this fashion. Controlled release of an immune modulator of the invention may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, controlled release may be achieved by using other polymer matrices, liposomes or microspheres.

The immune modulators of the present invention may also be administered to the respiratory tract as a nasal or pulmonary inhalation aerosol or solution for a nebulizer, or as a microfine powder for insufflation, alone or in combination with an inert carrier such as lactose, or with other pharmaceutically acceptable excipients. In some particulate embodiments of the present invention, the particles of a formulation may advantageously have diameters of less than 50 µm, suitably less than 10 µm.

In some particulate embodiments, the immune modulators are administered for active uptake by APC, for example by phagocytosis, as described for example in U.S. Pat. No. 5,783,567 (Pangaea). The phagocytosis by these cells may be improved by maintaining a particle size typically below about 20 µm, and preferably below about 11 µm.

In specific particulate embodiments, immune modulators in particulate form are delivered directly into the bloodstream (*i*.*e*., by intravenous or intra-arterial injection or infusion) if uptake by the phagocytic cells of the reticuloendothelial system (RES), including liver and spleen, is desired. Alternatively, one can target, *via* subcutaneous injection, take-up by phagocytic APC of the draining lymph nodes. Particles can also be introduced intradermally (*i*.*e*., to the APCs of the skin, such as dendritic cells and Langerhans cells) for example using ballistic or microneedle delivery. Illustrative particle-mediated delivery techniques include explosive, electric or gaseous discharge delivery to propel carrier particles toward target cells as described, for example, in U.S. Pat. Nos. 4,945,050, 5,120,657, 5,149,655 and 5,630,796. Non-limiting examples of microneedle delivery are disclosed in International Publication Nos. WO 2005/069736 and WO 2005/072630 and U.S. Pat. Nos. 6,503,231 and 5,457,041.

In other specific particulate embodiments, the route of particle delivery is *via* the gastrointestinal tract, *e*.*g*., orally. Alternatively, the particles can be introduced into organs such as the lung (*e.g.*, by inhalation of powdered microparticles or of a nebulized or aerosolized solution containing the microparticles), where the particles are picked up by the alveolar macrophages, or may be administered intranasally or buccally. Once a phagocytic APC phagocytoses the particle, the NF-κB inhibitor and optionally the aggrecan antigen are released into the interior of the cell.

Suitable routes for particle delivery include for example oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. For injection, particles may be formulated in aqueous solutions, suitably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

Pharmaceutical formulations for parenteral administration of particles include aqueous solutions of particles in water-soluble form. Additionally, suspensions of the particles may be prepared as appropriate oily injection suspensions. Suitable lipophilic solvents or vehicles include fatty oils such as sesame oil, or synthetic fatty acid esters, such as ethyl oleate or triglycerides. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Exemplary methods for particulate delivery of inhibitor (*e*.*g*., NF-κB inhibitor) and antigen are described for example in in U.S. Pat. Appl. Pub 20100151000.

The immune modulators of the invention (*e.g*., inhibitors(s), antigen, APCs, lymphocytes, fusion proteins, particles *etc*.) may be provided as salts with pharmaceutically compatible counterions. Pharmaceutically compatible salts may be formed with many acids, including but not limited to hydrochloric, sulfuric, acetic, lactic, tartaric, malic, succinic, *etc.* Salts tend to be more soluble in aqueous or other protonic solvents that are the corresponding free base forms.

Pharmaceutical compositions suitable for use in the present invention include compositions wherein the immune modulators are contained in an effective amount to achieve their intended purpose. The dose of an immune modulator administered to a patient should be sufficient to achieve a clinical improvement in the subject or to prevent joint damage or the symptoms of joint damage in a subject at risk of developing joint damage (including subjects at risk of developing RA). The quantity or dose frequency of the immune modulator(s) to be administered may depend on the subject to be treated inclusive of the mode of administration, age, sex, weight and general health condition thereof. In this regard, precise amounts of the immune modulator(s) for administration will depend on the judgment of the practitioner. In determining the effective amount of the immune modulator(s) to be administered in the treatment or prophylaxis of joint damage, the practitioner may evaluate inflammation, pro-inflammatory cytokine levels, lymphocyte proliferation, cytolytic T lymphocyte activity and regulatory T lymphocyte function that is specific to aggrecan. In any event, those of skill in the art may readily determine suitable dosages of immune modulator(s).

For any immune modulator used in the methods of the present invention, the therapeutically effective dose can be estimated initially from cell culture assays. For example, a dose can be formulated in animal models to achieve a circulating concentration range that includes the IC50 as determined in cell culture (*e.g*., the concentration of a test agent, which achieves a half-maximal reduction in NF-κB activity or mTOR activity or Syk activity, a maximal increase in aggrecan-specific antigen-presenting cells etc.). Such information can be used to more accurately determine useful doses in humans.

Toxicity and therapeutic efficacy of such immune modulators can be determined by standard pharmaceutical procedures in cell cultures or experimental animals, *e.g*., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Immune modulators that exhibit large therapeutic indices are preferred. The data obtained from these cell culture assays and animal studies can be used in formulating a range of dosage for use in human. The dosage of such immune modulators lies preferably within a range of circulating concentrations that include the ED50 with little or no toxicity. The dosage may vary within this range depending upon the dosage form employed and the route of administration utilized. The exact formulation, route of administration and dosage can be chosen by the individual physician in view of the patient's condition. (See for example Fingl et al., 1975, in "The Pharmacological Basis of Therapeutics", Ch. 1 p1).

Dosage amount and interval may be adjusted individually to provide plasma levels of the immune modulator, which are sufficient to enhance suppression of an immune response or tolerogenesis to an aggrecan antigen. Usual patient dosages of non-cellular immune modulators of the present invention for systemic administration range from 0.0001-2000 mg/day, commonly from 0.0001-250 mg/day, and typically from 0.001-150 mg/day.

The compositions of the invention may be administered over a period of hours, days, weeks, or months, depending on several factors, including the severity of the condition (*e.g*., joint damage including in RA) being treated, whether a recurrence of the condition is considered likely, etc. The administration may be constant, *e.g*., constant infusion over a period of hours, days, weeks, months, etc. Alternatively, the administration may be intermittent, *e.g*., immune modulators may be administered once a day over a period of days, once an hour over a period of hours, or any other such schedule as deemed suitable. In specific embodiments, the immune modulators of the present invention are administered on a regular regimen such as weekly, biweekly, monthly, quarterly, semi-annually or annually by one of the following routes: intradermally, intramuscularly or subcutaneously as well as cutaneous transdermal or nasal delivery in amounts of from 1-100, usually 10-50, micrograms per kilogram of body weight.

### 3.5 Methods for assessing antigen-specific tolerogenesis

The efficiency of inducing effector lymphocytes, especially effector T lymphocytes, to exhibit tolerance to an aggrecan antigen can be determined by assaying immune responses to that antigen including, but not limited to, assaying effector T lymphocyte cytolytic activity *in vitro* using for example aggrecan-specific antigen-presenting cells as targets of antigen-specific cytolytic T lymphocytes (CTL); assaying aggrecan-specific T lymphocyte proliferation, apoptosis or cytokine production (see, *e.g.,* Vollenweider and Groscurth, 1992. J. Immunol. Meth. 149:133-135), antigen-specific suppression of effector T cells, measuring B cell antibody response to the antigen using, for example, ELISPOT assays, and ELISA assays; interrogating cytokine profiles; or measuring delayed-type hypersensitivity (DTH) responses by test of skin reactivity to a specified antigen (see, *e.g.,* Chang et al. 1993, Cancer Res. 53, 1043-1050).

In some embodiments, the antigen-specific tolerance/anergy induced by the aggrecan-specific regulatory T lymphocytes reflects the inability of the antigen-specific immune effector cells (*e.g*., antigen-specific effector T and/or B lymphocytes) to respond to subsequent restimulation with the aggrecan antigen. These antigen-specific regulatory lymphocytes may be characterized by production of cytokines, such as IL-10 or IFN-γ in an antigen-specific manner. IL-10 and IFN-γ are cytokines with potent immunosuppressive properties produced by CD25⁻ CD127^{dim} CD4⁺ induced regulatory T cells in humans. Thus, in some embodiments, the presence of anergic T lymphocytes may be determined by assaying cytokine production using standard assays known in the art, such as intracellular staining (Haringer et al., 2009. J Exp Med 206(5):1009-1017). Alternatively there may be changes in the proportion of CD4⁺ regulatory T cells expressing CD25 and FoxP3, which are CD127dim (Fazekas de St Groth B et al., 2011. Methods Mol Biol 707:263-79).

Alternatively, or in addition, antigen-specific tolerance/anergy may be determined indirectly by giving the treated subject at least about one month after administration of the immune modulator/tolerogenic composition, a radiographic test that measures alleviation or healing of the joint damage, or slowing down of the progression of joint damage, including its signs and symptoms and/or structural damage, as compared to baseline prior to the administration, wherein the amount of immune modulator/tolerogenic composition administered is effective in achieving alleviation or healing of the joint damage, or slowing down of the progression of joint damage, including its signs and symptoms and/or structural damage, indicating that the subject has been successfully treated for the joint damage. In specific embodiments, the radiographic testing after administering the immune modulator/tolerogenic composition occurs at least about two months, at least about 10 weeks, at least about three months, at least about four months, at least about five months, at least about 24 weeks, at least about six months, or at least about 52 weeks after administering the immune modulator/tolerogenic composition. In illustrative examples of this type, the test measures a total modified Sharp score.

In order that the invention may be readily understood and put into practical effect, particular preferred embodiments will now be described by way of the following non-limiting examples.

### EXAMPLES

### EXAMPLE 1

### T CELLS PROLIFERATE POORLY BUT PRODUCE CYTOKINES IN RESPONSE TO CITRULLINATED AUTOANTIGENIC PEPTIDES

Citrullinated or unmodified peptide antigens were synthesized from the fibrinogen, vimentin, collagen and aggrecan protein sequences which had been identified, either based on predicted binding capacity to RA-associated DR molecules in a molecular model positioning citrulline at P4, or through previous studies in HLA-DR4-IE-transgenic mice (Table 5) (Hida et al., 2004. J. Autoimmun. 23:141-150; Hill J et al., 2008. J. Exp. Med. 205:967-979; von Delwig et al., 2010. Arthritis Rheum. 62:143-149).

**TABLE 5 SEQUENCES OF THE PEPTIDES USED IN THE EXAMPLES**

| **Native peptide sequences** | **Citrullinated peptide sequences** | **Human native protein** |
|---|---|---|
| QDFTNRINKLKNS | QDFTNCitINKLKNS | Fibrinogen-α 79-91 |
| VVLLVATEGRVRVNSAYQDK | VVLLVATEGCitVRVNSAYQDK | Aggrecan 84-103 |
| SAVRARSSVPGVR | SAVRACitSSVPGVR | Vimentin 66-78 |
| QYMRADQAAGGLR | QYMCitADQAAGGLR | Collagen II 1237-1249 |

In total, 21 SE⁺ RA patients and 6 SE⁺ healthy controls were studied. All except 4 RA patients were also ACPA⁺ (Table 6). Forty three percent of the RA patients were non-smokers, 38% past and 19% current smokers. One healthy control is a current smoker, and 2 have a family history of RA.The proliferative response of peripheral blood mononuclear cells (PBMC) was analyzed from SE⁺ RA patients and SE⁺ healthy controls to varying concentrations of citrullinated or unmodified peptide antigen. Proliferative responses to peptide antigens and tetanus toxoid were expressed as SI. SI >2 were considered significant. Mean proliferative SI to citrullinated and unmodified peptides were generally between 1 and 2 in RA patients and healthy controls, and significantly lower in each case than responses to tetanus toxoid (Figure 1). The proliferative SI in response to citrullinated aggrecan peptide was significantly higher than to native aggrecan peptide in RA patients (Figure 1). Proliferative responses to tetanus toxoid were significantly lower when comparing RA with healthy control PBMC.

In initial experiments, the present inventors determined that background cytokine secretion and proliferative responses were generally lower if peptide responses were assayed in the presence of human rather than fetal calf serum. Moreover, there was no difference in cytokine production when comparing assays carried out in the presence of 10% healthy or autologous or allogeneic RA donor serum, provided Hetero Block was added where rheumatoid factor (RF) titres were > 100 U/mL in order to prevent RF from binding capture and detection antibodies in ELISA reactions (Todd et al., 2011. Arthritis & Rheumatism 63:894-903). In the absence of peptide stimulation, RA patient PBMC secreted significantly higher concentrations of IFN-γ and IL-6 than other cytokines in the absence of peptides (Figure 2A). Net cytokine secretion was estimated as cytokine secreted upon stimulation with citrullinated peptides minus cytokine secreted in the absence of peptides. IL-6 secretion was highest of the cytokines measured, with net production of up to 60 ng/mL in response to 30 µg/mL citrullinated aggrecan. Consistent with the lack of binding of native epitopes to shared epitope HLA-DR alleles (Snir *et al*., 2011, *supra*), RA patients produced significantly greater amounts of IL-6, TNF and IL-10 in response to citrullinated than native aggrecan peptides (Figure 2B, Figure 3). IL-17 and to some extent IFN-γ were also secreted in response to several citrullinated peptides, but there was considerable variability among individuals and none of the differences was statistically significant (Figure 3). IL-2 and IL-4 responses were generally low. When comparing RA patients and healthy controls, RA patients secreted significantly more IL-10 and TNF in response to citrullinated aggrecan than healthy controls (p<0.05), and there was a similar trend for IL-17 secretion in response to citrullinated aggrecan (p=0.065) and citrullinated fibrinogen (p=0.08). Given the lack of stable HLA-DR binding of native self-peptides, the present inventors determined positive responses to citrullinated peptides to be greater than a threshold of the mean and 2 SD above responses to the corresponding native peptides (citrullinated vimentin responses could not be assessed as responses to native vimentin were not measured). When compared to SE⁺ healthy controls, SE⁺ RA patients' citrullinated peptide responses produced a more diverse array of cytokines. Notably, when the percentage of RA patients and healthy controls with positive responses to each citrullinated peptide was plotted, the regulatory cytokines IL-10 and IFN-γ were only produced by RA patients to the tested epitopes (Figure 4).

### EXAMPLE 2

### IL-6 RESPONSE AMONG RA PATIENTS VARIES WITH DISEASE DURATION.

To better understand the citrullinated peptide response pattern of individual RA patients and SE⁺ healthy controls, IL-6 dose response curves were plotted for each peptide for each individual in the study. PBMC from 4/6 healthy controls dose-dependently secreted IL-6 to citrullinated aggrecan and 3/6 secreted IL-6 in response to citrillated fibrinogen. Taking the same threshold for a positive response as described above, it was found that of 17 RA patients' PBMC responses, 6 secreted IL-6 in response to no epitopes, 8 to citrullinated aggrecan only, 0 to citrullinated fibrinogen, 0 to citrullinated collagen type II only and 3 to multiple citrullinated epitopes. Thus, IL-6 responses were highest and observed most frequently towards citrullinated aggrecan in both RA patients and healthy controls, suggesting this was the most immunogenic epitope tested. An IL-6 response to multiple citrullinated epitopes occurred more frequently among patients diagnosed with RA at least 5 years previously (Figure 5B).

### EXAMPLE 3

### EFFECTOR MEMORY CD4⁺ T CELLS SECRETE CYTOKINES IN RESPONSE TO CITRULLINATED PEPTIDES.

IL-6 is an important cytokine in RA, which could be produced upon stimulation of PBMC either by T cells or antigen presenting cells. To determine the origin of the cytokines secreted into supernatants of PBMC stimulated by citrullinated peptides, RA PBMC were incubated with citrullinated or native peptides for 5 days, with addition of Brefeldin-A for the last 18 hours, prior to intracellular cytokine staining along with analysis of cell surface markers. CD3⁺CD4⁺ T cells produced more intra-cellular IFN-γ and IL-6 when incubated with citrullinated aggrecan or fibrinogen relative to incubation in medium alone (Figure 6A, B). Fluorescence minus one (FMO) staining demonstrates the gating strategy to determine the threshold for positive staining, as described (Herzenberg et al., 2006. Nat Immunol 7(7):681-685) (Figure 6C). No intra-cellular cytokine staining was observed in these assays by CD4⁻CD28⁻ cells, most of which represent antigen-presenting cells (Figure 6D). Differentiated or ageing CD45RO⁻ memory cells have been shown to re-express CD45RA and to be characterized by the loss of CD27 and CD28 (Weng et al., 2009. Trends Immunol 30(7):306-312). In healthy controls, CD28⁻ cells comprised only a small proportion of the CD4⁺ T cells, and cytokine-secreting cells were exclusively CD4⁺CD28⁺. CD28⁻ cells were more abundant among CD4⁺ PB T cells in some, but not all, RA patients. Where CD4⁺CD28⁻ T cells were present, IL-6 and IFN-γ were secreted by both CD28⁻ and CD28⁺CD4⁺ T cells in response to citrullinated peptides. High background cytokine secretion was noted in these cultures, as the present inventors had observed earlier in analysis of supernatants (Figures 2A and 6A). IFN-γ⁺CD4⁺ and IL-6⁺CD4⁺ T cells included both CD45RO⁺ and CD45RO⁻ cells (Figure 6E). CXCR5⁺ follicular helper T cells did not express IL-6 or IFN-γ in response to citrullinated peptides.

### DISCUSSION OF EXAMPLES 1 - 3

The present inventors have shown herein that pro-inflammatory and regulatory cytokines, including IL-6, IFN-γ IL-10 and TNF were produced by CD4⁺ T cells in SE⁺ RA patients in response to citrullinated self-epitopes, of which citrullinated aggrecan was most immunogenic. These cytokine responses were observed in spite of weak peptide-specific T cell proliferative responses. SE⁺ healthy controls also produced cytokines in response to citrullinated aggrecan and citrullinated fibrinogen. Such T cell responses are not necessarily causally related to RA - rather they demonstrate the autoreactivity present in SE⁺ individuals towards citrullinated self-peptides, even in the absence of ACPA. Cytokine responses to citrullinated self-epitopes were, however, more diverse in RA than healthy control individuals. Indeed only RA patients secreted the regulatory cytokines IL-10 and IFN-γ in response to these epitopes (Haringer et al., 2009. J Exp Med 206(5):1009-1017). IFN-γ production by PB and synovial T cells is well-described (Steiner et al., 1999. Rheumatology 38(3):202-213), and IFN-regulated genes have been shown to be predictive of RA development in ACPA⁺ patients with arthralgia (van Baarsen et al., 2010. Arthritis & Rheumatism 62(3):694-704). By intra-cellular staining the present inventors demonstrated IL-6 and IFN-γ production by memory CD4⁺ T cells but not by CD4⁻ antigen-presenting cells in these cultures, at least when examined after 5 days of peptide stimulation. These data demonstrate that CD4⁺ T cells were capable of cytokine production but do not exclude the possibility that CD4⁻ antigen presenting cells such as moncytes, B cells and dendritic cells could also produce these cytokines during the culture, which could be secreted into the supernatant. IL-6 responses increased dose-dependently in response to citrullinated peptides. Intracellular cytokine staining confirmed a broad cytokine response profile, consistent with the phenotype of memory CD4⁺ T cells.

Various mechanisms could contribute to the low proliferative response exhibited by autoreactive T cells to self-peptides and tetanus toxoid antigen in RA. For instance, a number of studies implicate deficient signaling of RA T cells through the T cell receptor-CD3 complex (Emery et al., 1984. Clin. Exp. Immunol. 57:123-129; Seitz et al., 1988. Rheumatol. Int. 8:189-196; Allen et al., 1995. Eur. J. Immunol. 25:1547-1554; Thomas et al., 1992. Arthritis Rheum. 35:1455-1465; Berg et al., 2000. Arthritis Res. 2:75-84; Maurice et al., 1997. J. Immunol. 159:2973-2978; Berg et al., 2000. Clin. Exp. Immunol. 120:174-182). IL-2 might be rapidly consumed through binding to the high avidity CD25 receptor expressed by regulatory T cells and by effector memory T cells upon activation. This could limit availability of IL-2 for T cell proliferation in tissue culture (Wolf et al., 2001. Eur. J. Immunol 31:1637-1645; Ishimaru et al., 2006. Nat. Immunol. 7:763-772). Furthermore, autoreactive T cells are subject to the influence of regulatory cells and cytokines, as demonstrated here (Berg et al., 2001. Ann. Rheum. Dis. 60:133-139; van Amelsfort et al., 2004. Arthritis Rheum. 50:2775-2785). On the other hand, effector memory cells produce high levels of pro-inflammatory and regulatory cytokines, and cytokine production more effectively interrogates highly differentiated autoreactive effector memory T cells in both humans and mice (Garcia de Tena et al., 2006. J. Clin. Immunol. 26:233-242; Nanki et al., 2000. Arthritis Res. 2:415-423; Morita et al., 1998. Arthritis Rheum. 41:1669-1676).

The pro-inflammatory cytokine IL-6 stimulates B cell antibody production and plays a critical role in RA pathogenesis (Assier et al., 2010. Joint Bone Spine 77:532-536). In RA patients, intracellular IL-6 and IFN-γ were produced by CD28⁺ and CD28⁻ and CD45RO⁺ and CD45RO⁻ PB CD4⁺ T cells. Consistent with the poor proliferative but good cytokine response of RA T cells in response to citrullinated peptides in the current studies, CD4⁺CD45RB^{dim}CD2T⁻ memory T cells from healthy donors were previously shown to proliferate poorly but to produce large amounts of IL-4 and IL-10 in response to mitogen, and to provide effective B cell help for immunoglobulin production (Tortorella et al., 1995. J. Immunol. 155:149-162). In contrast, typical CXCR5⁺ follicular helper T cells, which have been shown to promote antibody production in lymphoid tissue *in vivo* (Chevalier et al., 2011. J Immunol 186:5556-5568), did not express cytokines in response to citrullinated peptides. Of interest, where CD28⁻ T cells were present in PB of RA patients, they also produced cytokine in response to citrullinated peptides. CD28⁻ T cells represent an important effector memory amplification response in the synovial environment, and have been shown to be more resistant to suppression by regulatory T cells (Weng et al., 2009. Trends Immunol. 30:306-312; Thewissen et al., 2007. J. Immunol 179:6514-6523). Together the data presented herein support the hypothesis that effector memory T cells reactive with a variety of citrullinated self-peptides and with the potentiator B cell help circulate in peripheral blood and are present in synovial fluid of SE⁺ individuals.

Previous studies in RA patients analyzed the PB CD4⁺ T cell response to a single citrullinated peptide specificity. Proliferative and IL-17 CD4⁺ T cell responses to citrullinated aggrecan 84-103 were demonstrated in RA patients but not in healthy controls, demonstrating the immunogenicity of this citrullinated aggrecan epitope (von Delwig et al., 2010. Arthritis Rheum 62(1):143-149). The unmodified aggrecan epitope is immunodominant in BALB/c mice immunized with aggrecan/proteoglycan (Buzás et al., 2005. Cell Immunol 235(2):98-108). This citrullinated aggrecan peptide was also the most immunogenic epitope in the current studies - with the highest frequency of responders, and the highest magnitude of IL-6 responses. It should be noted, however, that the Buzás study did not compare early RA and longstanding RA patients, nor did it investigate the host immune response to any other antigen beyond aggrecan.

In long-standing RA patients, the present inventors also found robust cytokine production was also found in response to citrullinated fibrinogen-α 79-91, which was shown to be the immunodominant epitope in HLA-DR4-IE transgenic mice immunized with citrullinated human fibrinogen (Hill et al., 2008. J. Exp. Med. 205:967-979). Stimulation with the citrullinated vimentin 66-78 epitope produced much weaker cytokine responses in the current studies, as was observed by Snir *et al.* (2011, *supra),* who found that HLA-DRB1*0401⁺ RA patient T cells produced a number of cytokines when incubated with citrullinated vimentin 59-78, but not 66-78. In that study, IFN-γ and TNF were expressed intracellularly by a small proportion of CD154⁺ CD4⁺ RA patient T cells in response to citrullinated but not native vimentin 59-78. The proportion of T cells staining for intracellular cytokine was much lower than in the current studies, in which the present inventors carried out staining 5 days after antigen stimulation and without a second restimulation. It is possible that anergy may have been induced by restimulation with peptide after an initial 5 day culture with peptide, or given their effector memory phenotype (Di Mitri et al. 2011. "Reversible Senescence in Human CD4+CD45RA+CD27- Memory T Cells." J. Immunol), that few T cells were capable of re-expressing CD154 after prolonged in vitro culture. Similar to the current study, Snir *et al.* (2011, *supra)* did not observe strong proliferative or IL-17A responses, unlike von Delwig *et al.* (2010, *supra).* Differences in cytokine secretion may relate to the inclusion of healthy human serum or of Hetero Block plus autologous serum in the current studies (Todd et al., 2011. Arthritis & Rheumatism 63:894-903). Finally, due to the hydrophobic amino acids at 84-88, the citrullinated aggrecan peptide was relatively insoluble in aqueous medium, which may also have affected antigen purity, concentration and cellular uptake in different laboratories.

The present inventors also observed T cell cytokine responses to citrullinated self-epitopes in SE⁺ RA patients, whether ACPA⁺ or not, as well as in ACPA⁻ healthy controls. HLA-DRB1*0401⁺ healthy control T cells were also observed to produce cytokine in response to citrullinated vimentin 59-78 (Snir *et al*., 2011, *supra*). After immunization of DR4-IE transgenic mice with citrullinated human fibrinogen, responses to unmodified native epitopes were stimulated in parallel with responses to citrullinated epitopes. From the previous and current analysis of HLA-DR binding and responses to unmodified epitopes, responses to citrullinated epitopes appear to be most specific to SE⁺ RA PB. In contrast, the magnitude and diversity of the responses to citrullinated epitopes in healthy controls was unexpected. There was no T cell autoreactivity towards citrullinated fibrinogen in DR4-IE transgenic mice primed with native human fibrinogen (Hill *et al*., 2008, *supra*). Spontaneous autoreactivity towards citrullinated fibrinogen in naive or stressed DR4-IE-transgenic mice has not been tested. However, it has been shown that CD4⁺ T cells in both SE⁺ RA patients and HLA-DR4⁺ healthy controls have reduced T cell receptor excision circles, overall telomere shortening, and reduced replicative capacity, which together imply a HLA-DR SE-associated reduction in T cell input to the peripheral repertoire, an increased proliferative drive for naïve T cells towards peripheral self-antigens and a limited diversity of the TCR repertoire (Fujii et al., 2009. Proc Natl Acad Sci U S A 106:4360-4365; Koetz et al., 2000. Proc Natl Acad Sci U S A 97:9203-9208; Schönland et al., 2003. Proc Natl Acad Sci U S A 100:13471-13476). The current and previous studies in humans suggest together that PB T cells in HLA-DR SE⁺ individuals may be predisposed to autoreactivity towards self-antigens including those modified by citrullination, potentially exposed during stress or pro-inflammatory settings, including joint trauma or smoking (Klareskog et al., 2006. Arthritis Rheum. 54:38-46; de Jong et al., 2010. Ann. Rheum. Dis. 69:255-262). Furthermore, unlike T cell autoreactivity, the development of B cell autoreactivity towards citrullinated epitopes appears to be a significant checkpoint in the progression to RA. It has been proposed that infection e.g. with *Porphyromonas gingivalis* in patients with periodontitis, may be a critical trigger for progress through this checkpoint, potentially due to cross reactivity with bacterial antigens, adjuvant effects of pathogen-associated molecular patterns, targeting of alternative antigen presenting cells, or a combination of these (Wegner et AL., 2010. Immunol. Rev. 233:34-54). Carbamylation of lysine residues to homocitrulline may be another factor enhancing the immunogenicity of RA autoantigens, including citrullinated antigens (Mydel et al., 2010. J Immunol. 184:6882-6890).

The data presented herein indicate that RA patients with long-standing disease are more likely to respond to multiple citrullinated epitopes, whereas patients with recent-onset RA (including those previously untreated) are more likely to respond either to no antigen or only to citrullinated aggrecan. These data also indicate that epitope spreading occurs as disease progresses. The assay developed herein, in which T cell cytokine production is interrogated in the presence of varying concentrations of a panel of citrullinated epitopes is proposed to be a useful biomarker to identify the most immunogenic peptide epitopes, and to correlate specific T cell responses with corresponding ACPA "fine specificities" (Willemze et al., 2011. Arthritis Rheum. 63:1823-1832). Adavantageously, these assays can be carried out in conjunction with analysis of tetramer-specific T cells. Contemplated applications include comparison of serial samples of PBMC from pre-RA through to diagnosis (Deane et al., 2010. Arthritis Rheum. 62:3161-3172), and analysis of PBMC from clinical trials of antigen-specific or non-specific immunotherapy, such as Abatacept (Yue et al., 2010. Arthritis Rheum. 62:2941-2952). Finally, analysis of specific peptide reactivity is proposed to be beneficial for stratifying patients and for identifying appropriate epitopes for personalized antigen-specific immunotherapy.

### MATERIALS AND METHODS

### PATIENTS

Twenty one patients who fulfilled the 1987 American College of Rheumatology (ACR) criteria for RA (Aletaha *et al*., 2010, *supra*) and 6 non-smoking ACPA⁻ SE⁺ healthy controls were included. All individuals provided peripheral blood (PB) samples, although in some cases the yield was insufficient for all assays. Patient demographic details are outlined in Table 6. HLA-DR genotyping was carried out at Queensland Health Pathology Services. The study was approved by the Human Research Ethics Committee of the Princess Alexandra Hospital and informed consent was obtained from each patient.

**TABLE 6 CHARACTERISTICS OF PATIENTS IN THIS STUDY**

| Pt | Ethnicity | HLA-DRB1 genotype | ACPA | Disease Duration (yr) | Treatment | CRP (mg/L) | Represented in Figure |
|---|---|---|---|---|---|---|---|
| RA1 | C | 03,0401 | + | 6 | M, S | 8 | 1 |
| RA2 | C | 03,0401 | + | <1 | Nil | 3 | 1-5 |
| RA3 | C/I | 0403, 0405 | - | <1 | M, S, H | 3 | 1 |
| RA4 | C | 0401, 0404 | + | <1 | L | 11 | 1-5 |
| RA5 | C | 0404, 1302 | + | 1 | M, S, H | 2 | 1 |
| RA6 | C | 0401 13 | + | >5 | M, H | 1 | 1-5 |
| RA7 | A | 0405 | - | 1 | M | 1 | 1-5 |
| RA8 | C | 0401, 0404 | + | >5 | L, H, S | 22 | 1-5 |
| RA9 | C | 0103,0101 | + | <1 | Nil | 4 | 1-5 |
| RA10 | C | 01, 1302 | + | 5 | H | 10 | 1-5 |
| RA11 | C | 04, 0301 | - | 4 | M, S, H | 3 | 1-5 |
| RA12* | C | 0401 | + | 0 | Nil | 45 | 1-5 |
| RA13 | C | 0101, 1301 | - | >5 | M | 20 | 1-6 |
| RA14 | C | 0408, 11 | + | >5 | M, S, H | 4.6 | 1-6 |
| RA15 | C | 0401, 0408 | + | 0 | Nil | 1 | 1-6 |
| RA16 | C | 0101, 0408 | + | 0 | Nil | 6 | 1-6 |
| RA17 | C | 0401, 03 | + | >5 | M, A | 33 | 6 |
| RA18 | C | 0401, 03 | ± | 3 | M | 2 | 1-5 |
| RA19 | C | 0101, 0103 | ± | 2 | M. S. H | 5.3 | 1-5 |
| RA20 | C | 0401, 1501 | | 2 | - | 9.9 | 1-5 |
| RA21 | C | 0401, 0101 | ± | >5 | M, P | 2 | 1-5 |
| HC1 | C | 01 | - | | | | 1-5 |
| HC2 | C | 03, 04 | - | | | | 1-5 |
| HC3 | C | 0401, 0701 | - | | | | 1-5 |
| HC4 | C | 0101, 08 | - | | | | 1-5 |
| HC5 | C | 1301, 0401 | - | | | | 1-6 |
| HC6 | C | 0401, 1301 | - | | | | 1-6 |

| | | | | | | | |
|---|---|---|---|---|---|---|---|
| CRP measured at the time of blood withdrawal for peptide response. M: methotrexate, S: sulfasalazine, H: hydroxychloroquine, L: leflunomide, A: abatacept. C Caucasian, A Asian, I Pacific Islander * Both PB and SF obtained. For RA patients mean age = 50 (range 22-65) of whom 89% were females, and for controls mean age = 38 (40-45), 50% females. | | | | | | | |

### PEPTIDE PREPARATION

Citrulline or arginine-containing SE-binding epitopes corresponding to vimentin, collagen type II, fibrinogen and aggrecan were synthesized by Auspep (NSW, Australia). All peptides were filtered, reconstituted to 300 µg/mL in sterile water and stored at - 70° C. Final dilutions for working stock were made with medium.

### PURIFICATION OF PB MONONUCLEAR CELLS AND ANTIGEN PRESENTATION ASSAYS

Mononuclear cells (MC) were isolated from PB and SF using Ficoll-Paque density gradients (Amersham Pharmacia Biotech, Uppsala, Sweden) and washed with 0.9 % saline then resuspended in RPMI and 10% human serum from healthy or autologous or allogeneic RA donors. Tetanus toxoid was used at a concentration of 4 Lfi/mL (Chiron Vaccinese). Two x 10⁵ PBMC or SFMC were incubated with 0, 3 and 30 µg/mL of each peptide in the presence of RPMI containing 10% human serum in a final volume of 200 µL in roundbottomed wells, for 5 days. T cell proliferation was assessed by addition of 1 µCi/well [³H]thymidine (ICN Biochemicals) for the final 18 hours. Cells were harvested onto glass fibre filter mats and [³H]-thymidine incorporation was determined by liquid scintillation spectroscopy (Packard Topcount, Packard Instrument Co.). IL-2, IL-4, IFNγ, IL-10, IL-6, IL-17 and TNF were measured in day 5 supernatants using BD Cytometric Bead Array (CBA) kits (BD Bioscience). Initial kinetic experiments demonstrated that peptide stimulation for 5 days optimally induced antigen-specific proliferative and cytokine responses, and that longer cultures did not yield greater responses. Where RA serum was used in cytokine production assays, 150 µg/mL HeteroBlock (Omega Biologicals) were added during CBA measurement (Todd *et al*., 2011, *supra*). Samples were read on the BD FACSArray™ bioanalyzer system. Stimulation indices (SI) for proliferative responses were calculated as fold increase in response to peptide over background. Net cytokine secretion was calculated for each response as [concentration with peptide stimulation] minus [concentration without peptide stimulation]. Positive response thresholds were 2 SD above the cytokine responses towards the corresponding native peptide, for both RA patients and healthy controls.

### FLOW CYTOMETRY AND INTRACELLULAR CYTOKINE STAINING

PBMC from SE⁺ RA patients or healthy controls were incubated with or without peptides at concentrations of 3 and 30 µg/mL for 5 days, with Brefeldin A (Sigma Aldrich) added for the final 18 hours. Cells were stained for CD3-FITC, CD4-APC/Cy7, CD28-FITC, CXCR5-PerCP/Cy5.5 and CD45RO-PerCP/Cy5.5 (Biolegend), followed by permeabilization and staining for intracellular IL-6-APC and IFN-γ-APC (Biolegend). Data were collected on the Gallios flow cytometer and analysed using Kaluza software (Beckman Coulter).

### STATISTICAL ANALYSIS

One-way non-parametric ANOVA with post-hoc correction compared multiple means. Unpaired Mann Whitney tests compared the tetanus toxoid proliferative responses between RA patients and healthy controls and specific cytokine responses to citrullinated vs native peptides. Significance is indicated as **P* < 0.05, ***P* < 0.005, and ***P* < 0.001. All error bars represent SEM.

### EXAMPLE 4

### INHIBITION OF APC FROM AN RA PATIENT WITH INHIBITORS OF NF-KB, MTOR OR SYK SUPPRESSES THEIR CAPACITY TO INDUCE T CELL CYTOKINE PRODUCTION IN RESPONSE TO CITRULLINATED AGGRECAN PEPTIDE

Peripheral blood mononuclear cells (PBMC) were extracted from an RA patient with disease duration 18 years, positive anti-CCP and HLA-DRB1*0401 shared epitope. Autologous CD4+ T cells were isolated using immunomagnetic beads. The non-T cell fraction consists of antigen-presenting cells (APC), including dendritic cells, monocytes and B cells; it was treated with Mitomycin C to prevent proliferation. The APC were pre-incubated for 1h without or with 3uM BAY7011-82, 20 µg/mL Curcumin (both inhibit NF-kB), 1 0ng/mL Rapamycin (inhibits mTOR) or 4 0µM Piceatannol (inhibits syk), then incubated with 100 ng/mL LPS for 1h. The APC were washed 3 times then incubated for 5 days with CD4+ T cells in the presence of 0 µg/mL, 3 µg/mL or 30 µg/mL Aggrecan peptide 84-103 cit 93. Cytokines were analysed in culture supernatants by cytokine bead array. The data show net (peptide minus no peptide wells) IL-6 and TNF production in response to 3 or 30 µg/mL peptide.

The results presented in Figure 7 show that pre-incubation of APC with Bay11-7082 partially suppressed, and the other inhibitors completely suppressed the capacity of APC presenting cit-peptide to induce T cell IL-6 production. All inhibitors completely suppressed the capacity of APC presenting cit-peptide to induce T cell TNF production.

### EXAMPLE 5

### CYTOKINE RESPONSE TO CITRULLINATED AGGRECAN G1 EPITOPE IN AN RA PATIENT

PBMC from the same RA patient as described in Example 4 were incubated with the native or citrulinated form of the 351 amino acid human aggrecan G1 epitope (contains dominant and subdominant epitopes in mice), for 5 days at 1 µg/mL, then IL-6 was measured in supernatant. Net IL-6 (antigen minus no antigen wells) production shown in Figure 8 clearly shows a significantly higher IL-6 response to the citrullinated G1 epitope as compared to the corresponding non-citrullinated epitope.

### EXAMPLE 6

### CIT AGGRECAN-SPECIFIC T CELLS ARE PRESENT IN PERIPHERAL BLOOD OF RHEUMATOID ARTHRITIS PATIENTS

PBMC from 2 HLA-DRB1* 0401⁺ ACPA⁺ RA patients were stained with CLIP, influenza hemagglutinin (HA), aggrecan 89-103 cit 93, 95 or vimentin 59-71 cit 64, 69, 71 DRB1*0401-tetramers in the presence of dasatinib. Plots were gated on live cells, CD4⁺ T cells, enriched for tetramer+ cells using immunomagnetic beads. The % tetramer⁺ of enriched PBMC is shown in Figure 9 for each plot. These results clearly show that citrullinated aggrecan-specific T cells are present in peripheral blood of rheumatoid arthritis patients.

### EXAMPLE 7

### NUMBER AND FLUORESCENCE INTENSITY OF CIT-AGGRECAN-SPECIFIC T CELLS IN PERIPHERAL BLOOD

PBMC from 6 RA patients and 3 healthy controls were stained as in Example 6 with inclusion of fluorescent counting beads, then the number of tetramer+ T cells was calculated per mL blood. The mean fluorescence intensity (MFI) of tetramer staining is an indicator of affinity of the T cell receptor for the antigen presented by the HLA-DR molecule. Cit-aggrecan-specific T cells circulate in RA patients and healthy controls of this genotype. The results presented in Figure 10 show that the number of cit-aggrecan-specific T cells is higher in a proportion of RA patients relative to the mean for the healthy controls, and in some RA patients these Ag-specific T cells have higher affinity for cit-aggrecan.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention.

## Claims

1. An agent or combination of agents that elicits an antigen-specific tolerogenic response to an aggrecan polypeptide selected from:
a)
(i) an antigen that corresponds in whole or in part to an aggrecan polypeptide, or a polynucleotide encoding an antigen that corresponds in whole or in part to an aggrecan polypeptide; co-administered together with
(ii) at least one NF-κB inhibitor, mTOR inhibitor or Syk inhibitor, or a polynucleotide encoding the at least one NF-κB inhibitor, mTOR inhibitor or Syk inhibitor; or
b) an antigen presenting cell (APC) to which:
(i) an antigen that corresponds in whole or in part to an aggrecan polypeptide, or a polynucleotide encoding an antigen that corresponds in whole or in part to an aggrecan polypeptide; and
(ii) at least one NF-κB inhibitor, mTOR inhibitor or Syk inhibitor, or a polynucleotide encoding at least one NF-κB inhibitor, mTOR inhibitor or Syk inhibitor,
have been delivered ex vivo; or
c) a T lymphocyte which has been contacted with the APC of (b); or
d) a complex comprising an antigen that corresponds in whole or in part to an aggrecan polypeptide and a Major Histocompatibility Complex (MHC) component; or
e) a B cell expressing a nucleic acid molecule that encodes an antigen that corresponds in whole or in part to an aggrecan polypeptide, wherein inducing expression leads to presentation of the antigen or processed form thereof on the surface of the B cell; or
f) a construct comprising an immunomodulatory peptide attached to an immune/T-cell-binding ligand (I/TCBL), wherein the immunomodulatory peptide comprises an amino acid sequence corresponding to a portion of an aggrecan polypeptide which binds to an antigen receptor on pro-inflammatory or autoreactive T lymphocytes, and wherein the I/TCBL binds to a class or subclass of T cell selected from the group consisting of helper T cells, suppressor T cells and cytotoxic T cells and modulates T cell activity; or
g) an altered peptide ligand (APL) that comprises or consists of an amino acid sequence corresponding to a portion of an aggrecan polypeptide which binds to an antigen receptor on pro-inflammatory or autoreactive T lymphocytes, wherein the APL amino acid sequence is distinguished from the amino acid sequence of the portion by at least one amino acid substitution, deletion or addition, and the APL interferes with normal signaling through an antigen receptor; or
h) a combination of (b) and (c),
for use in treating or preventing joint damage in a human subject with early RA or incipient RA, wherein the aggrecan polypeptide is a citrullinated aggrecan polypeptide.

2. An agent or combination of agents for use according to claim 1, wherein the subject is identified as having early RA or incipient RA prior to eliciting the antigen-specific tolerogenic response.

3. An agent or combination of agents for use according to claim 1 or claim 2, wherein the subject is positive for the shared epitope (SE).

4. An agent or combination of agents for use according to claim 3, wherein the subject is identified as being positive for SE prior to eliciting the antigen-specific tolerogenic response.

5. An agent or combination of agents for use according to any one of claims 1 to 4, wherein the subject has or is at risk of developing an immune response to the aggrecan polypeptide.

6. An agent or combination of agents for use according to claim 5, wherein the immune response comprises an effector T lymphocyte response.

7. An agent or combination of agents for use according to claim 6, wherein the immune response comprises production of at least one cytokine selected from the group consisting of interleukin-6 (IL-6), interferon-γ (IFN-γ), tumor necrosis factor (TNF), and interleukin-10 (IL-10).

8. An agent or combination of agents for use according to claim 5, wherein the immune response includes a pro-inflammatory T lymphocyte response, which comprises or consists of production of at least one cytokine selected from the group consisting of IL-6, IFN-γ and TNF.

9. An agent or combination of agents for use according to claim 8, wherein the pro-inflammatory T lymphocyte response comprises or consists of production of IL-6.

10. An agent or combination of agents for use according to claim 5, wherein immune response is produced at least in part by CD4⁺ lymphocytes.

11. An agent or combination of agents for use according to any one of claims 1 to 10, wherein the antigen-specific tolerogenic response is effected by:
(1) increasing the number of tolerogenic antigen-presenting cells (agg-tolAPC) in the subject, which present a peptide corresponding to a portion of the aggrecan polypeptide, wherein the portion is associated with a pro-inflammatory or autoreactive T lymphocyte response to the aggrecan polypeptide;
(2) inducing anergy or apoptosis in pro-inflammatory or autoreactive T lymphocytes in the subject, which are reactive against the aggrecan polypeptide; and
(3) increasing the number of regulatory or suppressor T lymphocytes in the subject, which suppress or otherwise reduce a pro-inflammatory or autoreactive T lymphocyte response to the aggrecan polypeptide.

12. An agent or combination of agents for use according to claim 11, comprising increasing the number of agg-tolAPC in the subject to thereby treat or prevent the joint damage.

13. An agent or combination of agents for use according to claim 12, wherein the agg-tolAPC stimulate the production of regulatory or suppressor T lymphocytes that suppress or otherwise reduce the pro-inflammatory or autoreactive T lymphocyte response to the aggrecan polypeptide.

14. An agent or combination of agents for use according to claim 1, wherein the antigen of (a), (b), (d), (e) or (h) comprises an amino acid sequence corresponding to a full-length aggrecan polypeptide.

15. An agent or combination of agents for use according to claim 1, wherein the antigen of (a), (b), (d), (e) or (h) comprises an amino acid sequence corresponding to a mature aggrecan polypeptide.

16. An agent or combination of agents for use according to claim 1, wherein the antigen of (a), (b), (d), (e) or (h) comprises an amino acid sequence corresponding to a domain of an aggrecan polypeptide selected from the G1 domain, the G2 domain, or the G3 domain.

17. An agent or combination of agents for use according to claim 1, wherein the antigen of (a), (b), (d), (e) or (h) comprises an amino acid sequence corresponding to a T cell epitope of an aggrecan polypeptide.

18. An agent or combination of agents for use according to claim 17, wherein the amino acid sequence is selected from any one of SEQ ID NO: 5-35, including citrullinated forms thereof.

19. An agent or combination of agents for use according to claim 17, wherein the amino acid sequence is selected from any one of SEQ ID NO: 32-35.

20. An agent or combination of agents for use according to claim 1, wherein the antigen of (a), (b), (d), (e), or (h) is HLA DR restricted and the subject is positive for an HLA DR allele.

21. An agent or combination of agents for use according to claim 1, wherein the antigen of (a), (b), (d), (e), or (h) is in the form of one or more peptides corresponding in whole or in part to an aggrecan polypeptide, including citrullinated forms thereof.

22. An agent or combination of agents for use according to claim 1, wherein the antigen of (a), (b), (d), (e), or (h) is in the form of a plurality of contiguous overlapping peptides whose sequences span at least a portion of an aggrecan polypeptide, including citrullinated forms thereof.

23. An agent or combination of agents for use according to claim 22, wherein the overlapping peptides comprise or consist of an amino acid sequence selected from SEQ ID NO: 49-531, including citrullinated forms thereof.

24. An agent or combination of agents for use according to claim 1, wherein the NF-κB inhibitor is curcumin or a curcumin derivative.

25. An agent or combination of agents for use according to claim 1, wherein the NF-κB inhibitor, mTOR inhibitor or Syk inhibitor and the antigenic molecule are co-administered in soluble form.

26. An agent or combination of agents for use according to claim 1, wherein the NF-κB inhibitor, mTOR inhibitor or Syk inhibitor and the antigenic molecule are co-administered in particulate form.

27. An agent or combination of agents for use according to claim 26, wherein the NF-κB inhibitor, mTOR inhibitor or Syk inhibitor and the antigenic molecule are co-administered in the same particle.

28. An agent or combination of agents for use according to claim 27, wherein the particle is a polymeric particle.

29. An agent or combination of agents for use according to claim 27, wherein the particle is a liposome.

30. An agent or combination of agents for use according to claim 1, wherein the nucleic acid molecule expressed in the B cell of (e) further encodes an immunoglobulin or an immunoglobulin fragment fused directly or indirectly to the antigen.

31. An agent or combination of agents for use according to claim 1, wherein the I/TCBL of (f) comprises at least a portion of a molecule selected from a MHC class I molecule, a MHC class II molecule, an accessory molecule such as β2-microglobulin, lymphocyte function associated molecule-3 (LFA-3), the Fc region of the heavy chain of an immunoglobulin molecule, Ia⁺ molecules, an antibody to CD2, an antibody to CD3, an antibody to CD4, an antibody to CD8, an antibody to lectin, a lymphokine.

## Patentansprüche

1. Wirkstoff oder Kombination von Wirkstoffen, der/die eine antigenspezifische tolerogene Antwort auf ein Aggrecan-Polypeptid hervorruft, ausgewählt aus:
a)
(i) einem Antigen, das vollständig oder teilweise einem Aggrecan-Polypeptid entspricht, oder einem Polynukleotid, das für ein Antigen codiert, das vollständig oder teilweise einem Aggrecan-Polypeptid entspricht; verabreicht gemeinsam mit
(ii) mindestens einem NF-κB-Inhibitor, mTOR-Inhibitor oder Syk-Inhibitor, oder einem Polynukleotid, das für den mindestens einen NF-κB-Inhibitor, mTOR-Inhibitor oder Syk-Inhibitor codiert; oder
b) einer antigenpräsentierenden Zelle (APC), der
(i) ein Antigen, das vollständig oder teilweise einem Aggrecan-Polypeptid entspricht, oder ein Polynukleotid, das für ein Antigen codiert, das vollständig oder teilweise einem Aggrecan-Polypeptid entspricht; und
(ii) mindestens ein NF-κB-Inhibitor, mTOR-Inhibitor oder Syk-Inhibitor, oder ein Polynukleotid, das für mindestens einen NF-κB-Inhibitor, mTOR-Inhibitor oder Syk-Inhibitor codiert,
ex vivo zugeführt wurden; oder
c) einem T-Lymphozyten, der mit der APC aus (b) in Kontakt gebracht wurde; oder
d) einem Komplex, umfassend ein Antigen, das vollständig oder teilweise einem Aggrecan-Polypeptid entspricht, und eine Haupt-Histokompatibilitäts-Komplex(MHC)-Komponente; oder
e) einer B-Zelle, exprimierend ein Nukleinsäuremolekül, das für ein Antigen codiert, das vollständig oder teilweise einem Aggrecan-Polypeptid entspricht, wobei das Einleiten der Expression zur Präsentation des Antigens oder einer verarbeiteten Form davon auf der Oberfläche der B-Zelle führt; oder
f) einem Konstrukt, umfassend ein immunmodulatorisches Peptid, das an einen Immun/T-Zellen-bindenden Liganden (I/TCBL) angefügt ist, wobei das immunmodulatorische Peptid eine Aminosäuresequenz umfasst, die einem Abschnitt eines Aggrecan-Polypeptids entspricht, der an einen Antigen-Rezeptor an proinflammatorischen oder autoreaktiven T-Lymphozyten bindet, und wobei der I/TCBL an eine Klasse oder Subklasse von T-Zellen bindet, ausgewählt aus der Gruppe bestehend aus T-Helferzellen, T-Suppressorzellen und cytotoxischen T-Zellen, und die T-Zellen-Aktivität moduliert; oder
g) einem veränderten Peptidliganden (APL), der eine Aminosäuresequenz, die einem Abschnitt eines Aggrecan-Polypeptids entspricht, der an einen Antigen-Rezeptor an proinflammatorischen oder autoreaktiven T-Lymphozyten bindet, umfasst oder daraus besteht, wobei sich die APL-Aminosäuresequenz von der Aminosäuresequenz des Abschnitts durch mindestens eine Aminosäure-Substitution, -Deletion oder - Addition unterscheidet und der APL die normale Signalübertragung durch einen Antigen-Rezeptor stört; oder
h) einer Kombination aus (b) und (c),
zur Verwendung beim Behandeln oder Verhindern eines Gelenkschadens an einem menschlichen Subjekt mit früher RA oder einsetzender RA, wobei das Aggrecan-Polypeptid ein citrulliniertes Aggrecan-Polypeptid ist.

2. Wirkstoff oder Kombination von Wirkstoffen zur Verwendung nach Anspruch 1, wobei bei dem Subjekt vor dem Hervorrufen der antigenspezifischen tolerogenen Antwort ein Vorliegen von früher RA oder einsetzender RA bestimmt wird.

3. Wirkstoff oder Kombination von Wirkstoffen zur Verwendung nach Anspruch 1 oder Anspruch 2, wobei das Subjekt für das Shared Epitope (SE) positiv ist.

4. Wirkstoff oder Kombination von Wirkstoffen zur Verwendung nach Anspruch 3, wobei das Subjekt vor dem Hervorrufen der antigenspezifischen tolerogenen Antwort als positiv für das SE bestimmt wird.

5. Wirkstoff oder Kombination von Wirkstoffen zur Verwendung nach einem der Ansprüche 1 bis 4, wobei das Subjekt eine Immunantwort auf das Aggrecan-Polypeptid oder das Risiko eines Entwickelns davon aufweist.

6. Wirkstoff oder Kombination von Wirkstoffen zur Verwendung nach Anspruch 5, wobei die Immunantwort eine Antwort durch Effektor-T-Lymphozyten umfasst.

7. Wirkstoff oder Kombination von Wirkstoffen zur Verwendung nach Anspruch 6, wobei die Immunantwort die Produktion von mindestens einem Cytokin umfasst, ausgewählt aus der Gruppe bestehend aus Interleukin-6 (IL-6), Interferon-γ (IFN-γ), Tumornekrosefaktor (TNF) und Interleukin-10 (IL-10).

8. Wirkstoff oder Kombination von Wirkstoffen zur Verwendung nach Anspruch 5, wobei die Immunantwort eine Antwort durch proinflammatorische T-Lymphozyten aufweist, die die Produktion von mindestens einem Cytokin, ausgewählt aus der Gruppe bestehend aus IL-6, IFN-γ und TNF, umfasst oder daraus besteht.

9. Wirkstoff oder Kombination von Wirkstoffen zur Verwendung nach Anspruch 8, wobei die Antwort durch proinflammatorische T-Lymphozyten die Produktion von IL-6 umfasst oder daraus besteht.

10. Wirkstoff oder Kombination von Wirkstoffen zur Verwendung nach Anspruch 5, wobei die Immunantwort zumindest teilweise durch CD4⁺-Lymphozyten erzeugt wird.

11. Wirkstoff oder Kombination von Wirkstoffen zur Verwendung nach einem der Ansprüche 1 bis 10, wobei die antigenspezifische tolerogene Antwort bewirkt wird durch:
(1) Erhöhen der Anzahl von tolerogenen antigenpräsentierenden Zellen (agg-tolAPC) in dem Subjekt, die ein Peptid präsentieren, das einem Abschnitt des Aggrecan-Polypeptids entspricht, wobei der Abschnitt mit einer Antwort auf das Aggrecan-Polypeptid durch proinflammatorische oder autoreaktive T-Lymphozyten zusammengehört;
(2) Herbeiführen von Anergie oder Apoptose in proinflammatorischen oder autoreaktiven T-Lymphozyten in dem Subjekt, die gegen das Aggrecan-Polypeptid reaktiv sind; und
(3) Erhöhen der Anzahl von regulatorischen oder Suppressor-T-Lymphozyten in dem Subjekt, die eine Antwort auf das Aggrecan-Polypeptid durch proinflammatorische oder autoreaktive T-Lymphozyten unterdrücken oder anderweitig verringern.

12. Wirkstoff oder Kombination von Wirkstoffen zur Verwendung nach Anspruch 11, umfassend Erhöhen der Anzahl von agg-tolAPC in dem Subjekt, um dadurch den Gelenkschaden zu behandeln oder zu verhindern.

13. Wirkstoff oder Kombination von Wirkstoffen zur Verwendung nach Anspruch 12, wobei die agg-tolAPC die Produktion von regulatorischen oder Suppressor-T-Lymphozyten stimulieren, die die Antwort auf das Aggrecan-Polypeptid durch proinflammatorische oder autoreaktive T-Lymphozyten unterdrücken oder anderweitig verringern.

14. Wirkstoff oder Kombination von Wirkstoffen zur Verwendung nach Anspruch 1, wobei das Antigen aus (a), (b), (d), (e) oder (h) eine Aminosäuresequenz umfasst, die einem Aggrecan-Polypeptid in voller Länge entspricht.

15. Wirkstoff oder Kombination von Wirkstoffen zur Verwendung nach Anspruch 1, wobei das Antigen aus (a), (b), (d), (e) oder (h) eine Aminosäuresequenz umfasst, die einem reifen Aggrecan-Polypeptid entspricht.

16. Wirkstoff oder Kombination von Wirkstoffen zur Verwendung nach Anspruch 1, wobei das Antigen aus (a), (b), (d), (e) oder (h) eine Aminosäuresequenz umfasst, die einer Domäne eines Aggrecan-Polypeptids entspricht, ausgewählt aus der G1-Domäne, der G2-Domäne oder der G3-Domäne.

17. Wirkstoff oder Kombination von Wirkstoffen zur Verwendung nach Anspruch 1, wobei das Antigen aus (a), (b), (d), (e) oder (h) eine Aminosäuresequenz umfasst, die einem T-Zell-Epitop eines Aggrecan-Polypeptids entspricht.

18. Wirkstoff oder Kombination von Wirkstoffen zur Verwendung nach Anspruch 17, wobei die Aminosäuresequenz aus einer von SEQ ID NO: 5-35 ausgewählt ist, einschließlich citrullinierter Formen davon.

19. Wirkstoff oder Kombination von Wirkstoffen zur Verwendung nach Anspruch 17, wobei die Aminosäuresequenz aus einer von SEQ ID NO: 32-35 ausgewählt ist.

20. Wirkstoff oder Kombination von Wirkstoffen zur Verwendung nach Anspruch 1, wobei das Antigen aus (a), (b), (d), (e) oder (h) HLA-DR-restringiert ist und das Subjekt positiv für ein HLA-DR-Allel ist.

21. Wirkstoff oder Kombination von Wirkstoffen zur Verwendung nach Anspruch 1, wobei das Antigen aus (a), (b), (d), (e) oder (h) in der Form eines oder mehrerer Peptide vorliegt, die vollständig oder teilweise einem Aggrecan-Polypeptid entsprechen, einschließlich citrullinierter Formen davon.

22. Wirkstoff oder Kombination von Wirkstoffen zur Verwendung nach Anspruch 1, wobei das Antigen aus (a), (b), (d), (e) oder (h) in der Form einer Mehrzahl von angrenzenden überlappenden Peptiden vorliegt, deren Sequenzen zumindest einen Abschnitt eines Aggrecan-Polypeptids überspannen, einschließlich citrullinierter Formen davon.

23. Wirkstoff oder Kombination von Wirkstoffen zur Verwendung nach Anspruch 22, wobei die überlappenden Peptide eine aus SEQ ID NO: 49-531 ausgewählte Aminosäuresequenz, einschließlich citrullinierter Formen davon, umfassen oder daraus bestehen.

24. Wirkstoff oder Kombination von Wirkstoffen zur Verwendung nach Anspruch 1, wobei der NF-κB-Inhibitor Curcumin oder ein Curcumin-Derivat ist.

25. Wirkstoff oder Kombination von Wirkstoffen zur Verwendung nach Anspruch 1, wobei der NF-κB-Inhibitor, mTOR-Inhibitor oder Syk-Inhibitor und das Antigenmolekül in löslicher Form gemeinsam verabreicht werden.

26. Wirkstoff oder Kombination von Wirkstoffen zur Verwendung nach Anspruch 1, wobei der NF-κB-Inhibitor, mTOR-Inhibitor oder Syk-Inhibitor und das Antigenmolekül in Partikelform gemeinsam verabreicht werden.

27. Wirkstoff oder Kombination von Wirkstoffen zur Verwendung nach Anspruch 26, wobei der NF-κB-Inhibitor, mTOR-Inhibitor oder Syk-Inhibitor und das Antigenmolekül in demselben Partikel gemeinsam verabreicht werden.

28. Wirkstoff oder Kombination von Wirkstoffen zur Verwendung nach Anspruch 27, wobei das Partikel ein Polymerpartikel ist.

29. Wirkstoff oder Kombination von Wirkstoffen zur Verwendung nach Anspruch 27, wobei das Partikel ein Liposom ist.

30. Wirkstoff oder Kombination von Wirkstoffen zur Verwendung nach Anspruch 1, wobei das in der B-Zelle aus (e) exprimierte Nukleinsäuremolekül weiter für ein Immunglobulin oder ein Immunglobulin-Fragment codiert, das direkt oder indirekt an das Antigen fusioniert wird.

31. Wirkstoff oder Kombination von Wirkstoffen zur Verwendung nach Anspruch 1, wobei der I/TCBL aus (f) zumindest einen Abschnitt eines Moleküls umfasst, ausgewählt aus einem Molekül der MHC-Klasse I, einem Molekül der MHC-Klasse II, einem akzessorischen Molekül wie β2-Mikroglobulin, dem lymphozytenfunktionsassoziierten Molekül 3 (LFA-3), der Fc-Region der schweren Kette eines Immunglobulin-Moleküls, Ia⁺-Molekülen, einem Antikörper für CD2, einem Antikörper für CD3, einem Antikörper für CD4, einem Antikörper für CD8, einem Antikörper für Lektin, einem Lymphokin.

## Revendications

1. Agent ou combinaison d'agents qui produit une réponse tolérogène spécifique à un antigène à un polypeptide d'aggrécane choisi parmi :
a)
(i) un antigène qui correspond en totalité ou en partie à un polypeptide d'aggrécane ou un polynucléotide codant pour un antigène qui correspond en totalité ou en partie à un polypeptide d'aggrécane ; coadministré(s) conjointement avec :
(ii) au moins un inhibiteur de NF-κB, un inhibiteur de mTOR ou un inhibiteur de Syk ou un polynucléotide codant pour le au moins un inhibiteur de NF-κB, un inhibiteur de mTOR ou un inhibiteur de Syk ; ou
b) une cellule présentant un antigène (APC) à laquelle :
(i) un antigène qui correspond en totalité ou en partie à un polypeptide d'aggrécane ou un polynucléotide codant pour un antigène qui correspond en totalité ou en partie à un polypeptide d'aggrécane ; et
(ii) un inhibiteur de NF-κB, un inhibiteur de mTOR ou un inhibiteur de Syk ou un polynucléotide codant pour au moins un inhibiteur de NF-κB, un inhibiteur de mTOR ou un inhibiteur de Syk,
ont été délivrés ex vivo ; ou
c) un lymphocyte T qui a été mis en contact avec l'APC de (b) ; ou
d) un complexe comprenant un antigène qui correspond en totalité ou en partie à un polypeptide d'aggrécane et un composant de complexe à histocompatibilité majeure (MHC) ; ou
e) une cellule B exprimant une molécule d'acide nucléique qui code un antigène qui correspond en totalité ou en partie à un polypeptide d'aggrécane, dans lequel ou laquelle l'induction de l'expression mène à la présentation de l'antigène ou de sa forme traitée sur la surface de la cellule B ; ou
f) un montage comprenant un peptide immunomodulateur fixé à un ligand immune/fixateur de cellules T (I/TCBL), dans lequel le peptide immunomodulateur comprend une séquence d'acides aminés correspondant à une partie d'un polypeptide d'aggrécane qui se lie à un récepteur d'antigène sur des lymphocytes T pro-inflammatoires ou auto-réactifs et dans lequel l'I/TCBL se lie à une classe ou une sous-classe de cellules T choisies dans le groupe constitué de cellules auxiliaires T, de cellules suppressives T et de cellules cytotoxiques T et module l'activité des cellules T ; ou
g) un ligand peptidique modifié (APL) qui comprend ou est constitué d'une séquence d'acides aminés correspondant à une partie d'un polypeptide d'aggrécane qui se lie à un récepteur d'antigène sur des lymphocytes T pro-inflammatoires ou auto-réactifs, dans lequel la séquence d'acides aminés APL se distingue de la séquence d'acides aminés de la partie par au moins une substitution, une délétion ou une addition d'acides aminés et l'APL interfère avec une signalisation normale à travers un récepteur d'antigène ; ou
h) une combinaison de (b) et de (c),
pour utilisation dans le traitement ou la prévention de dégâts d'articulation chez un humain sujet à une RA précoce ou une RA débutante, dans lequel ou laquelle le polypeptide d'aggrécane est un polypeptide d'aggrécane citrulliné.

2. Agent ou combinaison d'agents pour utilisation selon la revendication 1, dans lequel ou laquelle le sujet est identifié comme ayant une RA précoce ou une RA débutante avant de produire la réponse tolérogène spécifique à l'antigène.

3. Agent ou combinaison d'agents pour utilisation selon la revendication 1 ou la revendication 2, dans lequel ou laquelle le sujet est positif pour l'épitope partagé (SE).

4. Agent ou combinaison d'agents pour utilisation selon la revendication 3, dans lequel ou laquelle le sujet est identifié comme étant positif pour le SE avant de produire la réponse tolérogène spécifique à l'antigène.

5. Agent ou combinaison d'agents pour utilisation selon l'une quelconque des revendications 1 à 4, dans lequel ou laquelle le sujet a ou risque d'avoir un développement d'une réponse immunitaire au polypeptide d'aggrécane.

6. Agent ou combinaison d'agents pour utilisation selon la revendication 5, dans lequel ou laquelle la réponse immunitaire comprend une réponse des lymphocytes T effecteurs.

7. Agent ou combinaison d'agents pour utilisation selon la revendication 6, dans lequel ou laquelle la réponse immunitaire comprend la production d'au moins une cytokine choisie dans le groupe constitué de l'interleukine-6 (IL-6), de l'interféron-γ (IFN-γ), du facteur de nécrose tumorale (TNF) et de l'interleukine-10 (IL-10).

8. Agent ou combinaison d'agents pour utilisation selon la revendication 5, dans lequel ou laquelle la réponse immunitaire comprend une réponse des lymphocytes T pro-inflammatoires qui comprend ou consiste en la production d'une moins une cytokine choisie dans le groupe constitué de l'IL-6, de l'IFN-γ et de la TNF.

9. Agent ou combinaison d'agents pour utilisation selon la revendication 8, dans lequel ou laquelle la réponse des lymphocytes T pro-inflammatoires comprend ou consiste en la production d'IL-6.

10. Agent ou combinaison d'agents pour utilisation selon la revendication 5, dans lequel ou laquelle la réponse immunitaire est produite au moins en partie par des lymphocytes CD4⁺.

11. Agent ou combinaison d'agents pour utilisation selon l'une quelconque des revendications 1 à 10, dans lequel ou laquelle la réponse tolérogène spécifique à l'antigène est effectuée par :
(1) l'augmentation du nombre de cellules présentant des antigènes tolérogènes (agg-tolAPC) chez le sujet, qui présentent un peptide correspondant à une partie du polypeptide d'aggrécane, dans lequel la partie est associée à une réponse aux lymphocytes T pro-inflammatoires ou auto-réactifs au polypeptide d'aggrécane ;
(2) l'induction d'anergie ou d'apoptose dans les lymphocytes T pro-inflammatoires ou auto-réactifs chez le sujet, qui sont réactifs contre le polypeptide d'aggrécane ; et
(3) l'augmentation du nombre de lymphocytes T régulateurs ou suppresseurs chez le sujet, qui suppriment ou réduisent autrement une réponse pro-inflammatoire ou auto-réactives des lymphocytes T au polypeptide d'aggrécane.

12. Agent ou combinaison d'agents pour utilisation selon la revendication 11, comprenant l'augmentation du nombre d'agg-tolAPC chez le sujet pour ainsi traiter ou empêcher les dégâts dans les articulations.

13. Agent ou combinaison d'agents pour utilisation selon la revendication 12, dans lequel ou laquelle les agg-tolAPC stimulent la production de lymphocytes T régulateurs ou suppresseurs qui suppriment ou autrement réduisent la réponse pro-inflammatoire ou auto-réactive des lymphocytes T au polypeptide d'aggrécane.

14. Agent ou combinaison d'agents pour utilisation selon la revendication 1, dans lequel ou laquelle l'antigène de (a), (b), (d), (e) ou (h) comprend une séquence d'acides aminés correspondant à un polypeptide d'aggrécane de pleine longueur.

15. Agent ou combinaison d'agents pour utilisation selon la revendication 1, dans lequel ou laquelle l'antigène (a), (b), (d), (e) ou (h) comprend une séquence d'acides aminés correspondant à un polypeptide d'aggrécane mature.

16. Agent ou combinaison d'agents pour utilisation selon la revendication 1, dans lequel ou laquelle l'antigène de (a), (b), (d), (e) ou (h) comprend une séquence d'acides aminés correspondant à un domaine d'un polypeptide d'aggrécane choisi parmi le domaine G1, le domaine G2 ou le domaine G3.

17. Agent ou combinaison d'agents pour utilisation selon la revendication 1, dans lequel ou laquelle l'antigène de (a), (b), (d), (e) ou (h) comprend une séquence d'acides aminés correspondant à un épitope de cellules T d'un polypeptide d'aggrécane.

18. Agent ou combinaison d'agents pour utilisation selon la revendication 17, dans lequel ou laquelle la séquence d'acides aminés est choisie parmi l'une quelconque des SEQ ID NO : 5-35, incluant leurs formes citrullinées.

19. Agent ou combinaison d'agents pour utilisation selon la revendication 17, dans lequel ou laquelle la séquence d'acides aminées est choisie parmi l'une quelconque des SEQ ID NO : 32-35.

20. Agent ou combinaison d'agents pour utilisation selon la revendication 1, dans lequel ou laquelle l'antigène de (a), (b), (d), (e) ou (h) est limité au HLA DR et le sujet est positif pour un allèle de HLA DR.

21. Agent ou combinaison d'agents pour utilisation selon la revendication 1, dans lequel ou laquelle l'antigène de (a), (b), (d), (e) ou (h) se présente sous la forme d'un ou plusieurs peptides correspondant en totalité ou en partie à un polypeptide d'aggrécane, incluant ses formes citrullinées.

22. Agent ou combinaison d'agents pour utilisation selon la revendication 1, dans lequel ou laquelle l'antigène de (a), (b), (d), (e) ou (h) se présente sous la forme d'une pluralité de peptides chevauchants contigus dont les séquences couvrent au moins une partie d'un polypeptide d'aggrécane, incluant ses formes citrullinées.

23. Agent ou combinaison d'agents pour utilisation selon la revendication 22, dans lequel ou laquelle les peptides chevauchants comprennent ou sont constitués d'une séquence d'acides aminés choisie parmi les SEQ ID NO : 49-531, incluant leurs formes citrullinées.

24. Agent ou combinaison d'agents pour utilisation selon la revendication 1, dans lequel ou laquelle l'inhibiteur de NF-κB est la curcumine ou un dérivé de la curcumine.

25. Agent ou combinaison d'agents pour utilisation selon la revendication 1, dans lequel ou laquelle l'inhibiteur de NF-κB, l'inhibiteur de mTOR ou l'inhibiteur de Syk et la molécule antigénique sont coadministrés sous forme soluble.

26. Agent ou combinaison d'agents pour utilisation selon la revendication 1, dans lequel ou laquelle l'inhibiteur de NF-κB, l'inhibiteur de mTOR ou l'inhibiteur de Syk et la molécule antigénique sont coadministrés sous forme particulaire.

27. Agent ou combinaison d'agents pour utilisation selon la revendication 26, dans lequel ou laquelle l'inhibiteur de NF-κB, l'inhibiteur de mTOR ou l'inhibiteur de Syk et la molécule antigénique sont coadministrés dans la même particule.

28. Agent ou combinaison d'agents pour utilisation selon la revendication 27, dans lequel ou laquelle la particule est une particule polymère.

29. Agent ou combinaison d'agents pour utilisation selon la revendication 27, dans lequel ou laquelle la particule est un liposome.

30. Agent ou combinaison d'agents pour utilisation selon la revendication 1, dans lequel ou laquelle la molécule d'acide nucléique exprimée dans la cellule B de (e) code en outre pour une immunoglobuline ou un fragment d'immunoglobuline fusionné(e) directement ou indirectement sur l'antigène.

31. Agent ou combinaison d'agents pour utilisation selon la revendication 1, dans lequel ou laquelle l'I/TCBL de (f) comprend au moins une partie d'une molécule choisie parmi une molécule MHC de classe I, une molécule MHC de classe II, une molécule accessoire telle que la β2-microglobuline, une molécule-3 associée à une fonction lymphocytaire (LFA-3), la région Fc de la chaîne lourde d'une molécule d'immunoglobuline, des molécules Ia⁺, un anticorps à la CD2, un anticorps à la CD3, un anticorps à la CD4, un anticorps à la CD8, un anticorps à la lectine, une lymphokine.
